(19) Europäisches Patentamt
European Patent Office
Office européen des brevets

(11) EP 4 257 584 A1

(12) EUROPEAN PATENT APPLICATION
published in accordance with Art. 153(4) EPC

(43) Date of publication:
**11.10.2023 Bulletin 2023/41**

(21) Application number: **21900025.4**

(22) Date of filing: **01.12.2021**

(51) International Patent Classification (IPC):
***C07D 401/04*** (2006.01) ***C07D 471/04*** (2006.01)
***C07D 487/04*** (2006.01) ***C07D 403/04*** (2006.01)
***A61K 31/506*** (2006.01) ***A61P 35/00*** (2006.01)

(52) Cooperative Patent Classification (CPC):
**A61K 31/506; A61P 35/00; C07D 401/04; C07D 403/04; C07D 471/04; C07D 487/04**

(86) International application number:
**PCT/CN2021/134790**

(87) International publication number:
**WO 2022/116995 (09.06.2022 Gazette 2022/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.12.2020 CN 202011400431**

(71) Applicant: **Abbisko Therapeutics Co., Ltd.**
**Shanghai 201203 (CN)**

(72) Inventors:
- **ZHAO, Baowei**
  **Shanghai 201203 (CN)**
- **ZHANG, Mingming**
  **Shanghai 201203 (CN)**
- **YU, Hongping**
  **Shanghai 201203 (CN)**
- **CHEN, Zhui**
  **Shanghai 201203 (CN)**
- **XU, Yaochang**
  **Shanghai 201203 (CN)**

(74) Representative: **Potter Clarkson**
**Chapel Quarter**
**Mount Street**
**Nottingham NG1 6HQ (GB)**

# (54) 2,3-DIHYDRO-1H-PYRROLO[3,2-B]PYRIDINE DERIVATIVE, PREPARATION METHOD THEREFOR, AND APPLICATION THEREOF

(57) The present invention relates to a 2,3-dihydro-1H-pyrrolo[3,2-b]pyridine derivative, a preparation method therefor, and an application thereof, and in particular to an EGFR inhibitor having the structure of formula (I), a preparation method therefor, a pharmaceutical composition containing same, a use of same as an EGFR inhibitor, and a use of same in the treatment and/or prevention of cancers, tumors, or metastatic diseases at least partially related to EGFR exon 20 insertion or deletion mutations, especially a use in the treatment of hyperproliferative diseases and dysfunction in cell death induction. The definition of each substituent in formula (I) is the same as that in the description.

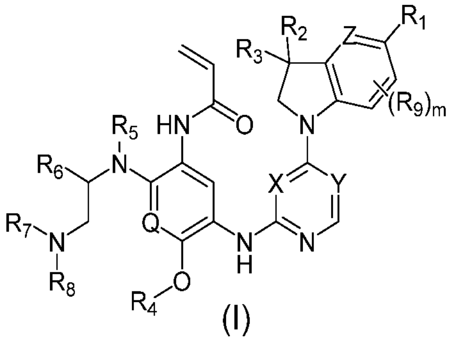
(I)

EP 4 257 584 A1

## Description

## TECHNICAL FIELD

The present invention belongs to the field of medicament synthesis, and in particular relates 2,3-dihydro-1H-pyrrolo[3,2-b]pyridine derivative, preparation method therefor and an application thereof.

## BACKGROUND

Lung cancer is the leading cause of deaths from cancers around the world, with non-small cell lung cancer (NSCLC) accounting for 85%. Multi-target therapies targeting epidermal growth factor receptor (EGFR) mutations, anaplastic lymphoma kinase (ALK) translocations, ROS1 proto-oncogene receptor tyrosine kinase (ROS1) rearrangements, and B-raf proto-oncogene, serine/threonine kinase (BRAF) have been developed and clinically validated. EGFR can be inhibited to significantly improve the progression-free survival of adenocarcinoma NSCLC, and after its acquired drug-resistant mutations, it can be subsequently targeted by a third-generation inhibitor.

Despite the successful inhibition of classical EGFR activating mutations (exons 19 and 21) and drug-resistant mutations (T790M), the intra-frame insertion of an exon 20 also leads to structural activation of EGFR signaling, and it is associated with the de novo resistance to existing EGFR inhibitors. The exon 20 mutations are heterogeneous and include the in-frame insertion or duplication of 1-7 amino acids among 762-774 amino acids of the EGFR protein. In NSCLC, the frequency of EGFR exon 20 mutations accounts for 4-10% of all EGFR mutations. These mutations are mutually exclusive with other known oncogenic gene driver mutations, and are enriched in adenocarcinomas in women, non-smokers, Asian populations, and patients with non-small cell lung cancer. In addition to NSCLC, the EGFR exon 20 insertion mutations are also found in a rare form of head and neck cancer, namely, nasal squamous cell carcinoma (SNSCC). Furthermore, a structurally similar exon 20 insertion mutation is also found in HER2, another member of the EGFR family of receptor tyrosine kinase (RTK).

Multiple retrospective analyses have shown limited efficacy of currently available first, second and third generations of EGFR inhibitors against the exon 20 insertion mutations, with the exception of A763-Y764insFQEA mutations. An irreversible inhibitor Poziotinib and an EGFR/MET bispecific antibody amivantamab are in course of clinical trials. Several small molecule inhibitors, including TAK-788 and TAS-6417, have shown clinically significant efficacy in patients with non-small cell lung cancer induced by EGFR exon 20. However, due to limited selectivity for EGFR WT (wild type), the adverse effects of these inhibitors are unavoidable and may lead to dose-limiting toxicity. Therefore, for these patients, there is an urgent need for a highly selective small molecule inhibitor targeting the EGFR exon 20 insertion mutations.

## SUMMARY

An object of the present invention is to provide 2,3-dihydro-1H-pyrrolo[3,2-b]pyridine derivative, preparation method therefor and application thereof. A series of compounds of the present invention has a strong inhibitory effect on the insertion, deletion or other mutant cytological activities of EGFR exon 20, and shows high selectivity for EGFR wild type. They can be widely used in the preparation of a medicament for treatment and/or prevention of cancers, tumors or metastatic diseases at least partially associated with the insertion, deletion or other mutations of EGFR exon 20, in particular a medicament for treatment of hyperproliferative diseases and induced cell death disorders, whereby a new generation of EGFR inhibitors is expected to be developed.

The first aspect of the present invention provides a compound of formula (I), or a stereoisomer or pharmaceutically acceptable salt thereof:

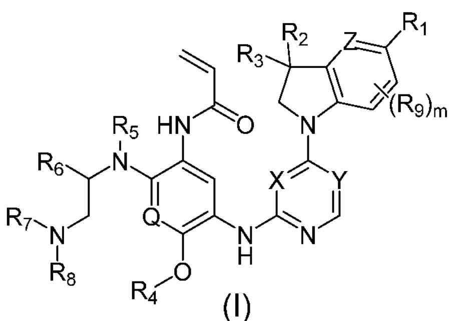

(I)

,

wherein, X and Y are each independently $CR_{10}$ or N; Z is $CR_{11}$ or N; Q is CH or N;

$R_1$ is selected from the group consisting of hydrogen, deuterium, halogen, cyano, nitro, azido, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$, the above groups are optionally further substituted by one or more substituents selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$,

or, when $m \geq 1$, $R_1$ and adj acent $R_9$, together with the moiety to which they are directly attached, form a $C_{5-6}$ cycloalkyl or 5-6 membered heterocyclyl;

$R_2$ and $R_3$ are each independently selected from the group consisting of hydrogen, deuterium, halogen, cyano, nitro, azido, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl and 5-10 membered heteroaryl,

or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

$R_4$ is selected from the group consisting of hydrogen, deuterium, $C_{1-10}$ alkyl, $C_{2-10}$ alkenyl, $C_{3-12}$ cycloalkyl, 3-12 membered heterocyclyl, $C_{6-10}$ aryl and 5-10 membered heteroaryl, the above groups are optionally further substituted by one or more substituents selected from the group consisting of deuterium, halogen, hydroxy, =O, cyano, $C_{1-10}$ alkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

$R_5$ is selected from the group consisting of hydrogen, deuterium, hydroxy, $C_{1-10}$ alkyl, $C_{1-10}$ haloalkyl, $C_{1-10}$ deuterioalkyl, $C_{2-4}$ alkenyl, $C_{3-6}$ cycloalkyl and 3-6 membered heterocyclyl;

$R_6$ is selected from the group consisting of hydrogen, deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

or, $R_5$ and $R_6$, together with the moiety to which they are directly attached, form a 4-6 membered heterocyclyl, above 4-6 membered heterocyclyl is optionally further substituted by one or more substituents selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, $- C(=NR_{15})R_{14}$, $-N(R_{15})-C(=NR_{16})R_{14}$, $-C(O)NR_{15}R_{16}$ and $-N(R_{15})-C(O)R_{14}$;

$R_7$ and $R_8$ are each independently selected from the group consisting of hydrogen, deuterium, hydroxy, $C_{1-10}$ alkyl, $C_{2-4}$ alkenyl, $C_{3-6}$ cycloalkyl and 3-6 membered heterocyclyl, or, $R_7$ and $R_8$, together with the nitrogen atom to which they are directly attached, form a 3-12 membered heterocyclyl, the above groups are optionally further substituted by one or more substituents selected from the group consisting of deuterium, halogen, hydroxy, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

each $R_9$ is independently selected from the group consisting of hydrogen, deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, $- C(=NR_{15})R_{14}$, $-N(R_{15})-C(=NR_{16})R_{14}$, $-C(O)NR_{15}R_{16}$ and $-N(R_{15})-C(O)R_{14}$, or, when m=2, two $R_9$, together with the moiety to which they are directly attached, form a $C_{3-12}$ cycloalkyl or 3-12 membered heterocyclyl, the above groups are optionally further substituted by one or more substituents selected from the group consisting of deuterium, halogen, hydroxy, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

each $R_{10}$ is independently selected from the group consisting of hydrogen, deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, $- C(=NR_{15})R_{14}$, $-N(R_{15})-C(=NR_{16})R_{14}$, $-C(O)NR_{15}R_{16}$ and $-N(R_{15})-C(O)R_{14}$;

$R_{11}$ is selected from the group consisting of hydrogen, deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl,

5-10 membered heteroaryl, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

each $R_{12}$ is independently selected from the group consisting of hydrogen, deuterium, hydroxy, $C_{1-10}$ alkyl, $C_{2-10}$ alkenyl, $C_{3-12}$ cycloalkyl, 3-12 membered heterocyclyl, $C_{6-10}$ aryl, 5-10 membered heteroaryl and $-NR_{15}R_{16}$, the above groups are optionally further substituted by one or more substituents selected from the group consisting of deuterium, halogen, hydroxy, oxo, $C_{1-10}$ alkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

each $R_{13}$ is independently selected from the group consisting of hydrogen, deuterium, $C_{1-10}$ alkyl, $C_{2-10}$ alkenyl, $C_{3-12}$ cycloalkyl, 3-12 membered heterocyclyl, $C_{6-10}$ aryl and 5-10 membered heteroaryl, the above groups are optionally further substituted by one or more substituents selected from the group consisting of deuterium, halogen, hydroxy, oxo, cyano, $C_{1-10}$ alkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

each $R_{14}$ is independently selected from the group consisting of hydrogen, deuterium, hydroxy, $C_{1-10}$ alkyl, $C_{1-10}$ alkoxy, $C_{2-10}$ alkenyl, $C_{2-10}$ alkynyl, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$, the above groups are optionally further substituted by one or more substituents selected from the group consisting of deuterium, halogen, hydroxy, cyano, $C_{1-10}$ alkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

$R_{15}$ and $R_{16}$ are each independently selected from the group consisting of hydrogen, deuterium, hydroxy, $C_{1-10}$ alkoxy, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl, amino, mono$C_{1-10}$ alkylamino, di$C_{1-10}$ alkylamino and $C_{1-10}$ alkanoyl, the above groups are optionally further substituted by one or more substituents selected from the group consisting of deuterium, halogen, hydroxy, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy, amino, mono$C_{1-10}$ alkylamino, di$C_{1-10}$ alkylamino and $C_{1-10}$ alkanoyl,

or, $R_{15}$ and $R_{16}$, together with the nitrogen atom to which they are directly attached, form a 4-10 membered heterocyclyl or 5-10 membered heteroaryl, the above groups are optionally further substituted by one or more substituents selected from the group consisting of deuterium, halogen, hydroxy, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy, amino, mono$C_{1-10}$ alkylamino, di$C_{1-10}$ alkylamino and $C_{1-10}$ alkanoyl;

m is 0, 1 or 2;

each r is independently 0, 1 or 2.

As a preferred embodiment, in the compound of formula (I), the stereoisomer or pharmaceutically acceptable salt thereof, $R_1$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$, or, when m≥1, $R_1$ and adjacent $R_9$, together with the moiety to which they are directly attached, form a $C_{5-6}$ cycloalkyl or 5-6 membered heterocyclyl;

$R_2$ and $R_3$ are each independently selected from the group consisting of hydrogen, deuterium, halogen, cyano, nitro, azido, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl and 5-8 membered heteroaryl, or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

$R_4$ is selected from the group consisting of hydrogen, deuterium, $C_{1-4}$ alkyl, $C_{2-4}$ alkenyl, $C_{3-6}$ cycloalkyl, 3-6 membered heterocyclyl, $C_{6-8}$ aryl and 5-8 membered heteroaryl, the above groups are optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_5$ is selected from the group consisting of hydrogen, deuterium, hydroxy, $C_{1-4}$ alkyl, $C_{1-4}$ haloalkyl, $C_{1-4}$ deuterioalkyl, $C_{2-4}$ alkenyl, $C_{3-6}$ cycloalkyl and 3-6 membered heterocyclyl;

$R_6$ is selected from the group consisting of hydrogen, deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, - $S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

or, $R_5$ and $R_6$, together with the moiety to which they are directly attached, form a 4-6 membered heterocyclyl, above 4-6 membered heterocyclyl is optionally further substituted by one or more substituents selected from the group consisting of deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ and $-N(R_{15})-C(O)R_{14}$;

$R_7$ and $R_8$ are each independently selected from the group consisting of hydrogen, deuterium, hydroxy, $C_{1-4}$ alkyl, $C_{2-4}$ alkenyl, $C_{3-6}$ cycloalkyl and 3-6 membered heterocyclyl, or, $R_7$ and $R_8$, together with the nitrogen atom to which they are directly attached, form a 3-6 membered heterocyclyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

each $R_9$ is independently selected from the group consisting of hydrogen, deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ and $-N(R_{15})-C(O)R_{14}$, or, when m=2, two $R_9$, together with the moiety to which they are directly attached, form a $C_{3-6}$ cycloalkyl or 3-6 membered heterocyclyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

each $R_{10}$ is independently selected from the group consisting of hydrogen, deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ and $-N(R_{15})-C(O)R_{14}$;

$R_{11}$ is selected from the group consisting of hydrogen, deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, - $S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

wherein, $R_{12}$, $R_{13}$, $R_{14}$, $R_{15}$, $R_{16}$ and r are defined as those in the compound of formula (I).

As a preferred embodiment, in the compound of formula (I), the stereoisomer or pharmaceutically acceptable salt thereof, the compound of formula (I) is a compound of formula (IIa) as below:

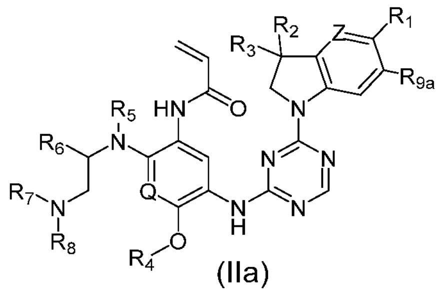

(IIa)

,

wherein, Z is $CR_{11}$ or N; Q is CH or N;

$R_1$ is selected from hydrogen, chlorine, bromine and $C_{1-4}$ alkyl, the $C_{1-4}$ alkyl is optionally further substituted by one or more substituents selected from the group consisting of deuterium, halogen, cyano, hydroxy, amino, dimethylamino, $C_{3-6}$ cycloalkyl and 3-6 membered heterocyclyl;

$R_2$ and $R_3$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

$R_4$ is selected from the group consisting of hydrogen, deuterium, $C_{1-4}$ alkyl, $C_{2-4}$ alkenyl, $C_{3-6}$ cycloalkyl and 3-6 membered heterocyclyl, the above groups are optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_5$ is selected from the group consisting of hydrogen, deuterium, $C_{1-4}$ alkyl, $C_{1-4}$ haloalkyl, $C_{1-4}$ deuterioalkyl and $C_{2-4}$ alkenyl;

$R_6$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl and 5-8 membered heteroaryl;

$R_7$ and $R_8$ are each independently selected from the group consisting of hydrogen, deuterium, hydroxy, $C_{1-4}$ alkyl and $C_{2-4}$ alkenyl, or, $R_7$ and $R_8$, together with the nitrogen atom to which they are directly attached, form a 3-6 membered heterocyclyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_{9a}$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl and $C_{6-8}$ aryl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_{11}$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl and 3-6 membered heterocyclyl;

wherein, $R_{12}$, $R_{13}$, $R_{14}$, $R_{15}$, $R_{16}$ and r are defined as those in the compound of formula (I).

As a further preferred embodiment, in the compound of formula (I), the stereoisomer or pharmaceutically acceptable salt thereof, the compound of formula (I) is a compound of formula ($IIIa_1$) as below:

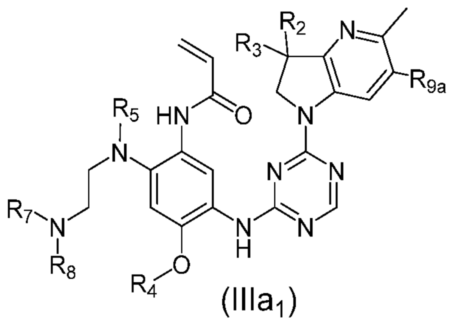

($IIIa_1$) ,

wherein, $R_2$ and $R_3$ are each independently selected from the group consisting of hydrogen, deuterium, halogen, $C_{1-4}$ alkyl, $C_{1-4}$ haloalkyl, $C_{1-4}$ deuterioalkyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl;

$R_4$ is selected from hydrogen, deuterium, $C_{1-4}$ alkyl and $C_{3-6}$ cycloalkyl, the above groups are optionally further substituted by one or more substituents selected from the group consisting of deuterium, halogen, hydroxy, cyano, $C_{1-4}$ alkyl, $C_{1-4}$ alkoxy, $C_{3-6}$ cycloalkyl, $C_{3-6}$ cycloalkyloxy, 3-6 membered heterocyclyl and 3-6 membered heterocyclyloxy;

$R_5$, $R_7$ and $R_8$ are each independently hydrogen or methyl;

$R_{9a}$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl and $C_{6-8}$ aryl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$.

As a more further preferred embodiment, in the compound of formula (I), the stereoisomer or pharmaceutically acceptable salt thereof, $R_2$ and $R_3$ are each independently selected from the group consisting of hydrogen, deuterium, fluorine, chlorine, bromine, methyl, ethyl, propyl, isopropyl, trifluoromethyl, difluoromethyl, trideuteriomethyl, dideuteriomethyl, cyclopropyl, cyclobutyl, oxacyclobutyl and azacyclobutyl, or, $R_2$ and $R_3$, together with the carbon atom to which they are directly attached, form a cyclopropyl, cyclobutyl, cyclopentyl, cyclohexyl, oxacyclobutyl, azacyclobutyl, oxacyclopentyl or azacyclopentyl, above cyclopropyl, cyclobutyl, cyclopentyl, cyclohexyl, oxacyclobutyl, azacyclobutyl, oxacyclopentyl or azacyclopentyl is optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl and cyclobutyl;

$R_4$ is selected from the group consisting of hydrogen, deuterium, methyl, ethyl, propyl, isopropyl, cyclopropyl and cyclobutyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl;

$R_5$, $R_7$ and $R_8$ are each independently hydrogen or methyl;

$R_{9a}$ is selected from the group consisting of hydrogen, deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl, cyclobutyl and phenyl, the phenyl is optionally further substituted by one or more substituents selected from the group consisting of deuterium, fluorine, chlorine, bromine, hydroxy, methyl, ethyl, propyl, isopropyl, vinyl, ethynyl, trifluoromethyl, difluoromethyl, trideuteriomethyl, dideuteriomethyl, methoxy, ethoxy, cyclopropyl, cyclobutyl, oxacyclobutyl and azacyclobutyl.

As a further preferred embodiment, in the compound of formula (I), the stereoisomer or pharmaceutically acceptable salt thereof, the compound of formula (I) is a compound of formula ($IIIa_2$) as below:

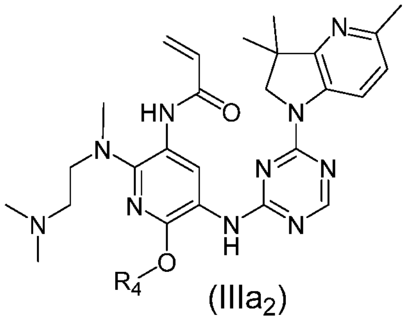

($IIIa_2$)

,

wherein, $R_4$ is isopropyl and cyclopropyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl.

As a further preferred embodiment, in the compound of formula (I), the stereoisomer or pharmaceutically acceptable salt thereof, the compound of formula (I) is a compound of formula ($IIIa_3$) as below:

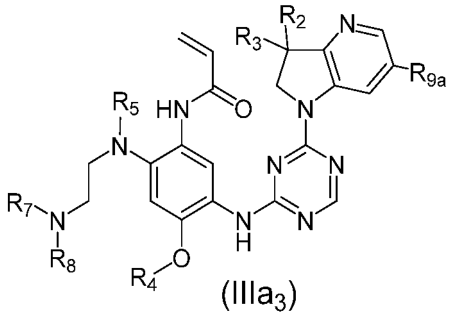

(IIIa$_3$)

,

wherein, $R_2$ and $R_3$ are each independently selected from the group consisting of hydrogen, deuterium, fluorine, chlorine, bromine, methyl, ethyl, propyl, isopropyl, trifluoromethyl, difluoromethyl, trideuteriomethyl, dideuteriomethyl, cyclopropyl, cyclobutyl, oxacyclobutyl and azacyclobutyl, or, $R_2$ and $R_3$, together with the carbon atom to which they are directly attached, form a cyclobutyl, cyclopentyl, cyclohexyl, oxacyclobutyl, azacyclobutyl, oxacyclopentyl or azacyclopentyl, above cyclopropyl, cyclobutyl, cyclopentyl, cyclohexyl, oxacyclobutyl, azacyclobutyl, oxacyclopentyl or azacyclopentyl is optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl and cyclobutyl;

$R_4$ is selected from the group consisting of hydrogen, deuterium, methyl, ethyl, propyl, isopropyl, cyclopropyl and cyclobutyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl;

$R_5$, $R_7$ and $R_8$ are each independently hydrogen or methyl;

$R_{9a}$ is selected from the group consisting of hydrogen, deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl and cyclobutyl,

provided that, when $R_{9a}$ is hydrogen, $R_2$ and $R_3$, together with the carbon atom to which they are directly attached, form a cyclobutyl, cyclopentyl, cyclohexyl, oxacyclobutyl, azacyclobutyl, oxacyclopentyl or azacyclopentyl.

As a further preferred embodiment, in the compound of formula (I), the stereoisomer or pharmaceutically acceptable salt thereof, the compound of formula (I) is a compound of formula (IIIa$_4$) as below:

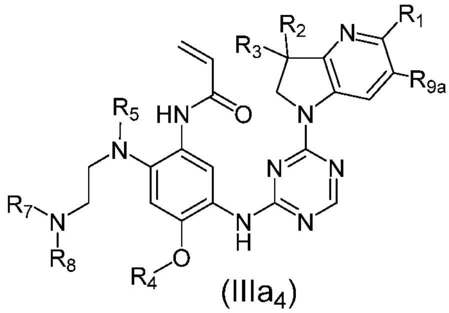

(IIIa$_4$)

,

wherein, $R_1$ is chlorine or bromine;

$R_2$ and $R_3$ are each independently selected from the group consisting of hydrogen, deuterium, fluorine, chlorine, bromine, methyl, ethyl, propyl, isopropyl, trifluoromethyl, difluoromethyl, trideuteriomethyl, dideuteriomethyl, cyclopropyl, cyclobutyl, oxacyclobutyl and azacyclobutyl, or, $R_2$ and $R_3$, together with the carbon atom to which they are directly attached, form a cyclopropyl, cyclobutyl, cyclopentyl, cyclohexyl, oxacyclobutyl, azacyclobutyl, oxacyclopentyl or azacyclopentyl, above cyclopropyl, cyclobutyl, cyclopentyl, cyclohexyl, oxacyclobutyl, azacyclobutyl, oxacyclopentyl or azacyclopentyl is optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl and cyclobutyl;

$R_4$ is selected from the group consisting of hydrogen, deuterium, methyl, ethyl, propyl, isopropyl, cyclopropyl and cyclobutyl, the above groups are optionally further substituted by one or more substituents selected from the group consisting of deuterium, fluorine, chlorine, bromine, hydroxy, cyano, methyl, ethyl, propyl, isopropyl, methoxy, ethoxy,

cyclopropyl, cyclobutyl, oxacyclobutyl and azacyclobutyl;
$R_5$, $R_7$ and $R_8$ are each independently hydrogen or methyl;
$R_{9a}$ is selected from the group consisting of hydrogen, deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl, cyclobutyl and phenyl, the phenyl is optionally further substituted by one or more substituents selected from the group consisting of deuterium, fluorine, chlorine, bromine, hydroxy, methyl, ethyl, propyl, isopropyl, vinyl, ethynyl, trifluoromethyl, difluoromethyl, trideuteriomethyl, dideuteriomethyl, methoxy, ethoxy, cyclopropyl, cyclobutyl, oxacyclobutyl and azacyclobutyl.

As a preferred embodiment, in the compound of formula (I), the stereoisomer or pharmaceutically acceptable salt thereof, the compound of formula (I) is a compound of formula (IIb) as below:

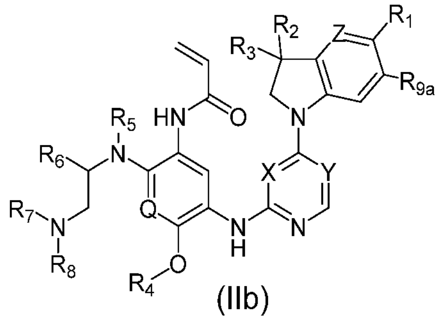
(IIb)
,

wherein, one of X and Y is CH, the other is N; Z is $CR_{11}$ or N; Q is CH or N;
$R_1$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl and $-SF_5$, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$,$-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$,
or, $R_1$ and $R_{9a}$, together with the moiety to which they are directly attached, form a $C_{5-6}$ cycloalkyl or 5-6 membered heterocyclyl;
$R_2$ and $R_3$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;
$R_4$ is selected from the group consisting of hydrogen, deuterium, $C_{1-4}$ alkyl, $C_{2-4}$ alkenyl, $C_{3-6}$ cycloalkyl and 3-6 membered heterocyclyl, the above groups are optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;
$R_5$ is selected from the group consisting of hydrogen, deuterium, $C_{1-4}$ alkyl, $C_{1-4}$ haloalkyl, $C_{1-4}$ deuterioalkyl and $C_{2-4}$ alkenyl;
$R_6$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl and 5-8 membered heteroaryl;
or, $R_5$ and $R_6$, together with the moiety to which they are directly attached, form a 4-6 membered heterocyclyl, above 4-6 membered heterocyclyl is optionally further substituted by one or more substituents selected from the group consisting of deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, $-SF_5$, $-S(O)_r12$, $-OR_{13}$, $-C(O)OR_{13}$, $-C(O)R_{14}$, $-O-C(O)R_{14}$, $-NR_{15}R_{16}$, $- C(=NR_{15})R_{14}$, $-N(R_{15})-C(=NR_{16})R_{14}$, $-C(O)NR_{15}R_{16}$ and $-N(R_{15})-C(O)R_{14}$;
$R_7$ and $R_8$ are each independently selected from the group consisting of hydrogen, deuterium, hydroxy, $C_{1-4}$ alkyl and $C_{2-4}$ alkenyl, or, $R_7$ and $R_8$, together with the nitrogen atom to which they are directly attached, form a 3-6

membered heterocyclyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_{9a}$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl and 5-8 membered heteroaryl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_{11}$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl and 3-6 membered heterocyclyl;

wherein, $R_{12}$, $R_{13}$, $R_{14}$, $R_{15}$, $R_{16}$ and r are defined as those in the compound of formula (I).

As a further preferred embodiment, in the compound of formula (I), the stereoisomer or pharmaceutically acceptable salt thereof, the compound of formula (I) is a compound of formula ($IIIb_1$) or ($IIIb_2$) as below:

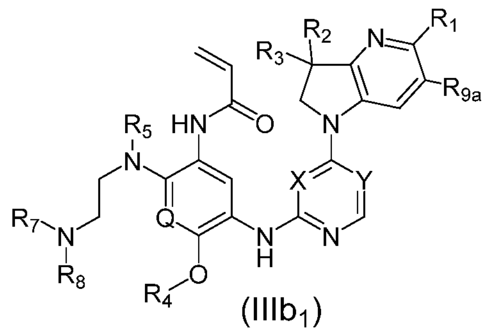

($IIIb_1$)

or

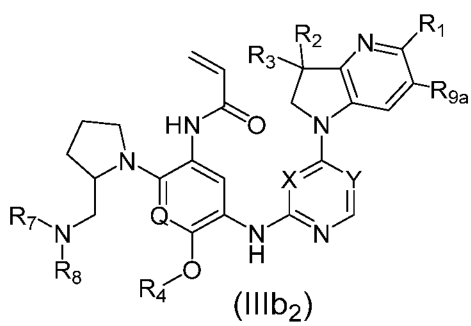

($IIIb_2$)

,

wherein, one of X and Y is CH, the other is N; each Q is CH or N;

each $R_1$ is independently selected from the group consisting of hydrogen, deuterium, halogen, cyano, $C_{1-4}$ alkyl, $C_{2-4}$ alkynyl, $C_{3-6}$ cycloalkyl and 5-8 membered heteroaryl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl and 3-6 membered heterocyclyl,

or, $R_1$ and $R_{9a}$, together with the moiety d to which they are irectly attached, form a $C_{5-6}$ cycloalkyl or 5-6 membered heterocyclyl;

$R_2$ and $R_3$ are each independently selected from the group consisting of hydrogen, deuterium, halogen, $C_{1-4}$ alkyl, $C_{1-4}$ haloalkyl, $C_{1-4}$ deuterioalkyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, $C_{1-4}$ alkyl, $C_{2-4}$ alkenyl, $C_{2-4}$ alkynyl, $C_{1-4}$ haloalkyl, $C_{1-4}$ deuterioalkyl and $C_{3-6}$ cycloalkyl;

each $R_4$ is independently selected from hydrogen, deuterium, $C_{1-4}$ alkyl and $C_{3-6}$ cycloalkyl, the above groups are optionally further substituted by one or more substituents selected from the group consisting of deuterium, halogen, hydroxy, cyano, $C_{1-4}$ alkyl, $C_{1-4}$ alkoxy, $C_{3-6}$ cycloalkyl, $C_{3-6}$ cycloalkyloxy, 3-6 membered heterocyclyl and 3-6 membered heterocyclyloxy;

in the compound of formula ($IIIb_1$), $R_5$ is selected from hydrogen, deuterium, $C_{1-4}$ alkyl, $C_{1-4}$ haloalkyl and $C_{1-4}$ deuterioalkyl;

$R_7$ and $R_8$ are each independently selected from hydrogen, deuterium and $C_{1-4}$ alkyl, or, $R_7$ and $R_8$, together with the nitrogen atom to which they are directly attached, form a 3-6 membered heterocyclyl;

each $R_{9a}$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl and $C_{6-8}$ aryl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl and 3-6 membered heterocyclyloxy.

As a more further preferred embodiment, in the compound of formula (I), the stereoisomer or pharmaceutically acceptable salt thereof, each $R_1$ is independently selected from the group consisting of hydrogen, deuterium, methyl,

ethyl, propyl, isopropyl, ethynyl, cyclopropyl, cyclobutyl, cyclopentyl,

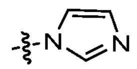 and 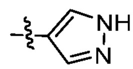 ,

the above groups are optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl, cyclobutyl, oxacyclobutyl and azacyclobutyl;

or, $R_1$ and $R_{9a}$, together with the moiety to which they are directly attached, form a cyclopentyl;

$R_2$ and $R_3$ are each independently selected from the group consisting of hydrogen, deuterium, fluorine, chlorine, bromine, methyl, ethyl, propyl, isopropyl, trifluoromethyl, difluoromethyl, trideuteriomethyl, dideuteriomethyl, cyclopropyl, cyclobutyl, oxacyclobutyl and azacyclobutyl, or, $R_2$ and $R_3$, together with the carbon atom to which they are directly attached, form a $C_{3-6}$ cycloalkyl or 3-6 membered heterocyclyl, above $C_{3-6}$ cycloalkyl or 3-6 membered heterocyclyl is optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl and cyclobutyl;

each $R_4$ is independently selected from the group consisting of hydrogen, deuterium, methyl, ethyl, propyl, isopropyl, cyclopropyl and cyclobutyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl;

in the compound of formula ($IIIb_1$), $R_5$ is selected from hydrogen, deuterium and methyl;

$R_7$ and $R_8$ are each independently selected from hydrogen, deuterium and methyl;

each $R_{9a}$ is independently selected from the group consisting of hydrogen, deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl, cyclobutyl and phenyl, the phenyl is optionally further substituted by one or more substituents selected from the group consisting of deuterium, fluorine, chlorine, bromine, hydroxy, methyl, ethyl, propyl, isopropyl, vinyl, ethynyl, trifluoromethyl, difluoromethyl, trideuteriomethyl, dideuteriomethyl, methoxy, ethoxy, cyclopropyl, cyclobutyl, oxacyclobutyl and azacyclobutyl.

As a preferred embodiment, in the compound of formula (I), the stereoisomer or pharmaceutically acceptable salt thereof, the compound of formula (I) is a compound of formula (IIc) as below:

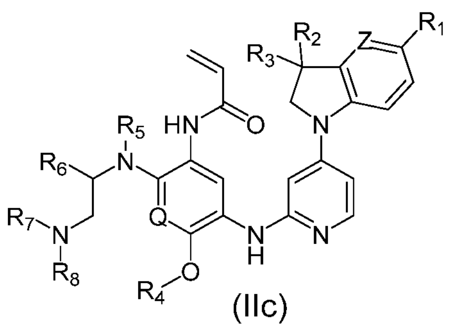

(IIc) ,

wherein, Z is $CR_{11}$ or N; Q is CH or N;

$R_1$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl and $-SF_5$, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$,$-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

$R_2$ and $R_3$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl,

3-6 membered heterocyclyl, $C_{6-8}$ aryl, 5-8 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

$R_4$ is selected from the group consisting of hydrogen, deuterium, $C_{1-4}$ alkyl, $C_{2-4}$ alkenyl, $C_{3-6}$ cycloalkyl and 3-6 membered heterocyclyl, the above groups are optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_5$ is selected from the group consisting of hydrogen, deuterium, $C_{1-4}$ alkyl, $C_{1-4}$ haloalkyl, $C_{1-4}$ deuterioalkyl and $C_{2-4}$ alkenyl;

$R_6$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl and 5-8 membered heteroaryl;

or, $R_5$ and $R_6$, together with the moiety to which they are directly attached, form a 4-6 membered heterocyclyl, above 4-6 membered heterocyclyl is optionally further substituted by one or more substituents selected from the group consisting of deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ and $-N(R_{15})-C(O)R_{14}$;

$R_7$ and $R_8$ are each independently selected from the group consisting of hydrogen, deuterium, hydroxy, $C_{1-4}$ alkyl and $C_{2-4}$ alkenyl, or, $R_7$ and $R_8$, together with the nitrogen atom to which they are directly attached, form a 3-6 membered heterocyclyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_{11}$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl and 3-6 membered heterocyclyl;

wherein, $R_{12}$, $R_{13}$, $R_{14}$, $R_{15}$, $R_{16}$ and r are defined as those in the compound of formula (I).

As a further preferred embodiment, in the compound of formula (I), the stereoisomer or pharmaceutically acceptable salt thereof, the compound of formula (I) is a compound of formula ($IIIc_1$) or ($IIIc_2$) as below:

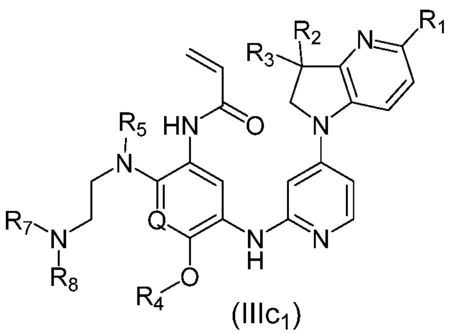

($IIIc_1$)

or

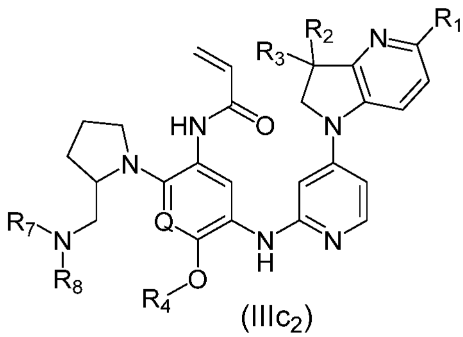

($IIIc_2$)

,

wherein, each Q is CH or N;

each $R_1$ is independently selected from the group consisting of hydrogen, deuterium, halogen, cyano, $C_{1-4}$ alkyl and 5-8 membered heteroaryl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl and 3-6 membered heterocyclyl;

$R_2$ and $R_3$ are each independently selected from the group consisting of hydrogen, deuterium, halogen, $C_{1-4}$ alkyl, $C_{1-4}$ haloalkyl, $C_{1-4}$ deuterioalkyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl;

each $R_4$ is independently selected from hydrogen, deuterium, $C_{1-4}$ alkyl and $C_{3-6}$ cycloalkyl, the above groups are optionally further substituted by one or more substituents selected from the group consisting of deuterium, halogen, hydroxy, cyano, $C_{1-4}$ alkyl, $C_{1-4}$ alkoxy, $C_{3-6}$ cycloalkyl, $C_{3-6}$ cycloalkyloxy, 3-6 membered heterocyclyl and 3-6 membered heterocyclyloxy;

in the compound of formula ($IIIc_1$), $R_5$ is selected from hydrogen, deuterium, $C_{1-4}$ alkyl, $C_{1-4}$ haloalkyl and $C_{1-4}$ deuterioalkyl;

$R_7$ and $R_8$ are each independently selected from hydrogen, deuterium and $C_{1-4}$ alkyl, or, $R_7$ and $R_8$, together with the nitrogen atom to which they are directly attached, form a 3-6 membered heterocyclyl.

As a more further preferred embodiment, in the compound of formula (I), the stereoisomer or pharmaceutically acceptable salt thereof, each $R_1$ is independently selected from the group consisting of hydrogen, deuterium, fluorine, chlorine, bromine, cyano, methyl, ethyl, propyl, isopropyl,

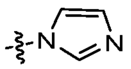 and 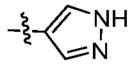 ,

the above groups are optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl, cyclobutyl, oxacyclobutyl and azacyclobutyl;

$R_2$ and $R_3$ are each independently selected from the group consisting of hydrogen, deuterium, fluorine, chlorine, bromine, methyl, ethyl, propyl, isopropyl, trifluoromethyl, difluoromethyl, trideuteriomethyl, dideuteriomethyl, cyclopropyl, cyclobutyl, oxacyclobutyl and azacyclobutyl;
each $R_4$ is independently selected from the group consisting of hydrogen, deuterium, methyl, ethyl, propyl, isopropyl, cyclopropyl and cyclobutyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl;
in the compound of formula ($IIIc_1$), $R_5$ is selected from hydrogen, deuterium and methyl;
$R_7$ and $R_8$ are each independently selected from hydrogen, deuterium and methyl.

As a preferred embodiment, in the compound of formula (I), the stereoisomer or pharmaceutically acceptable salt thereof, each $R_{12}$ is independently selected from the group consisting of hydrogen, deuterium, hydroxy, $C_{1-4}$ alkyl, $C_{2-4}$ alkenyl, $C_{3-6}$ cycloalkyl, 3-6 membered heterocyclyl, $C_{6-8}$ aryl, 5-8 membered heteroaryl and $-NR_{15}R_{16}$, the above groups are optionally further substituted by one or more substituents selected from the group consisting of deuterium, halogen, hydroxy, oxo, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

each $R_{13}$ is independently selected from the group consisting of hydrogen, deuterium, $C_{1-4}$ alkyl, $C_{2-4}$ alkenyl, $C_{3-6}$ cycloalkyl, 3-6 membered heterocyclyl, $C_{6-8}$ aryl and 5-8 membered heteroaryl, the above groups are optionally further substituted by one or more substituents selected from the group consisting of deuterium, halogen, hydroxy, oxo, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;
each $R_{14}$ is independently selected from the group consisting of hydrogen, deuterium, hydroxy, $C_{1-4}$ alkyl, $C_{1-4}$ alkoxy, $C_{2-4}$ alkenyl, $C_{2-4}$ alkynyl, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$, the above groups are optionally further substituted by one or more substituents selected from the group consisting of deuterium, halogen, hydroxy, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;
$R_{15}$ and $R_{16}$ are each independently selected from the group consisting of hydrogen, deuterium, hydroxy, $C_{1-4}$ alkoxy, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl, amino, mono$C_{1-4}$ alkylamino, di$C_{1-4}$ alkylamino and $C_{1-4}$ alkanoyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy, amino, mono$C_{1-4}$ alkylamino, di$C_{1-4}$ alkylamino and $C_{1-4}$ alkanoyl,
or, $R_{15}$ and $R_{16}$, together with the nitrogen atom to which they are directly attached, form a 5-8 membered heterocyclyl or 5-8 membered heteroaryl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy, amino, mono$C_{1-4}$ alkylamino, di$C_{1-4}$ alkylamino and $C_{1-4}$ alkanoyl.

As the most preferred embodiment, the compound of formula (I), the stereoisomer or pharmaceutically acceptable salt thereof comprises, but is not limited to, the following compounds:

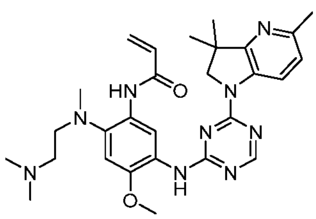

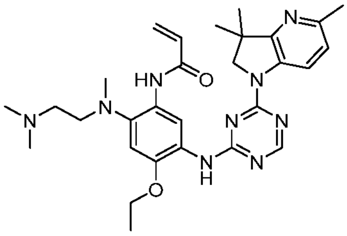

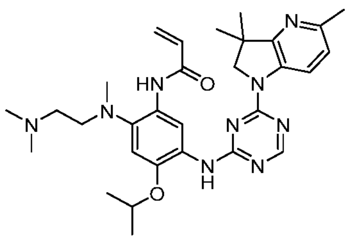

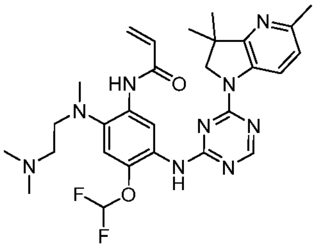

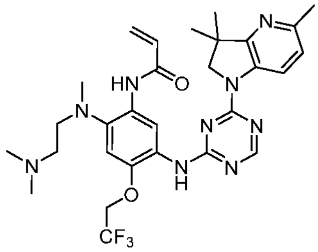

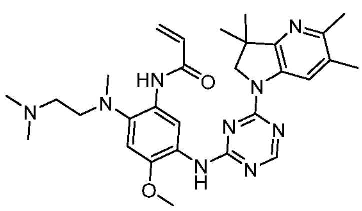

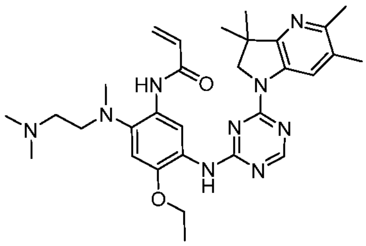

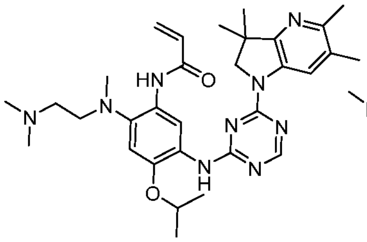

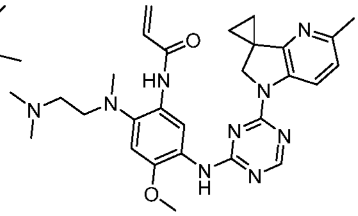

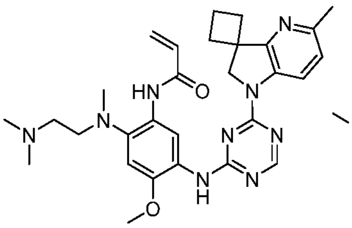

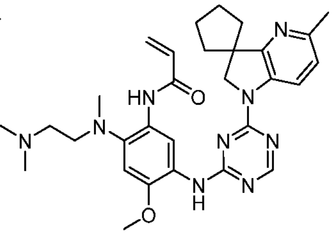

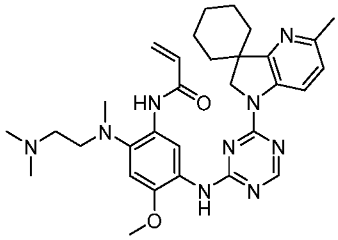

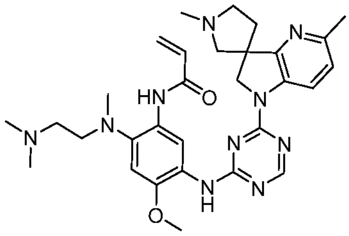

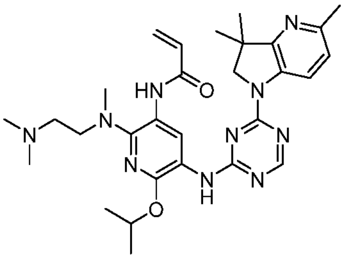

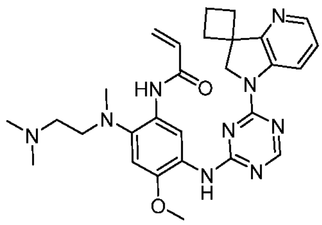

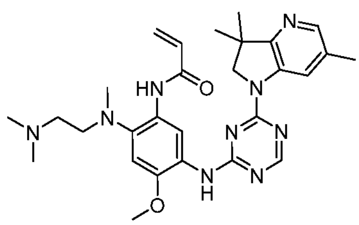

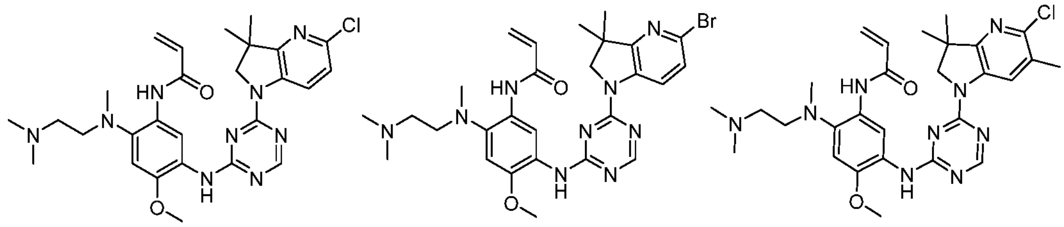

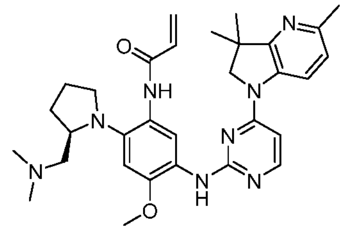

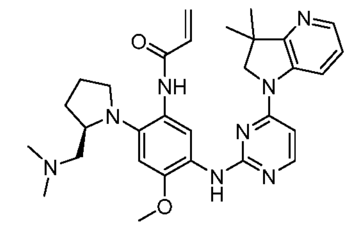

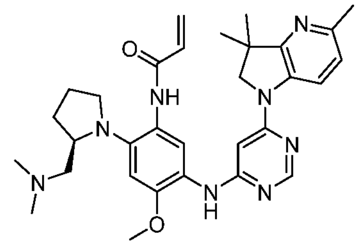

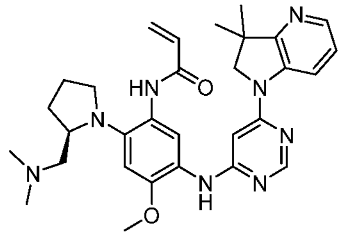

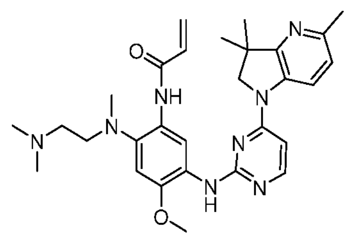

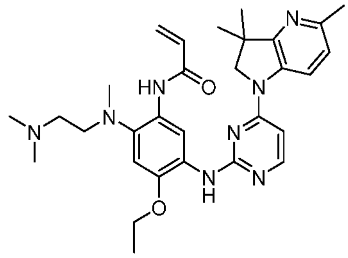

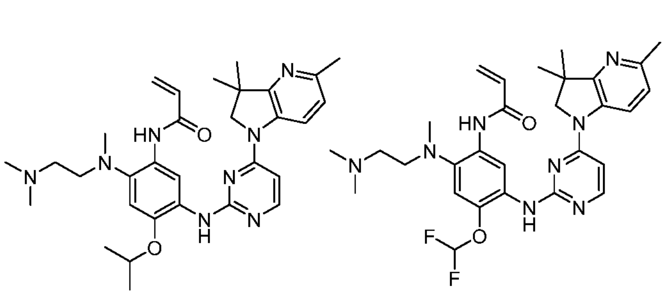

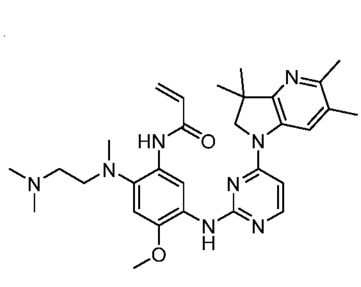

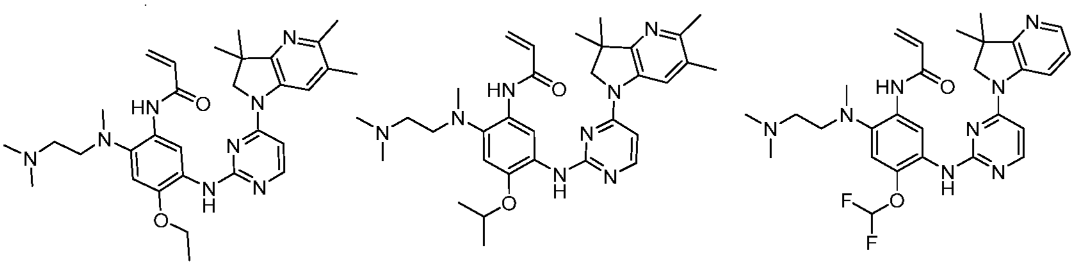

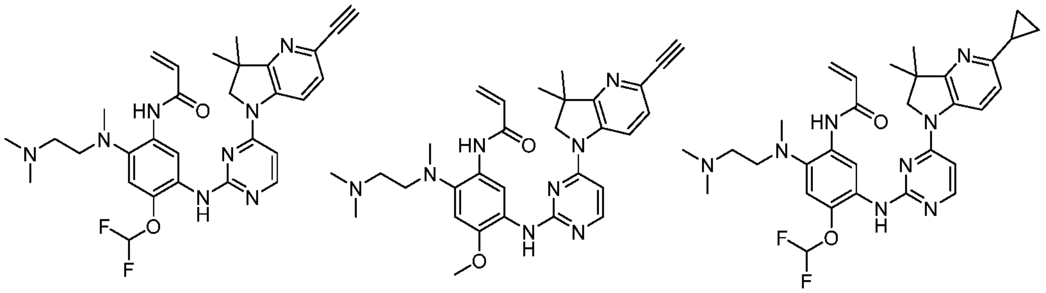

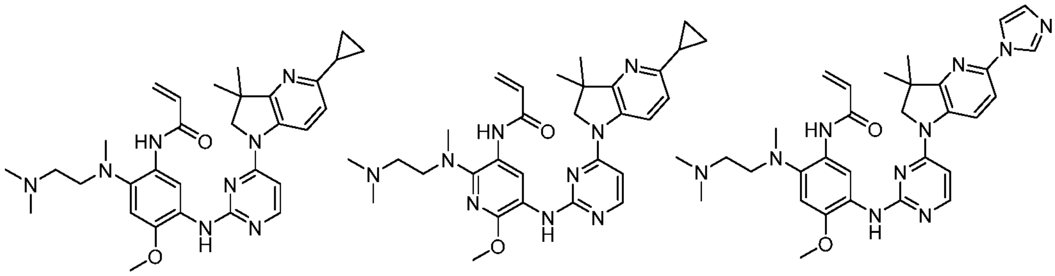

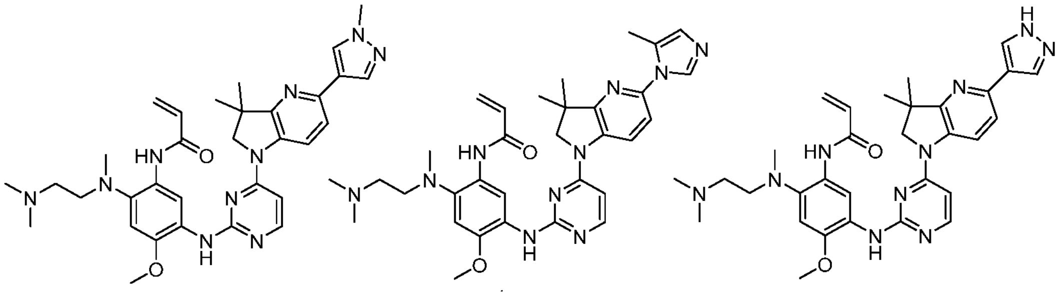

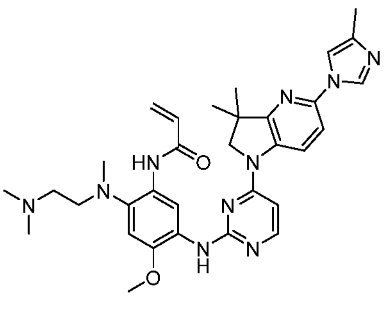

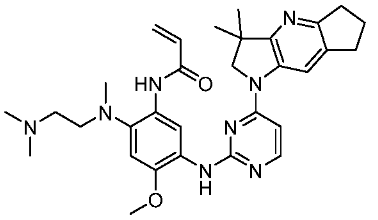

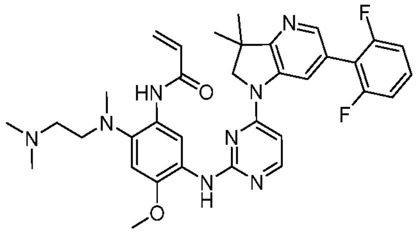

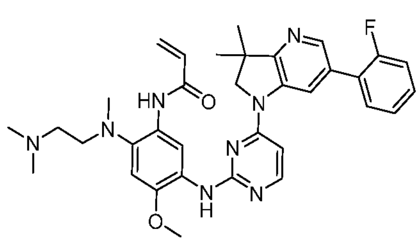

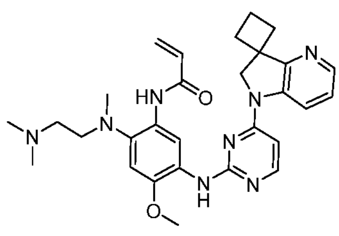

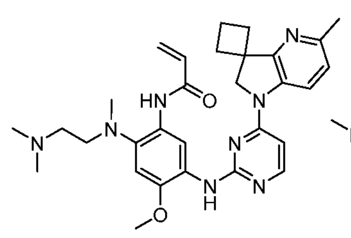

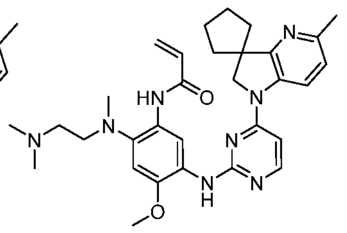

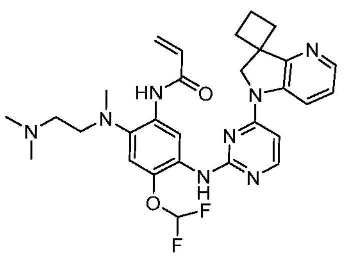

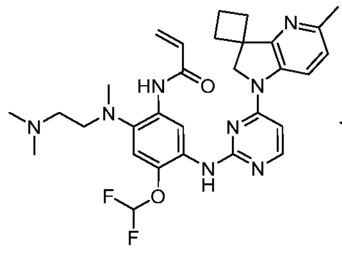

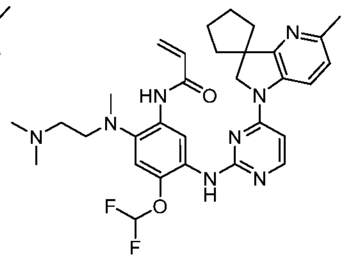

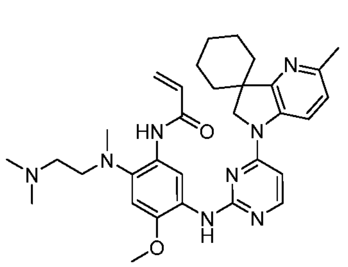

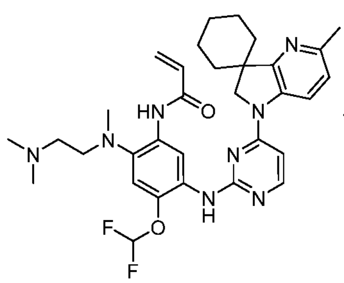

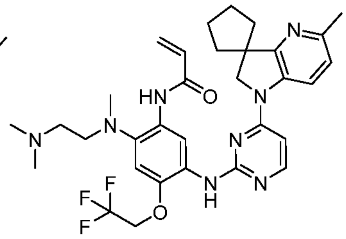

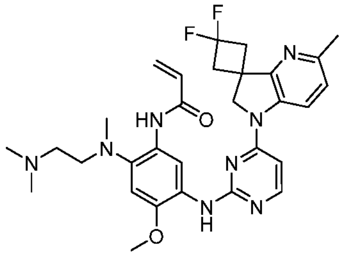

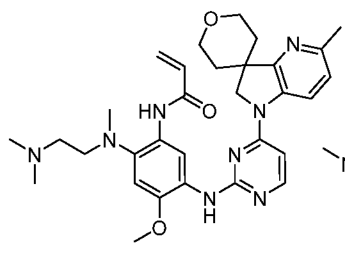

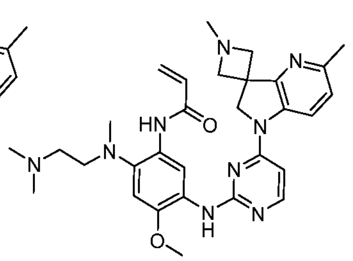

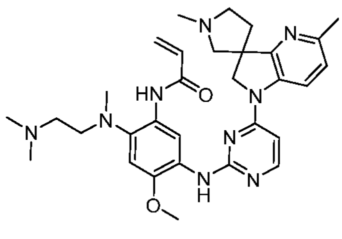

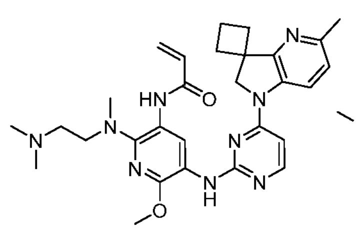

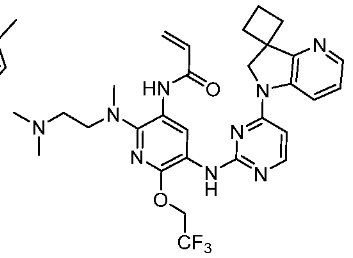

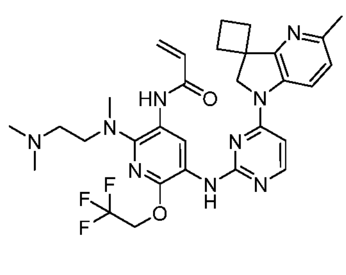
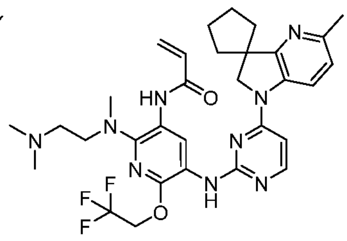
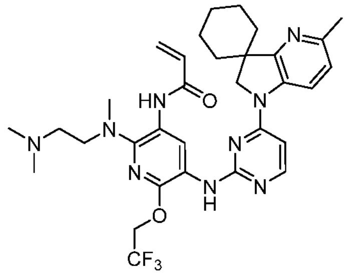

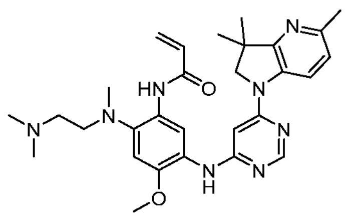
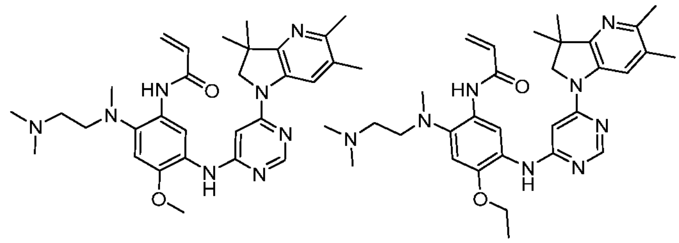

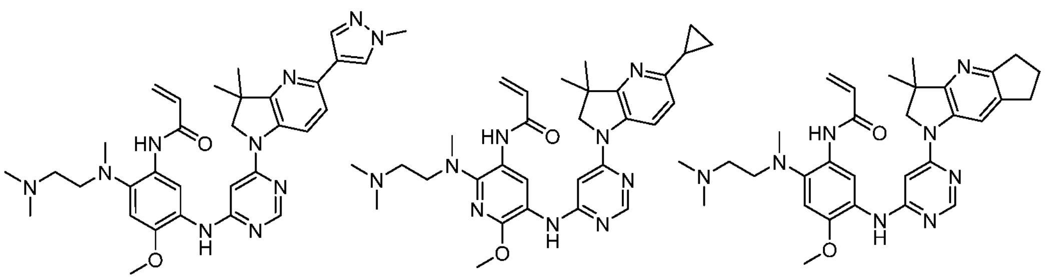

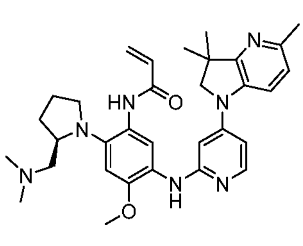
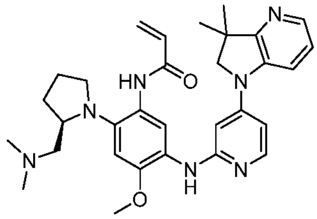
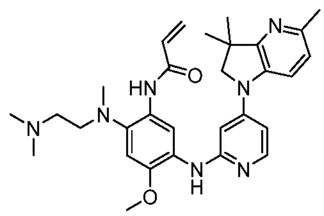

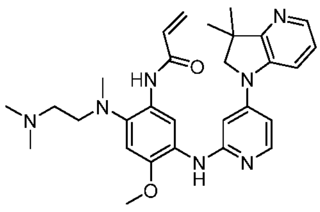
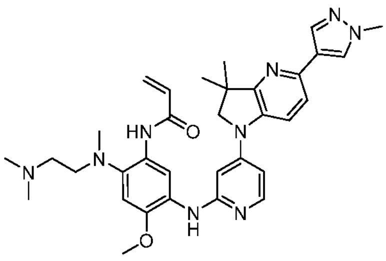

and

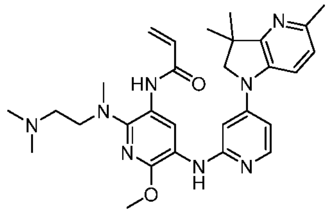

.

The second aspect of the present invention provides a preparation method for the compound of formula (I), the stereoisomer, or pharmaceutically acceptable salt thereof, comprising the following steps:

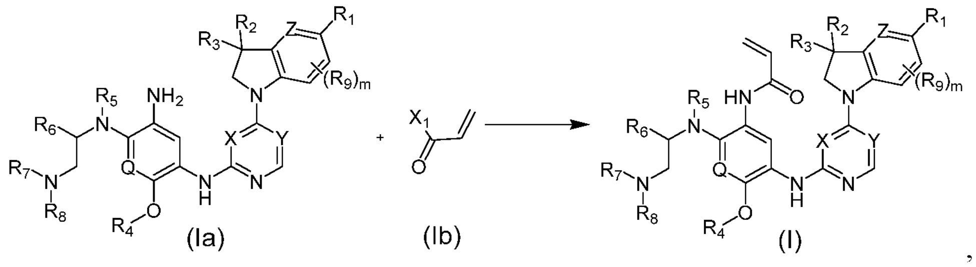

wherein, $X_1$ is halogen, preferably selected from fluorine, chlorine and bromine; $R_1$, $R_2$, $R_3$, $R_4$, $R_5$, $R_6$, $R_7$, $R_8$, $R_9$, X, Y, Z, Q and m are defined as those in the compound of formula (I).

The third aspect of the present invention provides a pharmaceutical composition, comprising the compound of formula (I), the stereoisomer or pharmaceutically acceptable salt thereof as defined, and a pharmaceutically acceptable carrier.

The present invention also relates to use of the compound of formula (I), the stereoisomer or pharmaceutically acceptable salt thereof as defined in preparation of a medicament for treatment and/or prevention of cancers, tumors or metastatic diseases at least partially associated with the insertion, deletion or other mutations of EGFR exon 20.

The present invention also relates to use of the compound of formula (I), the stereoisomer or pharmaceutically acceptable salt thereof as defined in preparation of a medicament for prevention and/or treatment of cancers, tumors or metastatic diseases caused by hyperproliferation and dysfunction in cell death induction.

The present invention also relates to use of the compound of formula (I), the stereoisomer or pharmaceutically acceptable salt thereof as previously defined in the preparation of a medicament for prevention and/or treatment of lung cancer, colon cancer, pancreatic cancer, head and neck cancer, breast cancer, ovarian cancer, uterine cancer, gastric cancer, non-small cell lung cancer, leukemia, myelodysplastic syndrome, malignant lymphoma, head and neck tumor, thoracic tumor, gastrointestinal tumor, endocrine tumor, breast and other gynecological tumor, urological tumor, skin tumor, sarcoma, sinonasal inverted papilloma, or sinonasal squamous cell carcinoma associated with sinonasal inverted papilloma, which is at least partially associated with the insertion, deletion or other mutations of EGFR exon 20.

The present invention also relates to the compound of formula (I), the stereoisomer or pharmaceutically acceptable salt thereof as defined for use as a medicament.

The present invention also relates to use of the compound of formula (I), the stereoisomer or pharmaceutically acceptable salt thereof as defined in the treatment and/or prevention of cancers, tumors or metastatic diseases at least partially associated with the insertion, deletion or other mutations of EGFR exon 20.

The present invention also relates to the compound of formula (I), the stereoisomer or pharmaceutically acceptable salt thereof as defined for use in the prevention and/or treatment of tumors, cancers or metastatic diseases caused by hyperproliferation and dysfunction in cell death induction

The present invention also relates to the compound of formula (I), the stereoisomer or pharmaceutically acceptable salt thereof as defined for use in the treatment and/or prevention of lung cancer, colon cancer, pancreatic cancer, head and neck cancer, breast cancer, ovarian cancer, uterine cancer, gastric cancer, non-small cell lung cancer, leukemia, myelodysplastic syndrome, malignant lymphoma, head and neck tumor, thoracic tumor, gastrointestinal tumor, endocrine tumor, breast and other gynecological tumor, urological tumor, skin tumor, sarcoma, sinonasal inverted papilloma, or sinonasal squamous cell carcinoma associated with sinonasal inverted papilloma, which is at least partially associated with the insertion, deletion or other mutations of EGFR exon 20.

The present invention also relates to a method for treating and/or preventing cancers, tumors or metastatic diseases at least partially associated with the insertion, deletion or other mutations of EGFR exon 20, and the method comprises: administrating a therapeutically effective amount of the compound of formula (I), the stereoisomer or pharmaceutically acceptable salt thereof as defined to a patient in need thereof.

The present invention also relates to a method for preventing and/or treating tumors, cancers or metastatic diseases caused by hyperproliferation and induced cell death disorders, and the method comprises: administrating a therapeutically effective amount of the compound of formula (I), the stereoisomer or pharmaceutically acceptable salt thereof as defined to a patient in need thereof.

The present invention also relates to a method for treating and/or preventing lung cancer, colon cancer, pancreatic cancer, head and neck cancer, breast cancer, ovarian cancer, uterine cancer, gastric cancer, non-small cell lung cancer, leukemia, myelodysplastic syndrome, malignant lymphoma, head and neck tumor, thoracic tumor, gastrointestinal tumor, endocrine tumor, breast and other gynecological tumor, urological tumor, skin tumor, sarcoma, sinonasal inverted papilloma, or sinonasal squamous cell carcinoma associated with sinonasal inverted papilloma, which is at least partially associated with the insertion, deletion or other mutations of EGFR exon 20, and the method comprises: administrating a therapeutically effective amount of the compound of formula (I), the stereoisomer or pharmaceutically

acceptable salt thereof as defined to a patient in need thereof.

## DETAILED DESCRIPTION OF THE INVENTION

After extensive and in-depth studies, the inventor of the present application has developed for the first time an EGFR inhibitor having the structure represented by formula (I). The series of compounds of the present invention can be widely used in the preparation of a medicament for treatment and/or prevention of cancers, tumors or metastatic diseases at least partially associated with the insertion, deletion or other mutations of EGFR exon 20, in particular a medicament for treatment of hyperproliferative diseases and induced cell death disorders, whereby a new generation of EGFR inhibitors is expected to be developed. On such basis, the present invention has been completed.

Detailed description: Unless otherwise stated to the contrary or specifically noted, the following terms used in the specification and claims have the following meanings.

"Alkyl" refers to linear or branched saturated aliphatic alkyl groups, preferably linear or branched alkyl group containing 1 to 10 carbon atoms or 1 to 6 carbon atoms or 1 to 4 carbon atoms, which includes, but is not limited to methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, etc. "$C_{1-10}$ alkyl" refers to linear or branched alkyl group containing 1 to 10 carbon atoms, and "$C_{1-4}$ alkyl" refers to linear or branched alkyl group containing 1 to 4 carbon atoms.

Alkyl may be optionally substituted or unsubstituted, and when it is substituted, the substituent is preferably one or more (preferably 1, 2, 3 or 4) of the following groups independently selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ or $-N(R_{15})-C(O)R_{14}$.

"Cycloalkyl" or "carbocycle" refers to a monocyclic or polycyclic cyclic hydrocarbon substituent that is saturated or partially unsaturated. The partially unsaturated cyclic hydrocarbon means a cyclic hydrocarbon that may contain one or more (preferably 1, 2 or 3) double bonds, but none of rings has a fully conjugated $\pi$-electron system. The cycloalkyl includes monocyclic cycloalkyl and polycyclic cycloalkyl, preferably including a cycloalkyl grouping containing 3 to 12 or 3 to 8 or 3 to 6 carbon atoms. For example, "$C_{3-12}$ cycloalkyl" means a cycloalkyl group containing 3 to 12 carbon atoms, and "$C_{3-6}$ cycloalkyl" means a cycloalkyl group containing 3 to 6 carbon atoms, wherein:

monocyclic cycloalkyl includes, but is not limited to cyclopropyl, cyclobutyl, cyclopentyl, cyclopentenyl, cyclohexyl, cyclohexenyl, cyclohexadienyl, cycloheptyl, cycloheptatrienyl, cyclooctyl and the like;

and polycyclic cycloalkyl includes spiro, fused, and bridged cycloalkyls. "Spirocycloalkyl" refers to a polycyclic group in which a carbon atom (called spiro-atom) is shared among monocyclic rings, wherein those rings may contain one or more (preferably, 1, 2 or 3) double bonds, but none of them has a fully conjugated $\pi$-electron system. According to the number of the spiro-atoms shared among the rings, the spirocycloalkyl may be monospirocycloalkyl, bispirocycloalkyl or polyspirocycloalkyl, including but not limited to:

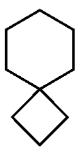 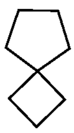 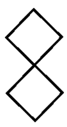 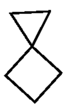 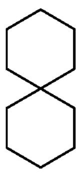 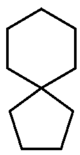 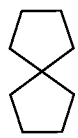 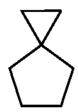.

"Fused cycloalkyl" refers to an all-carbon polycyclic group in which each ring shares a pair of adjacent carbon atoms with the other rings in the system, wherein one or more of the rings may contain one or more (preferably, 1, 2 or 3) double bonds, but none of them has a fully conjugated $\pi$-electron system. According to the number of formed rings, the fused cycloalkyl may be bicyclic, tricyclic, tetracyclic or polycyclic, including but not limited to:

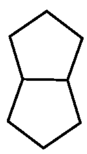 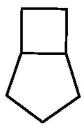 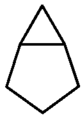 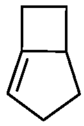 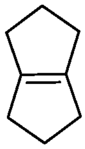

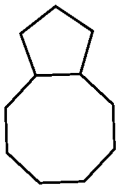 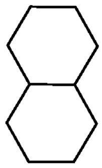 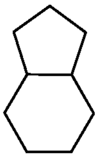 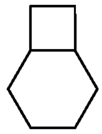 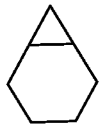

"Bridged cycloalkyl" refers to an all-carbon polycyclic group in which any two rings share two carbon atoms that are not directly connected to each other, wherein these rings may contain one or more (preferably, 1, 2 or 3) double bonds, but none of them has a fully conjugated $\pi$-electron system. According to the number of formed rings, the bridged cycloalkyl may be bicyclic, tricyclic, tetracyclic or polycyclic, including but not limited to:

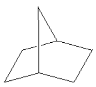 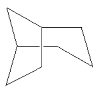 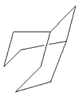 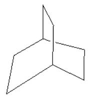 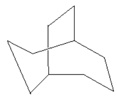 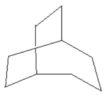 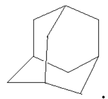

The cycloalkyl ring can be fused to an aryl, heteroaryl or heterocycloalkyl ring, wherein the ring attached to the parent structure is cycloalkyl, which includes, but is not limited to, indanyl, tetrahydronaphthyl, benzocycloheptyl, etc.

Cycloalkyl may be optionally substituted or unsubstituted, and when it is substituted, the substituent is preferably one or more (preferably 1, 2, 3 or 4) of the following groups independently selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ or $-N(R_{15})-C(O)R_{14}$.

"Heterocyclyl" or "heterocycle" refers to a monocyclic or polycyclic cyclic hydrocarbon substituent that is saturated or partially unsaturated. The partially unsaturated cyclic hydrocarbon means a cyclic hydrocarbon that may contain one or more (preferably 1, 2 or 3) double bonds, but none of rings has a fully conjugated $\pi$-electron system. One or more (preferably 1, 2, 3 or 4) ring atoms in the heterocyclyl are selected from heteroatoms of nitrogen, oxygen, or $S(O)_r$ (wherein r is an integer of 0, 1, or 2), but excluding the ring moiety of -O-O-, -O-S- or -S-S-, and the remaining ring atoms are carbon. The heterocyclyl preferably includes the one containing 3 to 12 or 3 to 8 or 3 to 6 ring atoms. For example, "3-6 membered heterocyclyl" refers to a ring group containing 3 to 6 ring atoms; "4-6 membered heterocyclyl" refers to a ring group containing 4 to 6 ring atoms; "4-10 membered heterocyclyl" refers to a ring group containing 4 to 10 ring atoms; and "3-12 membered heterocyclyl" refers to a ring group containing 3 to 12 ring atoms.

Monocyclic heterocyclyl includes, but is not limited to pyrrolidinyl, piperidinyl, piperazinyl, morpholinyl, thiomorpholinyl, homopiperazinyl, oxocyclobutane, tetrahydrofuranyl and the likes.

Polycyclic heterocyclyl includes spiro, fused, and bridged heterocyclyls. "Spiroheterocyclyl" refers to a polycyclic heterocyclyl group that shares an atom (called a spiro atom) between the monocyclic rings, wherein one or more (preferably 1, 2, 3 or 4) of the ring atoms are heteroatoms selected from nitrogen, oxygen, or $S(O)_r$ (wherein r is an integer of 0, 1, 2), and the remaining ring atoms are carbon atoms. These groups may contain one or more (preferably 1, 2 or 3) double bonds, but none of the rings have a fully conjugated $\pi$-electron system. The spiroheterocyclyl may be a monospiroheterocyclyl, a bispiroheterocyclyl or a polyspiroheterocyclyl according to the number of spiro atoms shared between the rings. Spiroheterocyclyl includes, but is not limited to:

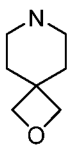 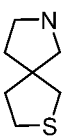 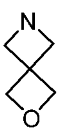 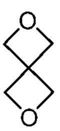 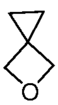 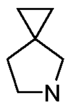 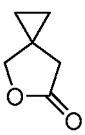

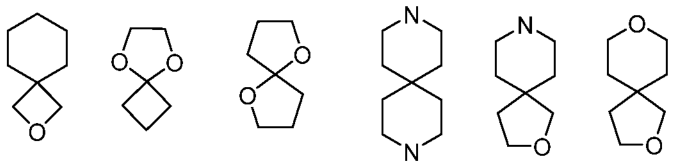

.

"Fused heterocyclyl" refers to a polycyclic heterocyclyl group in which each ring shares an adjacent pair of carbon atoms with other rings in the system, wherein one or more (preferably 1, 2, 3 or 4) of the rings may contain one or more (preferably 1, 2 or 3) double bonds, but none of the rings have a fully conjugated $\pi$-electron system, wherein one or more (preferably 1, 2, 3 or 4) of the ring atoms are heteroatoms selected from nitrogen, oxygen or $S(O)_r$ (wherein r is an integer of 0, 1, 2), and the remaining ring atoms are carbon atoms. Depending on the number of rings, it may be bicyclic, tricyclic, tetracyclic or polycyclic, fused heterocyclyl includes, but is not limited to:

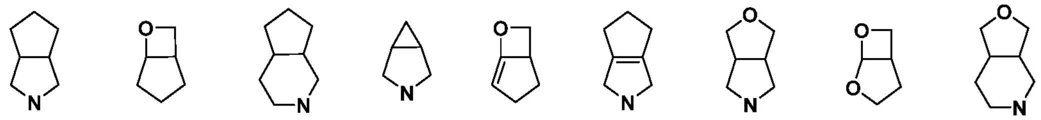

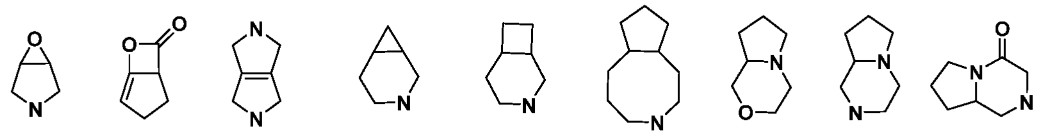

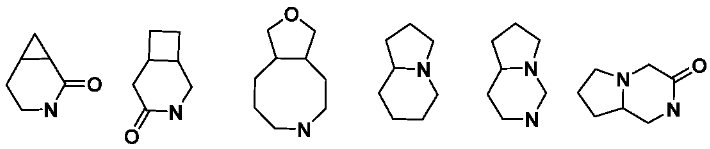

"Bridged heterocyclyl" refers to a polycyclic heterocyclyl group in which any two rings share two atoms that are not directly bonded, which may contain one or more (preferably 1, 2 or 3) double bonds, but none of the rings have a fully conjugated $\pi$-electron system, wherein one or more (preferably 1, 2, 3 or 4) of the ring atoms are heteroatoms selected from nitrogen, oxygen or $S(O)_r$ (wherein r is an integer of 0, 1, 2), and the remaining ring atoms are carbon atoms. Depending on the number of rings, it may be bicyclic, tricyclic, tetracyclic or polycyclic, bridged heterocyclyl includes, but is not limited to:

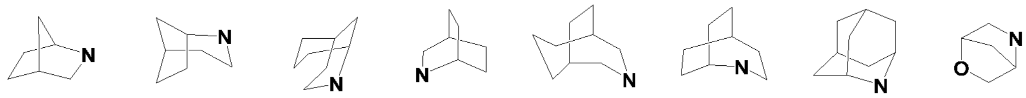

.

The ring of the heterocyclyl may be fused to a ring of aryl, heteroaryl or cycloalkyl wherein the ring attached to the parent structure is a heterocyclyl, which includes, but is not limited to:

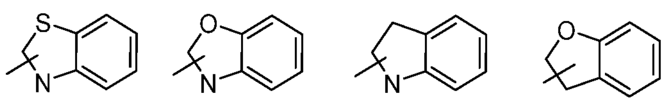

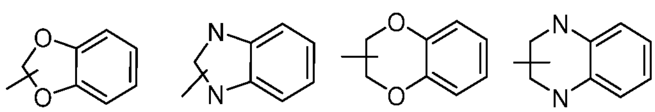

.

Heterocyclyl may be optionally substituted or unsubstituted, and when it is substituted, the substituent is preferably one or more (preferably 1, 2, 3 or 4) of the following groups independently selected from the group consisting of

deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ or $-N(R_{15})-C(O)R_{14}$.

"Aryl" or "aromatic ring" refers to an all-carbon monocyclic or fused polycyclic (i.e., a ring that shares a pair of adjacent carbon atoms) group, and a polycyclic group having a conjugated π-electron system (i.e., a ring with adjacent pairs of carbon atoms). All-carbon aryl containing 5 to 10 or 5 to 8 carbons is preferred. For example, "$C_{6-10}$ aryl" refers to all-carbon aryl containing 6 to 10 carbons, including but not limited to phenyl and naphthyl; and "$C_{6-8}$ aryl" refers to all-carbon aryl containing 6 to 8 carbons. An aryl ring can be fused to a ring of heteroaryl, heterocyclyl or cycloalkyl, wherein the ring attached to the parent structure is an aryl ring, which includes, but is not limited to:

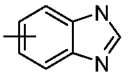 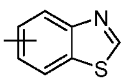 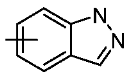 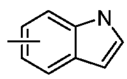 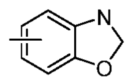

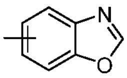 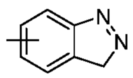 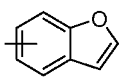 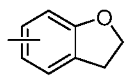 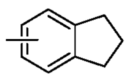

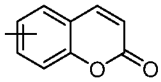 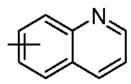 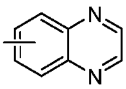 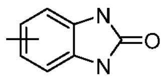 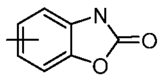 .

"Aryl" may be substituted or unsubstituted, and when it is substituted, the substituent is preferably one or more (preferably, 1, 2, 3 or 4) of the following groups independently selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ or $-N(R_{15})-C(O)R_{14}$.

"Heteroaryl" refers to a heteroaromatic system containing one or more (preferably 1, 2, 3 or 4) heteroatoms including a heteroatom selected from nitrogen, oxygen or $S(O)_r$ (wherein r is an integer of 0, 1, 2). The heteroaromatic system containing 5-10 or 5-8 or 5-6 ring atoms is preferred. For example, 5-6 membered heteroaryl refers to a heteroaromatic system containing 5 to 6 ring atoms, 5-8 membered heteroaryl refers to a heteroaromatic system containing 5 to 8 ring atoms, and 5-10 membered heteroaryl refers to a heteroaromatic system containing 5 to 10 ring atom, including but not limited to furyl, thiophenyl, pyridyl, pyrrolyl, N-alkylpyrrolyl, pyrimidinyl, pyrazinyl, imidazolyl, tetrazolyl or the likes. The heteroaryl ring may be fused to a ring of aryl, heterocyclyl or cycloalkyl wherein the ring attached to the parent structure is a heteroaryl ring, which includes, but is not limited to:

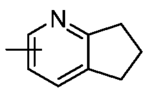 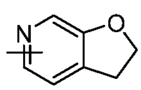 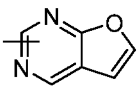 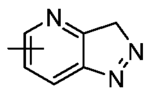 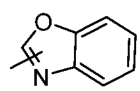

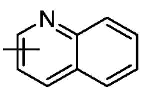 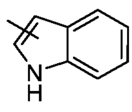 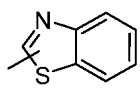 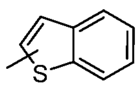 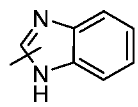 .

"Heteroaryl" may be optionally substituted or unsubstituted, and when it is substituted, the substituent is preferably one or more (preferably 1, 2, 3 or 4) of the following groups independently selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ or $-N(R_{15})-C(O)R_{14}$.

"Alkenyl" refers to an alkyl group defined as above consisting of at least two carbon atoms and at least one carbon-carbon double bond, preferably a linear or branched alkenyl containing 2-10 or 2-4 carbons. For example, $C_{2-10}$ alkenyl refers to a linear or branched alkenyl containing 2 to 10 carbons, $C_{2-4}$ alkenyl refers to a linear or branched alkenyl containing 2 to 4 carbons. Alkenyl includes, but is not limited to, vinyl, 1-propenyl, 2-propenyl, 1-, 2- or 3-butenyl, and the likes.

"Alkenyl" may be substituted or unsubstituted, and when it is substituted, the substituent is preferably one or

more (preferably 1, 2, 3 or 4) of the groups independently selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ or $-N(R_{15})-C(O)R_{14}$.

"Alkynyl" refers to an alkyl group defined as above consisting of at least two carbon atoms and at least one carbon-carbon triple bond, preferably a linear or branched alkynyl containing 2-10 or 2-4 carbons. For example, $C_{2-10}$ alkynyl refers to a linear or branched alkynyl containing 2 to 10 carbons, and $C_{2-4}$ alkynyl refers to a linear or branched alkynyl containing 2 to 4 carbons. Alkynyl includes, but is not limited to, ethynyl, 1-propynyl, 2-propynyl, 1-, 2- or 3-butynyl, and the likes.

"Alkynyl" may be substituted or unsubstituted, and when it is substituted, the substituent is preferably one or more (preferably 1, 2, 3 or 4) of the groups independently selected from the following group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ or $-N(R_{15})-C(O)R_{14}$.

"Alkoxy" refers to -O-alkyl, wherein alkyl is defined as above. For example, "$C_{1-10}$ alkoxy" refers to alkyloxy containing 1 to 10 carbons, and $C_{1-4}$ alkoxy refers to alkyloxy containing 1-4 carbons. Alkoxy includes, but is not limited to, methoxy, ethoxy, propoxy, butoxy, and the likes.

"Alkoxy" may be optionally substituted or unsubstituted, and when it is substituted, the substituent is preferably one or more (preferably 1, 2, 3 or 4) of the following groups independently selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ or $-N(R_{15})-C(O)R_{14}$.

"Cycloalkyloxy" refers to -O-cycloalkyl, wherein the cycloalkyl is as defined above. For example, "$C_{3-12}$ cycloalkyloxy" refers to a cycloalkyloxy containing 3 to 12 carbons, and "$C_{3-6}$ cycloalkyloxy" refers to a cycloalkyloxy containing 3 to 6 carbons. Cycloalkyloxy includes, but is not limited to, cyclopropyloxy, cyclobutyloxy, cyclopentyloxy, cyclohexyloxy and the likes.

"Cycloalkyloxy" may be optionally substituted or unsubstituted, and when it is substituted, the substituent is preferably one or more (preferably 1, 2, 3 or 4) of the following groups independently selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ or $-N(R_{15})-C(O)R_{14}$.

"Heterocyclyloxy" refers to -O-heterocyclyl, wherein the heterocyclyl is defined as above. The heterocyclyloxy includes, but is not limited to, azacyclobutyloxy, oxacyclobutyloxy, azacyclopentyloxy, nitrogen, oxacyclohexyloxy, etc.

"Heterocyclyloxy" may be optionally substituted or unsubstituted, and when it is substituted, the substituent is preferably one or more (preferably 1, 2, 3 or 4) of the following groups independently selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ or $-N(R_{15})-C(O)R_{14}$.

"$C_{1-10}$ alkanoyl" refers to a monovalent atomic group obtained by removing hydroxy from $C_{1-10}$ alkyl acid, and it is also generally represented as "$C_{0-9}$ alkyl-C(O)-". For example, "$C_1$ alkyl-C(O)-" refers to acetyl; "$C_2$ alkyl-C(O)-" refers to propionyl; and "$C_3$ alkyl-C(O)-" refers to butyryl or isobutyryl.

"$C_{1-10}$ haloalkyl" refers to an alkyl group having 1 to 10 carbon atoms, any hydrogen atom on which is optionally substituted with F, Cl, Br or I atom. It includes, but is not limited to, difluoromethyl, dichloromethyl, dibromomethyl, trifluoromethyl, trichloromethyl, tribromomethyl, and the likes.

"$C_{1-10}$ haloalkoxy" means an alkoxy group having 1 to 10 carbon atoms, wherein any hydrogen atom on which is optionally substituted with F, Cl, Br or I atom. It includes, but is not limited to, difluoromethoxy, dichloromethoxy, dibromomethoxy, trifluoromethoxy, trichloromethoxy, tribromomethoxy, and the likes.

"$C_{1-10}$ deuterioalkyl" means an alkyl group having 1 to 10 carbon atoms, wherein any hydrogen atom on which is optionally substituted with deuterium atom. It includes, but is not limited to, monodeuterioethoxy, dideuteriomethoxy, trideuteriomethoxy, and the likes.

"Halogen" means F, Cl, Br or I.

"Optional" or "optionally" means that the event or environment subsequently described may, but need not, occur, including where the event or environment occurs or does not occur, that is, including both substituted and unsubstituted situations. For example, "heterocyclyl optionally substituted by alkyl" means that an alkyl group may be, but is not necessarily, present, and the description includes the case where the heterocyclyl is substituted with an alkyl and the case where the heterocyclyl is not substituted with an alkyl.

The term "substituted" means that one or more "hydrogen atoms" in the group are each independently substituted by a corresponding number of substituents. It goes without saying that a substituent is only in its possible chemical

position, which is consistent with the valence-bond theory of chemistry. Those skilled in the art will be able to determine possible or impossible substitution (by experiments or theories) without undue efforts. For example, it may be unstable when an amino group or a hydroxyl group having a free hydrogen is attached with a carbon atom having an unsaturated bond (such as an olefin).

"Stereoisomer" means an isomer produced due to a different spatial arrangement of atoms in the molecules, and can be classified into either cis-trans isomers and enantiomers, or enantiomers and diastereomers. Stereoisomers resulting from the rotation of a single bond are called conformational stereo-isomers, and sometimes also called rotamers. Stereoisomers induced by reasons such as bond lengths, bond angles, double bonds in molecules and rings are called configuration stereo-isomers, which are classified into two categories. Among them, isomers induced by the double bonds or single bonds of ring-forming carbon atoms that cannot rotate freely are called geometric isomers, also known as cis-trans isomers, which are divided into two configurations including Z and E. For example: cis-2-butene and trans-2-butene are a pair of geometric isomers. Stereoisomers with different optical activities due to the absence of anti-axial symmetry in the molecules are called optical isomers, which are classified into two configurations including R and S. Unless otherwise specified, the "stereoisomer" in the present invention can be understood to include one or several of the above-mentioned enantiomers, configurational isomers and conformational isomers.

"Pharmaceutically acceptable salt" in the present invention refers to pharmaceutically acceptable acid addition salts, including inorganic acid salts and organic acid salts, and these salts can be prepared by methods known in the art.

"Pharmaceutical composition" refers to a mixture comprising one or more of the compounds described herein, or a physiologically/pharmaceutically acceptable salt or pro-drug thereof, and other chemical components, for example physiological/pharmaceutically acceptable carriers and excipients. The purpose of the pharmaceutical composition is to promote the administration to an organism, which facilitates the absorption of the active ingredient thereby exerting biological activities.

The present invention will be further described in detail below in conjunction with the embodiments which is not intended to limit the present invention. The present invention is also not limited to the contents of the embodiments.

The structure of the compound of the present invention is determined by nuclear magnetic resonance (NMR) or/and liquid chromatography-mass spectrometry (LC-MS). The NMR chemical shift ($\delta$) is given in parts per million (ppm). The NMR is measured by a Bruker AVANCE-400/500 nuclear magnetic apparatus, and the solvent is deuterated dimethyl sulfoxide (DMSO-$d_6$), deuterated methanol ($CD_3OD$) and deuterated chloroform ($CDCl_3$), and the internal standard is tetramethylsilane (TMS).

The measurement of LC-MS is performed by using an Agilent 6120 mass spectrometer. The measurement of HPLC is performed by using an Agilent 1200 DAD high pressure liquid chromatograph (Sunfire C18 150 $\times$ 4.6 mm column) and a Waters 2695-2996 high pressure liquid chromatograph (Gimini C18 150 $\times$ 4.6 mm column).

The thin layer chromatography silica gel plate is Yantai Yellow Sea HSGF254 or Qingdao GF254 silica gel plate. The specification of TLC is 0.15 mm - 0.20 mm, and the specification for thin layer chromatography separation and purification is 0.4 mm - 0.5 mm. 200-300 mesh silica gel (Yantai Huanghai silica gel) as a carrier is generally used in column chromatography.

The starting materials in the embodiments of the present invention are known and commercially available or can be synthesized according to methods known in the art.

Unless otherwise stated, all reactions of the present invention are carried out under continuous magnetic stirring in a dry nitrogen or argon atmosphere, the solvent is a dry solvent, and the unit of the reaction temperature is Celsius degree (°C).

**I. Preparation of Intermediates**

**Intermediate A1: Preparation of 3,3-dimethyl-2,3-dihydro-1*H*-pyrrolo[3,2-b]pyridine**

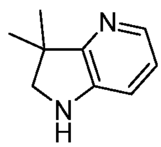

**Step 1: Synthesis of 2-iodo-*N*-(2-methylallyl)pyridin-3-amine**

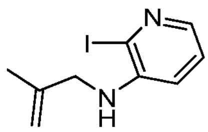

At room temperature, potassium tert-butoxide in tetrahydrofuran solution (27 mL, 1M, 27.2 mmol) was added to the solution of 2-iodopyridin-3-amine (5 g, 22.7 mmol) in tetrahydrofuran (100 mL). The mixture was stirred for 15 minutes at room temperature. Then, 3-bromo-2-methylprop-1-ene (3.68 g, 27.2 mmol) was slowly added dropwise to the mixture. The reaction mixture was stirred for 2 h at room temperature. After the reaction was completed, the mixture was concentrated under reduced pressure to remove the solvent. The residue was separated by flash silicagel columns [eluent: ethyl acetate/petroleum ether: 0-20%] to obtain 2-iodo-*N*-(2-methylallyl)pyridin-3-amine (2.7 g, yield: 43%), ESI-MS: 275.0 $[M+1]^+$.

**Step 2: Synthesis of 3,3-dimethyl-2,3-dihydro-1*H*-pyrrolo[3,2-*b*]pyridine**

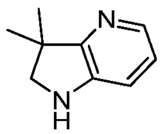

2-iodo-*N*-(2-methylallyl)pyridin-3-amine (2.7 g, 10 mmol), sodium formate (816 mg, 12 mmol), tetrabutylammonium chloride (3.3 g, 12 mmol), triethylamine (3 g, 30 mmol), palladium acetate (448 mg, 2 mmol), dimethylsulfoxide (50 mL) and water (3 mL) were added to a reaction flask. The mixture was subjected to nitrogen displacement three times, and under the protection of nitrogen, was heated to 120 °C and stirred for 1 h. The reaction mixture was filtered. The filtrate was washed with water and extracted with ethyl acetate. The organic layer was dried over anhydrous sodium sulfate, and distilled under reduced pressure to obtain a crude product, which was separated by flash silicagel columns [eluent: ethyl acetate/petroleum ether: 0-30%] to obtain 3,3-dimethyl-2,3-dihydro-1*H*-pyrrolo[3,2-*b*]pyridine (612 mg, yield: 41%), ESI-MS: 149.0 $[M+1]^+$.

**Intermediate A2: Preparation of 5-chloro-3,3-dimethyl-2,3-dihydro-1*H*-pyrrolo[3,2-*b*]pyridine**

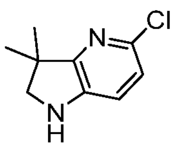

**Step 1: Synthesis of 6-chloro-2-iodopyridin-3-amine**

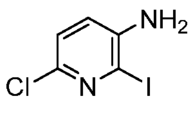

*N*-iodosuccinimide (23.3 g, 103.5 mmol) was added to the solution of 6-chloropyridin-3-amine (12.1 g, 94.1 mmol) in *N,N*-dimethylformamide (200 mL). The reaction mixture was stirred for 16 hrs at room temperature. The reaction mixture was poured into water, and ethyl acetate was added for extraction. The organic phases were combined, the saturated saline water was added for washing, and the organic phases were concentrated and then separated by column chromatography [petroleum ether:ethyl acetate=4: 1] to obtain 6-chloro-2-iodopyridin-3-amine (17.4 g, yield: 72.65%). ESI-MS: 254.8 $[M+1]^+$.

$^1H$ NMR (400 MHz, DMSO-$d_6$) δ 7.17 (d, $J$ = 8.4 Hz, 1H), 7.03 (d, $J$ = 8.4 Hz, 1H), 5.56 (s, 2H).

**Step 2: Synthesis of 6-chloro-2-iodo-*N*-(2-methylallyl)pyridin-3-amine**

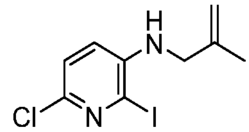

3-bromo-2-methylprop-1-ene (11.0 g, 82.0 mmol) and potassium tert-butoxide in tetrahydrofuran solution (82.0 mL, 1M, 82.0 mmol) were added to the solution of 6-chloro-2-iodopyridin-3-amine (17.4 g, 68.3 mmol) in tetrahydrofuran (200 mL). The reaction mixture was stirred for 2 hrs at room temperature. The reaction mixture was poured into water, and ethyl acetate was added for extraction. The organic phases were combined, the saturated saline water was added for washing, and the organic phases were concentrated and then separated by column chromatography [petroleum ether:ethyl acetate=4: 1] to obtain 6-chloro-2-iodo-*N*-(2-methylallyl)pyridin-3-amine (19.4 g, yield: 91.9%). ESI-MS: 308.8 $[M+1]^+$.

**Step 3: Synthesis of 5-chloro-3,3-dimethyl-2,3-dihydro-1*H*-pyrrolo[3,2-*b*]pyridine**

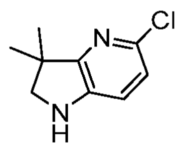

Sodium formate (4.1 g, 60.2 mmol), tetrabutylammonium chloride (16.7 g, 60.2 mmol), triethylamine (15.2 g, 150.7 mmol) and palladium acetate (1.7 g, 7.5 mmol) were added to the solution of 6-chloro-2-iodo-*N*-(2-methylallyl)pyridin-3-amine (15.5 g, 50.2 mmol) in dimethylsulfoxide/water (200 mL/6mL). After reaction, evacuation and nitrogen displacement were conducted, the mixture was stirred for 2 h at 120 °C. The reaction mixture was poured into water, and ethyl acetate was added for extraction. The organic phases were combined, the saturated saline water was added for washing, and the organic phases were concentrated and then separated by column chromatography [petroleum ether:ethyl acetate=4: 1] to obtain 5-chloro-3,3-dimethyl-2,3-dihydro-1*H*-pyrrolo[3,2-*b*]pyridine (6.5 g, yield: 70.8%). ESI-MS: 183.1 $[M+1]^+$.

**Intermediate A3: Preparation of 3,3,5-trimethyl-2,3-dihydro-1*H*-pyrrolo[3,2-*b*]pyridine**

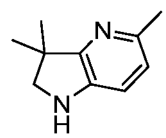

Methylboronic acid (10.7 g, 178.7 mmol), potassium phosphate (22.6 g, 106.7 mmol), tricyclohexylphosphine (3.0 g, 10.6 mmol) and palladium acetate (1.2 g, 5.3 mmol) were added to the solution of 5-chloro-3,3-dimethyl-2,3-dihydro-1*H*-pyrrolo[3,2-*b*]pyridine (6.5 g, 35.5 mmol) in toluene (150 mL). After reaction, evacuation and nitrogen displacement were conducted, the mixture was stirred for 18 h at 110 °C. The reaction mixture was poured into water, and ethyl acetate was added for extraction. The organic phases were combined, the saturated saline water was added for washing, and the organic phases were concentrated and then separated by column chromatography [petroleum ether: ethyl acetate=3: 1] to obtain 3,3,5-trimethyl-2,3-dihydro-1*H*-pyrrolo[3,2-*b*]pyridine (1.1 g, yield: 19.0%). ESI-MS: 163.0 $[M+1]^+$.

**Intermediate A4: Preparation of 5-cyclopropyl-3,3-dimethyl-2,3-dihydro-1*H*-pyrrolo[3,2-*b*]pyridine**

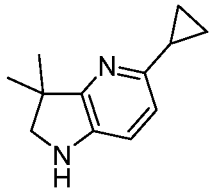

5-chloro-3,3-dimethyl-2,3-dihydro-1*H*-pyrrolo[3,2-*b*]pyridine (450 mg, 2.5 mmol), cyclopropylboronic acid (1.1g, 12.4 mmol), potassium phosphate (1.94 g, 9.1 mmol), tricyclohexylphosphine (138 mg, 0.5 mmol), palladium acetate (55 mg, 0.3 mmol), toluene (30 mL) were added to a reaction flask. The mixture was subjected to nitrogen displacement three times, and under the protection of nitrogen, was heated to 110 °C and stirred for 6 h. The reaction mixture was filtered. The filtrate was washed with water and extracted with ethyl acetate. The organic layer was dried over anhydrous sodium sulfate, and distilled under reduced pressure to obtain a crude product, which was separated by flash silicagel columns [eluent: ethyl acetate/petroleum ether: 0-30%] to obtain 5-cyclopropyl-3,3-dimethyl-2,3-dihydro-1*H*-pyrrolo[3,2-*b*]pyridine (152 mg, yield: 33.0%). ESI-MS: 189.0 $[M+1]^+$.

**Intermediate A5: Preparation of 3,3-dimethyl-5-(1-methyl-1*H*-pyrazol-4-yl)-2,3-dihydro-1*H*-pyrrolo[3,2-*b*]pyridine**

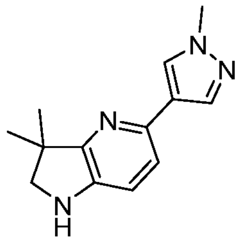

5-chloro-3,3-dimethyl-2,3-dihydro-1*H*-pyrrolo[3,2-*b*]pyridine (274 mg, 1.5 mmol), 1-methyl-4-(4,4,5,5-tetramethyl-1,3,2-dioxaborolane-2-yl)-1*H*-pyrazol (624 mg, 3.0 mmol), potassium phosphate (955 mg, 4.5 mmol), tricyclohexylphosphine (168 mg, 0.6 mmol), palladium acetate (67.3 mg, 0.3 mmol), and toluene (50 mL) were added to a reaction flask. The mixture was subjected to nitrogen displacement three times, and under the protection of nitrogen, was heated to 110 °C and stirred for 16 h. The reaction mixture was filtered. The filtrate was washed with water and extracted with ethyl acetate. The organic layer was dried over anhydrous sodium sulfate, and distilled under reduced pressure to obtain a crude product, which was separated by flash silicagel columns [eluent: ethyl acetate/petroleum ether: 0-30%] to obtain 3,3-dimethyl-5-(1-methyl-1*H*-pyrazol-4-yl)-2,3-dihydro-1*H*-pyrrolo[3,2-*b*]pyridine (125 mg, yield: 35.7%). ESI-MS: 229.0 $[M+1]^+$.

**Intermediate A6: Preparation of 5-bromo-3,3-dimethyl-2,3-dihydro-1*H*-pyrrolo[3,2-*b*]pyridine**

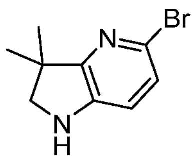

**Step 1: Synthesis of 6-bromo-2-iodopyridin-3-amine**

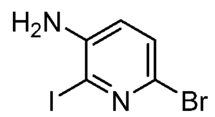

At room temperature, *N*-iodosuccinimide (2.70g, 12.0 mmol) was added to the solution of 6-brominepyridin-3-amine (1.73g, 10 mmol) in *N*,*N*-dimethylformamide (50 mL). The mixture was stirred overnight at room temperature. After the reaction was completed, the reaction mixture was washed with water and extracted with ethyl acetate. The organic phase was dried over anhydrous sodium sulfate, and concentrated under reduced pressure to remove the solvent. The residue was separated by flash silicagel columns [eluent: ethyl acetate/petroleum ether: 0-20%] to obtain 6-bromo-2-iodopyridin-3-amine (2.1 g, yield: 66.4%). ESI-MS: 298.8, 300.8 $[M+1]^+$.

**Step 2: Synthesis of 6-bromo-2-iodo-*N*-(2-methallyl)pyridin-3-amine**

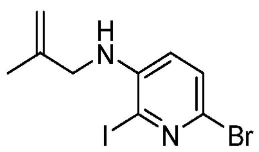

At room temperature, potassium tert-butoxide (8.4 mL, 8.4 mmol, 1M/mL) was added to the solution of 6-bromo-2-iodopyridin-3-amine (2.09 g, 7.0 mmol) in tetrahydrofuran (50 mL). The mixture was stirred for 15 minutes at room temperature. Then, 3-bromo-2-methylprop-1-ene (1.04 g, 7.7 mmol) was slowly added dropwise to the mixture. The reaction mixture was stirred for 2 h at room temperature. After the reaction was completed, the mixture was concentrated under reduced pressure to remove the solvent. The residue was separated by flash silicagel columns [eluent: ethyl acetate/petroleum ether: 0-20%] to obtain 6-bromo-2-iodo-*N*-(2-methallyl)pyridin-3-amine (2.1 g, yield: 69.8%). ESI-MS: 352.8, 354.8 $[M+1]^+$.

**Step 3: Synthesis of 5-bromo-3,3-dimethyl-2,3-dihydro-1*H*-pyrrolo[3,2-*b*]pyridine**

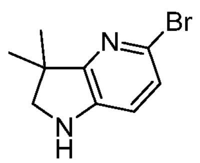

6-bromo-2-iodo-*N*-(2-methallyl)pyridin-3-amine (2.1 g, 5.9 mmol), sodium formate (0.49g, 7.1 mmol), tetrabutylammonium chloride (1.98 g, 7.1 mmol), triethylamine (1.8 g, 17.8 mmol), palladium acetate (0.2g, 0.9 mmol), dimethylsulfoxide (20 mL) and water (2 mL) were added to a reaction flask. The mixture was subjected to nitrogen displacement three times, and under the protection of nitrogen, was heated to 120 °C and stirred for 1 h. The reaction mixture was filtered. The filtrate was washed with water and extracted with ethyl acetate. The organic layer was dried over anhydrous sodium sulfate, and distilled under reduced pressure to obtain a crude product, which was separated by flash silicagel columns [eluent: ethyl acetate/petroleum ether: 0-30%] to obtain 5-bromo-3,3-dimethyl-2,3-dihydro-1*H*-pyrrolo[3,2-*b*]pyridine (0.6g, yield: 38.6%). ESI-MS: 226.9, 228.9, $[M+1]^+$.

**Intermediate A7: Preparation of 5-(1*H*-imidazol-1-yl)-3,3-dimethyl-2,3-dihydro-1*H*-pyrrolo[3,2-*b*]pyridine**

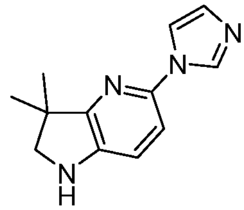

5-bromo-3,3-dimethyl-2,3-dihydro-1*H*-pyrrolo[3,2-*b*]pyridine (500 mg, 2.2 mmol), imidazole (300 mg, 4.4 mmol), potassium carbonate (913 mg, 6.6 mmol), cuprous iodide (83.9 mg, 0.44 mmol), (1*S*,2*S*)-$N^1$,$N^2$-dimethyl cyclohexane-1,2-diamine (125 mg, 0.88 mmol), and dimethylsulfoxide (8 mL) were added to a reaction flask. The mixture was subjected to nitrogen displacement three times, and under the protection of nitrogen, was heated to 110 °C and stirred for 16 h. The reaction mixture was filtered. The filtrate was washed with water and extracted with ethyl acetate. The organic layer was dried over anhydrous sodium sulfate, and distilled under reduced pressure to obtain a crude product, which was separated by flash silicagel columns [eluent: methanol/dichloromethane: 0-10%] to obtain 5-(1*H*-imidazol-1-yl)-3,3-dimethyl-2,3-dihydro-1*H*-pyrrolo[3,2-*b*]pyridine (135 mg, yield: 26.6%). ESI-MS: 215.0 $[M+1]^+$.

**Intermediate A8: Preparation of 5-chloro-3,3,6-trimethyl-2,3-dihydro-1*H*-pyrrolo[3,2-*b*]pyridine**

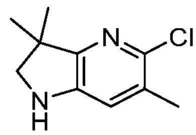

**Step 1: Synthesis of 6-chloro-2-iodo-5-methylpyridin-3-amine**

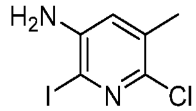

*N*-iodosuccinimide (10.2 g, 45.6 mmol) was added to the solution of 6-chloro-5-methylpyridin-3-amine (5.0 g, 35.1 mmol) in *N,N*-dimethylformamide (100 mL). The reaction mixture was stirred for 16 hrs at room temperature. The reaction mixture was poured into water, and ethyl acetate was added for extraction. The organic phases were combined, the saturated saline water was added for washing, and the organic phases were concentrated and then separated by column chromatography [petroleum ether:ethyl acetate=4: 1] to obtain 6-chloro-2-iodo-5-methylpyridin-3-amine (8.18 g, yield: 78.2 %). ESI-MS: 269.0 $[M+1]^+$.

**Step 2: Synthesis of 6-chloro-2-iodo-5-methyl-*N*-(2-methallyl)pyridin-3-amine**

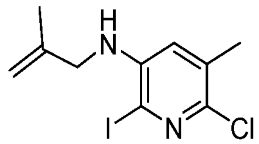

3-bromo-2-methylprop-1-ene (3.32 g, 24.6 mmol) and potassium tert-butoxide in tetrahydrofuran solution (24.6 mL, 1M, 24.6 mmol) were added to the solution of 6-chloro-2-iodo-5-methylpyridin-3-amine (5.5 g, 20.5 mmol) in tetrahydrofuran (50 mL). The reaction mixture was stirred for 20 minutes at room temperature. The reaction mixture was poured into water, and ethyl acetate was added for extraction. The organic phases were combined, the saturated saline water was added for washing, and the organic phases were concentrated and then separated by column chromatography

[petroleum ether:ethyl acetate=4: 1] to obtain 6-chloro-2-iodo-5-methyl-*N*-(2-methallyl)pyridin-3-amine (3.55 g, yield: 53%). ESI-MS: 323.0 [M+1]$^+$.

**Step 3: Synthesis of 5-chloro-3,3,6-trimethyl-2,3-dihydro-1*H*-pyrrolo[3,2-*b*]pyridine**

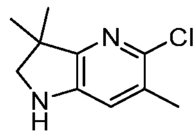

Sodium formate (0.85 g, 12.5 mmol), tetrabutylammonium chloride (3.47 g, 12.5 mmol), triethylamine (31.2 g, 31.2 mmol) and palladium acetate (0.35 g, 1.5 mmol) were added to the solution of 6-chloro-2-iodo-5-methyl-*N*-(2-methallyl)pyridin-3-amine (3.35 g, 10.4 mmol) in dimethylsulfoxide/water (60 mL/2.6mL). After reaction, evacuation and nitrogen displacement were conducted, the mixture was stirred for 2 h at 120 °C. The reaction mixture was poured into water, and ethyl acetate was added for extraction. The organic phases were combined, the saturated saline water was added for washing, and the organic phases were concentrated and then separated by column chromatography [petroleum ether:ethyl acetate=4: 1] to obtain 5-chloro-3,3,6-trimethyl-2,3-dihydro-1*H*-pyrrolo[3,2-*b*]pyridine (1.5 g, yield: 67.2%). ESI-MS: 197.0 [M+1]$^+$.

**Intermediate A9: Preparation of 3,3,5,6-tetramethyl-2,3-dihydro-1*H*-pyrrolo[3,2-*b*]pyridine**

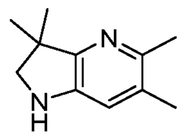

Trimethylboroxine (3.5 mL, 3.5 M, 12.3 mmol), potassium carbonate (1.02 g, 7.38 mmol), and [1,1'-bis(diphenylphosphine)ferrocene]palladium dichloride dichloromethane complex (199 mg, 0.24 mmol) were added to the solution of 5-chloro-3,3,6-trimethyl-2,3-dihydro-1*H*-pyrrolo[3,2-*b*]pyridine (480 mg, 2.46 mmol) in 1,2-dichloroethane (150 mL). After reaction, evacuation and nitrogen displacement were conducted, the mixture was stirred for 1 h at 120 °C. The reaction mixture was poured into water, and ethyl acetate was added for extraction. The organic phases were combined, the saturated saline water was added for washing, and the organic phases were concentrated and then separated by column chromatography [dichloromethane:methanol=5: 1] to obtain 3,3,5,6-tetramethyl-2,3-dihydro-1*H*-pyrrolo[3,2-*b*]pyridine (376 mg, yield: 77.80%). ESI-MS: 177.0 [M+1]$^+$.

**Intermediate A10: Preparation of 3,3,6-trimethyl-2,3-dihydro-1H-pyrrolo[3,2-b]pyridine**

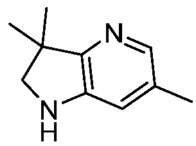

**Step 1: Synthesis of ethyl 2-(5-bromo-3-nitropyridin-2-yl)acetate**

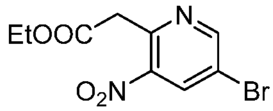

5-bromo-2-chloro-3-nitropyridine (2.5 g, 10.53 mmol) was dissolved in acetonitrile (50 mL). 3-ethoxy-3-potassium carbonylpropionate (2.15 g, 12.64 mmol), magnesium chloride (1.5 g, 15.79 mmol) and triethylamine (2.93 mL, 21.06 mmol) were added. The reaction mixture was stirred for 16 hrs at 70 °C. The reaction mixture was adjusted with 1N hydrochloric acid to pH=7, and extracted with dichloromethane (100 mL). The organic phase was washed with water (50 mL) and saturated saline water (50 mL) in sequence. The organic phase was concentrated, and the residue was separated by flash silicagel columns [petroleum ether: ethyl acetate=3:1] to obtain ethyl 2-(5-bromo-3-nitropyridin-2-yl)acetate (890 mg, yield: 29%). ESI-MS: 289.0, 290.9 $[M+1]^+$.

**Step 2: Synthesis of ethyl 2-(5-bromo-3-nitropyridin-2-yl)-2-methylpropionate**

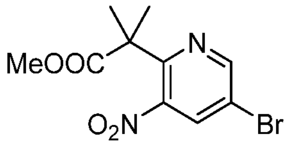

Ethyl 2-(5-bromo-3-nitropyridin-2-yl)acetate (710 mg, 2.46 mmol) was dissolved in tetrahydrofuran (50 mL). 18-crown ether-6 (64.9 mg, 0.25 mmol), iodomethane (0.46 mL, 7.37 mmol) and sodium hydride (177 mg, 7.37 mmol) were added to the reaction mixture. The reaction mixture was stirred for 3 hrs at 0 °C under the protection of nitrogen. After the reaction was completed, the reaction mixture was stratified with ethyl acetate (100 mL) and saturated saline water (100 mL), and the organic phase was washed with saturated saline water (50 mL). The resulting organic phase was concentrated, and the residue was treated with flash silicagel columns [petroleum ether/ethyl acetate=3/1] to obtain ethyl 2-(5-bromo-3-nitropyridin-2-yl)-2-methylpropionate (532 mg, yield: 68%). ESI-MS: 317.0, 319.0 $[M+1]^+$.

**Step 3: Synthesis of 6-bromo-3,3-dimethyl-1,3-dihydro-2*H*-pyrrolo[3,2-*b*]pyridin-2-one**

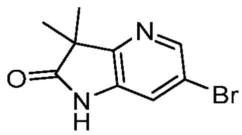

Ethyl 2-(5-bromo-3-nitropyridin-2-yl)-2-methylpropionate (532 mg, 1.68 mmol), and iron powder (940 mg, 16.77 mmol) were dissolved in glacial acetic acid (10 mL). The reaction mixture was stirred for 16 hrs at 80 °C. The reaction mixture was filtered through diatomite, and the filtrate was concentrated. The resulting residue was stratified with ethyl acetate (50 mL) and saturated saline water (50 mL). The organic phase was concentrated, and the residue was separated by flash silicagel columns [petroleum ether/ethyl acetate=2/1] to obtain 6-bromo-3,3-dimethyl-1,3-dihydro-2*H*-pyrrolo[3,2-*b*]pyridin-2-one (270 mg, yield: 66%). ESI-MS: 241.0, 243.0 $[M+1]^+$.

**Step 4: Synthesis of 3,3,6-trimethyl-1,3-dihydro-2*H*-pyrrolo[3,2-*b*]pyridin-2-one**

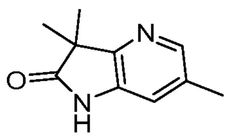

6-bromo-3,3-dimethyl-1,3-dihydro-2*H*-pyrrolo[3,2-*b*]pyridin-2-one (140 mg, 0.58 mmol), trimethylboroxine (108 mg, 0.87 mmol), [1,1'-bis(diphenylphosphine)ferrocene]palladium dichloride (42 mg, 0.058 mmol) and potassium carbonate (161 mg, 1.16 mmol) were dissolved in dimethoxyethylene glycol (5 mL). The reaction mixture was stirred for 1 hr at 90 °C under the protection of nitrogen, and the reaction was completed. The reaction mixture was stratified with ethyl acetate (50 mL) and saturated saline water (50 mL), and the organic phase was washed with saturated saline water (50 mL). The resulting organic phase was concentrated, and the residue was separated by flash silicagel columns [petroleum ether/ethyl acetate=2/1] to obtain 3,3,6-trimethyl-1,3-dihydro-2H-pyrrolo[3,2-*b*]pyridin-2-one (85 mg, yield: 83%). ESI-MS: 177.0 $[M+1]^+$.

**Step 5: Synthesis of 3,3,6-trimethyl-2,3-dihydro-1*H*-pyrrolo[3,2-*b*]pyridine**

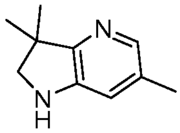

3,3,6-trimethyl-1,3-dihydro-2*H*-pyrrolo[3,2-*b*]pyridin-2-one (85 mg, 0.48 mmol) was dissolved in tetrahydrofuran (20 mL). A borane tetrahydrofuran solution (1.4 mL, 1.4 mmol) was added to the reaction mixture, which was stirred for 16 hrs at 70 °C under the protection of nitrogen, and the reaction was completed. The reaction mixture was stratified with ethyl acetate (50 mL) and saturated saline water (50 mL), and the organic phase was washed with saturated saline water (50 mL). The resulting organic phase was concentrated, and the residue was separated by flash silicagel columns [petroleum ether/ethyl acetate=2/1] to obtain 3,3,6-trimethyl-2,3-dihydro-1*H*-pyrrolo[3,2-*b*]pyridine (63 mg, yield: 80%). ESI-MS: 163.0 $[M+1]^+$.

**Intermediates A11-A12 were prepared according to the preparation method for Intermediate A10:**

| Intermediate No. | Structural Formula | Chemical Name | ESI-MS: [M+1]+ |
|---|---|---|---|
| A11 | 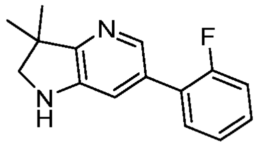 | 6-(2-fluorophenyl)-3,3- dimethyl-1*H*-pyrrolo[3,2-*b*] pyridine | 243.0 |
| A12 | 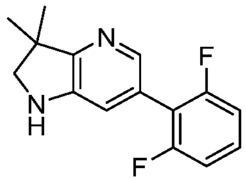 | 6-(2,6-difluorophenyl)-3,3-dimethyl-1*H*-pyrrolo [3,2-*b*]pyridine | 261.3 |

**Intermediate A13: Preparation of 5'-methyl-1',2'-dihydrospiro[cyclopropane-1,3'-pyrrolo[3,2-*b*]pyridine]**

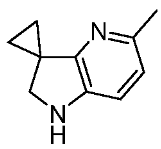

**Step 1: Synthesis of diethyl-2-(6-methyl-3-nitropyridin-2-yl)malonate**

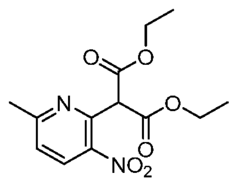

At 0 °C, diethyl malonate (11.14 g, 69.54 mmol) was slowly added dropwise to the suspension solution of sodium hydride (3.01 g, 75.33 mmol) in dimethylsulfoxide (140 mL). The mixture was stirred for 0.5 hrs at room temperature. Then, 2-chloro-6-methyl-3-nitropyridine (10 g, 57.95 mmol) was added to the mixture. The reaction mixture was stirred for 1.5 hrs at 100 °C. After the reaction was completed, the mixture was cooled to 0 °C, and saturated sodium bicarbonate was slowly added to quench the reaction. The mixture was washed with water and extracted with ethyl acetate. The organic layer was dried over anhydrous sodium sulfate, and distilled under reduced pressure to obtain a crude product, which was separated by flash silicagel column chromatography [eluent: ethyl acetate/petroleum ether: 0-50%] to obtain diethyl-2-(6-methyl-3-nitropyridin-2-yl)malonate (8.66 g, yield: 45.9%). ESI-MS: 297.1 $[M+1]^+$.

**Step 2: Synthesis of ethyl-2-(6-methyl-3-nitropyridin-2-yl)acetate**

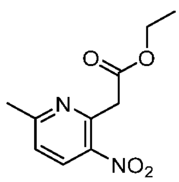

Water (10 mL) and lithium chloride (2.74 mL, 132.99 mmol) were added to the solution of diethyl-2-(6-methyl-3-nitropyridin-2-yl)malonate (8.66 g, 26.60 mmol) in dimethylsulfoxide (65 mL). The mixture was stirred for 4 days at 100 °C. The reaction mixture was cooled to room temperature, washed with water and extracted with ethyl acetate. The organic layer was dried over anhydrous sodium sulfate, and distilled under reduced pressure to obtain a crude product, which was separated by flash silicagel column chromatography [eluent: ethyl acetate/petroleum ether: 0-50%] to obtain ethyl-2-(6-methyl-3-nitropyridin-2-yl)acetate (2.1 g, yield: 33.45%). ESI-MS: 225.0 $[M+1]^+$.

**Step 3: Synthesis of ethyl-1-(6-methyl-3-nitropyridin-2-yl)cyclopropan-1-carboxylate**

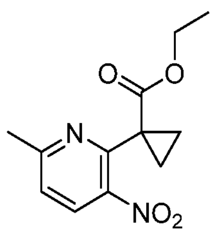

Under the protection of nitrogen at room temperature, diphenyl(vinyl)sulfonium trifluoromethanesulfonate (565 mg, 1.56 mmol) was added to the solution of ethyl-2-(6-methyl-3-nitropyridin-2-yl)acetate (180 mg, 0.80 mmol) in dimethylsulfoxide (10 mL). The mixture was stirred for 10 minutes at room temperature, and then dry 1,8-diazabicycloundecanon-7-ene (DBU) (0.36 mL, 2.41 mmol) was added. After the reaction was completed, the resultant was washed with water and extracted with ethyl acetate, and the organic layer was dried over anhydrous sodium sulfate, and distilled under reduced pressure to obtain a crude product, which was assayed by flash silicagel column chromatography [eluent: ethyl acetate/petroleum ether: 0-25%] to obtain ethyl-1-(6-methyl-3-nitropyridin-2-yl)cyclopropane-1-carboxylate (144 mg,

yield: 68.09%). ESI-MS: 251.2 $[M+1]^+$.

**Step 4: Synthesis of 5'-methylspiro[cyclopropane-1,3'-pyrrolo[3,2-b]pyridin]-2'(1'*H*)-one**

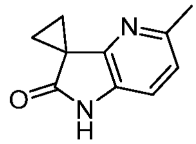

Ammonium formate (0.14 mL, 2.73 mmol) and 10% palladium on carbon (20 mg) were added to the solution of ethyl-1-(6-methyl-3-nitropyridin-2-yl)cyclopropane-1-carboxylate (144 mg, 0.55 mmol) in ethanol (20 mL). The mixture was stirred at 90 °C to react for 18 hrs. After the reaction was completed, the reaction mixture was filtered, and the filtrate was concentrated. The residue was washed with water and extracted with ethyl acetate. The organic phase was dried over anhydrous sodium sulfate, and distilled under reduced pressure to obtain a crude product 5'-methylspiro[cyclopropane-1,3'-pyrrolo[3,2-*b*]pyridin]-2'(1'*H*)-one (80 mg, yield: 77.29%), which was directly used in the next step. ESI-MS: 175.0 $[M+1]^+$.

**Step 5: Synthesis of 5'-methyl-1',2'-dihydrospiro[cyclopropane-1,3'-pyrrolo[3,2-*b*]pyridine]**

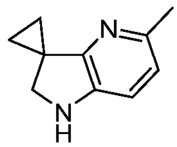

5'-methylspiro[cyclopropane-1,3'-pyrrolo[3,2-*b*]pyridin]-2'(1'*H*)-one (80 mg, 0.42 mmol) was dissolved in tetrahydrofuran (10 mL). The mixture was cooled to 0 °C. Lithium aluminum hydride in tetrahydrofuran solution (0.83 mL, 2.07 mmol) was added dropwise to the mixture. The mixture was stirred for 3 hrs at 50 °C. After the reaction was completed, the reaction mixture was quenched with sodium sulfate decahydrate until no bubbles were formed. The mixture was filtered, and the filtrate was distilled under reduced pressure to obtain 5'-methyl-1',2'-dihydrospiro[cyclopropane-1,3'-pyrrolo[3,2-b]pyridine] (64 mg, yield: 78.28%). ESI-MS: 161.0 $[M+1]^+$.

**Intermediate A14: Preparation of 5'-methyl-1',2'-dihydrospiro[cyclobutane-1,3'-pyrrolo[3,2-*b*]pyridine]**

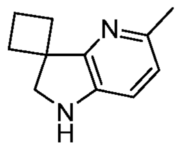

**Step 1: Synthesis of 1-(tert-butyl)3-ethyl 2-(6-methyl-3-nitropyridin-2-yl)malonate**

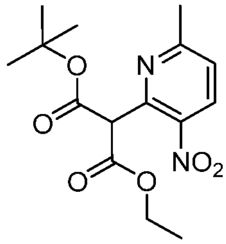

At 0 °C, 1-tert-butyl 3-ethylmalonate (35.45 g, 188.3 mmol) was slowly added dropwise to the suspension solution of sodium hydride (6.95 g, 173.8 mmol) in tetrahydrofuran (200 mL). The mixture was stirred for 0.5 hrs under an ice bath. Then, 2-chloro-6-methyl-3-nitropyridine (25 g, 144.8 mmol) was added to the mixture. The reaction mixture was stirred for 18 hrs at 60 °C. After the reaction was completed, the mixture was cooled to 0 °C, and ice water was slowly added to quench the reaction. The mixture was washed with water and extracted with ethyl acetate. The organic layer was dried over anhydrous sodium sulfate, and distilled under reduced pressure to obtain a crude product, which was separated by flash silicagel columns [eluent: ethyl acetate/petroleum ether: 0-20%] to obtain 1-(tert-butyl) 3-ethyl 2-(6-methyl-3-nitropyridin-2-yl)malonate (41 g, yield: 73.3%). ESI-MS: 325.0 $[M+1]^+$.

**Step 2: Synthesis of ethyl 2-(6-methyl-3-nitropyridin-2-yl)acetate**

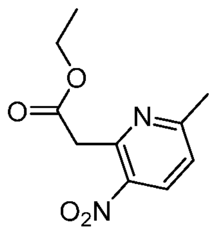

Trifluoroacetic acid (100 mL) was added to 1-(tert-butyl) 3-ethyl 2-(6-methyl-3-nitropyridin-2-yl)malonate (41g, 106.2 mmol), and the mixture was stirred for 2 hrs at 60 °C. The reaction mixture was distilled under reduced pressure. A crude product was diluted with dichloromethane, and washed with saturated sodium bicarbonate. The organic layer was dried over anhydrous sodium sulfate, and distilled under reduced pressure to obtain a crude product, which was separated by flash silicagel columns [eluent: ethyl acetate/petroleum ether: 0-15%] to obtain ethyl 2-(6-methyl-3-nitropyridin-2-yl)acetate (22 g, yield: 89%). ESI-MS: 225.0 $[M+1]^+$.

**Step 3: Synthesis of ethyl 2-(3-amino-6-methylpyridin-2-yl)acetate**

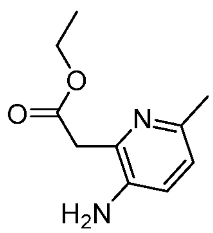

10% palladium on carbon (3.0 g) was added to the solution of ethyl 2-(6-methyl-3-nitropyridin-2-yl)acetate (22 g, 95.4 mmol) in methanol (150 mL). The mixture was stirred overnight at room temperature in the presence of hydrogen. After the reaction was completed, the mixture was filtered and distilled under reduced pressure to obtain ethyl 2-(3-amino-6-methylpyridin-2-yl)acetate (17.5 g, yield: 82%). ESI-MS: 195.0 $[M+1]^+$.

**Step 4: Synthesis of 5-methyl-1,3-dihydro-2*H*-pyrrolo[3,2-*b*]pyridin-2-one**

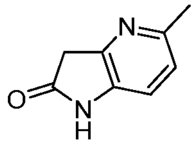

Ethyl 2-(3-amino-6-methylpyridin-2-yl)acetate (17.5 g, 78.4 mmol) was added to the solution of hydrochloric acid (1M) (100mL), and the mixture was stirred at 55 °C to react for 5 hrs. After the reaction was completed, the mixture was adjusted to alkalinity by using saturated sodium bicarbonate, and extracted multiple times with the solvent of dichloromethane:methanol=10:1. The organic layer was dried over anhydrous sodium sulfate, and distilled under reduced pressure to obtain a crude product, which was separated by flash silicagel columns [eluent: dichloromethane/methanol: 0-10%] to obtain 5-methyl-1,3-dihydro-2*H*-pyrrolo[3,2-*b*]pyridin-2-one (7.8 g, yield: 67%). ESI-MS: 149.0 $[M+1]^+$.

**Step 5: Synthesis of 5'-methylspiro[cyclobutane-1,3'-pyrrolo[3,2-*b*]pyridina]-2'(1'*H*)-one**

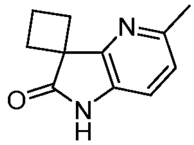

Sodium hydride (674.9 mg, 16.8 mmol) was dissolved in *N*,*N*-dimethylformamide (20 mL) and hexamethyl phosphoric triamide (2mL), and the mixture was cooled to 0 °C. The solution of 5-methyl-1,3-dihydro-2*H*-pyrrolo[3,2-*b*]pyridin-2-one (1.0 g, 6.7 mmol) and 1,3-diiodopropane (0.78 mL, 6.7 mmol) in *N,N*-dimethylformamide (20 mL) was added dropwise to the mixture. The mixture was stirred for 1 hr at 0 °C. After the reaction was completed, the reaction mixture was poured into ice water, and extracted with ethyl acetate. The organic layer was dried over anhydrous sodium sulfate, and distilled under reduced pressure to obtain a crude product, which was separated by flash silicagel columns [eluent: petroleum ether/ethyl acetate: 0-30%] to obtain 5'-methylspiro[cyclobutane-1,3'-pyrrolo[3,2-*b*]pyridina]-2'(1'*H*)-one (260 mg, yield: 20%). ESI-MS: 189.0 $[M+1]^+$.

**Step 6: Synthesis of 5'-methyl-1',2'-dihydrospiro[cyclobutane-1,3'-pyrrolo[3,2-*b*]pyridine]**

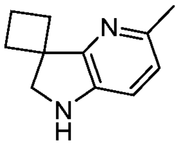

5'-methylspiro[cyclobutane-1,3'-pyrrolo[3,2-b]pyridina]-2'(1'H)-one (263 mg, 1.4 mmol) was dissolved in tetrahydrofuran (20 mL), and the mixture was cooled to 0 °C. Lithium aluminum hydride in tetrahydrofuran solution (1.7 mL, 2.5 M) was added dropwise to the mixture. The mixture was stirred for 2 hrs at 50 °C. After the reaction was completed, the reaction mixture was quenched with sodium sulfate decahydrate until no bubbles were formed. The mixture was filtered, and the filtrate was distilled under reduced pressure to obtain a crude product of 5'-methyl-1',2'-dihydrospiro[cyclobutane-1,3'-pyrrolo[3,2-*b*]pyridine] (260 mg, yield: 76%). ESI-MS: 175.0 $[M+1]^+$.

**Intermediate A15: Preparation of 5'-methyl-1',2'-dihydrospiro[cyclopentane-1,3'-pyrrolo[3,2-*b*]pyridine]**

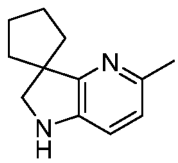

**Step 1: Synthesis of 5'-methylspiro[cyclopentane-1,3'-pyrrolo[3,2-*b*]pyridina]-2'(1'*H*)-one**

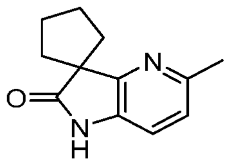

5-methyl-1,3-dihydro-2*H*-pyrrolo[3,2-b]pyridin-2-one (800 mg, 5.4 mmol) was dissolved in anhydrous tetrahydrofuran (40 mL) and hexamethyl phosphoric triamide (4.7 mL), and the mixture was cooled to -78 °C. N-butyllithium in tetrahydrofuran solution (6.5 mL, 16.2 mmol) was slowly added dropwise to the mixture. The reaction mixture was stirred for 30 minutes, and 1,4-diiodobutane (1.4 mL, 9.5 mmol) was added dropwise. The mixture was stirred for 1 hr at -20 °C. After the reaction was completed, the reaction mixture was poured into ice water, and extracted with ethyl acetate. The organic layer was dried over anhydrous sodium sulfate, and distilled under reduced pressure to obtain a crude product, which was separated by flash silicagel columns [eluent: petroleum ether/ethyl acetate: 0-30%] to obtain 5'-methylspiro[cyclopentane-1,3'-pyrrolo[3,2-*b*]pyridina]-2'(1'*H*)-one (450 mg, yield: 41%). ESI-MS: 203.0 $[M+1]^+$.

**Step 2: Synthesis of 5'-methyl-1',2'-dihydrospiro[cyclopentane-1,3'-pyrrolo[3,2-*b*]pyridine]**

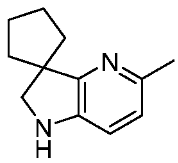

5'-methylspiro[cyclopentane-1,3'-pyrrolo[3,2-*b*]pyridina]-2'(1'*H*)-one (545 mg, 2.7mmol) was dissolved in tetrahydrofuran (20 mL), and the mixture was cooled to 0 °C. Lithium aluminum hydride in tetrahydrofuran solution (1.7 mL, 2.5 M) was added dropwise to the mixture. The mixture was stirred for 2 hrs at 50 °C. After the reaction was completed, the reaction mixture was quenched with sodium sulfate decahydrate until no bubbles were formed. The mixture was filtered, and the filtrate was distilled under reduced pressure to obtain a crude product of 5'-methyl-1',2'-dihydrospiro[cyclopentane-1,3'-pyrrolo[3,2-*b*]pyridine] (465 mg, yield: 83%). ESI-MS: 189.0 $[M+1]^+$.

**Intermediate A16: Preparation of 5'-methyl-1',2'-dihydrospiro[cyclohexane-1,3'-pyrrolo[3,2-*b*]pyridine]**

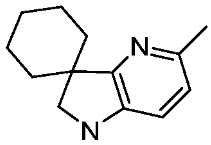

**Step 1: Synthesis of 5'-methylspiro[cyclohexane-1,3'-pyrrolo[3,2-*b*]pyridina]-2'(1'*H*)-one**

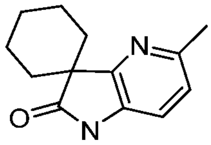

5-methyl-1,3-dihydro-2*H*-pyrrolo[3,2-b]pyridin-2-one (200 mg, 1.35 mmol) was dissolved in anhydrous tetrahydrofuran (15 mL) and hexamethyl phosphoric triamide (1.5 mL), and the mixture was cooled to -78 °C. N-butyllithium in tetrahydrofuran solution (1.89 mL, 4.73 mmol) was slowly added dropwise to the mixture. The reaction mixture was stirred for 30 minutes, and 1,5-diiodopentane (0.60 mL, 4.05 mmol) was added dropwise. The mixture was stirred for 1 hr at -20 °C. After the reaction was completed, the reaction mixture was poured into ice water, and extracted with ethyl acetate. The organic layer was dried over anhydrous sodium sulfate, and distilled under reduced pressure to obtain a crude product, which was separated by flash silicagel columns [eluent: petroleum ether/ethyl acetate: 0-30%] to obtain 5'-methylspiro[cyclohexane-1,3'-pyrrolo[3,2-*b*]pyridina]-2'(1'*H*)-one (220 mg, yield: 75.36%). ESI-MS: 217.0 $[M+1]^+$.

**Step 2: Synthesis of 5'-methyl-1',2'-dihydrospiro[cyclohexane-1,3'-pyrrolo[3,2-*b*]pyridine]**

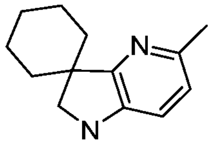

5'-methylspiro[cyclohexane-1,3'-pyrrolo[3,2-*b*]pyridina]-2'(1'*H*)-one (220 mg, 1.01mmol) was dissolved in tetrahydrofuran (10 mL), and the mixture was cooled to 0 °C. Lithium aluminum hydride in tetrahydrofuran solution (1.02 mL, 2.5 M) was added dropwise to the mixture. The mixture was stirred for 2 hrs at 50 °C. After the reaction was completed, the reaction mixture was quenched with sodium sulfate decahydrate until no bubbles were formed. The mixture was filtered, and the filtrate was distilled under reduced pressure to obtain a crude product of 5'-methyl-1',2'-dihydrospiro[cyclohexane-1,3'-pyrrolo[3,2-*b*]pyridine] (190 mg, yield: 92.2%). ESI-MS: 203.0$[M+1]^+$.

**Intermediate A17: Preparation of 5'-methyl-2,3,5,6-tetrahydrogen-2'*H*-1'l2-spiro[pyran-4,3'-pyrrolo[3,2-*b*]pyridine]**

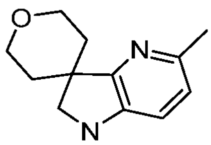

**Step 1: Synthesis of 5'-methyl-2,3,5,6-tetrahydrogen-2'*H*-1'l2-spiro[pyran-4,3'-pyrrolo[3,2-*b*]pyridina]-2'-one**

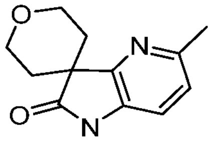

5-methyl-1,3-dihydro-2*H*-pyrrolo[3,2-*b*]pyridin-2-one (450 mg, 3.04 mmol) was dissolved in anhydrous tetrahydrofuran (25 mL) and hexamethyl phosphoric triamide (5 mL), and the mixture was cooled to -78 °C. N-butyllithium in tetrahydrofuran solution (3.65 mL, 9.11 mmol) was slowly added dropwise to the mixture. The reaction mixture was stirred for 30 minutes, and 1-iodo-2-(2-iodoethoxy)ethane (1.980 g, 6.08 mmol) was added dropwise. The mixture was stirred for 1 hr at -20 °C. After the reaction was completed, the reaction mixture was poured into ice water, and extracted

with ethyl acetate. The organic layer was dried over anhydrous sodium sulfate, and distilled under reduced pressure to obtain a crude product, which was separated by flash silicagel columns [eluent: petroleum ether/ethyl acetate: 0-30%] to obtain 5'-methyl-2,3,5,6-tetrahydrogen-2'*H*-1'l2-spiro[pyran-4,3'-pyrrolo[3,2-*b*]pyridina]-2'-one (191 mg, yield: 28.81%). ESI-MS: 219.3 $[M+1]^+$.

**Step 2: Synthesis of 5'-methyl-2,3,5,6-tetrahydrogen-2'*H*-1'l2-spiro[pyran-4,3'-pyrrolo[3,2-*b*]pyridine]**

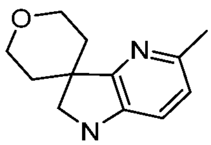

5'-methyl-2,3,5,6-tetrahydrogen-2'*H*-1'l2-spiro[pyran-4,3'-pyrrolo[3,2-*b*]pyridina]-2'-one (191 mg, 0.88mmol) was dissolved in tetrahydrofuran (8 mL), and the mixture was cooled to 0 °C. Lithium aluminum hydride in tetrahydrofuran solution (0.88 mL, 2.5 M) was added dropwise to the mixture. The mixture was stirred for 2 hrs at 50 °C. After the reaction was completed, the reaction mixture was quenched with sodium sulfate decahydrate until no bubbles were formed. The mixture was filtered, and the filtrate was distilled under reduced pressure to obtain a crude product of 5'-methyl-2,3,5,6-tetrahydrogen-2'*H*-1'l2-spiro[pyran-4,3'-pyrrolo[3,2-*b*]pyridine] (175 mg, yield: 97.9%). ESI-MS: 205.2 $[M+1]^+$.

**Intermediate A18: Preparation of 1',2'-dihydrospiro[cyclobutane-1,3'-pyrrolo[3,2-*b*]pyridine]**

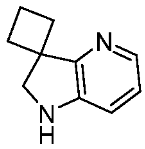

**Step 1: Synthesis of 1-(tert-butyl)3-ethyl 2-(3-nitropyridin-2-yl)malonate**

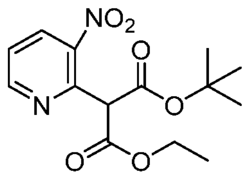

At 0 °C, tert-butylethylmalonate (44.52g, 236.5 mmol) was slowly added dropwise to the suspension solution of sodium hydride (9.46 g, 236.5 mmol) in tetrahydrofuran (200 mL). The mixture was stirred for 0.5 hrs at room temperature. Then, 2-chloro-3-nitropyridine (25.0 g, 157.7 mmol) was added to the mixture. The reaction mixture was stirred for 1.5 hrs at 60 °C. After the reaction was completed, the mixture was cooled to 0 °C, and the solution of saturated ammonium chloride was slowly added to quench the reaction. The mixture was washed with water and extracted with ethyl acetate. The organic layer was dried over anhydrous sodium sulfate, and distilled under reduced pressure to obtain a crude product, which was separated by flash silicagel columns [eluent: ethyl acetate/petroleum ether: 0-50%] to obtain 1-(tert-butyl) 3-ethyl 2-(3-nitropyridin-2-yl)malonate (32.7 g, yield: 66.8%). ESI-MS: 255.0 $[M-55]^+$.

**Step 2: Synthesis of ethyl 2-(3-nitropyridin-2-yl)acetate**

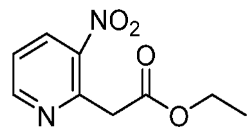

Trifluoroacetic acid (18.8 mL, 252.9 mmol) was added to 1-(tert-butyl) 3-ethyl 2-(3-nitropyridin-2-yl)malonate (32.7 g, 84.3 mmol). The mixture was stirred for 1 hr at 60 °C. The reaction mixture was cooled to room temperature, and distilled under reduced pressure to remove trifluoroacetic acid. The residue was added to the solution of saturated sodium bicarbonate and extracted with ethyl acetate. The organic phase was dried over anhydrous sodium sulfate, and distilled under reduced pressure to obtain ethyl 2-(3-nitropyridin-2-yl)acetate (17.7 g, yield: 91.9%). ESI-MS: 211.0 $[M+1]^+$.

**Step 3: Synthesis of ethyl 2-(3-aminopyridin-2-yl)acetate**

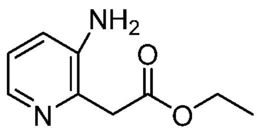

10% palladium on carbon (3.0 g) was added to the solution of ethyl 2-(3-nitropyridin-2-yl)acetate (22 g, 104.7 mmol) in methanol (150 mL). The mixture was stirred overnight at room temperature in the presence of hydrogen. After the reaction was completed, the mixture was filtered and distilled under reduced pressure to obtain ethyl 2-(3-aminopyridin-2-yl)acetate (17.5 g, yield: 90%). ESI-MS: 181.0 $[M+1]^+$.

**Step 4: Synthesis of 1,3-dihydro-2*H*-pyrrolo[3,2-*b*]pyridin-2-one**

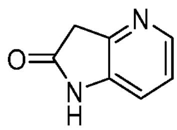

Ethyl 2-(3-aminopyridin-2-yl)acetate (17.5 g, 96.6 mmol) was added to the solution of hydrochloric acid (1M) (100mL), and the mixture was stirred at 55 °C to react for 5 hrs. After the reaction was completed, the mixture was adjusted to alkalinity by using saturated sodium bicarbonate, and extracted multiple times with the solvent of dichloromethane:methanol=10:1. The organic layer was dried over anhydrous sodium sulfate, and distilled under reduced pressure to obtain a crude product, which was separated by flash silicagel columns [eluent: dichloromethane/methanol: 0-10%] to obtain 1,3-dihydro-2*H*-pyrrolo[3,2-*b*]pyridin-2-one (7.8 g, yield: 60%). ESI-MS: 135.0 $[M+1]^+$.

**Step 5: Synthesis of spiro[cyclobutane-1,3'-pyrrolo[3,2-*b*]pyridin]-2'(1'*H*)-one**

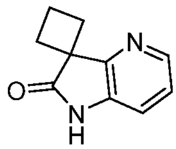

Sodium hydride (3.0 g, 74.5 mmol) and hexamethyl phosphoric triamide (12 mL) were dissolved in anhydrous *N,N*-dimethylformamide (60 mL). 1,3-dihydro-2*H*-pyrrolo[3,2-*b*]pyridin-2-one (4.0 g, 29.8 mmol), and 1,3-diiodopropane (8.8 g, 29.8 mmol) were added to the reaction mixture. The reaction mixture was stirred for 1 hr at 0 °C under the protection of nitrogen. After the reaction was completed, the reaction mixture was stratified with ethyl acetate (100 mL)

and saturated saline water (100 mL), and the organic phase was washed with saturated saline water (50 mL). The resulting organic phase was concentrated, and the residue was separated by flash silicagel columns [petroleum ether/ethyl acetate=3:1] to obtain spiro[cyclobutane-1,3'-pyrrolo[3,2-*b*]pyridin]-2'(1'*H*)-one (1.2 g, yield: 23%). ESI-MS: 175.0[M+1]$^+$.

**Step 6: Synthesis of 1',2'-dihydrospiro[cyclobutane-1,3'-pyrrolo[3,2-*b*]pyridine]**

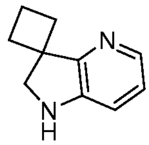

Spiro[cyclobutane-1,3'-pyrrolo[3,2-*b*]pyridin]-2'(1'*H*)-one (240 mg, 1.38 mmol) was dissolved in tetrahydrofuran (10 mL). The solution of dimethylsulfide borane (1.4 mL, 14 mmol) was added to the reaction mixture, which was stirred for 16 hrs at 25 °C under the protection of nitrogen, and the reaction was completed. The reaction mixture was stratified with ethyl acetate (50 mL) and saturated saline water (50 mL), and the organic phase was washed with saturated saline water (50 mL). The resulting organic phase was concentrated, and the residue was separated by flash silicagel columns [petroleum ether/ethyl acetate=2:1] to obtain 1',2'-dihydrospiro[cyclobutane-1,3'-pyrrolo[3,2-*b*]pyridine] (210 mg, yield: 95%). ESI-MS: 161.0[M+1]$^+$.

**Intermediate A19: Preparation of 3,3-difluoro-5'-methyl-1',2'-dihydrospiro[cyclobutane-1,3'-pyrrolo[3,2-*b*]pyridine]**

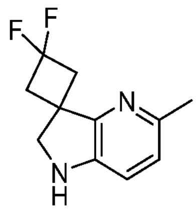

**Step 1: Synthesis of *N*-(2-bromo-6-methylpyridin-3-yl)-3,3-difluorocyclobutane-1-carboxamide**

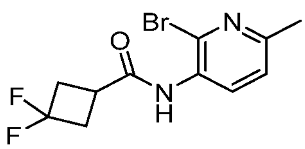

2-bromo-6-methylpyridin-3-amine (5 g, 26.732 mmol), 3,3-difluoro cyclobutane-1-carboxylic acid (4.37 g, 32.079 mmol), and 1-methylimidazole (6.58 g, 80.197 mmol) were dissolved in acetonitrile (150 mL), *N*,*N*,*N*',*N*'-tetramethylchloroformamidinium hexafluorophosphate (9.00 g, 32.078 mmol) was added, and the mixture was stirred for 3 hrs at room temperature. After the reaction was completed, the reaction mixture was poured into water, and extracted with ethyl acetate. The organic layer was dried over anhydrous sodium sulfate, and distilled under reduced pressure to obtain a crude product, which was separated by flash silicagel columns [eluent: petroleum ether/ethyl acetate: 0-30%] to obtain N (2-bromo-6-methylpyridin-3-yl)-3,3-difluorocyclobutane-1-carboxamide (7.8 g, yield: 95%). ESI-MS: 304.8 [M+1]$^+$.

**Step 2: Synthesis of *N*-(2-bromo-6-methylpyridin-3-yl)-3,3-difluoro-*N*-(4-methoxybenzyl)cyclobutane-1-carboxamide**

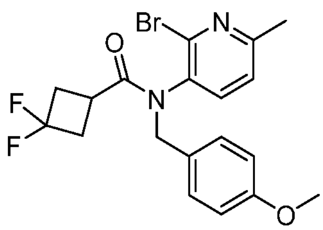

1-(chloromethyl)-4-metoxybenzene (2.01 mL, 14.75 mmol) and potassium carbonate (4.08 g, 29.50 mmol) were added to the solution of *N*-(2-bromo-6-methylpyridin-3-yl)-3,3-difluoro cyclobutane-1-carboxamide (3.0 g, 9.8 mmol) in acetonitrile (50 mL). The mixture was stirred at 90 °C to react for 18 hrs. After the reaction was completed, the mixture was filtered and distilled under reduced pressure to obtain a crude product, which was separated by flash silicagel columns [eluent: petroleum ether/ethyl acetate: 0-25%] to obtain *N*-(2-bromo-6-methylpyridin-3-yl)-3,3-difluoro-*N*-(4-methoxybenzyl)cyclobutane-1-carboxamide (3.8 g, yield: 90%). ESI-MS: 425.0 $[M+1]^+$.

**Step 3: Synthesis of 3,3-difluoro-1'-(4-methoxybenzyl)-5'-methylspiro[cyclobutane-1,3'-pyrrolo[3,2-*b*]pyridin]-2'(1'*H*)-one**

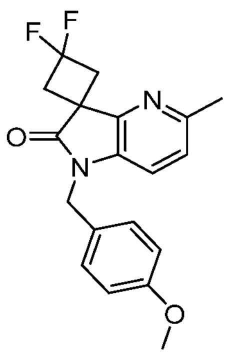

[1,3-bis(2,6-diisopropylbenzene)imidazole-2-diyl](3-chloropyridina)palladium dichloride (512 mg, 0.7 mmol), and sodium tert-butoxide (1.45 g, 15.0 mmol) were added to the solution of *N*-(2-bromo-6-methylpyridin-3-yl)-3,3-difluoro-*N*-(4-methoxybenzyl) cyclobutane-1-formamide (3.2 g, 7.5mmol) in dioxane (50 mL), and the mixture was stirred at 100 °C under the protection of nitrogen to react for 5 hrs. After the reaction was completed, the reaction mixture was poured into water, and extracted with ethyl acetate. The organic layer was dried over anhydrous sodium sulfate, and distilled under reduced pressure to obtain a crude product, which was separated by flash silicagel columns [eluent: petroleum ether/ethyl acetate: 0-30%] to obtain 3,3-difluoro-1'-(4-methoxybenzyl)-5'-methylspiro[cyclobutane-1,3'-pyrrolo[3,2-*b*]pyridin]-2'(1'*H*)-one (2.0 g, yield: 77%). ESI-MS: 345.0 $[M+1]^+$.

**Step 4: Synthesis of 3,3-difluoro-5'-methylspiro[cyclobutane-1,3'-pyrrolo[3,2-*b*]pyridina]-2'(1'*H*)-one**

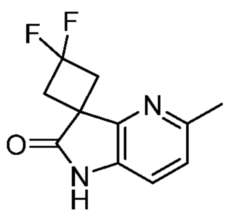

3,3-difluoro-1'-(4-methoxybenzyl)-5'-methylspiro[cyclobutane-1,3'-pyrrolo[3,2-*b*]pyridina]-2'(1'*H*)-one (1.8 g, 5.2 mmol) was dissolved in dichloromethane (3mL), and trifluoromethanesulfonic acid (3.5 mL) was added to the mixture. The mixture was stirred overnight at room temperature. After the reaction was completed, the crude product was diluted

with dichloromethane, and washed with saturated sodium bicarbonate. The organic layer was dried over anhydrous sodium sulfate, and distilled under reduced pressure to obtain a crude product, which was separated by flash silicagel columns [eluent: ethyl acetate/petroleum ether: 0-50%] to obtain 3,3-difluoro-5'-methylspiro[cyclobutane-1,3'-pyrrolo[3,2-*b*]pyridina]-2'(1'*H*)-one (1.1 g, yield: 93%). ESI-MS: 225.0 $[M+1]^+$.

**Step 5: Synthesis of 3,3-difluoro-5'-methyl-1',2'-dihydrospiro[cyclobutane-1,3'-pyrrolo[3,2-*b*]pyridine]**

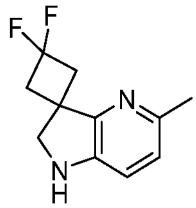

3,3-difluoro-5'-methylspiro[cyclobutane-1,3'-pyrrolo[3,2-*b*]pyridina]-2'(1'*H*)-one (250 mg, 1.1 mmol) was dissolved in tetrahydrofuran (20 mL), and the mixture was cooled to 0 °C. Lithium aluminum hydride in tetrahydrofuran solution (1.3 mL, 2.5 M) was added dropwise to the mixture. The mixture was stirred for 2 hrs at 50 °C. After the reaction was completed, the reaction mixture was quenched with sodium sulfate decahydrate until no bubbles were formed. The mixture was filtered, and the filtrate was distilled under reduced pressure to obtain a crude product of 3,3-difluoro-5'-methyl-1',2'-dihydrospiro[cyclobutane-1,3'-pyrrolo[3,2-*b*]pyridine] (250 mg, yield: 100%). ESI-MS: 211.0 $[M+1]^+$.

**Intermediate A20: Preparation of tert-butyl 5'-methyl-1',2'-dihydrospiro[azetidine-3,3'-pyrrolo[3,2-*b*]pyridine]-1-carboxylate**

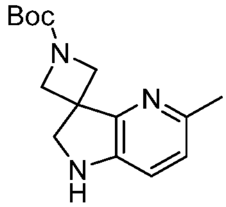

**Step 1: Synthesis of tert-butyl 3-((2-bromo-6-methylpyridin-3-yl)carbamyl)azetidine-1-carboxylate**

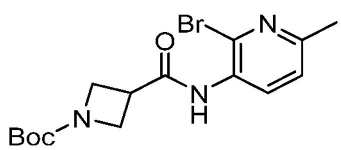

2-bromo-6-methylpyridin-3-amine (3.5 g, 18.7 mmol), 1-(tert-butoxycarbonyl)azetidine-3-carboxylic acid (4.52 g, 22.5 mmol), and *N*-methylimidazole (6.45 g, 78.6 mmol) were dissolved in acetonitrile (55 mL). *N,N,N',N'*-tetramethylchloroformamidinium hexafluorophosphate (TCFH) (7.35 g, 26.42mmol) was added. The reaction mixture was stirred for 2 hrs at room temperature. The reaction mixture was extracted with ethyl acetate (100 mL), and the organic phase was washed with water (50 mL) and saturated saline water (50 mL) in sequence. The organic phase was concentrated, and the residue was separated by flash silicagel columns [petroleum ether: ethyl acetate=1:1] to obtain a crude product tert-butyl 3-((2-bromo-6-methylpyridin-3-yl)carbamyl)azetidine-1-carboxylate (10.2 g, yield: 147%). ESI-MS: 370.0,372.0 $[M+1]^+$.

**Step 2: Synthesis of tert-butyl 3-((2-bromo-6-methylpyridin-3-yl)(4-methoxybenzyl)carbamoyl)azetidine-1-carboxylate**

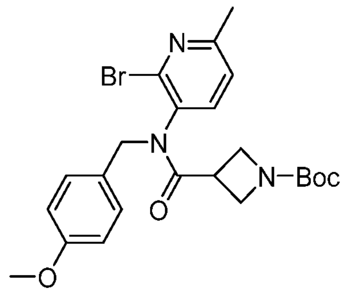

Tert-butyl 3-((2-bromo-6-methylpyridin-3-yl)carbamyl)azetidine-1-carboxylate (10.2 g, 22.03 mmol) was dissolved in acetonitrile (1800 mL). P-methoxybenzyl chloride (9.0 mL, 66.0 mmol) and potassium carbonate (5.5 g, 39.8 mmol) were added to the reaction mixture. The reaction mixture was stirred for 4 hrs at 110 °C under the protection of nitrogen. After the reaction was completed, the reaction mixture was stratified with ethyl acetate (100 mL) and saturated saline water (100 mL), and the organic phase was washed with saturated saline water (50 mL). The resulting organic phase was concentrated, and the residue was treated with flash silicagel columns [petroleum ether/ethyl acetate=2/1] to obtain tert-butyl 3-((2-bromo-6-methylpyridin-3-yl)(4-methoxybenzyl)carbamyl)azetidine-1-carboxylate (3.5 g, yield: 32.4%). ESI-MS: 434.0, 436.0 $[M+1]^+$.

**Step 3: Synthesis of tert-butyl 1'-(4-methoxybenzyl)-5'-methyl-2'-oxo-1',2'-dihydrospiro[azetidine-3,3'-pyrrolo[3,2-*b*]pyridine]-1-carboxylate**

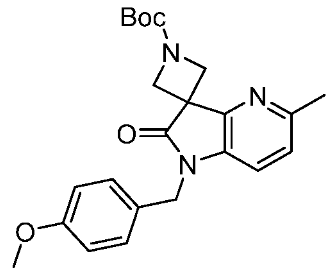

Tert-butyl 3-((2-bromo-6-methylpyridin-3-yl)(4-methoxybenzyl)carbamyl)azetidine-1-carboxylate (3.5 g, 7.1 mmol), [1,3-bis(2,6-diisopropylbenzene)imidazole-2-diyl](3-chloropyridina)palladium dichloride(PEPPSI-iPr) (240 mg, 0.36 mmol), and sodium tert-butoxide (1.03 g, 10.7 mmol) were dissolved in 1,4-dioxane (60 mL). The reaction mixture was stirred for 18 hrs at 110 °C under microwaves. The reaction mixture was filtered through diatomite, and the filtrate was concentrated. The resulting residue was stratified with ethyl acetate (50 mL) and saturated saline water (50 mL). The organic phase was concentrated, and the residue was separated by flash silicagel columns [petroleum ether/ethyl acetate=3/1] to obtain tert-butyl 1'-(4-methoxybenzyl)-5'-methyl-2'-oxo-1',2'-dihydrospiro[azetidine-3,3'-pyrrolo[3,2-*b*]pyridine]-1-carboxylate (640 mg, yield: 21.9%). ESI-MS: 410.2 $[M+1]^+$.

**Step 4: Synthesis of tert-butyl 5'-methyl-2'-oxo-1',2'-dihydrospiro[azetidine-3,3'-pyrrolo[3,2-*b*]pyridine]-1-carboxylate**

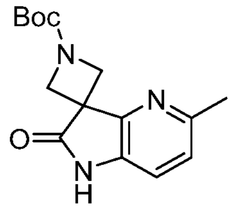

Tert-butyl 1'-(4-methoxybenzyl)-5'-methyl-2'-oxo-1',2'-dihydrospiro[azetidine-3,3'-pyrrolo[3,2-*b*]pyridine]-1-carboxylate (640 mg, 1.56 mmol) was dissolved in dichloromethane (2 mL), and trifluoromethanesulfonic acid (3 mL) was added. The reaction mixture was stirred for 18 hrs at room temperature, and the reaction was completed. The reaction mixture was stratified with ethyl acetate (50 mL) and saturated saline water (50 mL), and the organic phase was washed with saturated saline water (50 mL). The resulting organic phase was concentrated, and the residue was separated by flash silicagel columns [petroleum ether/ethyl acetate=1/1] to obtain tert-butyl 5'-methyl-2'-oxo-1',2'-dihydrospiro[azetidine-3,3'-pyrrolo[3,2-*b*]pyridine]-1-carboxylate (370 mg, yield: 81.82%). ESI-MS: 234.0 $[M+1]^+$.

**Step 5: Synthesis of tert-butyl 5'-methyl-1',2'-dihydrospiro[azetidine-3,3'-pyrrolo[3,2-*b*]pyridin]-1-carboxylate**

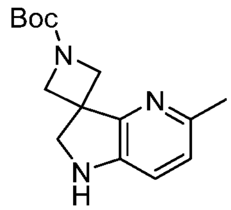

Tert-butyl 5'-methyl-2'-oxo-1',2'-dihydrospiro[azetidine-3,3'-pyrrolo[3,2-*b*]pyridine]-1-carboxylate (370 mg, 1.28 mmol) was dissolved in tetrahydrofuran (6 mL), and the solution of borane tetrahydrofuran (6.4 mL, 12.8 mmol) was added to the reaction mixture, which was then stirred for 3 hrs at room temperature under the protection of nitrogen, and the reaction was completed. The reaction mixture was stratified with ethyl acetate (50 mL) and saturated saline water (50 mL), and the organic phase was washed with saturated saline water (50 mL). The resulting organic phase was concentrated, and the residue was separated by flash silicagel columns [petroleum ether/ethyl acetate=1/1] to obtain tert-butyl 5'-methyl-1',2'-dihydrospiro[azetidine-3,3'-pyrrolo[3,2-*b*]pyridine]-1-carboxylate (264 mg, yield: 75.0%). ESI-MS: 276.0 $[M+1]^+$.

**Intermediate A21: Preparation of tert-butyl 5'-methyl-1',2'-dihydrospiro[pyrrolidine-3,3'-pyrrolo[3,2-*b*]pyridine]-1-carboxylate**

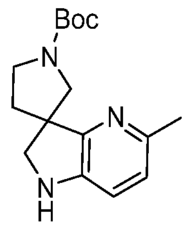

**Step 1: Synthesis of tert-butyl 3-((2-bromo-6-methylpyridin-3-yl)carbamyl)pyrrolidine-1-carboxylate**

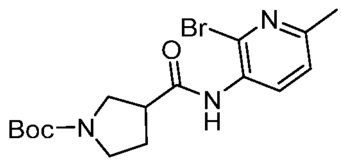

2-bromo-6-methylpyridin-3-amine (3.0 g, 16.0 mmol), 1-(tert-butoxycarbonyl)-pyrrolidine-3-formic acid (3.45 g, 16.0 mmol), and *N*-methylimidazole (5.370 mL, 67.4 mmol) were dissolved in acetonitrile (100 mL). *N,N,N',N'*-tetramethylchloroformamidinium hexafluorophosphate (TCFH) (6.3 g, 22.4 mmol) was added. The reaction mixture was stirred for 2 hrs at room temperature. The reaction mixture was extracted with ethyl acetate (100 mL), and the organic phase was washed with water (50 mL) and saturated saline water (50 mL) in sequence. The organic phase was concentrated, and the residue was separated by flash silicagel columns [petroleum ether: ethyl acetate=1:1] to obtain tert-butyl 3-((2-bromo-6-methylpyridin-3-yl)carbamyl)pyrrolidine-1-carboxylate (5.1 g, yield: 83%). ESI-MS: 384.3,386.3 $[M+1]^+$.

**Step 2: Synthesis of tert-butyl 3-((2-bromo-6-methylpyridin-3-yl)(4-methoxybenzyl)carbamoyl)pyrrolidine-1-carboxylate**

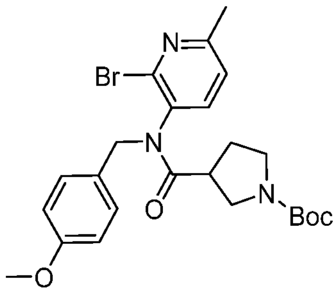

Tert-butyl 3-((2-bromo-6-methylpyridin-3-yl)carbamyl)pyrrolidine-1-carboxylate (5.1 g, 13.3 mmol) was dissolved in acetonitrile (50 mL), and p-methoxybenzyl chloride (2.7 mL, 20.0 mmol) and potassium carbonate (5.5 g, 39.8 mmol) were added to the reaction mixture. The reaction mixture was stirred for 18 hrs at 90 °C under the protection of nitrogen. After the reaction was completed, the reaction mixture was stratified with ethyl acetate (100 mL) and saturated saline water (100 mL), and the organic phase was washed with saturated saline water (50 mL). The resulting organic phase was concentrated, and the residue was treated with flash silicagel columns [petroleum ether/ethyl acetate=2/1] to obtain tert-butyl 3-((2-bromo-6-methylpyridin-3-yl)(4-methoxybenzyl)carbamoyl)pyrrolidine-1-carboxylate (3.5 g, yield: 53%). ESI-MS: 448.2, 450.2 $[M+1]^+$.

**Step 3: Synthesis of tert-butyl 1'-(4-methoxybenzyl)-5'-methyl-2'-oxo-1',2'-dihydrospiro[pyrrolidine-3,3'-pyrrolo[3,2-*b*]pyridine]-1-carboxylate**

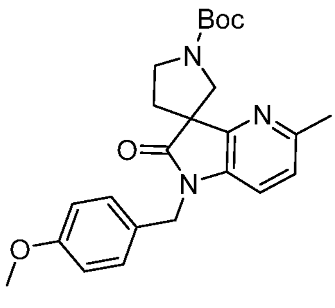

Tert-butyl 3-((2-bromo-6-methylpyridin-3-yl)(4-methoxybenzyl)carbamyl)pyrrolidine-1-carboxylate (600 mg, 1.19 mmol), [1,3-bis(2,6-diisopropylbenzene)imidazole-2-diyl](3-chloropyridina)palladium dichloride(PEPPSI-iPr)

(136.20 mg, 0.2 mmol), and sodium tert-butoxide (343 mg, 3.57 mmol) were dissolved in 1,4-dioxane (10 mL). The reaction mixture was stirred for 5 hrs at 110 °C under microwaves. The reaction mixture was filtered through diatomite, and the filtrate was concentrated. The resulting residue was stratified with ethyl acetate (50 mL) and saturated saline water (50 mL). The organic phase was concentrated, and the residue was separated by flash silicagel columns [petroleum ether/ethyl acetate=3/1] to obtain tert-butyl 1'-(4-methoxybenzyl)-5'-methyl-2'-oxo-1',2'-dihydrospiro[pyrrolidine-3,3'-pyrrolo[3,2-*b*]pyridine]-1-carboxylate (350 mg, yield: 69%). ESI-MS: 424.2 $[M+1]^+$.

**Step 4: Synthesis of tert-butyl 5'-methyl-2'-oxo-1',2'-dihydrospiro[pyrrolidine-3,3'-pyrrolo[3,2-*b*]pyridine]-1-carboxylate**

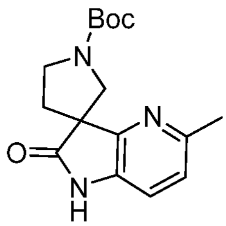

Tert-butyl 1'-(4-methoxybenzyl)-5'-methyl-2'-oxo-1',2'-dihydrospiro[pyrrolidine-3,3'-pyrrolo[3,2-*b*]pyridine]-1-carboxylate (350 mg, 0.83 mmol) was dissolved in dichloromethane (10 mL), and trifluoromethanesulfonic acid (0.74 mL, 8.3 mmol) was added. The reaction mixture was stirred for 18 hrs at room temperature, and the reaction was completed. The reaction mixture was stratified with ethyl acetate (50 mL) and saturated saline water (50 mL), and the organic phase was washed with saturated saline water (50 mL). The resulting organic phase was concentrated, and the residue was separated by flash silicagel columns [petroleum ether/ethyl acetate=1/1] to obtain tert-butyl 5'-methyl-2'-oxo-1',2'-dihydrospiro[pyrrolidine-3,3'-pyrrolo[3,2-*b*]pyridine]-1-carboxylate (220 mg, yield: 88%). ESI-MS: 304.0 $[M+1]^+$.

**Step 5: Synthesis of tert-butyl 5'-methyl-1',2'-dihydrospiro[pyrrolidine-3,3'-pyrrolo[3,2-*b*]pyridine]-1-carboxylate**

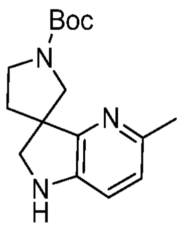

Tert-butyl 5'-methyl-2'-oxo-1',2'-dihydrospiro[pyrrolidine-3,3'-pyrrolo[3,2-*b*]pyridine]-1-carboxylate (220 mg, 0.73 mmol) was dissolved in tetrahydrofuran (10 mL), and the solution of borane tetrahydrofuran (7.2 mL, 7.2 mmol) was added to the reaction mixture, which was then stirred for 16 hrs at 70 °C under the protection of nitrogen, and the reaction was completed. The reaction mixture was stratified with ethyl acetate (50 mL) and saturated saline water (50 mL), and the organic phase was washed with saturated saline water (50 mL). The resulting organic phase was concentrated, and the residue was separated by flash silicagel columns [petroleum ether/ethyl acetate=1/1] to obtain tert-butyl 5'-methyl-1',2'-dihydrospiro[pyrrolidine-3,3'-pyrrolo[3,2-*b*]pyridine]-1-carboxylate (200 mg, yield: 95%). ESI-MS: 290.2 $[M+1]^+$.

**Intermediate A22: Preparation of 3,3-dimethyl-1,2,3,5,6,7-hexahydrocyclopenta[*b*]pyrrolo[2,3-e]pyridine**

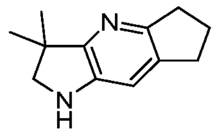

**Step 1: Synthesis of 3-nitro-1,5,6,7-tetrahydrogen-2H-cyclopenta[*b*]pyridin-2-one**

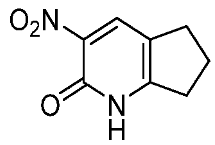

At 0 °C, nitric acid (65% in mass fraction, 5.4 g, 55.6 mmol) was slowly added dropwise to 1,5,6,7-tetrahydrogen-2*H*-cyclopentadieno[*b*]pyridin-2-one (450 mg, 2.5 mmol) in concentrated sulfuric acid (98% in mass fraction, 30 mL). The mixture was stirred for 1 hr at 0 °C, slowly poured into ice water, stirred for 1 h, and filtered. The filter cake was dried to obtain 3-nitro-1,5,6,7-tetrahydrogen-2H-cyclopenta[*b*]pyridin-2-one (3.5g, yield: 52.5%). ESI-MS: 181.0 $[M+1]^+$.

**Step 2: Synthesis of 2-chloro-3-nitro-6,7-dihydro-5H-cyclopenta[b]pyridine**

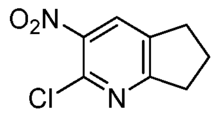

Phosphorus oxychloride (6.4 g, 41.6 mmol) and triethylbenzylammonium chloride (1.9 g, 7.0 mmol) were added to the solution of 3-nitro-1,5,6,7-tetrahydrogen-2*H*-cyclopenta[*b*]pyridin-2-one (2.5 g, 13.9 mmol) in acetonitrile (50 mL). The mixture was stirred for 1 hr at 80 °C, and concentrated under reduced pressure to remove the solvent. The residue was slowly poured into ice water, and stirred for 30 minutes. The mixture was extracted with dichloromethane. The organic layer was dried over anhydrous sodium sulfate, and distilled under reduced pressure to obtain a crude product, which was separated by flash silicagel columns [eluent: ethyl acetate/petroleum ether: 0-50%] to obtain 2-chloro-3-nitro-6,7-dihydro-5*H*-hydrocyclopenta[*b*]pyridine (985 mg, yield: 36.0%). ESI-MS: 198.9 $[M+1]^+$.

**Step 3: Synthesis of diethyl 2-(3-nitro-6,7-dihydro-5*H*-cyclopenta[*b*]pyridin-2-yl)malonate**

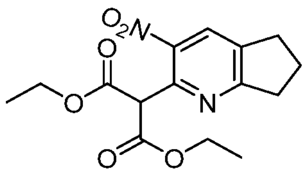

At 0 °C, sodium hydride (220 mg, 5.5mmol) was added to the solution of 2-chloro-3-nitro-6,7-dihydro-5*H*-hydrocyclopenta[*b*]pyridine (814 mg, 5.1 mmol) in dimethylsulfoxide (10 mL). The mixture was stirred for 0.5 hrs at 0dine (814 mg, 5.1 mmol) in dimethylsulfoxide (10 mL). The mixture was stirred for 0.5 hr into ice water, and and cooled to room temperature, and the solution of saturated ammonium chloride was used to quench the reaction. The reaction mixture was washed with water, and extracted with ethyl acetate. The organic layer was dried over anhydrous sodium sulfate, and distilled under reduced pressure to obtain a crude product, which was separated by flash silicagel columns [eluent: ethyl acetate/petroleum ether: 0-30%] to obtain diethyl 2-(3-nitro-6,7-dihydro-5*H*-hydrocyclopenta[*b*]pyridin-2-yl)malonate (409 mg, yield: 30.0%). ESI-MS: 323.0 $[M+1]^+$.

**Step 4: Synthesis of ethyl 2-(3-nitro-6,7-dihydro-5*H*-cyclopenta[*b*]pyridin-2-yl)acetate**

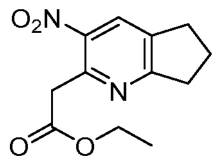

Water (0.91 mL, 5.1 mmol) and lithium chloride (267 mg, 6.4 mmol) were added to the solution of diethyl 2-(3-nitro-6,7-dihydro-5*H*-cyclopenta[*b*]pyridin-2-yl)malonate (409 mg, 1.3 mmol) in dimethylsulfoxide (5 mL). The mixture was stirred for 24 hrs at 100 °C. The reaction mixture was cooled to room temperature, washed with water and extracted with ethyl acetate. The organic layer was dried over anhydrous sodium sulfate, and distilled under reduced pressure to obtain a crude product, which was separated by flash silicagel columns [eluent: ethyl acetate/petroleum ether: 0-50%] to obtain ethyl 2-(3-nitro-6,7-dihydro-5*H*-hydrocyclopenta[*b*]pyridin-2-yl)acetate (240 mg, yield: 76.0%). ESI-MS: 251.0 $[M+1]^+$.

**Step 5: Synthesis of ethyl 2-methyl-2-(3-nitro-6,7-dihydro-5*H*-cyclopenta[*b*]pyridin-2-yl)propanoate**

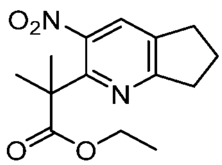

At 0 °C, iodomethane (300 mg, 2.1 mmol) and 18-crown ether-6 (26 mg, 0.1 mmol) were added to the solution of ethyl 2-(3-nitro-6,7-dihydro-5*H*-hydrocyclopenta[*b*]pyridin-2-yl)acetate (240 mg, 0.96 mmol) in *N,N* dimethylformamide (5 mL). Then, sodium hydride (88 mg, 2.2 mmol) was slowly added. The mixture was stirred for 1 h at 0 °C. After the reaction was completed, ice water was used to quench the reaction. The resultant was washed with water and extracted with ethyl acetate. The organic layer was dried over anhydrous sodium sulfate, and distilled under reduced pressure to obtain a crude product, which was separated by flash silicagel columns [eluent: ethyl acetate/petroleum ether: 0-25%] to obtain ethyl 2-methyl-2-(3-nitro-6,7-dihydro-5*H*-hydrocyclopenta[b]pyridin-2-yl)propanoate (150 mg, yield: 56.0%). ESI-MS: 279.0 $[M+1]^+$.

**Step 6: Synthesis of 3,3-dimethyl-3,5,6,7-tetrahydrocyclopenta[*b*]pyrrolo[2,3-e]pyridin-2(1*H*)-one**

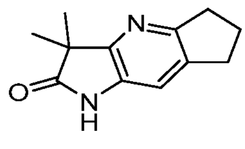

Ammonium formate (272 mg, 4.3 mmol) and 10% palladium on carbon (50mg) were added to the solution of 2-methyl-2-(3-nitro-6,7-dihydro-5*H*-cyclopenta[*b*]pyridin-2-yl)propanoate (150 mg, 0.54 mmol) in ethanol (5mL), and the mixture was stirred at 90 °C to react for 16 hrs. After the reaction was completed, the reaction mixture was filtered, the filtrate was concentrated, and the residue was washed with water and extracted with ethyl acetate. The organic layer was dried over anhydrous sodium sulfate, distilled under reduced pressure to obtain a crude product of 3,3-dimethyl-3,5,6,7-tetrahydrocyclopenta[*b*]pyrrolo[2,3-*e*]pyridin-2(1*H*)-one, which was directly used in the next step. ESI-MS: 203.0 $[M+1]^+$.

**Step 7: Synthesis of 3,3-dimethyl-1,2,3,5,6,7-hexahydrocyclopenta[*b*]pyrrolo[2,3-e]pyridine**

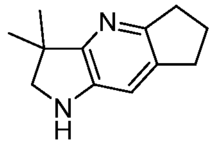

The crude product of 3,3-dimethyl-3,5,6,7-tetrahydrocyclopenta[*b*]pyrrolo[2,3-*e*]pyridin-2(1*H*)-one was dissolved in tetrahydrofuran (5 mL), and the mixture was cooled to 0 °C. Lithium aluminum hydride in tetrahydrofuran solution (2 mL, 2.5 M) was added dropwise to the mixture. The mixture was stirred for 4 hrs at room temperature. After the reaction was completed, the reaction mixture was quenched with sodium sulfate decahydrate until no bubbles were formed. The mixture was filtered, and the filtrate was distilled under reduced pressure to obtain a crude product of 3,3-dimethyl-1,2,3,5,6,7-hexahydrocyclopenta[*b*]pyrrolo[2,3-*e*]pyridine. ESI-MS: 189.0 $[M+1]^+$.

**Intermediate B1: Preparation of 1-(2-chloropyridin-4-yl)-3,3-dimethyl-2,3-dihydro-1*H*-pyrrolo[3,2-*b*]pyridine**

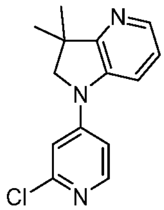

3,3-dimethyl-2,3-dihydro-1*H*-pyrrolo[3,2-*b*]pyridine (550 mg, 3.71 mmol) and 2-chloro-4-fluoropyridine (730 mg, 5.57 mmol) were dissolved in 10 mL of *N,N*-dimethylformamide. At room temperature, the solution of potassium tert-butoxide in tetrahydrofuran (5.6 mL, 1M, 5.57 mmol) was added to the mixture. The reaction mixture was stirred for 1 hr at 100 °C. Water was added to the mixture, which was then extracted with ethyl acetate three times. The organic phases were combined, then washed with saturated saline, and dried over anhydrous sodium sulfate. After the solvent was removed, the resultant was separated by silicagel column chromatography [petroleum ether:ethyl acetate=5: 1] to obtain 1-(2-chloropyridin-4-yl)-3,3-dimethyl-2,3-dihydro-1*H*-pyrrolo[3,2-*b*]pyridine (480 mg, yield: 60%). ESI-MS: 260.0 $[M+1]^+$.

**Intermediates B2-B3 were prepared according to the synthesis method for Intermediate B1:**

| Intermediate No. | Structural Formula | Chemical Name | ESI-MS: $[M+1]^+$ |
|---|---|---|---|
| **B2** | 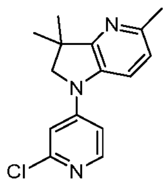 | 1-(2-chloropyridin-4-yl)-3,3,5-trimethyl-2,3-dihydro-1*H*-pyrrolo[3,2-*b*]pyridine | 274.0 |

(continued)

| Intermediate No. | Structural Formula | Chemical Name | ESI-MS: $[M+1]^+$ |
|---|---|---|---|
| **B3** | 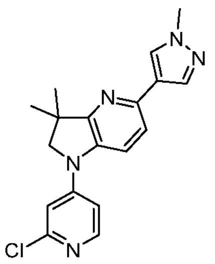 | 1-(2-chloropyridin-4-yl)-3,3-dimethyl-5-(1-methyl-1*H*-pyrazol-4-yl)-2,3-dihydro-1*H*-pyrrolo[3,2-*b*] pyridine | 340.0 |

**Intermediate B4: Preparation of 1-(2-chloropyrimidin-4-yl)-3,3,5-trimethyl-2,3-dihydro-1*H*-pyrrolo[3,2-*b*]pyridine**

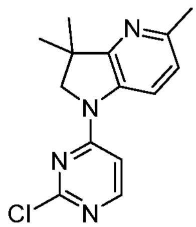

2,4-dichloropyrimidine (827 mg, 5.6 mmol), 3,3,5-trimethyl-2,3-dihydro-1*H*-pyrrolo[3,2-*b*]pyridine (600 mg, 3.7 mol), *N,N*-diisopropylethylamine (1.43 g, 11.1 mmol) and isopropanol (15 mL) were added into a microwave tube in sequence. The reaction mixture was treated with microwaves at 100 °C to react for 18 hrs. After the reaction was completed, the solvent was removed to obtain a crude product, which was separated by column chromatography [ethyl acetate/petroleum ether: 0-30%] to obtain 1-(2-chloropyrimidin-4-yl)-3,3,5-trimethyl-2,3-dihydro-1*H*-pyrrolo[3,2-*b*]pyridine (520 mg, yield: 50%), ESI-MS: 275.0 $[M+1]^+$.

**Intermediates B5-B19 were prepared according to the synthesis method for Intermediate B4:**

| Intermediate No. | Structural Formula | Chemical Name | ESI-MS: $[M+1]^+$ |
|---|---|---|---|
| **B5** | 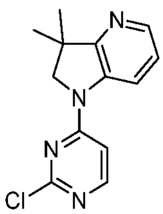 | 1-(2-chloropyrimidin-4-yl)-3,3 -dimethyl-2,3 -dihydro-1*H*-pyrrolo[3,2-*b*]pyridine | 261.0 |
| **B6** | 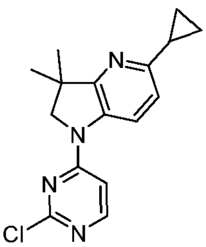 | 1-(2-chloropyrimidin-4-yl)-5-cyclopropyl-3,3-dimethyl-2,3-dihydro-1*H*-pyrrolo[3,2-*b*]pyridine | 301.0 |

(continued)

| Intermediate No. | Structural Formula | Chemical Name | ESI-MS: [M+1]+ |
|---|---|---|---|
| **B7** | 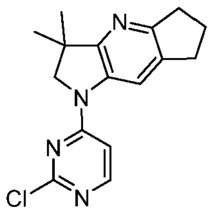 | 1-(2-chloropyrimidin-4-yl)-3,3-dimethyl-1,2,3,5,6,7-hexahydrocyclopenta[*b*]pyr rolo[2,3-*e*]pyridine | 301.0 |
| **B8** | 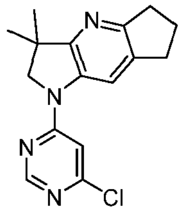 | 1-(6-chloropyrimidin-4-yl)-3,3-dimethyl-1,2,3,5,6,7-hexahydrocyclopenta[*b*]pyr rolo[2,3-*e*]pyridine | 301.0 |
| **B9** | 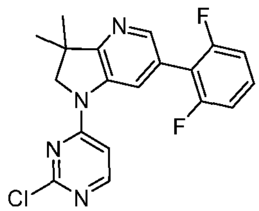 | 1-(2-chloropyrimidin-4-yl)-6-(2,6-difluorophenyl)-3,3-dimethyl-2,3-dihydro-1*H*-pyrrolo[3,2-*b*]pyridine | 373.1 |
| **B10** | 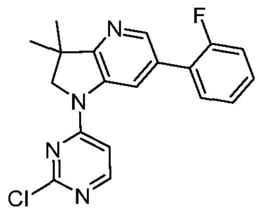 | 1-(2-chloropyrimidin-4-yl)-6-(2-fluorophenyl)-3,3-dimethyl-2,3-dihydro-1H-pyrrolo[3,2-*b*]pyridine | 355.1 |
| **B11** | 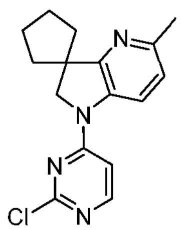 | 1'-(2-chloropyrimidin-4-yl)-5'-methyl-1',2'-dihydrospiro [cyclopentane-1,3'-pyrrolo[3,2-*b*]pyridine] | 301.1 |
| **B12** | 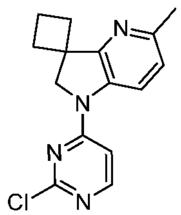 | 1'-(2-chloropyrimidin-4-yl)-5'-methyl-1',2'-dihydrospiro [cyclobutane-1,3'-pyrrolo[3,2-*b*]pyridine] | 287.0 |

(continued)

| Intermediate No. | Structural Formula | Chemical Name | ESI-MS: [M+1]+ |
|---|---|---|---|
| **B13** | 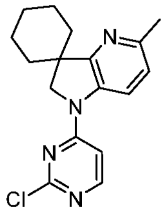 | 1'-(2-chloropyrimidin-4-yl)-5'-methyl-1',2'-dihydrospiro [cyclohexane-1,3'-pyrrolo[3,2-*b*]pyridine] | 315.0 |
| **B14** | 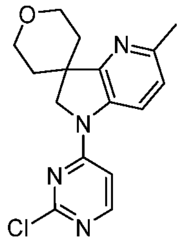 | 1'-(2-chloropyrimidin-4-yl)-5'-methyl-1',2,2',3,5,6-hexahydrogenspiro[pyran-4,3'-pyrrolo[3,2-*b*]pyridine] | 317.2 |
| **B15** | 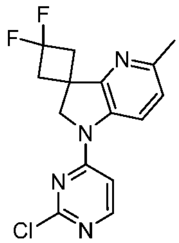 | 1'-(2-chloropyrimidin-4-yl)-3,3-difluoro-5'-methyl-1',2'-dihydrospiro[cyclobutane-1,3'-pyrrolo[3,2-*b*]pyridine] | 323.0 |
| **B16** | 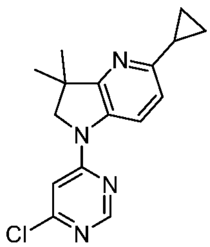 | 1-(6-chloropyrimidin-4-yl)-5-cyclopropyl-3,3-dimethyl-2,3-dihydro-1*H*-pyrrolo[3,2-*b*]pyridine | 301.1 |
| **B17** | 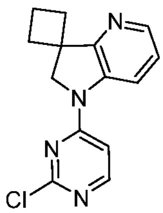 | 1'-(2-chloropyrimidin-4-yl)-1',2'-dihydrospiro [cyclobutane-1,3'-pyrrolo[3,2-*b*]pyridine] | 273.0 |
| **B18** | 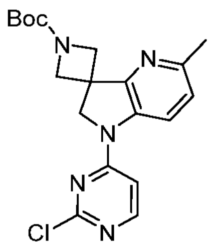 | tert-butyl 1'-(2-chloropyrimidin-4-yl)-5'-methyl-1',2'-dihydrospiro[azetidine-3,3'-pyrrolo[3,2-*b*]pyridine]-1-carboxylate | 388.0 |

(continued)

| Intermediate No. | Structural Formula | Chemical Name | ESI-MS: [M+1]+ |
|---|---|---|---|
| **B19** | 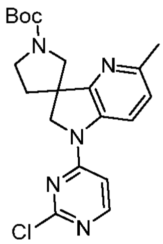 | tert-butyl 1'-(2-chloropyrimidin-4-yl)-5'-methyl-1',2'-dihydrospiro[pyrrolidine-3,3'-pyrrolo[3,2-*b*]pyridine]-1-carboxylate | 402.0 |

**Intermediate B20: Preparation of 1-(4-chloro-1,3,5-triazin-2-yl)-3,3,5-trimethyl-2,3-dihydro-1*H*-pyrrolo[3,2-*b*]pyridine**

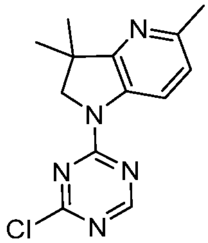

At room temperature, 2,4-dichloro-1,3,5-triazin (377 mg, 2.51 mmol) was dissolved in dichloromethane (10 mL), and *N,N*-diisopropylethylamine (542 mg, 4.19 mmol) was added. The solution of 3,3-dimethyl-5-(trifluoromethyl)-2,3-dihydro-1*H*-pyrrolo[3,2-*b*]pyridine (340 mg, 2.1 mmol) in dichloromethane (10 mL) was added dropwise over stirring. A resulting reaction mixture was stirred for 1 hr at room temperature. After the reaction was completed, the solvent was removed, and the resultant was separated by silicagel column chromatography [petroleum ether:ethyl acetate=4: 1] to obtain 1-(4-chloro-1,3,5-triazin-2-yl)-3,3,5-trimethyl-2,3-dihydro-1*H*-pyrrolo[3,2-*b*]pyridine (300 mg, yield: 52%), ESI-MS: 276.1 $[M+1]^+$.

**Intermediates B21 were prepared according to the synthesis method for Intermediate B20:**

| Intermediate No. | Structural Formula | Chemical Name | ESI-MS: [M+1]+ |
|---|---|---|---|
| **B21** | 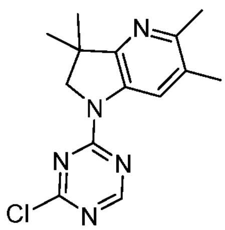 | 1-(4-chloro-1,3,5-triazin-2-yl)-3,3,5-trimethyl-2,3-dihydro-1*H*-pyrrolo[3,2-*b*]pyridine | 290.2 |

**Intermediate B22: Preparation of 1-(6-chloropyrimidin-4-yl)-3,3,5-trimethyl-2,3-dihydro-1*H*-pyrrolo[3,2-*b*]pyridine**

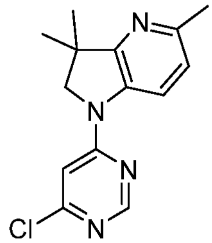

4,6-dichloropyrimidine (347 mg, 1.84 mmol) was dissolved in 15 mL of isopropanol. At room temperature, 3,3,5-trimethyl-2,3-dihydro-1*H*-pyrrolo[3,2-*b*]pyridine (250 mg, 1.53 mmol) and *N,N*-diisopropylethylamine (395 mg, 3.06 mmol) were added to the mixture. The reaction mixture was heated to 80 °C and stirred for 16 hrs. After the reaction was completed, the solvent was removed, and the resultant was separated by silicagel column chromatography [dichloromethane:methanol=10:1] to obtain 1-(6-chloropyrimidin-4-yl)-3,3,5-trimethyl-2,3-dihydro-1*H*-pyrrolo[3,2-*b*]pyridine (266 mg, yield: 63%). ESI-MS: 275.0 $[M+1]^+$.

**Intermediates B23-B25 were prepared according to the synthesis method for Intermediate B22:**

| Intermediate No. | Structural Formula | Chemical Name | ESI-MS: $[M+1]^+$ |
|---|---|---|---|
| B23 | 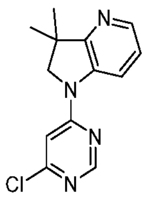 | 1-(6-chloropyrimidin-4-yl)-3,3-dimethyl-2,3-dihydro-1*H*-pyrrolo[3,2-*b*]pyridine | 261.0 |
| B24 | 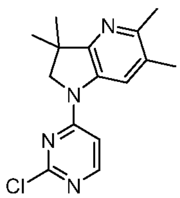 | 2-chloro-4-(3,3,5,6-tetramethyl-2,3-dihydro-1*H*-pyrrolo[3,2-*b*]pyridin-1-yl)pyrimidine | 289.0 |
| B25 | 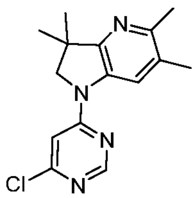 | 4-chloro-6-(3,3,5,6-tetramethyl-2,3-dihydro-1*H*-pyrrolo[3,2-*b*]pyridin-1-yl)pyrimidine | 289.0 |

**Intermediate B26: Preparation of 1-(2-chloropyrimidin-4-yl)-3,3-dimethyl-5-(1-methyl-1*H*-pyrazol-4-yl)-2,3-dihydro-1*H*-pyrrolo[3,2-*b*]pyridine**

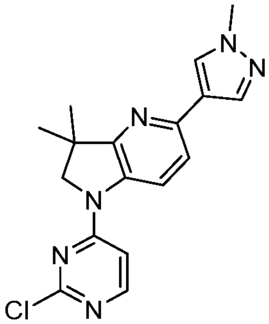

3,3-dimethyl-5-(1-methyl-1*H*-pyrazol-4-yl)-2,3-dihydro-1*H*-pyrrolo[3,2-*b*]pyridine (100 mg, 0.44 mmol) was dissolved in 8 mL of *N,N*-dimethylformamide. The resulting solution was cooled to 0 °C, added with sodium hydrogen (70 mg, 1.75 mmol), and stirred for 0.5 hrs. Then, 2,4-dichloropyrimidine (261 mg, 1.75 mmol) was added to the mixture. The reaction mixture was stirred for 1 h at 0 °C, and cooled to room temperature. The reaction was quenched with little water. The resultant was washed with water, extracted with ethyl acetate, and dried to remove the solvent. The residue was treated by column chromatography to obtain a product 1-(2-chloropyrimidin-4-yl)-3,3-dimethyl-5-(1-methyl-1*H*-pyrazol-4-yl)-2,3-dihydro-1*H*-pyrrolo[3,2-*b*]pyridine (100 mg, yield: 64.4%). ESI-MS: 341.0 [M+1]$^+$.

**Intermediates B27-B28 were prepared according to the synthesis method for Intermediate B26:**

| Intermediate No. | Structural Formula | Chemical Name | ESI-MS: [M+1]$^+$ |
|---|---|---|---|
| B27 | 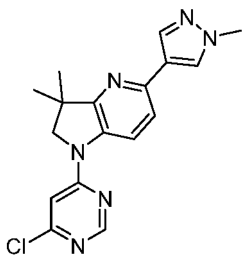 | 1-(6-chloropyrimidin-4-yl)-3,3-dimethyl-5-(1-methyl-1*H*-pyrazol-4-yl)-2,3-dihydro-1*H*-pyrrolo[3,2-*b*]pyridine | 341.0 |
| B28 | 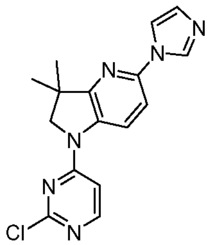 | 1-(2-chloropyrimidin-4-yl)-5-(1*H*-imidazole-1-yl)-3,3-dimethyl-2,3-dihydro-1*H*-pyrrolo[3,2-*b*]pyridine | 327.0 |

**Intermediate B29: Preparation of 5-bromo-3,3-dimethyl-1-(2-(methylsulfonyl)pyrimidin-4-yl)-2,3-dihydro-1*H*-pyrrolo[3,2-*b*]pyridine**

**Step 1: Synthesis of 5-bromo-3,3-dimethyl-1-(2-(methylthio)pyrimidin-4-yl)-2,3-dihydro-1*H*-pyrrolo[3,2-*b*]pyridine**

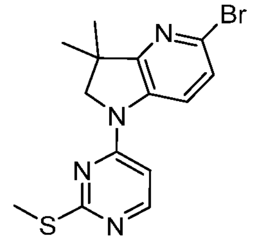

5-bromo-3,3-dimethyl-2,3-dihydro-1*H*-pyrrolo[3,2-*b*]pyridine (800 mg, 3.52 mmol) was dissolved in 8 mL of *N,N*-dimethylformamide. The resulting solution was cooled to 0 °C, added with sodium hydride (563.6 mg, 14 mmol), and stirred for 0.5 hrs. Then, 4-chloro-2-(methylthio)pyrimidine (1.13g, 7.0 mmol) was added to the mixture. The reaction mixture was stirred for 18 hrs at 0 °C, and cooled to room temperature. The reaction was quenched with little water. The resultant was washed with water, extracted with ethyl acetate, and dried to remove the solvent. The residue was treated by column chromatography to obtain a product 5-bromo-3,3-dimethyl-1-(2-(methylthio)pyrimidin-4-yl)-2,3-dihydro-1H-pyrrolo[3,2-b]pyridine (941 mg, yield: 60.8%). ESI-MS: 351.0, 353.0 $[M+1]^+$.

**Step 2: Synthesis of 5-bromo-3,3-dimethyl-1-(2-(methylsulfonyl)pyrimidin-4-yl)-2,3-dihydro-1*H*-pyrrolo[3,2-*b*]pyridine**

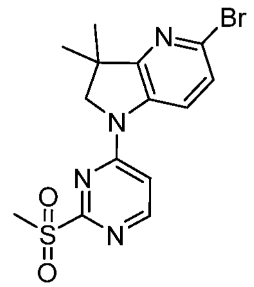

5-bromo-3,3-dimethyl-1-(2-(methylthio)pyrimidin-4-yl)-2,3-dihydro-1*H*-pyrrolo[3,2-*b*]pyridine was dissolved in a mixed solvent (941 mg, 2.68 mmol) of tetrahydrofuran (10mL), methanol (10mL) and water (3mL), and then, potassium peroxodisulfate (3.29g, 5.36mmol) was added. The reaction mixture was stirred overnight at room temperature, and filtered. The filtrate was washed with water, extracted with ethyl acetate, dried, and distilled under reduced pressure to remove the organic solvent. The residue was treated by column chromatography to obtain 5-bromo-3,3-dimethyl-1-(2-(methylsulfonyl)pyrimidin-4-yl)-2,3-dihydro-1*H*-pyrrolo[3,2-*b*]pyridine (642 mg, yield: 59.4%). ESI-MS: 383.0, 385.0 $[M+1]^+$.

**Intermediate C1: Preparation of *N¹*-(2-(dimethylamino)ethyl)-5-methoxy-*N¹*-methyl-2-nitrobenzene-1,4-diamine**

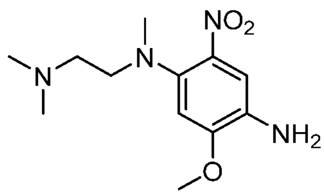

4-fluoro-2-methoxy-5-nitroaniline (1.86 g, 10.0 mmol) was dissolved in 10 mL of *N,N*-dimethylformamide. At room temperature, $N^1,N^1,N^2$-trimethylethane-1,2-diamine (1.53 g, 15.0 mmol) and potassium carbonate (2.76 g, 20.0 mmol) were added to the resulting solution. The reaction mixture was stirred for 3 hrs at 85 °C. Water was added to the solution, which was extracted with dichloromethane three times. The organic phases were combined, then washed with saturated saline, and dried over anhydrous sodium sulfate. After the solvent was removed, the resultant was separated

by silicagel column chromatography [dichloromethane:methanol=10:1] to obtain *N*[1]-(2-(dimethylamino)ethyl)-5-methoxy-*N*[1]-methyl-2-nitrobenzene-1,4-diamine (2.5 g, yield: 93%). ESI-MS: 269.0 $[M+1]^+$.

**Intermediates C2 were prepared according to the synthesis method for Intermediate C1:**

| Intermediate No. | Structural Formula | Chemical Name | ESI-MS: $[M+1]^+$ |
|---|---|---|---|
| C2 | 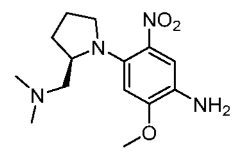 | (*R*)-4-(2-((dimethylamino)methyl) pyrrolidin-1-yl)-2-methoxy-5-nitroaniline | 295.0 |

**Intermediate C3: Preparation of 5-(difluoromethoxy)-*N*[1]-(2-(dimethylamino)ethyl)-*N*[1]-methyl-2-nitrobenzene-1,4-diamine**

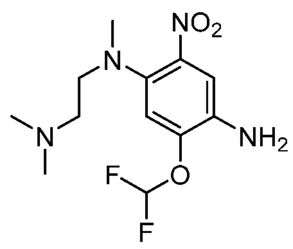

**Step 1: Synthesis of 2-(difluoromethoxy)-4-fluoro-1-nitrobenzene**

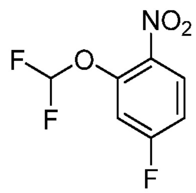

5-fluoro-2-nitrophenol (10.00 g, 63.65 mmol) and sodium carbonate (20.24 g, 190.96 mmol) were dissolved in *N,N*-dimethylformamide (100 mL), 2-chloro-2,2-sodium difluoroacetate (33.97 g, 222.79 mmol) was added in portions at 90 °C, and the reaction mixture was stirred for 3 hrs at the current temperature, and TLC monitoring was conducted. The reaction mixture was cooled to room temperature, then poured into ice water, and extracted with ethyl acetate three times. The organic phases were combined, then washed with saturated saline water, dried over anhydrous sodium sulfate, concentrated under reduced pressure, and then separated by column chromatography [eluent: ethyl acetate/petroleum ether: 0-10%] to obtain 2-(difluoromethoxy)-4-fluoro-1-nitrobenzene (10.3 g, 49.57 mmol, yield: 77.88%).

$^1H$ NMR (400 MHz, $CDCl_3$) $\delta$ 7.96 (dd, *J* = 9.1, 5.6 Hz, 1H), 7.11 - 6.97 (m, 2H), 6.57 (t, *J* = 72.4 Hz, 1H).

**Step 2: Synthesis of 2-(difluoromethoxy)-4-fluoroaniline**

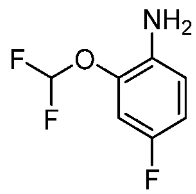

2-(difluoromethoxy)-4-fluoro-1-nitrobenzene (10.3 g, 49.73 mmol) was dissolved in ethanol (80 mL), to which palladium on carbon (1.0 g, 10%w/w) was then added. The reaction mixture was stirred at room temperature overnight in the presence of hydrogen. The reaction mixture was filtered with diatomite, and the filtrate was concentrated to obtain 2-(difluoromethoxy)-4-fluoroaniline (8.1 g, 42.99 mmol, yield: 86.42%). ESI-MS: 178.1 $[M+1]^+$.

**Step 3: Synthesis of 2-(difluoromethoxy)-4-fluoro-5-nitroaniline**

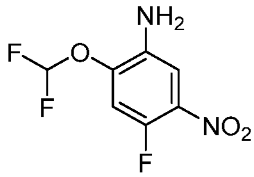

2-(difluoromethoxy)-4-fluoroaniline (8.1 g, 45.73 mmol) was dissolved in concentrated sulfuric acid (40 mL), and then, potassium nitrate (5.09 g, 50.30 mmol) was added at 0 °C. The reaction mixture slowly returned to room temperature, and was stirred for 3 hrs, and TLC monitoring was conducted. The reaction mixture was poured into ice water, and extracted with ethyl acetate three times. The organic phases were combined, then washed with saturated saline water, dried over anhydrous sodium sulfate, concentrated under reduced pressure, and then separated by column chromatography [petroleum ether100%-ethyl acetate15%] to obtain 2-(difluoromethoxy)-4-fluoro-5-nitroaniline (8.0 g, 35.15 mmol, yield: 76.87%).

$^1$H NMR (400 MHz, $CDCl_3$) $\delta$ 7.49 (d, *J* = 7.1 Hz, 1H), 7.03 (d, *J* = 10.9 Hz, 1H), 6.61 (t, *J* = 72.1 Hz, 1H), 4.06 (s, 2H).

**Step 4: Synthesis of 5-(difluoromethoxy)-*N*^1^-(2-(dimethylamino)ethyl)-*N*^1^-methyl-2-nitrobenzene-1,4-diamine**

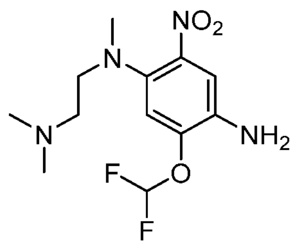

2-(difluoromethoxy)-4-fluoro-5-nitroaniline (1 g, 4.50 mmol) was dissolved in acetonitrile (30 mL), and then potassium carbonate (1.24 g, 9.00 mmol) and $N^1,N^1,N^2$-trimethylethane-1,2-diamine (0.863 mL, 6.753 mmol) were added. The reaction mixture was heated to 80 °C and stirred for 3 hrs, and TLC monitoring was conducted. The reaction mixture was cooled to room temperature and then filtered. The filtrate was concentrated under reduced pressure, and then separated by column chromatography [eluent: dichloromethane/methanol: 0-10%] to obtain 5-(difluoromethoxy)-$N^1$-(2-(dimethylamino)ethyl)-$N^1$-methyl-2-nitrobenzene-1,4-diamine (1.25 g, 3.74 mmol, yield: 83.03%). ESI-MS: 305.2 $[M+1]^+$.

Intermediates C4-C5 were prepared by referring to the synthesis method for Intermediate C3: sodium 2-chloro-2,2-difluoroacetate in Step 1 was changed with iodoethane or isoiodopropane; the reaction conditions were changed as follows: stirring for 18 h at 37 °C; and the remaining steps are the same.

| Intermediate No. | Structural Formula | Chemical Name | ESI-MS: [M+1]+ |
|---|---|---|---|
| C4 | 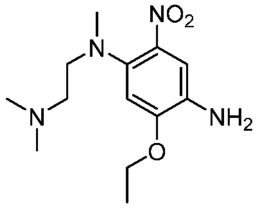 | $N^1$-(2-(dimethylamino)ethyl)-5-ethoxy-$N^1$-methyl-2-nitrobenzene-1,4-diamine | 283.1 |
| C5 | 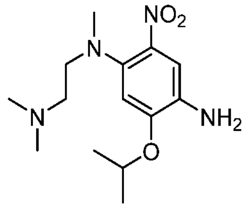 | $N^1$-(2-(dimethylamino)ethyl)-5-isopropoxy-$N^1$-methyl-2-nitrobenzene-1,4-diamine | 297.2 |

**Intermediate C6: Preparation of *N*-(4-((2-(dimethylamino)ethyl)(methyl)amino)-2-methoxy-5-nitrophenyl)formamide**

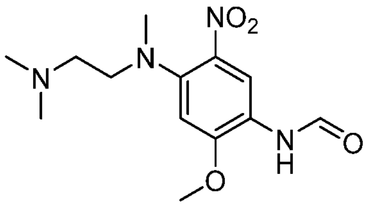

$N^1$-(2-(dimethylamino)ethyl)-5-methoxy-$N^1$-methyl-2-nitrobenzene-1,4-diamine (2.68g, 10mmol) and formic acid (20mL) were added to a reaction flask. The reaction mixture was stirred for 2 hrs at 100 °C. The resultant was distilled under reduced pressure to remove formic acid. The residue was separated by silicagel column chromatography [dichloromethane:methanol=10:1] to obtain *N*-(4-((2-(dimethylamino)ethyl)(methyl)amino)-2-methoxy-5-nitrophenyl)formamide (2.89g, yield: 93%). ESI-MS: 296.0[M+1]+.

**Intermediates C7 were prepared according to the synthesis method for Intermediate C6:**

| Intermediate No. | Structural Formula | Chemical Name | ESI-MS: [M+1]+ |
|---|---|---|---|
| C7 | 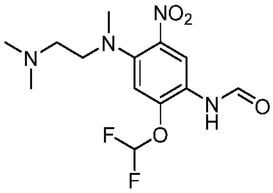 | *N*-(2-(difluoromethoxy)-4-((2-(dimethylamino)ethyl)(methyl)amino)-5-nitrophenyl)formamide | 333.0 |

**Intermediate C8: Preparation of *N[1]*-(2-(dimethylamino)ethyl)-*N[1]*-methyl-2-nitro-5-(2,2,2-trifluoroethoxy)benzene-1,4-diamine**

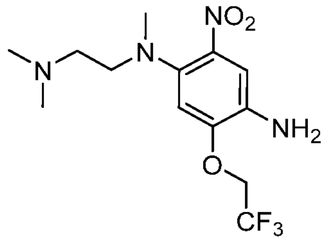

**Step 1: Synthesis of 4-fluoro-1-nitro-2-(2,2,2-trifluoroethoxy)benzene**

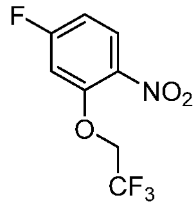

2,4-difluoro-1-nitrobenzene (12.00 g, 75.43 mmol) and cesium carbonate (24.58 g, 75.43 mmol) was dissolved in tetrahydrofuran (100 mL). At 0 °C, 2,2,2-trifluoroethane-1-ol (5.43 mL, 75.43 mmol) was added. The reaction mixture was stirred to room temperature overnight, and TLC monitoring was conducted. The reaction mixture was poured into water, and extracted with ethyl acetate three times. The organic phases were combined, then washed with saturated saline water, dried over anhydrous sodium sulfate, concentrated under reduced pressure, and then separated by column chromatography [eluent: ethyl acetate/petroleum ether: 0-10%] to obtain 4-fluoro-1-nitro-2-(2,2,2-trifluoroethoxy)benzene (15.8 g, 64.93 mmol, yield: 86.08%).

$^1H$ NMR (400 MHz, $CDCl_3$) $\delta$ 8.03 (ddd, *J* = 9.2, 5.8, 1.1 Hz, 1H), 7.00 - 6.81 (m, 2H), 4.51 (qd, *J* = 7.8, 1.1 Hz, 2H).

**Step 2: Synthesis of 4-fluoro-2-(2,2,2-trifluoroethoxy)aniline**

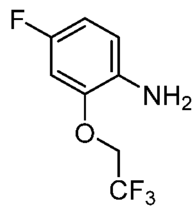

4-fluoro-1-nitro-2-(2,2,2-trifluoroethoxy)benzene (15.8 g, 66.08 mmol) was dissolved in ethanol (80 mL) and water (20 mL), to which iron powder (22.14 g, 396.45 mmol) was added. The reaction mixture was stirred for 5 hrs at 80 °C, and TLC detection was conducted. The reaction mixture was cooled to room temperature, and filtered with diatomite, and the filtrate was concentrated, and then extracted three times by adding water and ethyl acetate. The organic phases were combined, then washed with saturated saline water, dried over anhydrous sodium sulfate, concentrated under reduced pressure, and then separated by column chromatography [eluent: ethyl acetate/petroleum ether: 0-10%] to obtain 4-fluoro-2-(2,2,2-trifluoroethoxy)aniline (11.3 g, 52.01 mmol, yield: 78.71%). ESI-MS: 210.1 $[M+1]^+$.

**Step 3: Synthesis of 4-fluoro-5-nitro-2-(2,2,2-trifluoroethoxy)aniline**

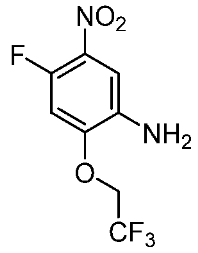

4-fluoro-2-(2,2,2-trifluoroethoxy)aniline (11.3 g, 54.03 mmol) was dissolved in concentrated sulfuric acid (40 mL), and then, potassium nitrate (6.01 g, 59.43 mmol) was added at 0 °C. The reaction mixture slowly returned to room temperature, and was stirred for 3 hrs, and TLC monitoring was conducted. The reaction mixture was poured into ice water, and extracted with ethyl acetate three times. The organic phases were combined, then washed with saturated saline water, dried over anhydrous sodium sulfate, concentrated under reduced pressure, and then separated by column chromatography [eluent: ethyl acetate/petroleum ether: 0-15%] to obtain 4-fluoro-5-nitro-2-(2,2,2-trifluoroethoxy)aniline (6.5 g, 24.29 mmol, yield: 44.97%).

$^1H$ NMR (400 MHz, $CDCl_3$) δ 7.46 (d, *J* = 7.3 Hz, 1H), 6.68 (d, *J* = 11.5 Hz, 1H), 4.47 (q, *J* = 7.7 Hz, 2H), 4.01 (s, 2H).

**Step 4: Synthesis of *N¹*-(2-(dimethylamino)ethyl)-*N¹*-methyl-2-nitro-5-(2,2,2-trifluoroethoxy)benzene-1,4-diamine**

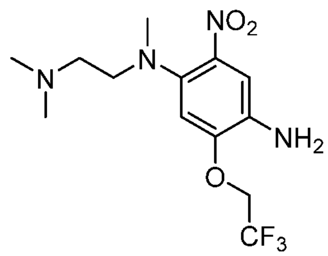

4-fluoro-5-nitro-2-(2,2,2-trifluoroethoxy)aniline (1.0 g, 3.93 mmol) was dissolved in acetonitrile (30 mL), and then, potassium carbonate (1.09 g, 7.87 mmol) and $N^1,N^1,N^2$-trimethylethane-1,2-diamine (0.754 mL, 5.90 mmol) were added. The reaction mixture was heated to 80 °C and stirred for 3 hrs, and TLC monitoring was conducted. The reaction mixture was cooled to room temperature and then filtered. The filtrate was concentrated under reduced pressure, and then separated by column chromatography [eluent: dichloromethane/methanol: 0-10%] to obtain $N^1$-(2-(dimethylamino)ethyl)-$N^1$-methyl-2-nitro-5-(2,2,2-trifluoroethoxy)benzene-1,4-diamine (1.1 g, 3.11 mmol, yield: 79.26%). ESI-MS: 337.1 $[M+1]^+$.

**Intermediate C9: Preparation of $N^2$-(2-(dimethylamino)ethyl)-6-methoxy-$N^2$-methyl-3-nitropyridin-2,5-diamine**

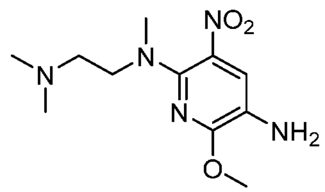

**Step 1: Synthesis of 6-bromo-2-methoxy-3-nitropyridine**

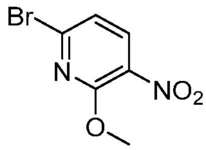

In an ice bath, sodium methoxide (5.3 g, 78.0 mmol) was added to the solution of 2,6-dibromine-3-nitropyridine (20 g, 70.9 mmol) in tetrahydrofuran (300 mL). The reaction mixture was stirred for 3 hrs at room temperature. The reaction mixture was poured into ice water, and ethyl acetate was added for extraction. The organic phases were combined, the saturated saline water was added for washing, and the organic phases were concentrated and then separated by column chromatography [petroleum ether:ethyl acetate=5:1] to obtain 6-bromo-2-methoxy-3-nitropyridine (13.9 g, yield: 85%). ESI-MS: 217.1 $[M-15]^+$.

**Step 2: Synthesis of 6-bromo-2-methoxypyridin-3-amine**

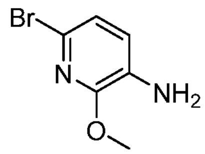

Iron powder (26.9 g, 480.8 mmol) and ammonium chloride (25.9 g, 480.8 mmol) were added to the solution of 6-bromo-2-methoxy-3-nitropyridine (13.9 g, 60.1 mmol) in [ethanol/water=2:1]. The reaction mixture was stirred for 3 hrs at 90 °C. Stratification was conducted with dichloromethane and water. The organic phase was concentrated and then separated by column chromatography [petroleum ether:ethyl acetate=3:1] to obtain 6-bromo-2-methoxypyridin-3-amine (9.1 g, yield: 75%). ESI-MS: 203.1 $[M+1]^+$.

1H NMR (400 MHz, DMSO-$d_6$) δ 6.89 (d, J = 7.9 Hz, 1H), 6.83 (d, J = 7.9 Hz, 1H), 5.10 (s, 2H), 3.84 (s, 3H).

**Step 3: Synthesis of *N*-(6-bromo-2-methoxypyridin-3-yl)acetamide**

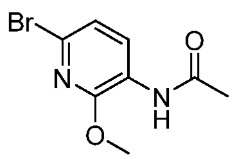

In an ice bath, triethylamine (6.7 g, 67.2 mmol, 1.5 eq.) and acetylchloride (3.8 g, 49.2 mmol, 1.1 eq.) were added to the solution of 6-bromo-2-methoxypyridin-3-amine (9.1 g, 44.8 mmol, 1 eq.) in dichloromethane (200 mL). The reaction mixture was stirred for 1 hr in the ice bath. Stratification was conducted with dichloromethane and water, and the organic phase was concentrated and then separated by column chromatography [petroleum ether: ethyl acetate=5:1] to obtain *N*-(6-bromo-2-methoxypyridin-3-yl)acetamide (9.5 g, yield: 86%), which was directly used in the next step.

**Step 4: Synthesis of *N*-(6-bromo-2-methoxy-5-nitropyridin-3-yl)acetamide**

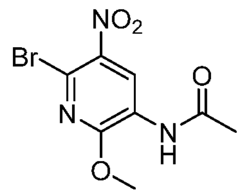

In an ice bath, concentrated nitric acid (65%, 46.6 mmol) was added to the solution of *N*-(6-bromo-2-methoxypyridin-3-yl)acetamide (9.5 g, 38.9 mmol) in trifluoroacetic anhydride (80 mL). The reaction mixture was stirred for 1 hr in the ice bath. The reaction mixture was slowly poured into ice water, stirred for 1 hr for solid precipitation, and then filtered by suction, and the filter cake was dried to obtain *N*-(6-bromo-2-methoxy-5-nitropyridin-3-yl)acetamide (11.5 g, yield: 100%). ESI-MS: 290.1 $[M+1]^+$.

$^1$H NMR (400 MHz, DMSO-$d_6$) δ 9.90 (s, 1H), 9.12 (s, 1H), 4.06 (s, 3H), 2.16 (s, 3H).

**Step 5: Synthesis of *N*-(6-((2-(dimethylamino)ethyl)(methyl)amino)-2-methoxy-5-nitropyridin-3-yl)acetamide**

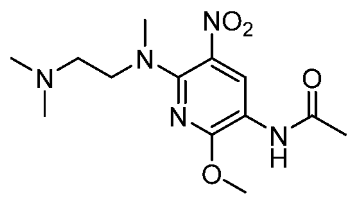

*N*$^1$,*N*$^1$,*N*$^2$-trimethylethane-1,2-diamine (520 mg, 5.1 mmol) was added to the solution of *N*-(6-bromo-2-methoxy-5-nitropyridin-3-yl)acetamide (1.0 g, 3.4 mmol) in acetonitrile (20 mL). The reaction mixture was stirred for 1 hr at 80 °C. After the solvent was removed, the resultant was separated by silicagel column chromatography [dichloromethane:methanol=10:1] to obtain *N*-(6-((2-(dimethylamino)ethyl)(methyl)amino)-2-methoxy-5-nitropyridin-3-yl)acetamide (756 mg, yield: 71%). ESI-MS: 312.3 $[M+1]^+$.

**Step 6: Synthesis of *N*$^2$-(2-(dimethylamino)ethyl)-6-methoxy-*N*$^2$-methyl-3-nitropyridin-2,5-diamine**

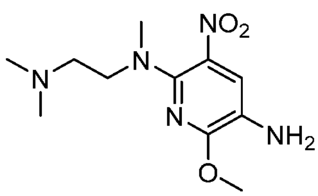

Concentrated hydrochloric acid (37%, 1.5mL, 18 mmol, 7.5 eq.) was added to the solution of *N*-(6-((2-(dimethylamino)ethyl)(methyl)amino)-2-methoxy-5-nitropyridin-3-yl)acetamide (756 mg, 2.4 mmol) in methanol (10 mL). The reaction mixture was stirred for 5 hrs at 60 °C. Stratification was conducted with the solution of saturated sodium bicarbonate, and dichloromethane. The organic phase was concentrated to obtain *N*$^2$-(2-(dimethylamino)ethyl)-6-methoxy-*N*$^2$-methyl-3-nitropyridin-2,5-diamine (645 mg, yield: 100%). ESI-MS: 270.3 $[M+1]^+$.

**Intermediates C10-C11 were prepared according to the preparation method for Intermediate C9:**

| Intermediate No. | Structural Formula | Chemical Name | ESI-MS: [M+1]+ |
|---|---|---|---|
| **C10** | 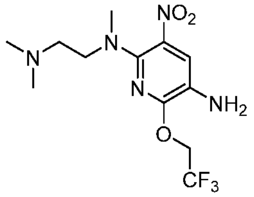 | $N^2$-(2-(dimethylamino)ethyl)-$N^2$-methyl-3-nitro-6-(2,2,2-trifluoroethoxy)pyridin-2,5-diamine | 338.0 |
| **C11** | 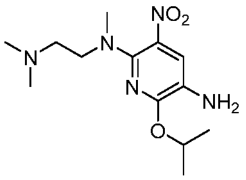 | $N^2$-(2-(dimethylamino)ethyl)-6-isopropoxy-$N^2$-methyl-3-nitropyridin-2,5-diamine | 298.3 |

**Intermediate D1: Preparation of *N*1-(4-(3,3-dimethyl-2,3-dihydro-1*H*-pyrrolo[3,2-*b*]pyridin-1-yl)pyridin-2-yl)-*N*4-(2-(dimethylamino)ethyl)-2-methoxy-*N*4-methyl-5-nitrobenzene-1,4-diamine**

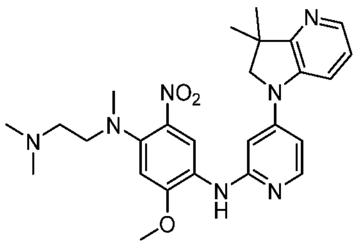

1-(2-chloropyridin-4-yl)-3,3-dimethyl-2,3-dihydro-1*H*-pyrrolo[3,2-*b*]pyridine (250 mg, 0.95 mmol) was dissolved in 20 mL of 1,4-dioxane. At room temperature, *N*1-(2-(dimethylamino)ethyl)-5-methoxy-*N*1-methyl-2-nitrobenzene-1,4-diamine (254 mg, 0.95 mmol), cesium carbonate (926 mg, 2.85 mmol), 1,1'-dinaphthalene-2,2'-bisdiphenylphosphine (177 mg, 0.285 mmol) and palladium acetate (31 mg, 0.145 mmol) were added to the mixture. After evacuation and nitrogen displacement were conducted three times, the reaction mixture was stirred for 2 hrs at 110 °C. Water was added to the solution, which was extracted with dichloromethane three times. The organic phases were combined, then washed with saturated saline, and dried over anhydrous sodium sulfate. After the solvent was removed, the resultant was separated by silicagel column chromatography [dichloromethane:methanol=10:1] to obtain *N*1-(4-(3,3-dimethyl-2,3-dihydro-1*H*-pyrrolo[3,2-*b*]pyridin-1-yl)pyridin-2-yl)-*N*4-(2-(dimethylamino)ethyl)-2-methoxy-*N*4-methyl-5-nitrobenzene-1,4-diamine (335 mg, yield: 71%). ESI-MS: 492.2 [M+1]+.

**Intermediates D2-D46, D47-2 and D48-2 were prepared according to the synthesis method for Intermediate D1:**

| Intermediate No. | Structural Formula | Chemical Name | ESI-MS: $[M+1]^+$ |
|---|---|---|---|
| D2 | 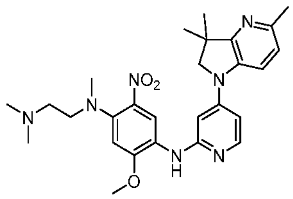 | *N*1-(2-(dimethylamino)ethyl)-5-methoxy-*N*1-methyl-2-nitro-*N*4-(4-(3,3,5-trimethyl-2,3-dihydro-1*H*-pyrrolo[3,2-*b*]pyridin-1-yl)pyridin-2-yl) benzene-1,4-diamine | 506.2 |
| D3 | 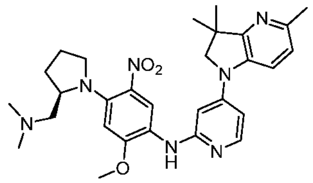 | (*R*)-*N*-(4-(2-((dimethylamino)methyl)pyrroli din-1-yl)-2-methoxy-5-nitrophenyl)-4-(3,3,5-trimethyl-2,3-dihydro-1*H*-pyrrolo[3,2-*b*]pyridin-1-yl)pyridin-2-amine | 532.2 |
| D4 | 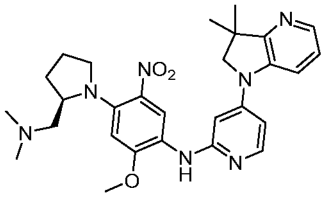 | (*R*)-4-(3,3-dimethyl-2,3-dihydro-1*H*-pyrrolo[3,2-*b*] pyridin-1-yl)-*N*-(4-(2-((dimethylamino)methyl) pyrroli din-1-yl)-2-methoxy-5-nitrophenyl)pyridin-2-amine | 518.2 |
| D5 | 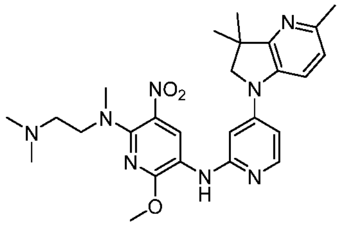 | *N*2-(2-(dimethylamino)ethyl)-6-methoxy-*N*2-methyl-3-nitro-*N*5-(4-(3,3,5-trimethyl-2,3-dihydro-1*H*-pyrrolo[3,2-*b*]pyridin-1-yl)pyridin-2-yl) pyridin-2,5-diamine | 507.2 |
| D6 | 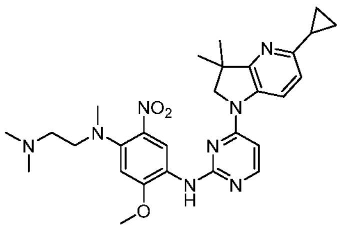 | *N*1-(4-(5-cyclopropyl-3,3-dimethyl-2,3-dihydro-1*H*-pyrrolo[3,2-*b*]pyridin-1-yl)pyrimidin-2-yl)-*N*4-(2-(dimethylamino)ethyl)-2-methoxy-*N*4-methyl-5-nitrobenzene-1,4-diamine | 533.3 |
| D7 | 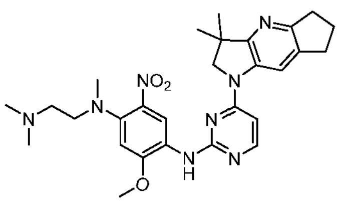 | *N*1-(4-(3,3-dimethyl-3,5,6,7-tetrahydrocyclopenta [*b*]pyrrolo[ 2,3-*e*]pyridin-1(2*H*)-yl)pyrimidin-2-yl)-*N*4-(2-(dimethylamino)ethyl)-2-methoxy-*N*4-methyl-5-nitrobenzene-1,4-diamine | 533.3 |
| D8 | 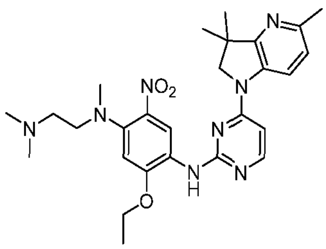 | *N*1-(2-(dimethylamino)ethyl)-5-ethoxy-*N*1-methyl-2-nitro-*N*4-(4-(3,3,5-trimethyl-2,3-dihydro-1*H*-pyrrolo[3,2-*b*]pyridin-1-yl)pyrimidin-2-yl) benzene-1,4-diamine | 521.2 |

(continued)

| Intermediate No. | Structural Formula | Chemical Name | ESI-MS: [M+1]+ |
|---|---|---|---|
| **D9** | 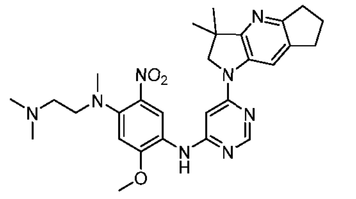 | $N^1$-(6-(3,3-dimethyl-3,5,6,7-tetrahydrocyclopenta [*b*]pyrrolo[ 2,3-*e*]pyridin-1(2*H*)-yl)pyrimidin-4-yl)-$N^4$-(2-(dimethylamino)ethyl)-2-methoxy-$N^4$-methyl-5-nitrobenzene-1,4-diamine | 533.3 |
| **D10** | 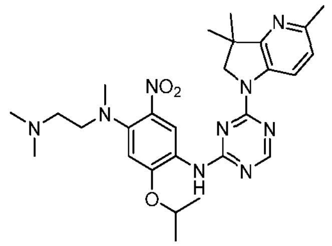 | $N^1$-(2-(dimethylamino)ethyl)-5-isopropoxy-$N^1$-methyl-2-nitro-$N^4$-(4-(3,3,5-trimethyl-2,3-dihydro-1*H*-pyrrolo[3,2-*b*]pyridin-1-yl)-1,3, 5-triazin-2-yl)benzene-1,4-diamine | 536.3 |
| **D11** | 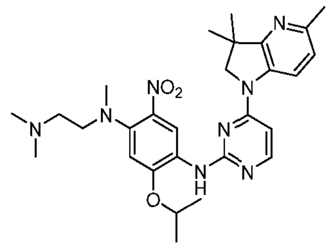 | $N^1$-(2-(dimethylamino)ethyl)-5-isopropoxy-$N^1$-methyl-2-nitro-$N^4$-(4-(3,3,5-trimethyl-2,3-dihydro-1*H*-pyrrolo[3,2-*b*]pyridin-1-yl)pyrimidin-2-yl)benzene-1,4-diamine | 535.2 |
| **D12** | 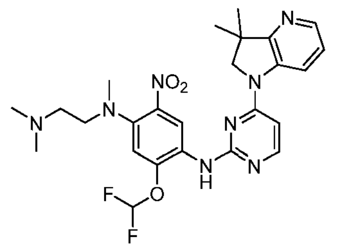 | 2-(difluoromethoxy)-$N^1$-(4-(3,3-dimethyl-2,3-dihydro-1*H*-pyrrolo[3,2-*b*]pyridin-1-yl)pyrimidin-2-yl)-$N^4$-(2-(dimethylamino)ethyl)-$N^4$-methyl-5-nitrobenzene-1,4-diamine | 529.2 |
| **D13** | 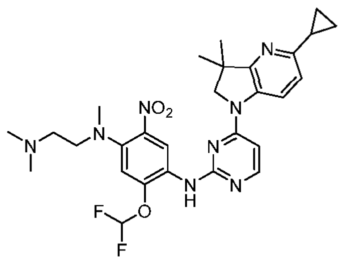 | $N^1$-(4-(5-cyclopropyl-3,3-dimethyl-2,3-dihydro-1*H*-pyrrolo[3,2-*b*]pyridin-1-yl)pyrimidin-2-yl)-2-(difluoromethoxy)-$N^4$-(2-(dimethylamino) ethyl)-$N^4$-methyl-5-nitrobenzene-1,4-diamine | 569.2 |
| **D14** | 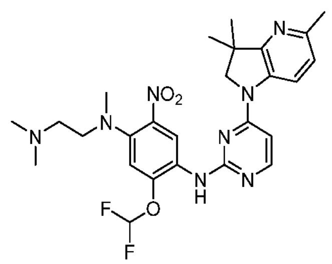 | 2-(difluoromethoxy)-$N^4$-(2-(dimethylamino) ethyl)-$N^4$-methyl-5-nitro-$N^1$-(4-(3,3,5-trimethyl-2,3-dihydro-1H-pyrrolo[3,2-*b*]pyridin-1-yl) pyrimidin-2-yl)benzene-1,4-diamine | 543.2 |

(continued)

| Intermediate No. | Structural Formula | Chemical Name | ESI-MS: [M+1]+ |
|---|---|---|---|
| **D15** | 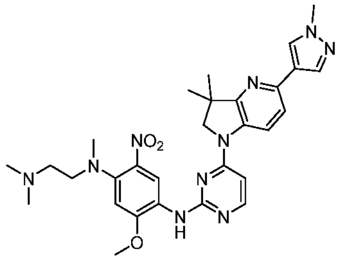 | *N*1-(4-(3,3-dimethyl-5-(1-methyl-1*H*-pyrazol-4-yl)-2,3-dihydro-1*H*-pyrrolo[3,2-*b*]pyridin-1-yl) pyrimidin-2-yl)-*N*4-(2-(dimethylamino)ethyl)-2-methoxy-*N*4-methyl-5-nitrobenzene-1,4-diamine | 573.3 |
| **D16** | 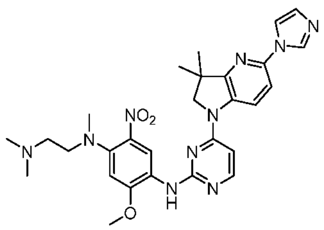 | *N*1-(4-(5-(1*H*-imidazole-1-yl)-3,3-dimethyl-2,3-dihydro-1*H*-pyrrolo[3,2-*b*]pyridin-1-yl)pyrimidin-2-yl)-*N*4-(2-(dimethylamino)ethyl)-2-methoxy-*N*4-methyl-5-nitrobenzene-1,4-diamine | 559.2 |
| **D17** | 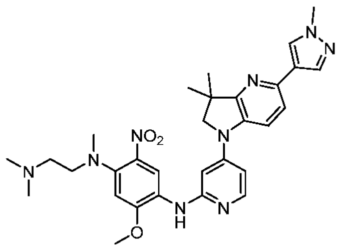 | *N*1-(4-(3,3-dimethyl-5-(1-methyl-1*H*-pyrazol-4-yl)-2,3-dihydro-1*H*-pyrrolo[3,2-*b*]pyridin-1-yl)pyridin-2-yl)-*N*4-(2-(dimethylamino)ethyl)-2-methoxy-*N*4-methyl-5-nitrobenzene-1,4-diamine | 572.2 |
| **D18** | 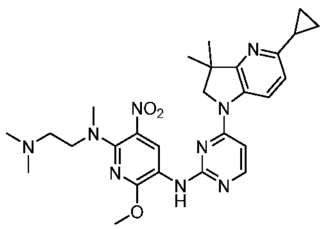 | *N*5-(4-(5-cyclopropyl-3,3-dimethyl-2,3-dihydro-1*H*-pyrrolo[3,2-*b*]pyridin-1-yl)pyrimidin-2-yl)-*N*2-(2-(dimethylamino)ethyl)-6-methoxy-*N*2-methyl-3-nitropyridin-2,5-diamine | 534.2 |
| **D19** | 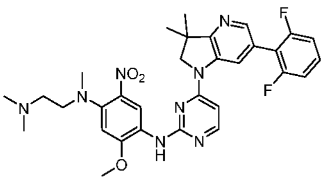 | *N*1-(4-(6-(2,6-difluorophenyl)-3,3-dimethyl-2,3-dihydro-1*H*-pyrrolo[3,2-*b*]pyridin-1-yl)pyrimidin-2-yl)-*N*4-(2-(dimethylamino)ethyl)-2-methoxy-*N*4-methyl-5-nitrobenzene-1,4-diamine | 605.3 |
| **D21** | 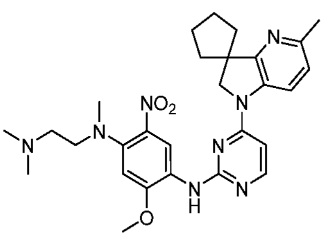 | *N*1-(2-(dimethylamino)ethyl)-5-methoxy-*N*1-methyl-*N*4-(4-(5'-methylspiro[cyclopentane-1,3'-pyrrolo[3,2-*b*]pyridin]-1'(2'*H*)-yl)pyrimidin-2-yl)-2-nitrobenzene-1,4-diamine | 533.3 |

(continued)

| Intermediate No. | Structural Formula | Chemical Name | ESI-MS: [M+1]+ |
|---|---|---|---|
| **D22** | 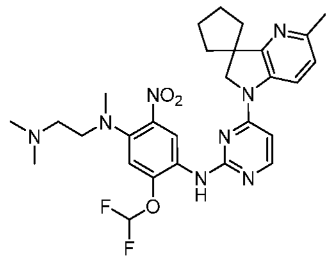 | 2-(difluoromethoxy)-*N*4-(2-(dimethylamino) ethyl)-*N*4-methyl-*N*1-(4-(5'-methylspiro [cyclopentane-1,3'-pyrrolo[3,2-*b*]pyridin]-1' (2'*H*)-yl)pyrimidin-2-yl)-5-nitrobenzene-1,4-diamine | 569.2 |
| **D23** | 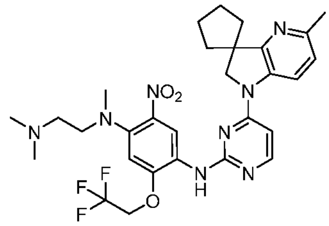 | *N*1-(2-(dimethylamino)ethyl)-*N*1-methyl-*N*4-(4-(5'-methylspiro[cyclopentane-1,3'-pyrrolo[3,2-*b*] pyridin]-1'(2'*H*)-yl)pyrimidin-2-yl)-2-nitro-5-(2,2,2-trifluoroethoxy)benzene-1,4-diamine | 601.2 |
| **D24** | 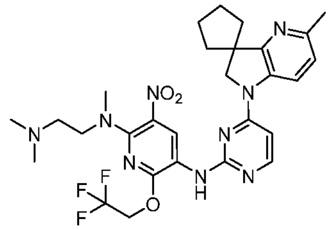 | *N*2-(2-(dimethylamino)ethyl)-*N*2-methyl-*N*5-(4-(5'-methylspiro[cyclopentane-1,3'-pyrrolo[3,2-*b*] pyridin]-1'(2'*H*)-yl)pyrimidin-2-yl)-3 -nitro-6-(2,2,2-trifluoroethoxy)pyridin-2,5-diamine | 602.2 |
| **D25** | 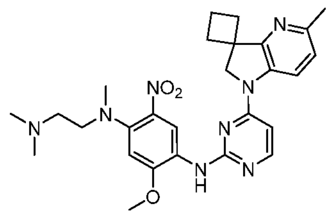 | *N*1-(2-(dimethylamino)ethyl)-5-methoxy-*N*1-methyl-*N*4-(4-(5'-methylspiro[cyclobutane-1,3'-pyrrolo[3,2-*b*]pyridin]-1'(2'*H*)-yl)pyrimidin-2-yl)-2-nitrobenzene-1,4-diamine | 519.2 |
| **D26** | 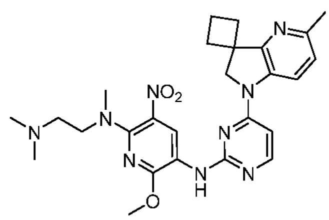 | *N*2-(2-(dimethylamino)ethyl)-6-methoxy-*N*2-methyl-*N*5-(4-(5'-methylspiro[cyclobutane-1,3'-pyrrolo[3,2-*b*]pyridin]-1'(2'*H*)-yl)pyrimidin-2-yl)-3 - nitropyridin-2,5-diamine | 520.2 |
| **D27** | 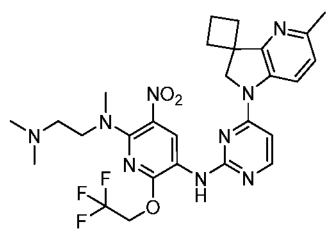 | *N*2-(2-(dimethylamino)ethyl)-*N*2-methyl-*N*5-(4-(5'-methylspiro[cyclobutane-1,3'-pyrrolo[3,2-*b*] pyridin]-1'(2'*H*)-yl)pyrimidin-2-yl)-3 -nitro-6-(2,2,2-trifluoroethoxy)pyridin-2,5-diamine | 588.2 |

(continued)

| Intermediate No. | Structural Formula | Chemical Name | ESI-MS: [M+1]+ |
|---|---|---|---|
| D28 | 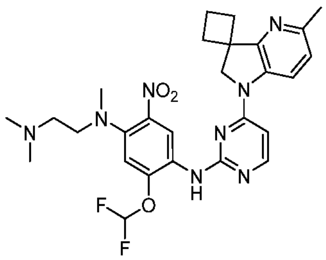 | 2-(difluoromethoxy)-*N*4-(2-(dimethylamino) ethyl)-*N*4-methyl-*N*1-(4-(5'-methylspiro [cyclobutane-1,3'-pyrrolo[3,2-*b*]pyridin]-1'(2'*H*)-yl) pyrimidin-2-yl)-5-nitrobenzene-1,4-diamine | 555.2 |
| D29 | 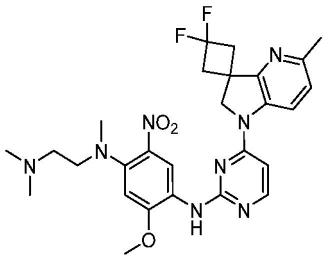 | *N*1-(4-(3,3-difluoro-5'-methylspiro[cyclobutane-1,3'-pyrrolo[3,2-*b*]pyridin]-1'(2'*H*)-yl)pyrimidin-2-yl)-*N*4-(2-(dimethylamino)ethyl)-2-methoxy-*N*4-methyl-5-nitrobenzene-1,4-diamine | 555.2 |
| D30 | 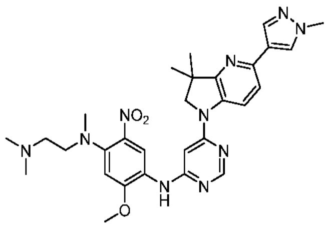 | *N*1-(6-(3,3-dimethyl-5-(1-methyl-1*H*-pyrazol-4-yl)-2,3-dihydro-1*H*-pyrrolo[3,2-*b*]pyridin-1-yl) pyrimidin-4-yl)-*N*4-(2-(dimethylamino)ethyl)-2-methoxy-*N*4-methyl-5-nitrobenzene-1,4-diamine | 573.2 |
| D31 | 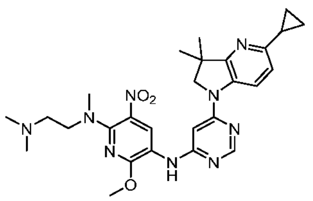 | *N*5-(6-(5-cyclopropyl-3,3-dimethyl-2,3-dihydro-1*H*-pyrrolo[3,2-*b*]pyridin-1-yl)pyrimidin-4-yl)-*N*2-(2-(dimethylamino)ethyl)-6-methoxy-*N*2-methyl-3-nitropyridin-2,5-diamine | 534.2 |
| D32 | 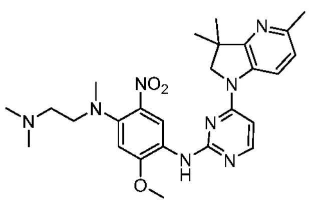 | *N*1-(2-(dimethylamino)ethyl)-5-methoxy-*N*1-methyl-2-nitro-*N*4-(4-(3,3,5-trimethyl-2,3-dihydro-1*H*-pyrrolo[3,2-*b*]pyridin-1-yl)pyrimidin-2-yl)benzene-1,4-diamine | 507.3 |
| D33 | 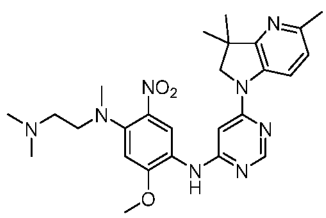 | *N*1-(2-(dimethylamino)ethyl)-5-methoxy-*N*1-methyl-2-nitro-*N*4-(6-(3,3,5-trimethyl-2,3-dihydro-1*H*-pyrrolo[3,2-*b*]pyridin-1-yl)pyrimidin-4-yl)benzene-1,4-diamine | 507.2 |

(continued)

| Intermediate No. | Structural Formula | Chemical Name | ESI-MS: [M+1]+ |
|---|---|---|---|
| **D34** | 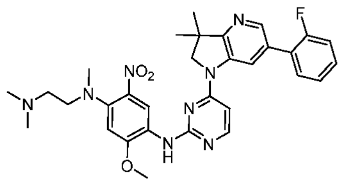 | *N*1-(2-(dimethylamino)ethyl)-*N*4-(4-(6-(2-fluorophenyl)-3,3 - dimethyl-2,3-dihydro-1*H*-pyrrolo[3,2-*b*]pyridin-1-yl)pyrimidin-2-yl)-5-methoxy-*N*1-methyl-2-nitrobenzene-1,4-diamine | 588.3 |
| **D35** | 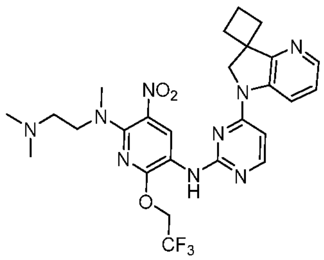 | *N*2-(2-(dimethylamino)ethyl)-*N*2-methyl-3-nitro-*N*5-(4-(spiro[cyclobutane-1,3'-pyrrolo[3,2-*b*] pyridin]-1'(2'*H*)-yl)pyrimidin-2-yl)-6-(2,2,2-trifluoroethoxy)pyridin-2, 5-diamine | 574.2 |
| **D36** | 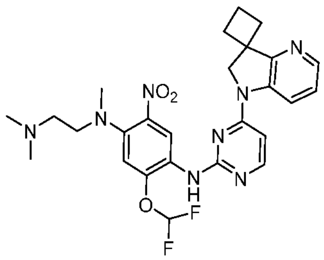 | 2-(difluoromethoxy)-*N*4-(2-(dimethylamino) ethyl)-*N*4-methyl-5-nitro-*N*1-(4-(spiro [cyclobutane-1,3'-pyrrolo[3,2-*b*]pyridin]-1'(2'*H*)-yl) pyrimidin-2-yl)benzene-1,4-diamine | 541.2 |
| **D37** | 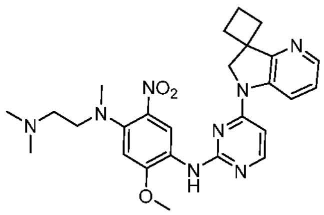 | *N*1-(2-(dimethylamino)ethyl)-5-methoxy-*N*1-methyl-2-nitro-*N*4-(4-(spiro[cyclobutane-1,3'-pyrrolo[3,2-*b*]pyridin]-1'(2'*H*-yl)pyrimidin-2-yl) benzene-1,4-diamine | 505.2 |
| **D38** | 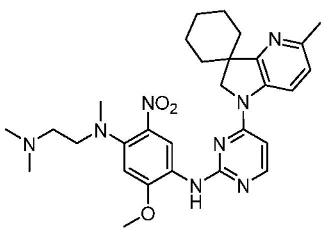 | *N*1-(2-(dimethylamino)ethyl)-5-methoxy-*N*1-methyl-*N*4-(4-(5'-methylspiro[cyclohexane-1,3'-pyrrolo[3,2-*b*]pyridin]-1'(2'*H*)-yl)pyrimidin-2-yl)-2-nitrobenzene-1,4-diamine | 547.2 |
| **D39** | 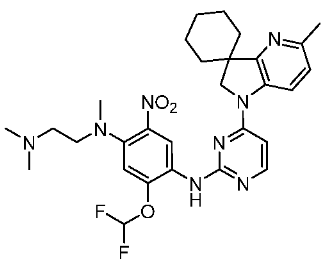 | 2-(difluoromethoxy)-*N*4-(2-(dimethylamino) ethyl)-*N*4-methyl-*N*1-(4-(5'-methylspiro [cyclohexane-1,3'-pyrrolo[3,2-*b*]pyridin]-1' (2'*H*)-yl)pyrimidin-2-yl)-5-nitrobenzene-1,4-diamine | 583.2 |

(continued)

| Intermediate No. | Structural Formula | Chemical Name | ESI-MS: [M+1]+ |
|---|---|---|---|
| **D40** | 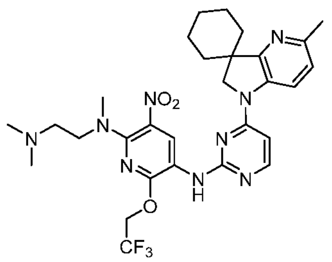 | $N^2$-(2-(dimethylamino)ethyl)-$N^2$-methyl-$N^5$-(4-(5'-methylspiro[cyclohexane-1,3'-pyrrolo[3,2-*b*] pyridin]-1'(2'*H*)-yl)pyrimidin-2-yl)-3 -nitro-6-(2,2,2-trifluoroethoxy)pyridin-2,5-diamine | 616.3 |
| **D41** | 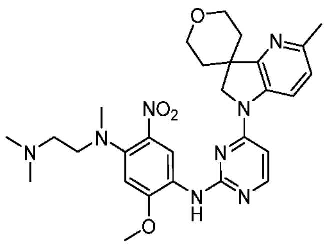 | $N^1$-(2-(dimethylamino)ethyl)-5-methoxy-$N^1$-methyl-$N^4$-(4-(5'-methyl-2,3,5,6-tetrahydrogenspiro[pyran-4,3'-pyrrolo[3,2-*b*] pyridin]-1'(2'H)-yl)pyrimidin-2-yl)-2-nitrobenzene-1,4-diamine | 549.2 |
| **D42** | 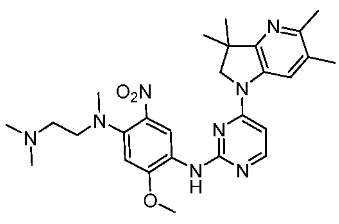 | $N^1$-[2-(dimethylamino)ethyl]-5-methoxy-$N^1$-methyl-2-nitro-$N^4$-(4-(3,3,5,6-tetramethyl-2,3-dihydro-1*H*-pyrrolo[3,2-*b*]pyridin-1-yl)pyrimidin-2-yl)benzene-1,4-diamine | 521.2 |
| **D43** | 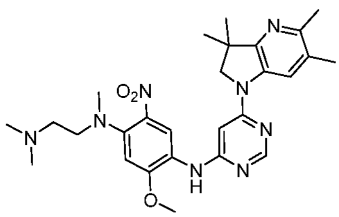 | $N^1$-[2-(dimethylamino)ethyl]-5-methoxy-$N^1$-methyl-2-nitro-$N^4$-(6-(3,3,5,6-tetramethyl-2,3-dihydro-1*H*-pyrrolo[3,2-*b*]pyridin-1-yl)pyrimidin-4-yl)benzene-1,4-diamine | 521.3 |
| **D44** | 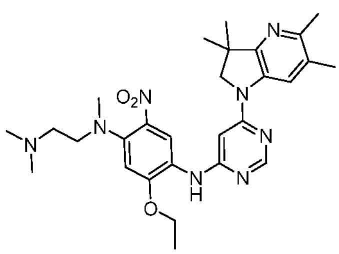 | $N^1$-[2-(dimethylamino)ethyl]-5-ethoxy-$N^1$-methyl-2-nitro-$N^4$-(6-(3,3,5,6-tetramethyl-2,3-dihydro-1*H*-pyrrolo[3,2-*b*]pyridin-1-yl)pyrimidin-4-yl) benzene-1,4-diamine | 535.3 |
| **D45** | 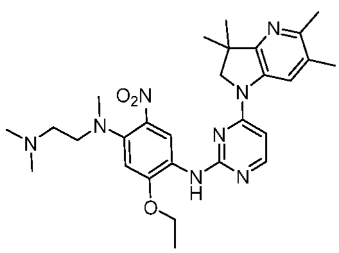 | $N^1$-[2-(dimethylamino)ethyl]-5-ethoxy-$N^1$-methyl-2-nitro-$N^4$-(4-(3,3,5,6-tetramethyl-2,3-dihydro-1*H*-pyrrolo[3,2-*b*]pyridin-1-yl)pyrimidin-2-yl) benzene-1,4-diamine | 535.2 |

(continued)

| Intermediate No. | Structural Formula | Chemical Name | ESI-MS: [M+1]+ |
|---|---|---|---|
| D46 | 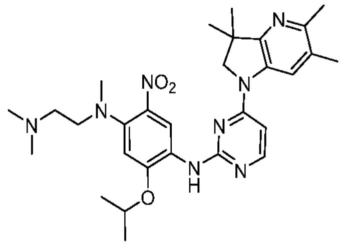 | $N^1$-[2-(dimethylamino)ethyl]-$N^1$-methyl-2-nitro-5-(propane-2-oxy)-$N^4$-(4-(3,3,5,6-tetramethyl-2,3-dihydro-1*H*-pyrrolo[3,2-*b*]pyridin-1-yl)pyrimidin-2-yl)benzene-1,4-diamine | 549.3 |
| D47-2 | 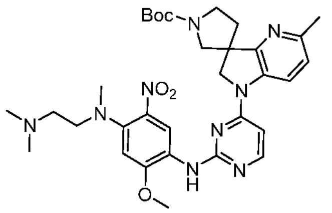 | tert-butyl 1'-(2-((4-((2-(dimethylamino)ethyl) (methyl) amino)-2-methoxy-5-nitrophenyl)amino) pyrimidin-4-yl)-5'-methyl-1',2'-dihydrospiro [pyrrolidin-3,3'-pyrrolo[3,2-*b*]pyridin]-1-carboxylate | 634.4 |
| D48-2 | 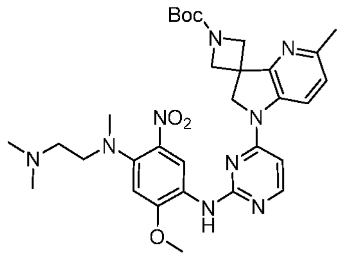 | tert-butyl 1'-(2-((4-((2-(dimethylamino)ethyl) (methyl) amino)-2-methoxy-5-nitrophenyl)amino) pyrimidin-4-yl)-5'-methyl-1',2'-dihydrospiro [azetidine-3,3'-pyrrolo[3,2-*b*]pyridin]-1-carboxylate | 620.2 |

**Intermediate D49-1: Preparation of 4-(3,3-dimethyl-2,3-dihydro-1*H*-pyrrolo[3,2-*b*]pyridin-1-yl)-*N*-(4-fluoro-2-methoxy-5-nitrophenyl)pyrimidin-2-amine**

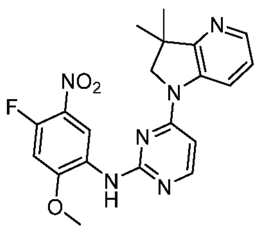

1-(2-chloropyrimidin-4-yl)-3,3-dimethyl-2,3-dihydro-1*H*-pyrrolo[3,2-*b*]pyridine (235 mg, 0.86 mmol), 4-fluoro-2-methoxy-5-nitroaniline (159.38 mg, 0.81 mmol), cesium carbonate (440.50 mg, 1.28 mmol), palladium acetate (20.24 mg, 0.09 mmol), 1,1'-dinaphthalene-2,2'-bisdiphenylphosphine (112.24 mg, 0.17 mmol) and 1,4-dioxane (15 mL) were added to a reaction flask in sequence. The reaction mixture was subjected to nitrogen displacement three times, stirred at 120 °C under the protection of nitrogen to react for 2 hrs, and filtered, and the filtrate was concentrated. The residue was separated by column chromatography [methanol/dichloromethane: 0-10%] to obtain 4-(3,3-dimethyl-2,3-dihydro-1*H*-pyrrolo[3,2-*b*]pyridin-1-yl)-*N*-(4-fluoro-2-methoxy-5-nitrophenyl)pyrimidin-2-amine (349 mg, yield: 89.38%), ESI-MS: 411.0 [M+1]+.

**Intermediates D50-1 and D51-1 were prepared according to the synthesis method for Intermediate D49-1:**

| Intermediate No. | Structural Formula | Chemical Name | ESI-MS: [M+1]+ |
|---|---|---|---|
| **D50-1** | 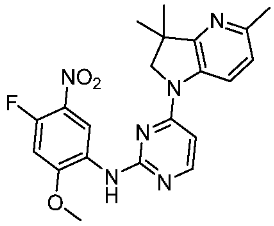 | *N*-(4-fluoro-2-methoxy-5-nitrophenyl)-4-(3,3,5 - trimethyl-2,3-dihydro-1*H*-pyrrolo[3,2-*b*]pyridin-1-yl) pyrimidin-2-amine | 425.0 |
| **D51-1** | 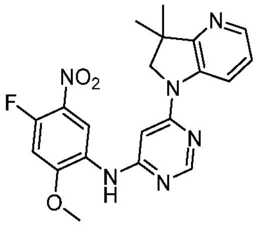 | 6-(3,3-dimethyl-2,3-dihydro-1*H*-pyrrolo[3,2-*b*] pyridin-1-yl)-*N*-(4-fluoro-2-methoxy-5-nitrophenyl) pyrimidin-4-amine | 411.0 |

**Intermediate D49: Preparation of (R)-4-(3,3-dimethyl-2,3-dihydro-1*H*-pyrrolo[3,2-*b*]pyridin-1-yl)-*N*-(4-(2-((dimethylamino)methyl)pyrrolidin-1-yl)-2-methoxy-5-nitrophenyl)pyrimidin-2-amine**

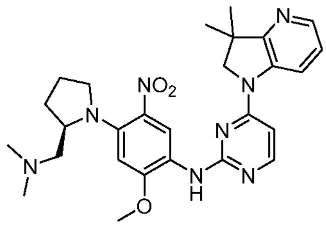

(R)-*N,N*-dimethyl-1-(pyrrolidin-2-yl)methylamine (93.12 mg, 0.44 mmol) and *N,N*-diisopropylethylamine (149.30 mg, 1.16 mmol) were added to the solution of 4-(3,3-dimethyl-2,3-dihydro-1*H*-pyrrolo[3,2-*b*]pyridin-1-yl)-*N*-(4-fluoro-2-methoxy-5-nitrophenyl)pyrimidin-2-amine (100 mg, 0.23 mmol) in 1,4-dioxane (20 mL). The reaction mixture was stirred for 18 hrs at 120 °C. Stratification was conducted with dichloromethane and water. The organic phase was washed with water and saturated sodium chloride in sequence, then dried over anhydrous sodium sulfate, filtered, concentrated, and then separated by column chromatography [dichloromethane:methanol=10:1] to obtain (R)-4-(3,3-dimethyl-2,3-dihydro-1*H*-pyrrolo[3,2-*b*]pyridin-1-yl)-*N*-(4-(2-((dimethylamino)methyl)pyrrolidin-1-yl)-2-methoxy-5-nitrophenyl)pyrimidin-2-amine (115 mg, yield: 91.01%). ESI-MS: 519.2 [M+1]+.

**Intermediates D50-D52 were prepared according to the synthesis method for Intermediate D49:**

| Intermediate No. | Structural Formula | Chemical Name | ESI-MS: [M+1]$^+$ |
|---|---|---|---|
| **D50** | 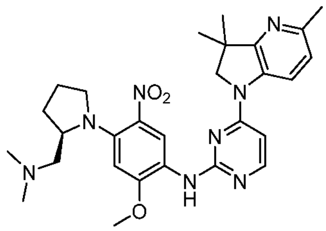 | (R)-*N*-(4-(2-((dimethylamino)methyl)py rrolidin-1-yl)-2-methoxy-5-nitrophenyl)-4-(3,3,5 - trimethyl-2,3-dihydro-1*H*-pyrrolo[3,2-*b*]pyridin-1-yl)pyrimidin-2-amine | 533.2 |
| **D51** | 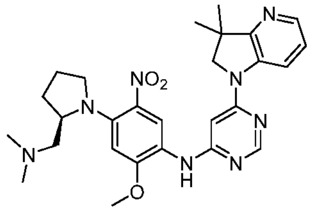 | (R)-6-(3,3-dimethyl-2,3 - dihydro-1*H*-pyrrolo[3,2-*b*] pyridin-1-yl)-*N*-(4-(2-((dimethylamino)methyl)py rrolidin-1-yl)-2-methoxy-5-nitrophenyl)pyrimidin-4-amine | 519.2 |
| **D52** | 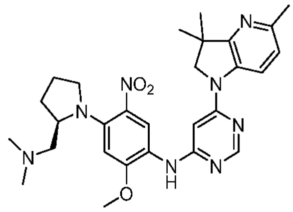 | (R)-6-(3,3,5-trimethyl-2,3-dihydro-1*H*-pyrrolo[3,2-*b*] pyridin-1-yl)-*N*-(4-(2-((dimethylamino)methyl)py rrolidin-1-yl)-2-methoxy-5-nitrophenyl)pyrimidin-4-amine | 533.2 |

**Intermediate D53-1: Preparation of *N*-(4-fluoro-2-methoxy-5-nitrophenyl)-4-(3,3,5-trimethyl-2,3-dihydro-1*H*-pyrrolo[3,2-*b*]pyridin-1-yl)-1,3,5-triazin-2-amine**

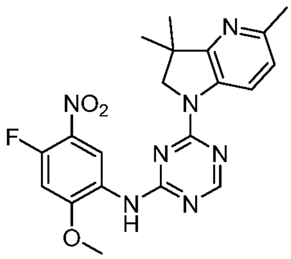

**Step 1: Synthesis of 4-chloro-*N*-(4-fluoro-2-methoxy-5-nitrophenyl)-1,3,5-triazin-2-amine**

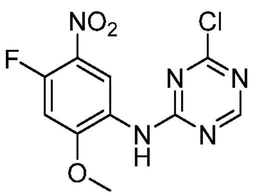

At room temperature, 2,4-dichloro-1,3,5-triazine (9.67 g, 64.46 mmol) was dissolved in dichloromethane (150 mL), and 4-fluoro-2-methoxy-5-nitroaniline (10 g, 53.72 mmol) and *N*,*N*-diisopropylethylamine (13.86 g, 107.44 mmol) were added in sequence. A resulting reaction mixture was stirred for 1 hr at room temperature. After the reaction was

completed, the solvent was removed to obtain a crude product, dichloromethane (80 mL) was added, and the resulting mixture was stirred for 30 minutes and then filtered. The obtained filter case was washed twice with dichloromethane, and dried to obtain 4-chloro-N (4-fluoro-2-methoxy-5-nitrophenyl)-1,3,5-triazin-2-amine (10.15 g, yield: 63%), ESI-MS: 300.1 [M+1]$^+$.

**Step 2: Synthesis of *N*-(4-fluoro-2-methoxy-5-nitrophenyl)-4-(3,3,5-trimethyl-2,3-dihydro-1*H*-pyrrolo[3,2-*b*]pyridin-1-yl)-1,3,5-triazin-2-amine**

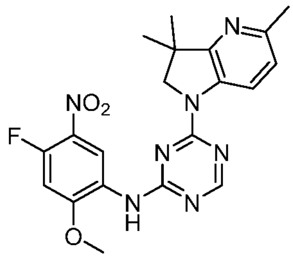

4-chloro-*N*-(4-fluoro-2-methoxy-5-nitrophenyl)-1,3,5-triazin-2-amine (185 g, 0.63 mmol, 1 eq.) and p-toluenesulfonate monohydrate (143 mg, 0.75 mmol) were added to the solution of 3,3,5-trimethyl-2,3-dihydro-1*H*-pyrrolo[3,2-*b*]pyridine (100 mg, 0.63 mol) in 1,4-dioxane (20 mL). The reaction mixture was stirred for 2 hrs at 120 °C. Stratification was conducted with dichloromethane and water. The organic phase was washed with water and saturated sodium chloride in sequence, then dried over anhydrous sodium sulfate, filtered, and concentrated to obtain *N*-(4-fluoro-2-methoxy-5-nitrophenyl)-4-(3,3,5-trimethyl-2,3-dihydro-1*H*-pyrrolo[3,2-b]pyridin-1-yl)-1,3,5-triazin-2-amine crude product, which was directly used in the next step. ESI-MS: 426.2 [M+1]$^+$.

**Intermediates D54-1 to 64-1 were prepared according to the synthesis method for Intermediate D53-1:**

| Intermediate No. | Structural Formula | Chemical Name | ESI-MS: [M+1]$^+$ |
|---|---|---|---|
| **D54-1** | 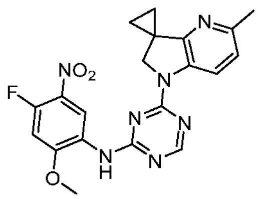 | *N*-(4-fluoro-2-methoxy-5-nitrophenyl)-4-(5'-methylspiro[cyclopropane-1,3'-pyrrolo[3,2-*b*]pyridin]-1'(2'*H*)-yl)-1,3,5-triazin-2-amine | 424.0 |
| **D55-1** | 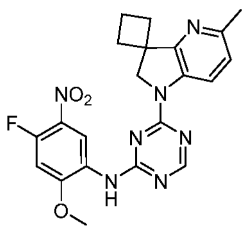 | *N*-(4-fluoro-2-methoxy-5-nitrophenyl)-4-(5'-methylspiro[cyclobutane-1,3'-pyrrolo[3,2-*b*]pyridin]-1'(2'*H*)-yl)-1,3,5-triazin-2-amine | 438.0 |

(continued)

| Intermediate No. | Structural Formula | Chemical Name | ESI-MS: [M+1]$^+$ |
|---|---|---|---|
| D56-1 | 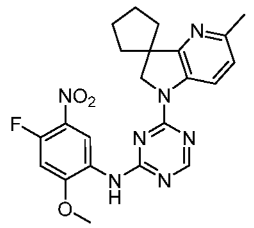 | *N*-(4-fluoro-2-methoxy-5-nitrophenyl)-4-(5'-methylspiro[cyclopentane-1,3'-pyrrolo[3,2-*b*]pyridin]-1'(2'*H*)-yl)-1,3,5-triazin-2-amine | 452.0 |
| D57-1 | 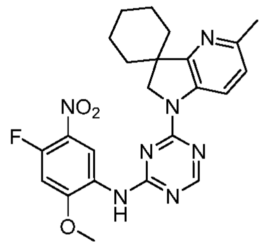 | *N*-(4-fluoro-2-methoxy-5-nitrophenyl)-4-(5'-methylspiro[cyclohexane-1,3'-pyrrolo[3,2-*b*]pyridin]-1'(2'*H*)-yl)-1,3,5-triazin-2-amine | 466.2 |
| D58-1 | 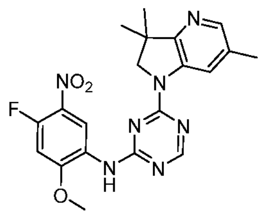 | *N*-(4-fluoro-2-methoxy-5-nitrophenyl)-4-(3,3,6-trimethyl-2,3-dihydro-1*H*-pyrrolo[3,2-*b*]pyridin-1-yl)-1,3,5-triazin-2-amine | 426.0 |
| D59-1 | 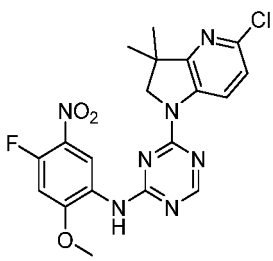 | 4-(5-chloro-3,3-dimethyl-2,3-dihydro-1*H*-pyrrolo[3,2-*b*]pyridin-1-yl)-*N*-(4-fluoro-2-methoxy-5-nitrophenyl)-1,3,5-triazin-2-amine | 446.2 |
| D60-1 | 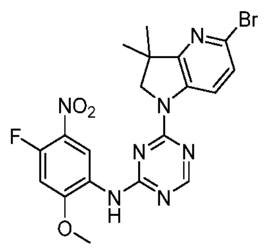 | 4-(5-bromo-3,3-dimethyl-2,3-dihydro-1*H*-pyrrolo[3,2-*b*]pyridin-1-yl)-*N*-(4-fluoro-2-methoxy-5-nitrophenyl)-1,3,5-triazin-2-amine | 490.2 492.2 |
| D61-1 | 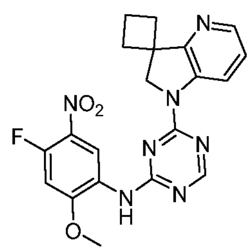 | *N*-(4-fluoro-2-methoxy-5-nitrophenyl)-4-(spiro[cyclobutane-1,3'-pyrrolo[3,2-*b*]pyridin]-1'(2'*H*)-yl)-1,3,5-triazin-2-amine | 424.2 |

(continued)

| Intermediate No. | Structural Formula | Chemical Name | ESI-MS: [M+1]+ |
|---|---|---|---|
| **D62-1** | 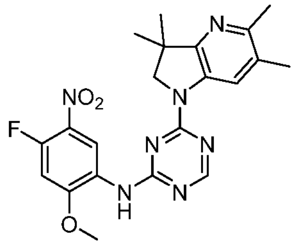 | *N*-(4-fluoro-2-methoxy-5-nitrophenyl)-4-(3,3,5,6-tetramethyl-2,3-dihydro-1*H*-pyrrolo[3,2-*b*]pyridin-1-yl)-1,3,5-triazin-2-amine | 440.2 |
| **D63-1** | 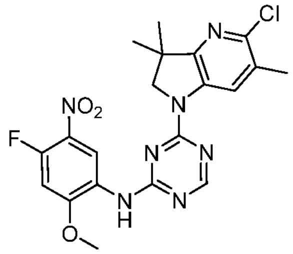 | *N*-(4-fluoro-2-methoxy-5-nitrophenyl)-4-(3,3,5,6-tetramethyl-2,3-dihydro-1*H*-pyrrolo[3,2-*b*]pyridin-1-yl)-1,3,5-triazin-2-amine | 460.1 |
| **D64-1** | 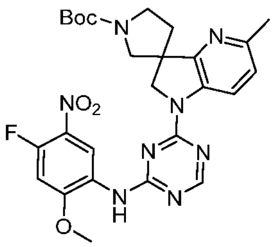 | tert-butyl 1'-(4-((4-fluoro-2-methoxy-5-nitrophenyl) amino)-1,3,5-triazin-2-yl)-5'-methyl-1',2'-dihydrospiro [pyrrolidin-3,3'-pyrrolo[3,2-*b*]pyridin]-1-carboxylate | 553.2 |

**Intermediate D53: Preparation of *N*1-(2-(dimethylamino)ethyl)-5-methoxy-*N*1-methyl-2-nitro-*N*4-(4-(3,3,5-trimethyl-2,3-dihydro-1*H*-pyrrolo[3,2-*b*]pyridin-1-yl)-1,3,5-triazin-2-yl)benzene-1,4-diamine**

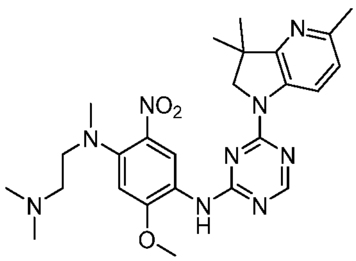

Dimethyl[2-(methylamino)ethyl]amine (95 mg, 0.95 mmol) and diisopropylethylamine (82 mg, 0.95 mmol) were added to the solution of the crude *N*-(4-fluoro-2-methoxy-5-nitrophenyl)-4-(3,3,5-trimethyl-2,3-dihydro-1*H*-pyrrolo[3,2-b]pyridin-1-yl)-1,3,5-triazin-2-amine in 1,4-dioxane (100 mL). The reaction mixture was stirred for 18 hrs at 120 °C. Stratification was conducted with dichloromethane and water. The organic phase was washed with water and saturated sodium chloride in sequence, then dried over anhydrous sodium sulfate, filtered, concentrated and then separated by column chromatography [dichloromethane:methanol=10:1] to obtain *N*1-(2-(dimethylamino)ethyl)-5-methoxy-*N*1-methyl-2-nitro-*N*4-(4-(3,3,5-trimethyl-2,3-dihydro-1*H*-pyrrolo[3,2-b]pyridin-1-yl)-1,3,5-triazin-2-yl)benzene-1,4-diamine (130 mg). ESI-MS: 508.2 $[M+1]^+$.

**Intermediates D54-D63 and D64-2 were prepared according to the synthesis method for Intermediate D53:**

| Intermediate No. | Structural Formula | Chemical Name | ESI-MS: $[M+1]^+$ |
|---|---|---|---|
| D54 | 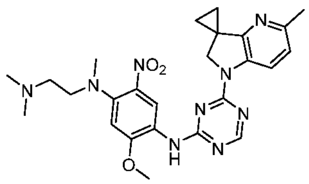 | $N^1$-(2-(dimethylamino)ethyl)-5-methoxy-$N^1$-methyl-$N^4$-(4-(5'-methylspiro[cyclopropane-1,3'-pyrrolo[3,2-b]pyridin]-1'(2'*H*)-yl)-1,3,5-triazin-2-yl)-2-nitrobenzene-1,4-diamine | 506.2 |
| D55 | 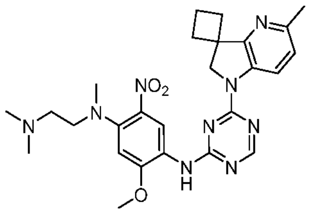 | $N^1$-(2-(dimethylamino)ethyl)-5-methoxy-$N^1$-methyl-$N^4$-(4-(5'-methylspiro[cyclobutane-1,3'-pyrrolo[3,2-*b*]pyridin]-1'(2'*H*)-yl)-1,3,5-triazin-2-yl)-2-nitrobenzene-1,4-diamine | 520.2 |
| D56 | 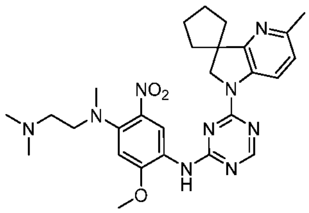 | $N^1$-(2-(dimethylamino)ethyl)-5-methoxy-$N^1$-methyl-$N^4$-(4-(5'-methylspiro[cyclopentane-1,3'-pyrrolo[3,2-*b*]pyridin]-1'(2'*H*)-yl)-1,3,5-triazin-2-yl)-2-nitrobenzene-1,4-diamine | 534.2 |
| D57 | 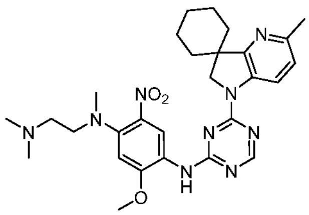 | $N^1$-(2-(dimethylamino)ethyl)-5-methoxy-$N^1$-methyl-$N^4$-(4-(5'-methylspiro[cyclohexane-1,3'-pyrrolo[3,2-*b*]pyridin]-1'(2'*H*)-yl)-1,3,5-triazin-2-yl)-2-nitrobenzene-1,4-diamine | 548.4 |
| D58 | 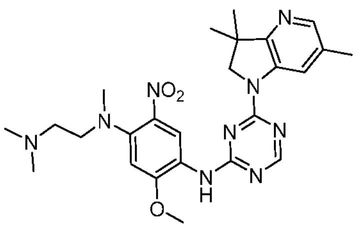 | $N^1$-(2-(dimethylamino)ethyl)-5-methoxy-$N^1$-methyl-2-nitro-$N^4$-(4-(3,3,6-trimethyl-2,3-dihydro-1*H*-pyrrolo[3,2-*b*]pyridin-1-yl)-1,3,5-triazin-2-yl)benzene-1,4-diamine | 508.2 |
| D59 | 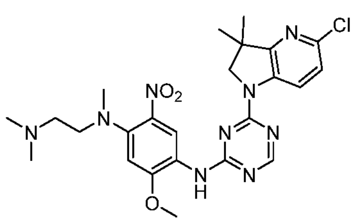 | $N^1$-(4-(5-chloro-3,3 -dimethyl-2,3-dihydro-1*H*-pyrrolo[3,2-*b*]pyridin-1-yl)-1,3,5-triazin-2-yl)-$N^4$-(2-(dimethylamino)ethyl)-2-methoxy-$N^4$-methyl-5-nitrobenzene-1,4-diamine | 528.2 |

(continued)

| Intermediate No. | Structural Formula | Chemical Name | ESI-MS: [M+1]+ |
|---|---|---|---|
| **D60** | 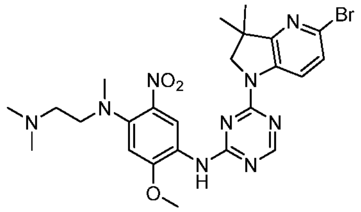 | $N^1$-(4-(5-bromo-3,3-dimethyl-2,3-dihydro-1*H*-pyrrolo[3,2-*b*]pyridin-1-yl)-1,3,5-triazin-2-yl)-$N^4$-(2-(dimethylamino)ethyl)-2-methoxy-$N^4$-methyl-5-nitrobenzene-1,4-diamine | 572.0 574.2 |
| **D61** | 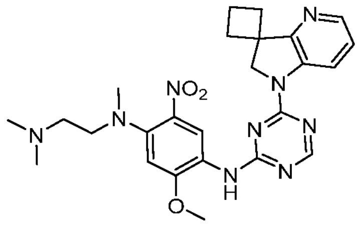 | *N*-(4-fluoro-2-methoxy-5-nitrophenyl)-4-(spiro [cyclobutane-1,3'-pyrrolo[3,2-*b*]pyridin]-1' (2'*H*)-yl)-1,3,5-triazin-2-amine | 506.4 |
| **D62** | 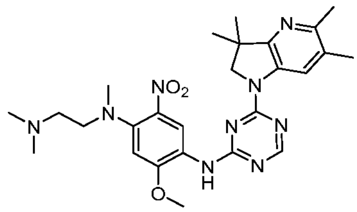 | $N^1$-[2-(dimethylamino)ethyl]-5-methoxy-$N^1$-methyl-2-nitro-$N^4$-(4-(3,3,5,6-tetramethyl-2,3-dihydro-1*H*-pyrrolo[3,2-*b*]pyridin-1-yl)-1,3,5-triazin-2-yl)benzene-1,4-diamine | 522.2 |
| **D63** | 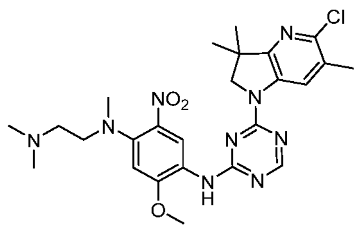 | $N^4$-(4-(5-chloro-3,3,6-trimethyl-2,3-dihydro-1*H*-pyrrolo[3,2-*b*]pyridin-1-yl)-1,3,5-triazizzzyy-2-yl)-$N^1$-[2-(dimethylamino)ethyl]-5-methoxy-$N^1$-methyl-2-nitrobenzene-1,4-diamine | 542.3 |
| **D64-2** | 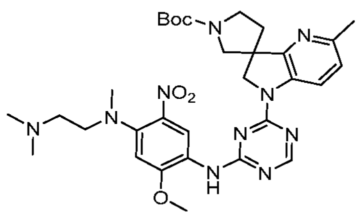 | tert-butyl 1'-(4-((4-((2-(dimethylamino)ethyl) (methyl )amino)-2-methoxy-5-nitrophenyl) amino)-1,3,5-triazin-2-yl)-5'-methyl-1',2'-dihydrospiro[pyrrolidin-3,3'-pyrrolo[3,2-*b*] pyridin]-1-carboxylate | 635.4 |

**Intermediate D47: Preparation of $N^1$-(2-(dimethylamino)ethyl)-$N^4$-(4-(1,5'-dimethylspiro[pyrrolidin-3,3'-pyrrolo[3,2-*b*]pyridin]-1'(2'*H*)-yl)pyrimidin-2-yl)-5-methoxy-$N^1$-methyl-2-nitrobenzene-1,4-diamine**

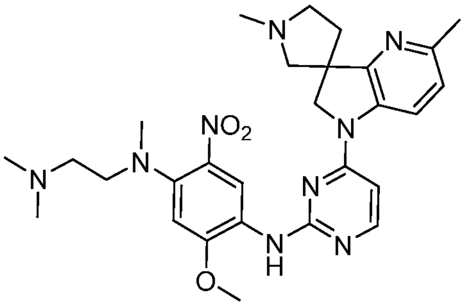

Tert-butyl 1'-(2-((4-((2-(dimethylamino)ethyl)(methyl)amino)-2-methoxy-5-nitrophenyl)amino)pyrimidin-4-yl)-5'-methyl-1',2'-dihydrospiro[pyrrolidin-3,3'-pyrrolo[3,2-*b*]pyridin]-1-carboxylate (50 mg, 0.079 mmol), trifluoroacetic acid (3 mL) was dissolved in dichloromethane (10 mL). The reaction mixture was stirred for 30 minutes at room temperature. After the reaction was completed, the reaction mixture was concentrated to dryness, and the residue was dissolved in methanol (3 mL) and water (20 mL). The aqueous solution of formaldehyde (5 mL) and diisopropylethylamine (51 mg, 0.39 mmol) were added to the reaction mixture, which was then stirred for 10 minutes, and sodium cyanoborohydride (50 mg, 0.80 mmol) was added. The reaction mixture was stirred for 30 minutes at room temperature. After the reaction was completed, the reaction mixture was stratified and extracted with dichloromethane (50 mL) and water (50 mL). The organic phase was dried and concentrated to obtain *N*1-(2-(dimethylamino)ethyl)-*N*4-(4-(1,5'-dimethylspiro[pyrrolidin-3,3'-pyrrolo[3,2-*b*]pyridin]-1'(2'*H*)-yl)pyrimidin-2-yl)-5-methoxy-*N*1-methyl-2-nitrobenzene-1,4-diamine (40 mg, 0.073 mmol, yield: 92.57%). ESI-MS:518.2 $[M+1]^+$.

**Intermediates D48 and D64 were prepared according to the synthesis method for Intermediate D47:**

| Intermediate No. | Structural Formula | Chemical Name | ESI-MS: $[M+1]^+$ |
|---|---|---|---|
| D48 | 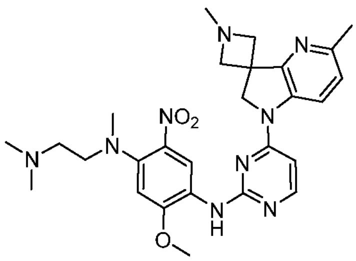 | *N*1-(2-(dimethylamino)ethyl)-*N*4-(4-(1,5'-dimethylspiro[azetidin-3,3'-pyrrolo[3,2-*b*] pyridin]-1'(2'*H*)-yl)pyrimidin-2-yl)-5-methoxy-*N*1-methyl-2-nitrobenzene-1,4-diamine | 534.3 |
| D64 | 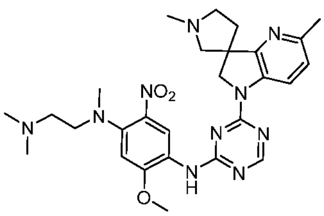 | *N*1-(2-(dimethylamino)ethyl)-*N*4-(4-(1,5'-dimethylspiro[pyrrolidin-3,3'-pyrrolo[3,2-*b*] pyridin]-1'(2'*H*)-yl)-1,3,5-triazin-2-yl)-5-methoxy-*N*1-methyl-2-nitrobenzene-1,4-diamine | 549.2 |

**Intermediate D65: Preparation of 2-(difluoromethoxy)-*N*4-(2-(dimethylamino)ethyl)-*N*4-methyl-5-nitro-*N*1-(4-(3,3,5-trimethyl-2,3-dihydro-1*H*-pyrrolo[3,2-b]pyridin-1-yl)-1,3,5-triazin-2-yl)benzene-1,4-diamine**

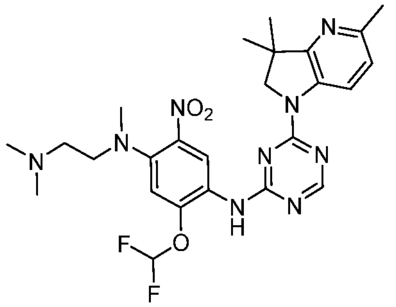

5-(difluoromethoxy)-$N^1$-(2-(dimethylamino)ethyl)-$N^1$-methyl-2-nitrobenzene-1,4-diamine (166 mg, 0.54 mmol) and p-toluenesulfonate monohydrate (125 mg, 0.54 mmol) were added to the solution of 1-(4-chloro-1,3,5-triazin-2-yl)-3,3,5-trimethyl-2,3-dihydro-1*H*-pyrrolo[3,2-b]pyridine (150 mg, 0.54 mmol) in 1,4-dioxane (10 mL). The reaction mixture was stirred for 2 hrs at 120 °C. Stratification was conducted with ethyl acetate and the aqueous solution of saturated sodium bicarbonate. The organic phase was washed with water and saturated sodium chloride in sequence, then dried over anhydrous sodium sulfate, filtered, concentrated, and then separated by silicagel column chromatography [dichloromethane:methanol=10:1] to obtain 2-(difluoromethoxy)-$N^4$-(2-(dimethylamino)ethyl)-$N^4$-methyl-5-nitro-$N^1$-(4-(3,3,5-trimethyl-2,3-dihydro-1*H*-pyrrolo[3,2-b]pyridin-1-yl)-1,3,5-triazin-2-yl)benzene-1,4-diamine (200 mg, yield: 67%). ESI-MS: 544.1 $[M+1]^+$.

**Intermediates D66 were prepared according to the synthesis method for Intermediate D65:**

| Intermediate No. | Structural Formula | Chemical Name | ESI-MS: $[M+1]^+$ |
|---|---|---|---|
| **D66** | 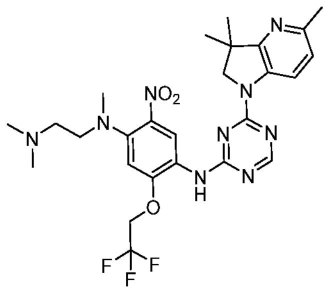 | $N^1$-(2-(dimethylamino)ethyl)-$N^1$-methyl-2-nitro-5-(2,2,2-trifluoroethoxy)-$N^4$-(4-(3,3,5-trimethyl-2,3-dihydro-1*H*-pyrrolo[3,2-*b*]pyridin-1-yl)-1,3,5-triazin-2-yl)benzene-1,4-diamine | 576.1 |

**Intermediate D67: Preparation of *N*[1]-(2-(dimethylamino)ethyl)-5-ethoxy-*N*[1]-methyl-2-nitro-*N*[4]-(4-(3,3,5-trimethyl-2,3-dihydro-1*H*-pyrrolo[3,2-*b*]pyridin-1-yl)-1,3,5-triazin-2-yl)benzene-1,4-diamine**

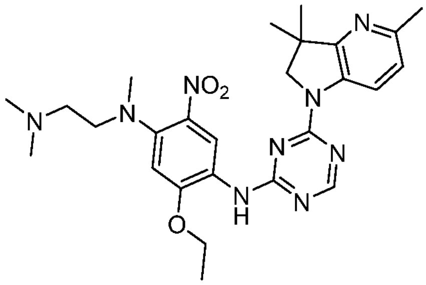

$N^1$-(2-(dimethylamino)ethyl)-5-ethoxy-$N^1$-methyl-2-nitrobenzene-1,4-diamine (153.6 mg, 0.54 mmol) and trifluoroacetic acid (124 mg, 1.09 mmol) were added to the solution of 1-(4-chloro-1,3,5-triazin-2-yl)-3,3,5-trimethyl-2,3-

dihydro-1*H*-pyrrolo[3,2-b]pyridine (150 mg, 0.54 mmol) in n-butanol (10 mL). The reaction mixture was stirred for 2 hrs at 120 °C. Stratification was conducted with ethyl acetate and the aqueous solution of saturated sodium bicarbonate. The organic phase was washed with water and saturated sodium chloride in sequence, then dried over anhydrous sodium sulfate, filtered, concentrated, and then separated by silicagel column chromatography [dichloromethane: methanol=10:1] to obtain *N*1-(2-(dimethylamino)ethyl)-5-ethoxy-*N*1-methyl-2-nitro-*N*4-(4-(3,3,5-trimethyl-2,3-dihydro-1*H*-pyrrolo[3,2-*b*]pyridin-1-yl)-1,3,5-triazin-2-yl)benzene-1,4-diamine (220 mg, yield: 62.8%). ESI-MS: 522.3 $[M+1]^+$.

**Intermediates D68-D70 were prepared according to the synthesis method for Intermediate D67:**

| Intermediate No. | Structural Formula | Chemical Name | ESI-MS: $[M+1]^+$ |
|---|---|---|---|
| **D68** | 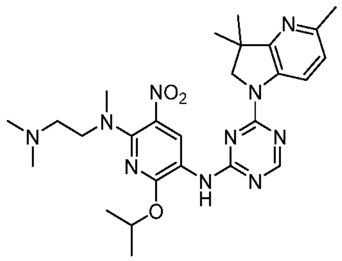 | *N*2-(2-(dimethylamino)ethyl)-6-isopropoxy-*N*2-methyl-3-nitro-*N*5-(4-(3,3,5-trimethyl-2,3-dihydro-1*H*-pyrrolo[3,2-*b*]pyridin-1-yl)-1,3,5-triazin-2-yl)pyridin-2,5-diamine | 537.2 |
| **D69** | 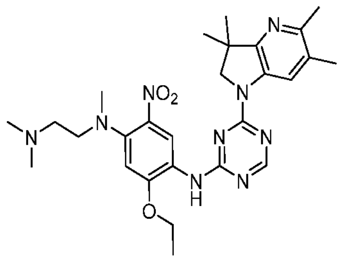 | *N*1-[2-(dimethylamino)ethyl]-5-ethoxy-*N*1-methyl-2-nitro-*N*4-(4-(3,3,5,6-tetramethyl-2,3-dihydro-1*H*-pyrrolo[3,2-*b*]pyridin-1-yl)-1,3,5-triazin-2-yl) benzene-1,4-diamine | 536.3 |
| D70 | 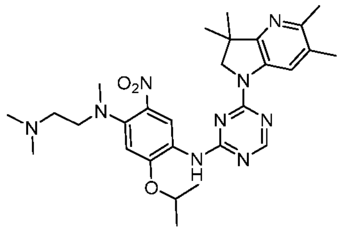 | *N*1-[2-(dimethylamino)ethyl]-*N*1-methyl-2-nitro-5-(propane-2-oxy)-*N*4-(4-(3,3,5,6-tetramethyl-2,3-dihydro-1*H*-pyrrolo[3,2-*b*]pyridin-1-yl)-1,3,5-triazin-2-yl)benzene-1,4-diamine | 549.3 |

**Intermediate D71: Preparation of *N*1-(4-(5-bromo-3,3-dimethyl-2,3-dihydro-1*H*-pyrrolo[3,2-*b*]pyridin-1-yl)pyrimidin-2-yl)-*N*4-(2-(dimethylamino)ethyl)-2-methoxy-*N*4-methyl-5-nitrobenzene-1,4-diamine**

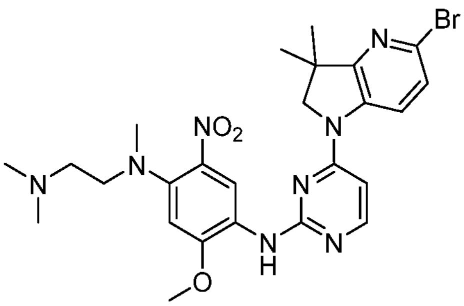

*N*-(4-((2-(dimethylamino)ethyl)(methyl)amino)-2-methoxy-5-nitrophenyl)formamide (309.28mg, 1.04 mmol) was dissolved in *N*,*N*-dimethylacetamide (20 mL). Sodium hydride (62 mg, 1.57 mmol) was added to the reaction mixture at 0 °C. The reaction mixture was stirred for 20 minutes at room temperature. 5-bromo-3,3-dimethyl-1-(2-(methylsulfonyl)pyrimidin-4-yl)-2,3-dihydro-1*H*-pyrrolo[3,2-*b*]pyridine (400mg, 1.04 mmol) was added, and the reaction mixture was continuously stirred for 2 hrs. Little water was added to the reaction mixture, which was then stirred for 1 hr. After the reaction was completed, stratification was conducted with ethyl acetate and water; the organic phase was washed with water and saturated sodium chloride in sequence, then dried over anhydrous sodium sulfate, filtered, concentrated, and then separated by silicagel column chromatography [petroleum ether: ethyl acetate=4:1] to obtain *N*$^1$-(4-(5-bromo-3,3-dimethyl-2,3-dihydro-1*H*-pyrrolo[3,2-*b*]pyridin-1-yl)pyrimidin-2-yl)-*N*$^4$-(2-(dimethylamino)ethyl)-2-methoxy-*N*$^4$-methyl-5-nitrobenzene-1,4-diamine (543 mg, yield: 91.2%). ESI-MS: 571.2, 573.2 $[M+1]^+$.

**Intermediates D72 were prepared according to the synthesis method for Intermediate D71**

| Intermediate No. | Structural Formula | Chemical Name | ESI-MS: $[M+1]^+$ |
|---|---|---|---|
| D72 | 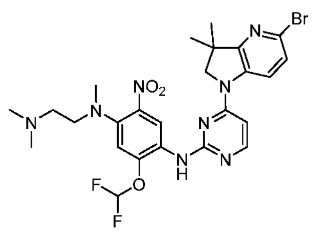 | *N*$^1$-(4-(5-bromo-3,3-dimethyl-2,3-dihydro-1*H*-pyrrolo [3,2-*b*]pyridin-1-yl)pyrimidin-2-yl)-2-(difluoromethoxy)-*N*$^4$-(2-(dimethylamino) ethyl)-*N*$^4$-methyl-5-nitrobenzene-1,4-diamine | 607.2 609.2 |

**Intermediate E1: Preparation of *N*$^4$-(4-(3,3-dimethyl-2,3-dihydro-1*H*-pyrrolo[3,2-*b*]pyridin-1-yl)pyridin-2-yl)-*N*$^1$-(2-(dimethylamino)ethyl)-5-methoxy-*N*$^1$-methylbenzene-1,2,4-triamine**

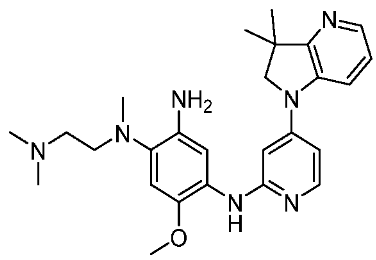

*N*$^1$-(4-(3,3-dimethyl-2,3-dihydro-1H-pyrrolo[3,2-*b*]pyridin-1-yl)pyridin-2-yl)-*N*$^4$-(2-(dimethylamino)ethyl)-2-methoxy-*N*$^4$-methyl-5-nitrobenzene-1,4-diamine (335 mg, 0.68 mmol) was dissolved in the mixed solvent of 20 mL/10mL of ethanol/water. At room temperature, iron powder (228 mg, 4.08 mmol) and ammonium chloride (228 mg, 4.08 mmol) were added to the mixture. The reaction mixture was stirred for 3 hrs at 90 °C. Water was added to the solution, which was extracted with dichloromethane three times. The organic phases were combined, then washed with saturated saline, and dried over anhydrous sodium sulfate. The solvent was removed to subsequently obtain *N*$^4$-(4-(3,3-dimethyl-2,3-dihydro-1H-pyrrolo[3,2-*b*]pyridin-1-yl)pyridin-2-yl)-*N*$^1$-(2-(dimethylamino)ethyl)-5-methoxy-*N*$^1$-methylbenzene-1,2,4-triamine (205 mg, yield: 65%). ESI-MS: 462.4 $[M+1]^+$.

**Intermediates E2-E29 were prepared according to the synthesis method for Intermediate E1:**

| Intermediate No. | Structural Formula | Chemical Name | ESI-MS: [M+1]$^+$ |
|---|---|---|---|
| **E2** | 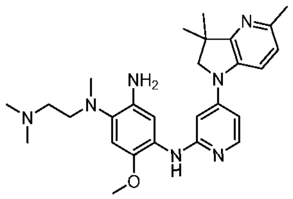 | *N*1-(2-(dimethylamino)ethyl)-5-methoxy-*N*1-methyl-*N*4-(4-(3,3, 5-trimethyl-2,3-dihydro-1*H*-pyrrolo[3,2-*b*]pyridin-1-yl)pyridin-2-yl) benzene-1,2,4-triamine | 476.4 |
| **E3** | 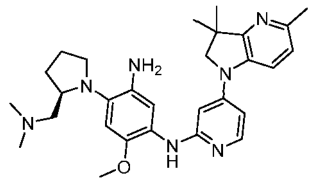 | (R)-4-(2-((dimethylamino)methyl)py rrolidin-1-yl)-6-methoxy-*N*1-(4-(3,3,5-trimethyl-2,3-dihydro-1*H*-pyrrolo[3,2-*b*]pyridin-1-yl)pyridin-2-yl) benzene-1,3 -diamine | 502.2 |
| **E4** | 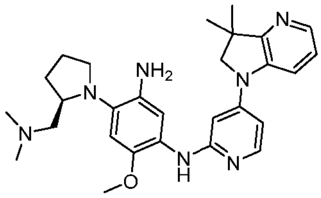 | (R)-*N*1-(4-(3,3-dimethyl-2,3-dihydro-1*H*-pyrrolo [3,2-*b*]pyridin-1-yl)pyridin-2-yl)-4-(2-((dimethylamino)methyl)py rrolidin-1-yl)-6-methoxybenzene-1,3-diamine | 488.2 |
| **E5** | 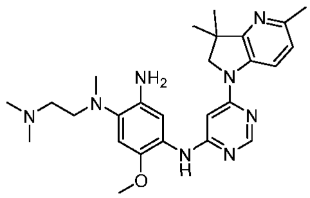 | *N*1-(2-(dimethylamino)ethyl)-5-methoxy-*N*1-methyl-*N*4-(6-(3,3, 5-trimethyl-2,3-dihydro-1*H*-pyrrolo[3,2-*b*]pyridin-1-yl)pyrimidin-4-yl) benzene-1,2,4-triamine | 477.2 |
| **E6** | 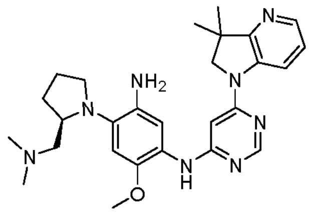 | (R)-*N*1-(6-(3,3-dimethyl-2,3-dihydro-1*H*-pyrrolo [3,2-*b*]pyridin-1-yl)pyrimidin-4-yl)-4-(2-((dimethylamino)methyl)py rrolidin-1-yl)-6-methoxybenzene-1,3-diamine | 489.2 |
| **E7** | 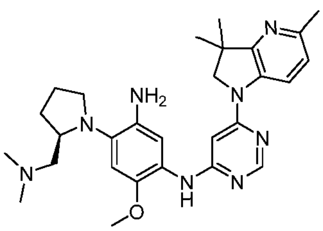 | (R)-*N*1-(6-(3,3,5-trimethyl-2,3-dihydro-1*H*-pyrrolo [3,2-*b*]pyridin-1-yl)pyrimidin-4-yl)-4-(2-((dimethylamino)methyl)py rrolidin-1-yl)-6-methoxybenzene-1,3-diamine | 503.2 |
| **E8** | 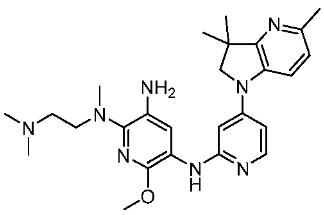 | *N*2-(2-(dimethylamino)ethyl)-6-methoxy-*N*2-methyl-*N*5-(4-(3,3, 5-trimethyl-2,3-dihydro-1*H*-pyrrolo[3,2-*b*]pyridin-1-yl)pyridin-2-yl)pyridin-2,3, 5-triamine | 477.2 |

(continued)

| Intermediate No. | Structural Formula | Chemical Name | ESI-MS: [M+1]+ |
|---|---|---|---|
| E9 | 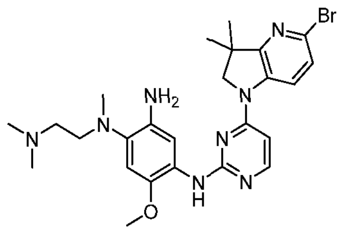 | $N^4$-(4-(5-bromo-3,3-dimethyl-2,3-dihydro-1*H*-pyrrolo[3,2-*b*]pyridin-1-yl)pyrimidin-2-yl)-$N^1$-(2-(dimethylamino)ethyl)-5-methoxy-$N^1$-methylbenzene-1,2,4-triamine | 541.2 543.2 |
| E10 | 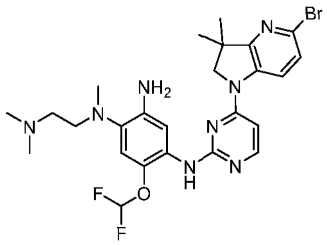 | $N^4$-(4-(5-bromo-3,3-dimethyl-2,3-dihydro-1*H*-pyrrolo[3,2-*b*]pyridin-1-yl)pyrimidin-2-yl)-5-(difluoromethoxy)-$N^1$-(2-(dimethylamino)ethyl)-$N^1$-methylbenzene-1,2,4-triamine | 577.2 579.2 |
| E11 | 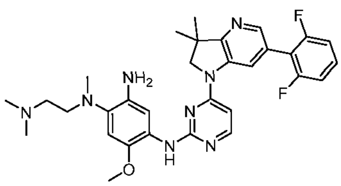 | $N^4$-(4-(6-(2,6-difluorophenyl)-3,3-dimethyl-2,3-dihydro-1*H*-pyrrolo[3,2-*b*]pyridin-1-yl)pyrimidin-2-yl)-$N^1$-(2-(dimethylamino)ethyl)-5-methoxy-$N^1$-methylbenzene-1,2,4-triamine | 575.3 |
| E12 | 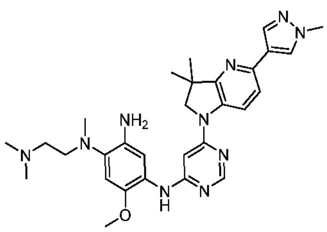 | $N^4$-(6-(3,3-dimethyl-5-(1-methyl-1*H*-pyrazol-4-yl)-2,3-dihydro-1*H*-pyrrolo[3,2-*b*]pyridin-1-yl)pyrimidin-4-yl)-$N^1$-(2-(dimethylamino)ethyl)-5-methoxy-$N^1$-methylbenzene-1,2,4-triamine | 543.2 |
| E13 | 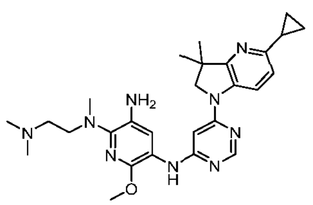 | $N^5$-(6-(5-cyclopropyl-3,3-dimethyl-2,3-dihydro-1*H*-pyrrolo[3,2-*b*]pyridin-1-yl)pyrimidin-4-yl)-$N^2$-(2-(dimethylamino)ethyl)-6-methoxy-$N^4$-methylpyridin-2,3,5-triamine | 504.4 |
| E14 | 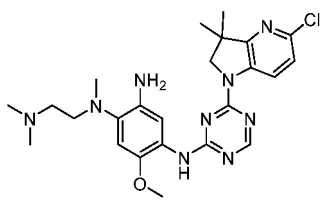 | $N^4$-(4-(5-chloro-3,3-dimethyl-2,3-dihydro-1*H*-pyrrolo[3,2-*b*]pyridin-1-yl)-1,3,5-triazin-2-yl)-$N^1$-(2-(dimethylamino)ethyl)-5-methoxy-$N^1$-methylbenzene-1,2,4-triamine | 498.2 |
| E15 | 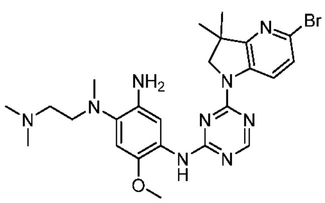 | $N^4$-(4-(5-bromo-3,3-dimethyl-2,3-dihydro-1*H*-pyrrolo[3,2-*b*]pyridin-1-yl)-1,3,5-triazin-2-yl)-$N^1$-(2-(dimethylamino)ethyl)-5-methoxy-$N^1$-methylbenzene-1,2,4-triamine | 542.2 544.2 |

(continued)

| Intermediate No. | Structural Formula | Chemical Name | ESI-MS: [M+1]+ |
|---|---|---|---|
| **E16** | 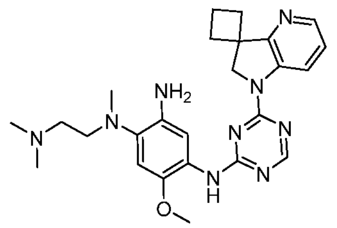 | *N*1-(2-(dimethylamino)ethyl)-5-methoxy-*N*1-methyl-*N*4-(4-(spiro[cyclobutane-1,3'-pyrrolo [3,2-*b*]pyridin]-1'(2'*H*)-yl)-1,3,5-triazin-2-yl) benzene-1,2,4-triamine | 476.2 |
| **E17** | 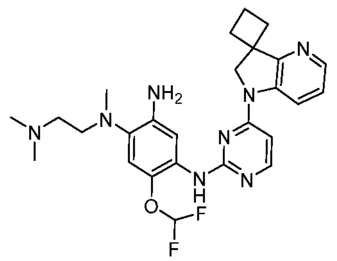 | 5-(difluoromethoxy)-*N*1-(2-(dimethylamino) ethyl)-*N*1-methyl-*N*4-(4-(spiro[cyclobutane-1,3'-pyrrolo[3,2-*b*]pyridin]-1'(2'*H*)-yl)pyrimidin-2-yl) benzene-1,2,4-triamine | 511.3 |
| **E18** | 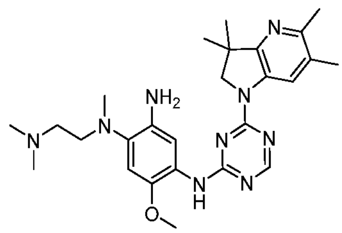 | *N*1-[2-(dimethylamino)ethyl]-5-methoxy-*N*1-methyl-*N*4-(4-(3,3,5,6-tetramethyl-2,3-dihydro-1*H*-pyrrolo[3,2-*b*]pyridin-1-yl)-1,3,5-triazin-2-yl)benzene-1,2,4-triamine | 492.2 |
| **E19** | 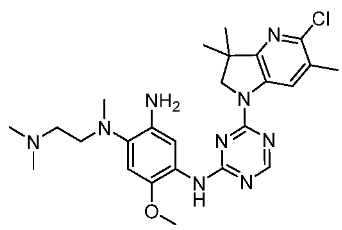 | *N*4-(4-(5-chloro-3,3,6-trimethyl-2,3-dihydro-1*H*-pyrrolo[3,2-*b*]pyridin-1-yl)-1,3,5-triazin-2-yl)-*N*1-[2-(dimethylamino)ethyl]-5-methoxy-*N*1-methylbenzene-1,2,4-triamine | 512.2 |
| **E20** | 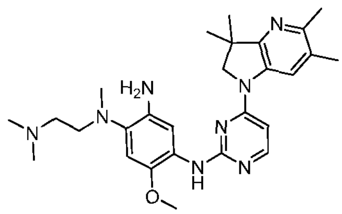 | *N*1-[2-(dimethylamino)ethyl]-5-methoxy-*N*1-methyl-*N*4-(4-(3,3,5 ,6-tetramethyl-2,3-dihydro -1*H*-pyrrolo[3,2-*b*]pyridin-1-yl)pyrimidin-2-yl)benzene-1,2,4-triamine | 491.3 |
| **E21** | 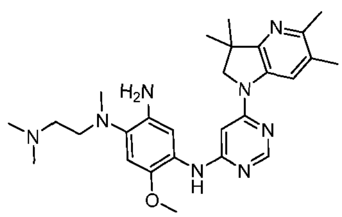 | *N*1-[2-(dimethylamino)ethyl]-5-methoxy-*N*1-methyl-*N*4-(6-(3,3,5,6-tetramethyl-2,3-dihydro-1*H*-pyrrolo[3,2-*b*]pyridin-1-yl)pyrimidin-4-yl)benzene-1,2,4-triamine | 491.2 |

(continued)

| Intermediate No. | Structural Formula | Chemical Name | ESI-MS: [M+1]+ |
|---|---|---|---|
| **E22** | 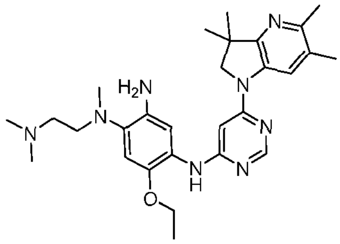 | *N*1-[2-(dimethylamino)ethyl]-5-ethoxy-*N*1-methyl-*N*4-(6-(3,3, 5, 6-tetramethyl-2, 3 - dihydro-1*H*-pyrrolo[3,2-*b*]pyridin-1-yl)pyrimidin-4-yl) benzene-1,2,4-triamine | 505.2 |
| **E23** | 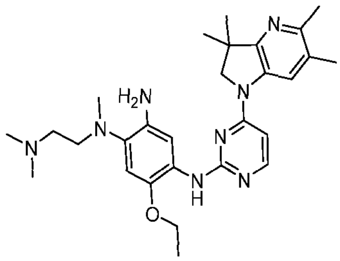 | *N*1-[2-(dimethylamino)ethyl]-5-ethoxy-*N*1-methyl-*N*4-(4-(3,3,5,6-tetramethyl-2,3-dihydro-1*H*-pyrrolo [3,2-*b*]pyridin-1-yl)pyrimidin-2-yl)benzene-1,2,4-triamine | 505.4 |
| **E24** | 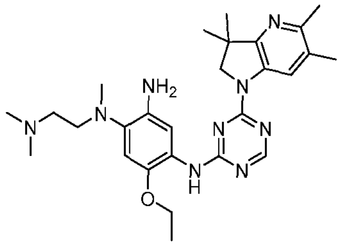 | 1-[2-(dimethylamino)ethyl]-5-ethoxy-*N*1-methyl-*N*4-(4-(3,3,5,6-tetramethyl-2,3-dihydro-1*H*-pyrrolo [3,2-*b*]pyridin-1-yl)-1,3,5-triazin-2-yl)benzene-1,2,4-triamine | 506.3 |
| **E25** | 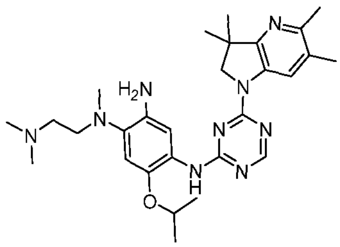 | *N*1-[2-(dimethylamino)ethyl]-*N*1-methyl-5-(propane-2-oxy)-*N*4-(4-(3,3,5,6-tetramethyl-2,3-dihydro-1*H*-pyrrolo[3,2-*b*]pyridin-1-yl)-1,3,5-triazin-2-yl)benzene-1,2,4-triamine | 520.3 |
| **E26** | 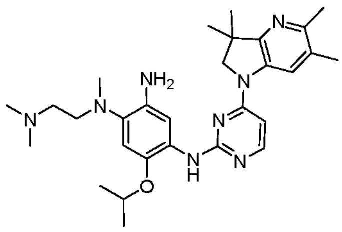 | *N*1-(2-(dimethylamino)ethyl)-5-isopropoxy-*N*1-methyl-*N*4-(4-(3,3,5,6-tetramethyl-2,3-dihydro-1*H*-pyrrolo[3,2-b]pyridin-1-yl)pyrimidin-2-yl)benzene-1,2,4-triamine | 519.3 |
| **E27** | 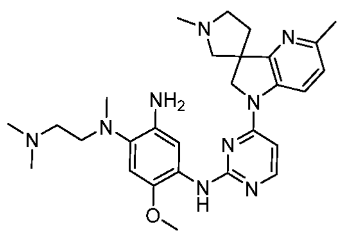 | *N*1-(2-(dimethylamino)ethyl)-*N*4-(4-(1,5'-dimethylspiro[pyrrolidin-3,3'-pyrrolo[3,2-*b*] pyridin]-1'(2'*H*)-yl)pyrimidin-2-yl)-5-methoxy-*N*1-methylbenzene-1,2,4-triamine | 518.2 |

(continued)

| Intermediate No. | Structural Formula | Chemical Name | ESI-MS: [M+1]+ |
|---|---|---|---|
| E28 | 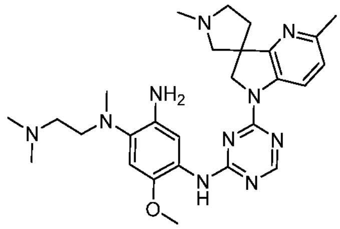 | $N^1$-(2-(dimethylamino)ethyl)-$N^4$-(4-(1,5'-dimethylspiro[pyrrolidin-3,3'-pyrrolo[3,2-*b*]pyridin]-1'(2'*H*)-yl)-1,3,5-triazin-2-yl)-5-methoxy-$N^1$-methylbenzene-1,2,4-triamine | 519.2 |
| E29 | 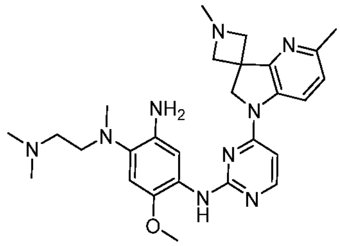 | $N^1$-(2-(dimethylamino)ethyl)-$N^4$-(4-(1,5'-dimethylspiro[azetidine-3,3'-pyrrolo[3,2-*b*]pyridin]-1'(2'*H*)-yl)pyrimidin-2-yl)-5-methoxy-$N^1$-methylbenzene-1,2,4-triamine | 504.3 |

**Intermediate E30: Preparation of *N*1-(2-(dimethylamino)ethyl)-5-methoxy-*N*1-methyl-*N*4-(4-(3,3,5-trimethyl-2,3-dihydro-1*H*-pyrrolo[3,2-*b*]pyridin-1-yl)pyrimidin-2-yl)benzene-1,2,4-triamine**

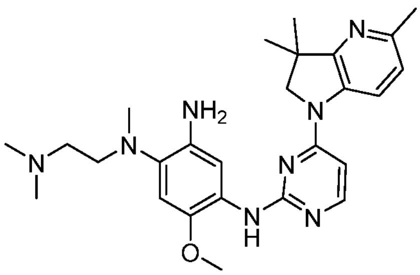

$N^1$-(2-(dimethylamino)ethyl)-5-methoxy-$N^1$-methyl-2-nitro-$N^4$-(4-(3,3,5-trimethyl-2,3-dihydro-1*H*-pyrrolo[3,2-*b*]pyridin-1-yl)pyrimidin-2-yl)benzene-1,4-diamine (201 mg, 0.40 mmol) was dissolved in 5 mL of methanol. 10% palladium on carbon (20 mg) was added to the mixture. The reaction mixture was subjected to hydrogen displacement three times, and stirred for 3 hrs at room temperature in the atmosphere of hydrogen. After the reaction was completed, the resultant was filtered with diatomite. The solvent was removed to subsequently obtain $N^1$-(2-(dimethylamino)ethyl)-5-methoxy-$N^1$-methyl-$N^4$-(4-(3,3,5-trimethyl-2,3-dihydro-1*H*-pyrrolo[3,2-*b*]pyridin-1-yl)pyrimidin-2-yl)benzene-1,2,4-triamine (150 mg, yield: 79.3%). ESI-MS: 477.3 $[M+1]^+$.

**Intermediates E31-E68 were prepared according to the synthesis method for Intermediate E30:**

| Intermediate No. | Structural Formula | Chemical Name | ESI-MS: [M+1]+ |
|---|---|---|---|
| **E31** | 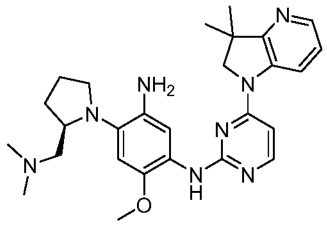 | (R)-$N^1$-(4-(3,3-dimethyl-2,3-dihydro-1*H*-pyrrolo [3,2-*b*]pyridin-1-yl)pyrimidin-2-yl)-4-(2-((dimethylamino)methyl)py rrolidin-1-yl)-6-methoxybenzene-1,3-diamine | 489.2 |
| **E32** | 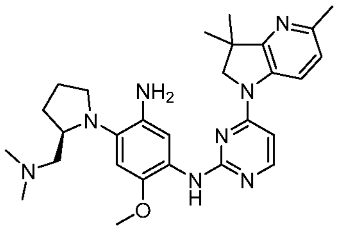 | (R)-4-(2-((dimethylamino)methyl)py rrolidin-1-yl)-6-methoxy-$N^1$-(4-(3,3,5-trimethyl-2,3-dihydro-1*H*-pyrrolo[3,2-*b*]pyridin-1-yl)pyrimidin-2-yl)benzene-1,3-diamine | 503.2 |
| **E33** | 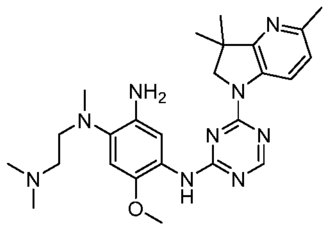 | $N^1$-(2-(dimethylamino)ethyl)-5-methoxy-$N^1$-methyl-$N^4$-(4-(3,3,5-trimethyl-2,3-dihydro-1*H*-pyrrolo[3,2-*b*]pyridin-1-yl)-1,3,5-triazin-2-yl) benzene-1,2,4-triamine | 478.2 |
| **E34** | 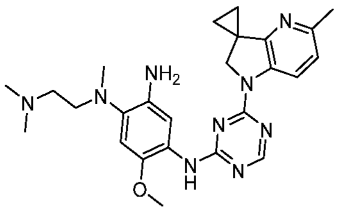 | $N^1$-(2-(dimethylamino)ethyl)-5-methoxy-$N^1$-methyl-$N^4$-(4-(5'-methylspiro[cyclopropane-1,3'-pyrrolo[3,2-*b*]pyridin]-1'(2'*H*)-yl)-1,3,5-triazin-2-yl)benzene-1,2,4-triamine | 476.2 |
| **E35** | 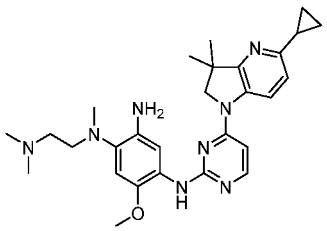 | $N^4$-(4-(5-cyclopropyl-3,3-dimethyl-2,3-dihydro-1*H*-pyrrolo[3,2-*b*]pyridin-1-yl)pyrimidin-2-yl)-$N^1$-(2-(dimethylamino)ethyl)-5-methoxy-$N^1$-methylbenzene-1,2,4-triamine | 503.3 |
| **E36** | 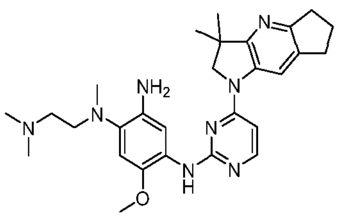 | $N^4$-(4-(3,3-dimethyl-3,5,6,7-tetrahydrocyclopenta[*b*]pyr rolo[2,3-e]pyridin-1 (2*H*)-yl)pyrimidin-2-yl)-$N^1$-(2-(dimethylamino) ethyl)-5-methoxy-$N^1$-methylbenzene-1,2,4-triamine | 503.2 |

(continued)

| Intermediate No. | Structural Formula | Chemical Name | ESI-MS: $[M+1]^+$ |
|---|---|---|---|
| **E37** | 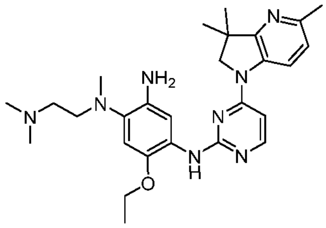 | *N*1-(2-(dimethylamino)ethyl)-5-ethoxy-*N*1-methyl-*N*1-(4-(3,3,5-trimethyl-2,3-dihydro-1*H*-pyrrolo[3,2-*b*]pyridin-1-yl)pyrimidin-2-yl) benzene-1,2,4-triamine | 491.2 |
| **E38** | 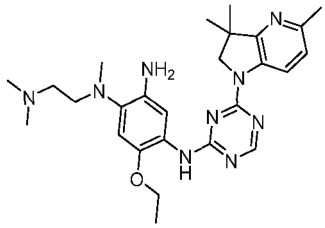 | *N*1-(2-(dimethylamino)ethyl)-5-ethoxy-*N*1-methyl-*N*4-(4-(3,3,5-trimethyl-2,3-dihydro-1*H*-pyrrolo[3,2-*b*]pyridin-1-yl)-1,3,5-triazin-2-yl) benzene-1,2,4-triamine | 492.1 |
| **E39** | 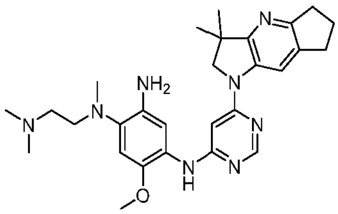 | *N*4-(6-(3,3-dimethyl-3,5,6,7-tetrahydrocyclopenta[*b*]pyr rolo[2,3-*e*]pyridin-1 (2*H*)-yl)pyrimidin-4-yl)-*N*1-(2-(dimethylamino) ethyl)-5-methoxy-*N*1-methylbenzene-1,2,4-triamine | 503.2 |
| **E40** | 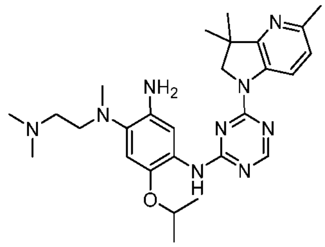 | *N*1-(2-(dimethylamino)ethyl)-5-isopropoxy-*N*1-methyl-*N*4-(4-(3,3,5-trimethyl-2,3-dihydro-1*H*-pyrrolo[3,2-*b*]pyridin-1-yl)-1,3,5-triazin-2-yl) benzene-1,2,4-triamine | 506.3 |
| **E41** | 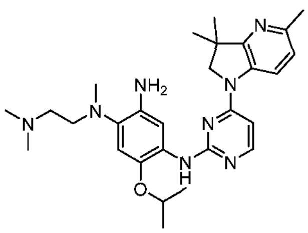 | *N*1-(2-(dimethylamino)ethyl)-5-isopropoxy-*N*1-methyl-*N*4-(4-(3,3,5-trimethyl-2,3 - dihydro-1*H*-pyrrolo[3,2-*b*]pyridin-1-yl)pyrimidin-2-yl) benzene-1,2,4-triamine | 505.3 |
| **E42** | 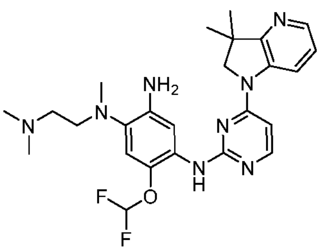 | 5-(difluoromethoxy)-*N*4-(4-(3,3 -dimethyl-2,3 -dihydro-1H-pyrrolo[3,2-*b*]pyridin-1-yl)pyrimidin-2-yl)-*N*1-(2-(dimethylamino) ethyl)-*N*1-methylbenzene-1,2,4-triamine | 499.2 |

(continued)

| Intermediate No. | Structural Formula | Chemical Name | ESI-MS: $[M+1]^+$ |
|---|---|---|---|
| **E43** | 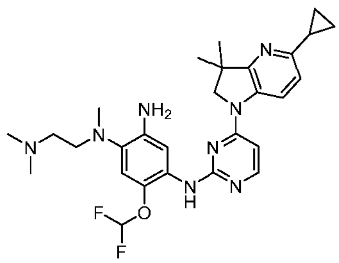 | $N^4$-(4-(5-cyclopropyl-3,3-dimethyl-2,3-dihydro-1*H*-pyrrolo[3,2-*b*]pyridin-1-yl)pyrimidin-2-yl)-5-(difluoromethoxy)-$N^1$-(2-(dimethylamino) ethyl)-$N^1$-methylbenzene-1,2,4-triamine | 539.2 |
| **E44** | 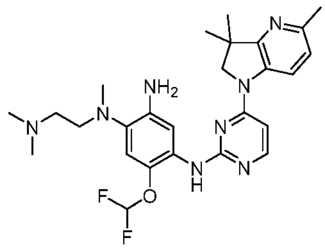 | 5-(difluoromethoxy)-$N^1$-(2-(dimethylamino) ethyl)-$N^1$-methyl-$N^4$-(4-(3,3,5-trimethyl-2,3-dihydro-1*H*-pyrrolo[3,2-*b*]pyridin-1-yl)pyrimidin-2-yl)benzene-1,2,4-triamine | 513.2 |
| **E45** | 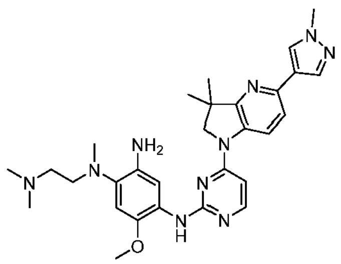 | $N^4$-(4-(3,3-dimethyl-5-(1-methyl-1*H*-pyrazol-4-yl)-2,3-dihydro-1*H*-pyrrolo[3,2-*b*]pyridin-1-yl) pyrimidin-2-yl)-$N^1$-(2-(dimethylamino)ethyl)-5-methoxy-$N^1$-methylbenzene-1,2,4-triamine | 543.2 |
| **E46** | 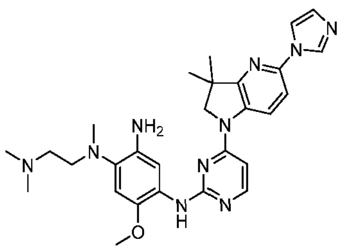 | $N^4$-(4-(5-(1*H*-imidazole-1-yl)-3,3-dimethyl-2,3-dihydro-1*H*-pyrrolo[3,2-*b*]pyridin-1-yl)pyrimidin-2-yl)-$N^1$-(2-(dimethylamino)ethyl)-5-methoxy-$N^1$-methylbenzene-1,2,4-triamine | 529.2 |
| **E47** | 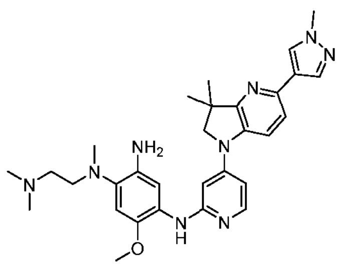 | $N^4$-(4-(3,3-dimethyl-5-(1-methyl-1*H*-pyrazol-4-yl)-2,3-dihydro-1*H*-pyrrolo[3,2-*b*]pyridin-1-yl) pyridin-2-yl)-$N^1$-(2-(dimethylamino)ethyl)-5-methoxy-$N^1$-methylbenzene-1,2,4-triamine | 542.2 |
| **E48** | 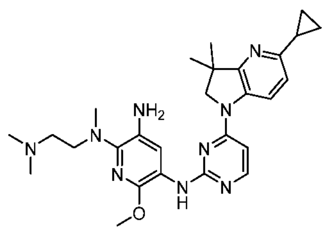 | $N^5$-(4-(5-cyclopropyl-3,3-dimethyl-2,3-dihydro-1*H*-pyrrolo[3,2-*b*]pyridin-1-yl)pyrimidin-2-yl)-$N^2$-(2-(dimethylamino)ethyl)-6-methoxy-$N^2$-methylpyridin-2,3,5-triamine | 504.2 |

(continued)

| Intermediate No. | Structural Formula | Chemical Name | ESI-MS: [M+1]$^+$ |
|---|---|---|---|
| **E49** | 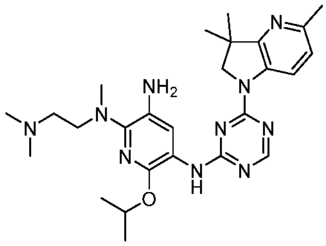 | N$^2$-(2-(dimethylamino)ethyl)-6-isopropoxy-N$^2$-methyl-N$^5$-(4-(3,3,5-trimethyl-2,3-dihydro-1*H*-pyrrolo[3,2-*b*]pyridin-1-yl)-1,3,5-triazin-2-yl)pyridin-2,3,5-triamine | 507.2 |
| **E50** | 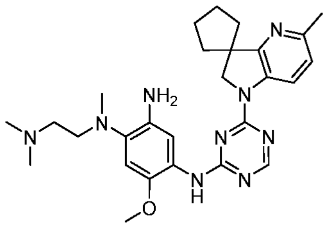 | *N*$^1$-(2-(dimethylamino)ethyl)-5-methoxy-*N*$^1$-methyl-*N*$^4$-(4-(5'-methylspiro[cyclopentane-1,3'-pyrrolo[3,2-*b*]pyridin]-1'(2'*H*)-yl)-1,3,5-triazin-2-yl)benzene-1,2,4-triamine | 504.4 |
| **E51** | 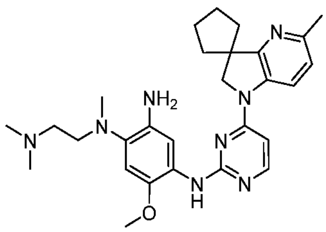 | *N*$^1$-(2-(dimethylamino)ethyl)-5-methoxy-*N*$^1$-methyl-*N*$^4$-(4-(5'-methylspiro[cyclopentane-1,3'-pyrrolo[3,2-*b*]pyridin]-1'(2'*H*)-yl)pyrimidin-2-yl)benzene-1,2,4-triamine | 503.2 |
| **E52** | 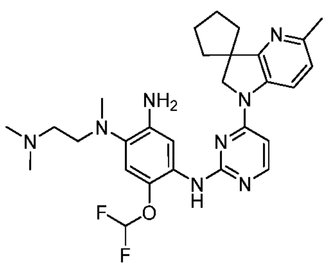 | 5-(difluoromethoxy)-*N*$^1$-(2-(dimethylamino)ethyl)-*N*$^1$-methyl-*N*$^4$-(4-(5'-methylspiro[cyclopentane-1,3'-pyrrolo[3,2-*b*]pyridin]-1'(2'*H*)-yl)pyrimidin-2-yl)benzene-1,2,4-triamine | 539.2 |
| **E53** | 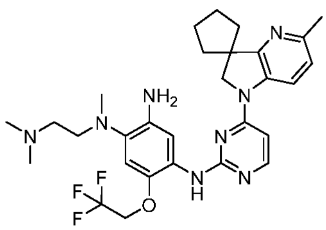 | *N*$^1$-(2-(dimethylamino)ethyl)-*N*$^1$-methyl-*N*$^4$-(4-(5'-methylspiro[cyclopentane-1,3'-pyrrolo[3,2-*b*]pyridin]-1'(2'*H*)-yl)pyrimidin-2-yl)-5-(2,2,2-trifluoroethoxy)benzene-1,2,4-triamine | 571.2 |
| **E54** | 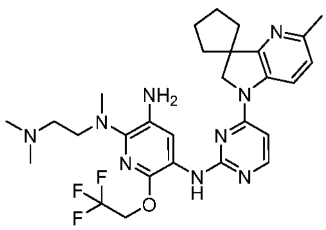 | *N*$^2$-(2-(dimethylamino)ethyl)-*N*$^2$-methyl-*N*$^5$-(4-(5'-methylspiro[cyclopentane-1,3'-pyrrolo[3,2-*b*]pyridin]-1'(2'*H*)-yl)pyrimidin-2-yl)-6-(2,2,2-trifluoroethoxy)pyridin-2,3,5-triamine | 572.3 |

(continued)

| Intermediate No. | Structural Formula | Chemical Name | ESI-MS: [M+1]+ |
|---|---|---|---|
| **E55** | 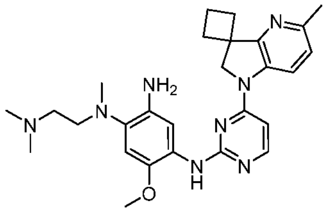 | *N*1-(2-(dimethylamino)ethyl)-5-methoxy-*N*1-methyl-*N*4-(4-(5'-methylspiro[cyclobutane-1,3'-pyrrolo[3,2-*b*]pyridin]-1'(2'*H*)-yl)pyrimidin-2-yl)benzene-1,2,4-triamine | 489.2 |
| **E56** | 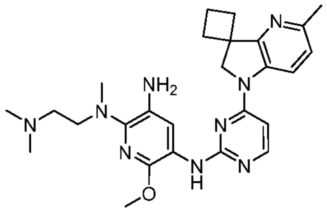 | *N*2-(2-(dimethylamino)ethyl)-6-methoxy-*N*2-methyl-*N*5-(4-(5'-methylspiro[cyclobutane-1,3'-pyrrolo[3,2-*b*]pyridin]-1'(2'*H*)-yl)pyrimidin-2-yl)pyridin-2,3,5-triamine | 490.2 |
| **E57** | 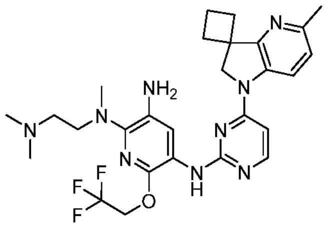 | *N*2-(2-(dimethylamino)ethyl)-*N*2-methyl-*N*5-(4-(5'-methylspiro[cyclobutane-1,3'-pyrrolo [3,2-*b*]pyridin]-1'(2'*H*)-yl)pyrimidin-2-yl)-6-(2,2,2-trifluoroethoxy)pyridin-2,3,5-triamine | 558.2 |
| **E58** | 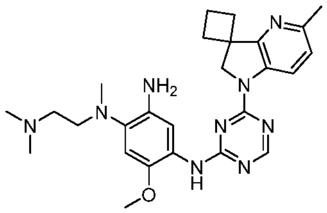 | *N*1-(2-(dimethylamino)ethyl)-5-methoxy-*N*1-methyl-*N*4-(4-(5'-methylspiro[cyclobutane-1,3'-pyrrolo[3,2-*b*]pyridin]-1'(2'*H*)-yl)-1,3,5-triazin-2-yl)benzene-1,2,4-triamine | 490.2 |
| **E59** | 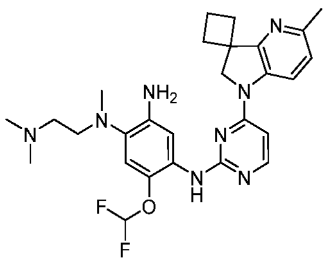 | 5-(difluoromethoxy)-*N*1-(2-(dimethylamino) ethyl)-*N*1-methyl-*N*4-(4-(5'-methylspiro [cyclobutane-1,3'-pyrrolo[3,2-*b*]pyridin]-1' (2'*H*)-yl)pyrimidin-2-yl)benzene-1,2,4-triamine | 525.2 |
| **E60** | 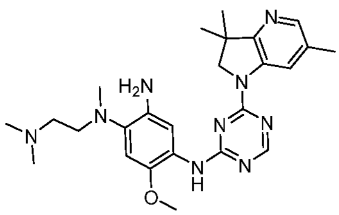 | *N*1-(2-(dimethylamino)ethyl)-5-methoxy-*N*1-methyl-*N*4-(4-(3,3,6-trimethyl-2,3-dihydro-1*H*-pyrrolo[3,2-*b*]pyridin-1-yl)-1,3,5-triazin-2-yl) benzene-1,2,4-triamine | 478.2 |

(continued)

| Intermediate No. | Structural Formula | Chemical Name | ESI-MS: [M+1]+ |
|---|---|---|---|
| **E61** | 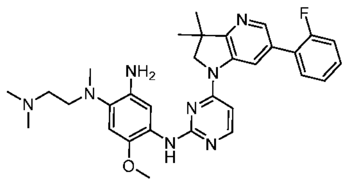 | *N*1-(2-(dimethylamino)ethyl)-*N*4-(4-(6-(2-fluorophenyl)-3,3 - dimethyl-2,3-dihydro-1*H*-pyrrolo[3,2-*b*]pyridin-1-yl)pyrimidin-2-yl)-5-methoxy-*N*1-methylbenzene-1,2,4-triamine | 558.3 |
| **E62** | 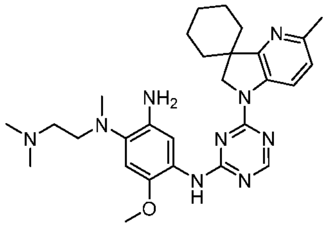 | *N*1-(2-(dimethylamino)ethyl)-5-methoxy-*N*1-methyl-*N*4-(4-(5'-methylspiro[cyclohexane-1,3'-pyrrolo[3,2-*b*]pyridin]-1'(2'*H*)-yl)-1,3,5-triazin-2-yl)benzene-1,2,4-triamine | 518.2 |
| **E63** | 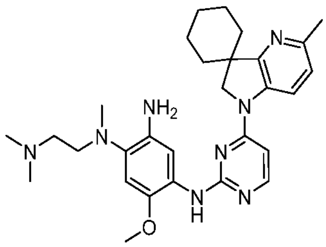 | *N*1-(2-(dimethylamino)ethyl)-5-methoxy-*N*1-methyl-*N*4-(4-(5'-methylspiro[cyclohexane-1,3'-pyrrolo[3,2-*b*]pyridin]-1'(2'*H*)-yl)pyrimidin-2-yl)benzene-1,2,4-triamine | 517.4 |
| **E64** | 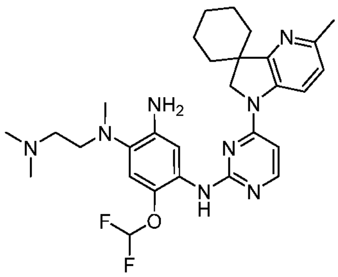 | 5-(difluoromethoxy)-*N*1-(2-(dimethylamino) ethyl)-*N*1-methyl-*N*4-(4-(5'-methylspiro [cyclohexane-1,3'-pyrrolo[3,2-*b*]pyridin]-1' (2'*H*)-yl)pyrimidin-2-yl)benzene-1,2,4-triamine | 553.4 |
| **E65** | 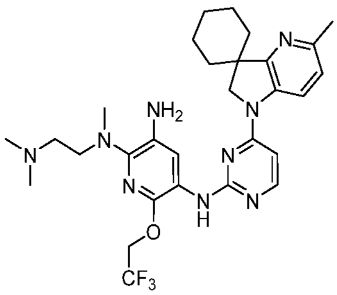 | *N*2-(2-(dimethylamino)ethyl)-*N*2-methyl-*N*5-(4-(5'-methylspiro[cyclohexane-1,3'-pyrrolo [3,2-*b*]pyridin]-1'(2'*H*)-yl)pyrimidin-2-yl)-6-(2,2,2-trifluoroethoxy)pyridin-2,3,5-triamine | 586.4 |
| **E66** | 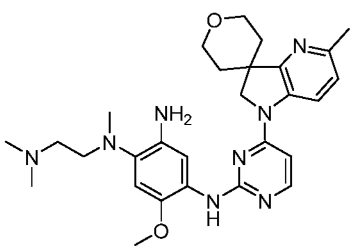 | *N*1-(2-(dimethylamino)ethyl)-5-methoxy-*N*1-methyl-*N*4-(4-(5'-methyl-2,3,5,6-tetrahydrogenspiro[pyran-4,3'-pyrrolo[3,2-*b*] pyridin]-1'(2'*H*)-yl)pyrimidin-2-yl)benzene-1,2,4-triamine | 519.4 |

(continued)

| Intermediate No. | Structural Formula | Chemical Name | ESI-MS: [M+1]+ |
|---|---|---|---|
| **E67** | 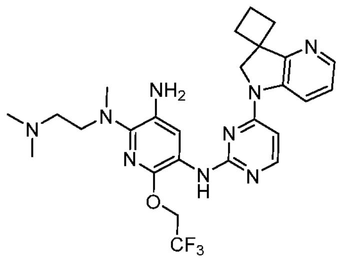 | *N*2-(2-(dimethylamino)ethyl)-*N*2-methyl-*N*5-(4-(spiro[cyclobutane-1,3'-pyrrolo[3,2-*b*]pyridin]-1'(2'*H*)-yl)pyrimidin-2-yl)-6-(2,2,2-trifluoroethoxy)pyridin-2,3,5-triamine | 544.2 |
| **E68** | 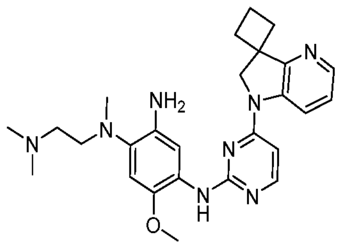 | *N*1-(2-(dimethylamino)ethyl)-5-methoxy-*N*1-methyl-*N*4-(4-(spiro[cyclobutane-1,3'-pyrrolo[3,2-*b*]pyridin]-1'(2'*H*)-yl)pyrimidin-2-yl)benzene-1,2,4-triamine | 475.3 |

**Intermediate E69: Preparation of 5-(difluoromethoxy)-*N*1-(2-(dimethylamino)ethyl)-*N*1-methyl-*N*4-(4-(3,3,5-trimethyl-2,3-dihydro-1*H*-pyrrolo [3,2-*b*] pyridin-1-yl)-1,3,5-triazin-2-yl)benzene-1,2,4-triamine**

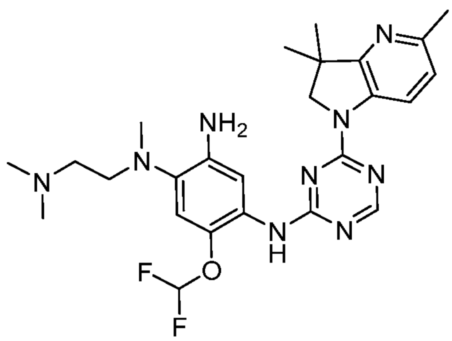

Zinc powder (482 mg, 7.36 mmol) and ammonium chloride (394 mg, 7.36 mmol) were added to the solution of 2-(difluoromethoxy)-*N*4-(2-(dimethylamino)ethyl)-*N*4-methyl-5-nitro-*N*1-(4-(3,3,5-trimethyl-2,3-dihydro-1*H*-pyrrolo[3,2-*b*]pyridin-1-yl)-1,3,5-triazin-2-yl)benzene-1,4-diamine (200 mg, 0.37 mmol) in methanol (6 mL). The reaction mixture was stirred for 3 hrs at normal temperature. The reaction mixture was filtered through diatomite, concentrated, and then separated by silicagel column chromatography [dichloromethane: methanol=10:1] to obtain 5-(difluoromethoxy)-*N*1-(2-(dimethylamino)ethyl)-*N*1-methyl-*N*4-(4-(3,3,5-trimethyl-2,3-dihydro-1*H*-pyrrolo[3,2-*b*]pyridin-1-yl)-1,3,5-triazin-2-yl)benzene-1,2,4-triamine (87 mg, yield: 42%). ESI-MS: 514.1 [M+1]+.

**Intermediates E70 were prepared according to the synthesis method for Intermediate E69:**

| Intermediate No. | Structural Formula | Chemical Name | ESI-MS: [M+1]+ |
|---|---|---|---|
| E70 | 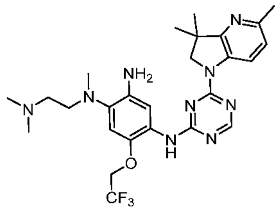 | *N*1-(2-(dimethylamino)ethyl)-*N*1-methyl-5-(2,2,2-trifluoroethoxy)-*N*4-(4-(3,3,5-trimethyl-2,3-dihydro-1*H*-pyrrolo[3,2-*b*]pyridin-1-yl)-1,3,5-triazin-2-yl) benzene-1,2,4-triamine | 546.1 |

**Intermediate F1: Preparation of *N*-(5-((4-(5-bromo-3,3-dimethyl-2,3-dihydro-1*H*-pyrrolo [3,2-*b*]pyridin-1-yl)pyrimidin-2-yl)amino)-2-((2-(dimethylamino)ethyl)(methyl)amino)-4-methoxyphenyl)acrylamide**

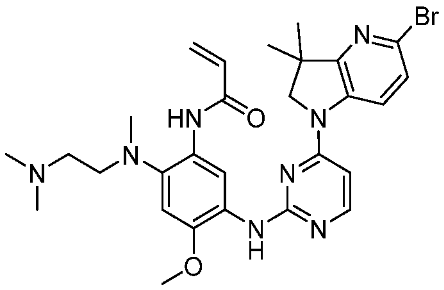

*N*4-(4-(5-bromo-3,3-dimethyl-2,3-dihydro-1*H*-pyrrolo[3,2-*b*]pyridin-1-yl)pyrimidin-2-yl)-*N*1-(2-(dimethylamino)ethyl)-5-methoxy-*N*1-methylbenzene-1,2,4-triamine (194 mg, 0.36 mmol) was dissolved in anhydrous acetonitrile/water (3mL/1mL). *N,N*-diisopropylethylamine (138 mg, 1.08 mmol) was added to the mixture. Acryloyl chloride (97.3 mg, 1.08 mmol) was added to the reaction mixture at 0 °C. After the reaction was completed, stratification was conducted with dichloromethane and water. The organic phase was washed with water and saturated sodium chloride in sequence, then dried over anhydrous sodium sulfate, filtered, concentrated, and then separated by reversed column chromatography [40-50% acetonitrile/water] to obtain *N* (5-((4-(5-bromo-3,3-dimethyl-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide (150 mg, yield: 56.2%). ESI-MS: 595.2,597.2 [M+1]+.

**Intermediates F2 were prepared according to the synthesis method for Intermediate F1:**

| Intermediate No. | Structural Formula | Chemical Name | ESI-MS: [M+1]+ |
|---|---|---|---|
| F2 | 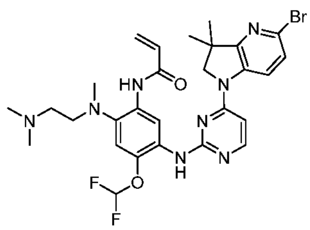 | *N*-(5-((4-(5-bromo-3,3-dimethyl-2,3-dihydro-1*H*-pyrrolo[3,2-*b*]pyridin-1-yl)pyrimidin-2-yl)amino)-4-(difluoromethoxy)-2-((2-(dimethylamino)ethyl) (methyl) amino)phenyl)acrylamide | 631.2 633.2 |

**Intermediate G1: Preparation of *N*-(5-((4-(3,3-dimethyl-5-((trimethylsilyl)ethynyl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide**

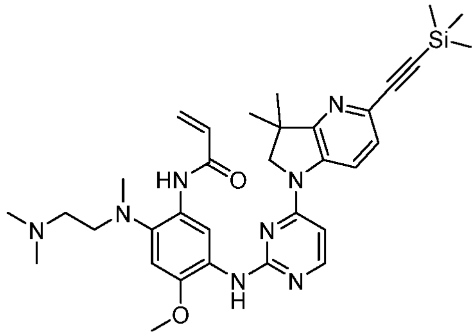

*N*-(5-((4-(5-bromo-3,3-dimethyl-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide (150 mg, 0.2 mmol), trimethylsilylacetylene (247 mg, 2.5 mmol), triethylamine (509 mg, 5.0 mmol), bis(triphenylphosphine) palladium dichloride (58 mg, 0.076 mmol) and cuprous iodide (14.4 mg, 0.076 mmol) were dissolved in tetrahydrofuran (6 mL). The reaction mixture was stirred for 3 hrs at room temperature under the protection of nitrogen, and the reaction was completed. The reaction mixture was filtered with diatomite. The resulting filtrate was concentrated, and the residue was separated by flash silicagel columns [dichloromethane: methanol=10:1] to obtain *N*-(5-((4-(3,3-dimethyl-5-((trimethylsilyl)ethynyl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide (100 mg, yield: 27.86%) ESI-MS: 613.4 $[M+1]^+$.

**Intermediates G2 were prepared according to the synthesis method for Intermediate G1:**

| Intermediate No. | Structural Formula | Chemical Name | ESI-MS: $[M+1]^+$ |
|---|---|---|---|
| G2 | 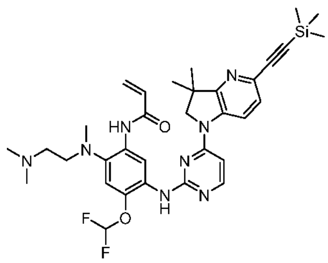 | *N*-(4-(difluoromethoxy)-5-((4-(3,3-dimethyl-5-((trimethylsilyl)ethynyl)-2,3-dihydro-1*H*-pyrrolo [3,2-*b*]pyridin-1-yl)pyrimidin-2-yl)amino)-2-((2-(dimethylamino)ethyl)(meth yl)amino)phenyl) acrylamide | 649.3 |

**II. Preparation of specific compounds of the examples**

**Example 1: Preparation of *N*-(5-((4-(3,3-dimethyl-2,3-dihydro-1*H*-pyrrolo[3,2-*b*]pyridin-1-yl)pyridin-2-yl)amino)-2-((2-(dimethylamino)ethyl)(methyl)amino)-4-methoxyphenyl)acrylamide**

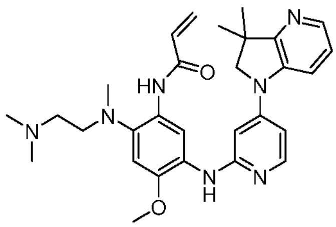

$N^4$-(4-(3,3-dimethyl-2,3-dihydro-1*H*-pyrrolo[3,2-*b*]pyridin-1-yl)pyridin-2-yl)-$N^1$-(2-(dimethylamino)ethyl)-5-methoxy-$N^1$-methylbenzene-1,2,4-triamine (205 mg, 0.44 mmol) was dissolved in anhydrous dichloromethane (5 mL). *N*,*N*-diisopropylethylamine (170 mg, 1.32 mmol) was added to the mixture. Acryloyl chloride (67 mg, 0.75 mmol) was added to the reaction mixture at 0 °C. After concentration, the resultant was separated by reversed column chromatography [40-50% acetonitrile/water] to obtain N-(5-((4-(3,3-dimethyl-2,3-dihydro-1*H*-pyrrolo[3,2-*b*]pyridin-1-yl)pyridin-2-yl)amino)-2-((2-(dimethylamino)ethyl)(methyl)amino)-4-methoxyphenyl)acrylamide (8.1 mg, yield: 3.5%). ESI-MS: 516.2 $[M+1]^+$.

$^1$H NMR (400 MHz, DMSO-$d_6$) $\delta$ 10.10 (s, 1H), 8.82 (s, 1H), 7.96 (d, *J* = 5.2 Hz, 2H), 7.81 (s, 1H), 7.68 (d, *J* = 8.2 Hz, 1H), 7.09(dd, *J* = 8.2, 4.8 Hz, 1H), 6.97 (s, 1H), 6.80 (s, 1H), 6.60 (d, *J* = 5.7 Hz, 1H), 6.38 (dd, *J* = 16.9, 10.0 Hz, 1H), 6.22 (d, *J* = 16.8 Hz, 1H), 5.74 (d, *J* = 9.9 Hz, 1H), 3.84 (s, 3H), 3.80 (s, 2H), 2.85 (t, *J* = 7.9 Hz, 2H), 2.68 (s, 3H), 2.27 (t, *J* = 7.9 Hz,2H), 2.19 (s, 6H), 1.32 (s, 6H).

**Example 9: Preparation of *N*-(4-(difluoromethoxy)-2-((2-(dimethylamino)ethyl)(methyl)amino)-5-((4-(3,3,5-trimethyl-2,3-dihydro-1*H*-pyrrolo[3,2-*b*]pyridin-1-yl)-1,3,5-triazin-2-yl)amino)phenyl)acrylamide**

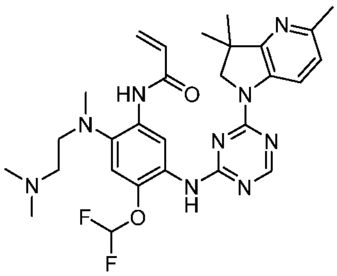

5-(difluoromethoxy)-$N^1$-(2-(dimethylamino)ethyl)-$N^1$-methyl-$N^4$-(4-(3,3,5-trimethyl-2,3 -dihydro- 1*H*-pyrrolo[3,2-b]pyridin-1-yl)-1,3,5-triazin-2-yl)benzene-1,2,4-triamine (87 mg, 0.17 mmol) was dissolved in acetonitrile/water (4:1, 5 mL). *N*,*N*-diisopropylethylamine (66 mg, 0.51 mmol) was added to the mixture. Acryloyl chloride (23 mg, 0.25 mmol) was added to the reaction mixture at 0 °C The reaction mixture was stirred for half an hr, quenched with 0.5 mL of methanol, and then subjected to preparative high-performance liquid chromatography to obtain *N*-(4-(difluoromethoxy)-2-((2-(dimethylamino)ethyl)(methyl)amino)-5-((4-(3,3,5-trimethyl-2,3-dihydro-1*H*-pyrrolo[3,2-*b*]pyridin-1-yl)-1,3,5-triazin-2-yl)amino)phenyl)acrylamide (47.8 mg, yield: 48%). ESI-MS: 568.2 $[M+1]^+$.

$^1$H NMR (400 MHz, DMSO-$d_6$) $\delta$ 10.19 (s, 1H), 9.16 (d, *J* = 209.4 Hz, 1H), 8.39 (d, *J*= 24.2 Hz, 2H), 7.34 - 6.65 (m, 3H), 6.44 (dd, *J* = 16.9, 10.1 Hz, 1H), 6.25 (t, *J* = 17.4 Hz, 1H), 5.79 (d, *J* = 10.1 Hz, 1H), 3.97 (d, *J* = 30.7 Hz, 2H), 2.87 (s, 2H), 2.72 (s, 3H), 2.37 (t, *J* = 17.7 Hz, 5H), 2.21 (s, 6H), 1.29 (s, 6H).

**The following examples were prepared according to the synthesis method for Example 1 or 9:**

| Example No. | Structural Formula | Chemical Name | ESI-MS: $[M+1]^+$ |
|---|---|---|---|
| Example 2 | 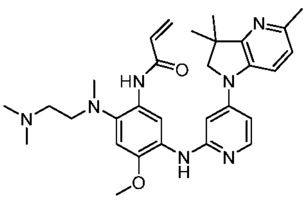 | *N*-(2-((2-(dimethylamino)ethyl)(m ethyl)amino)-4-methoxy-5-((4-(3, 3,5-trimethyl-2,3-dihydro-1*H*-pyrr olo[3,2-*b*]pyridin-1-yl)pyridin-2-y l)amino)phenyl) acrylamide | 530.2 |

(continued)

| Example No. | Structural Formula | Chemical Name | ESI-MS: $[M+1]^+$ |
|---|---|---|---|
| **Example 3** | 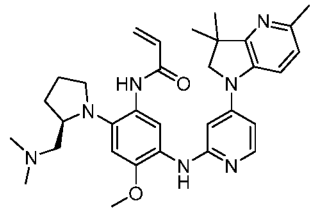 | (R)-*N*-(2-(2-((dimethylamino)met hyl)pyrrolidin-1-yl)-4-methoxy-5-((4-(3,3,5-trimethyl-2,3-dihydro-1 *H*-pyrrolo[3,2-*b*]pyridin-1-yl)pyri din-2-yl)amino) phenyl)acrylamid e | 556.3 |
| **Example 4** | 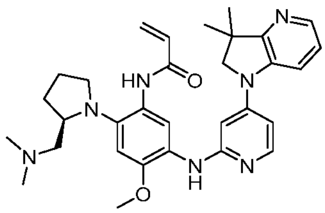 | (R)-*N*-(5-((4-(3,3-dimethyl-2,3-di hydro-1*H*-pyrrolo [3,2-*b*]pyridin-1 -yl)pyridin-2-yl)amino)-2-(2-((di methylamino)methyl)pyrrolidin-1 -yl)-4-methoxyphenyl)acrylamide | 542.2 |
| **Example 5** | 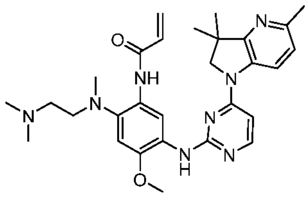 | *N*-(2-((2-(dimethylamino)ethyl)(m ethyl)amino)-4-methoxy-5-((4-(3, 3,5-trimethyl-2,3-dihydro-1*H*-pyrr olo[3,2-*b*]pyridin-1-yl)pyrimidin-2-yl)amino)phenyl) acrylamide | 531.3 |
| **Example 6** | 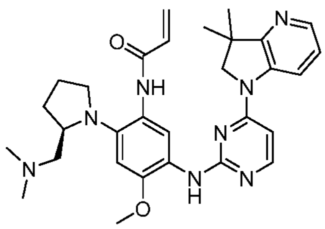 | (R)-*N*-(5-((4-(3,3-dimethyl-2,3-di hydro-1*H*-pyrrolo [3,2-b]pyridin-1 -yl)pyrimidin-2-yl)amino)-2-(2-((dimethylamino)methyl)pyrrolidi n-1-yl)-4-methoxyphenyl)acrylam ide | 543.2 |
| **Example 7** | 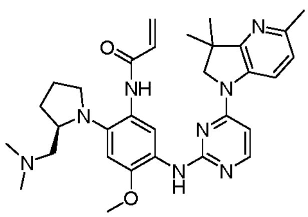 | (R)-*N*-(2-(2-((dimethylamino)met hyl)pyrrolidin-1-yl)-4-methoxy-5-((4-(3,3,5-trimethyl-2,3-dihydro-1 *H*-pyrrolo[3,2-*b*]pyridin-1-yl)pyri midin-2-yl)amino) phenyl)acrylam ide | 557.2 |
| **Example 8** | 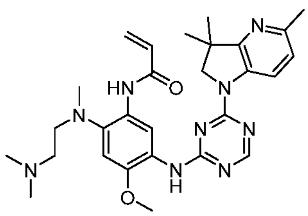 | *N*-(2-((2-(dimethylamino)ethyl)(m ethyl)amino)-4-methoxy-5-((4-(3, 3,5-trimethyl-2,3-dihydro-1*H*-pyrr olo[3,2-*b*]pyridin-1-yl)-1,3,5-triaz in-2-yl)amino) phenyl)acrylamide | 532.2 |
| **Example 10** | 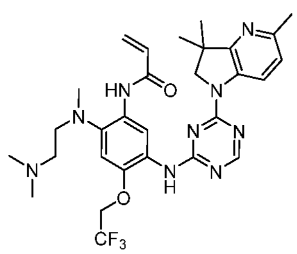 | *N*-(2-((2-(dimethylamino)ethyl)(m ethyl)amino)-4-(2,2,2-trifluoroeth oxy)-5 -((4-(3,3,5 -trimethyl-2,3 -di hydro-1*H*-pyrrolo[3,2-*b*]pyridin-1 -yl)-1,3,5-triazin-2-yl)amino)phen yl)acrylamide | 600.2 |

(continued)

| Example No. | Structural Formula | Chemical Name | ESI-MS: [M+1]+ |
|---|---|---|---|
| **Example 11** | 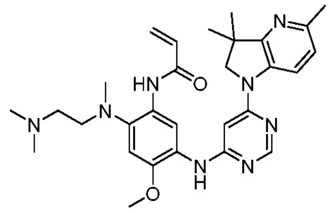 | *N*-(2-((2-(dimethylamino)ethyl)(m ethyl)amino)-4-methoxy-5-((6-(3, 3,5-trimethyl-2,3-dihydro-1*H*-pyrr olo[3,2-*b*]pyridin-1-yl)pyrimidin-4-yl)amino)phenyl) acrylamide | 531.4 |
| **Example 12** | 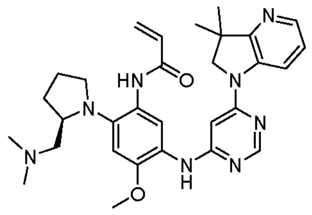 | (R)-*N*-(5-((6-(3,3-dimethyl-2,3-di hydro-1*H*-pyrrolo [3,2-*b*]pyridin-1 -yl)pyrimidin-4-yl)amino)-2-(2-((dimethylamino)methyl)pyrrolidi n-1-yl)-4-methoxyphenyl)acrylam ide | 543.2 |
| **Example 13** | 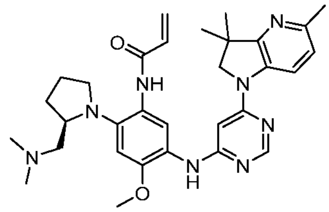 | (R)-*N*-(5-((6-(3,3,5-trimethyl-2,3-dihydro-1*H*-pyrrolo [3,2-*b*]pyridin -1-yl)pyrimidin-4-yl)amino)-2-(2-((dimethylamino)methyl)pyrrolidi n-1-yl)-4-methoxyphenyl)acrylam ide | 557.3 |
| **Example 14** | 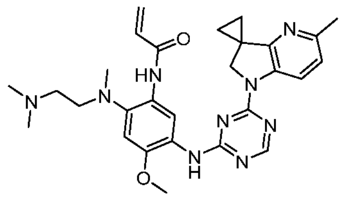 | *N*-(2-((2-(dimethylamino)ethyl)(m ethyl)amino)-4-methoxy-5-((4-(5' -methylspiro[cyclopropane-1,3'-p yrrolo[3,2-*b*]pyridin]-1'(2'*H*)-yl)-1,3,5-triazin-2-yl) amino)phenyl)a crylamide | 530.2 |
| **Example 15** | 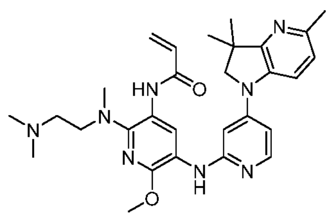 | *N*-(2-((2-(dimethylamino)ethyl)(m ethyl)amino)-6-methoxy-5-((4-(3, 3,5-trimethyl-2,3-dihydro-1*H*-pyrr olo[3,2-*b*]pyridin-1-yl)pyridin-2-y l)amino)pyridin-3-yl)acrylamide | 531.2 |
| **Example 16** | 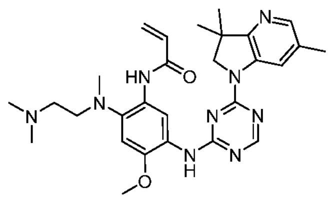 | N-(2-((2-(dimethylamino)ethyl) (methyl)amino)-4-methoxy-5-((4-(3,3,6-trimethyl-2,3-dihydro-1H-p yrrolo[3,2-b]pyridin-1 -yl)-1,3,5-tr iazin-2-yl)amino) phenyl)acrylami de | 532.2 |
| **Example 17** | 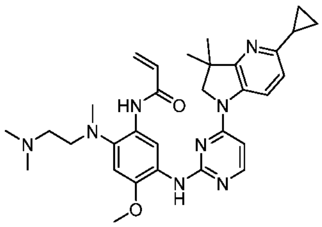 | *N (5-((4-(5-cyclopropyl-3,3-dimet* hyl-2,3-dihydro-1*H*-pyrrolo[3,2-*b*]pyridin-1-yl)pyrimidin-2-yl)ami no)-2-((2-(dimethylamino)ethyl) (methyl)amino)-4-methoxypheny l)acrylamide | 557.4 |

(continued)

| Example No. | Structural Formula | Chemical Name | ESI-MS: [M+1]+ |
|---|---|---|---|
| **Example 18** | 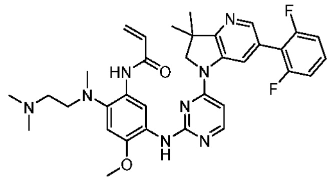 | *N*4-(4-(6-(2,6-difluorophenyl)-3,3-dimethyl-2,3-dihydro-1*H*-pyrrolo [3,2-*b*]pyridin-1-yl)pyrimidin-2-y 1)-*N*1-(2-(dimethylamino)ethyl)-5-methoxy-*N*1-methylbenzene-1,2,4 -triamine | 575.3 |
| **Example 19** | 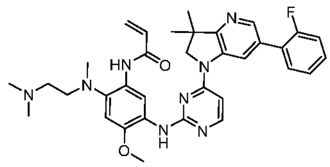 | *N*-(2-((2-(dimethylamino)ethyl)(m ethyl)amino)-5-((4-(6-(2-fluoroph enyl)-3,3-dimethyl-2,3-dihydro-1 *H*-pyrrolo[3,2-*b*]pyridin-1-yl)pyri midin-2-yl)amino)-4-methoxyphe nyl)acrylamide | 611.4 |
| **Example 20** | 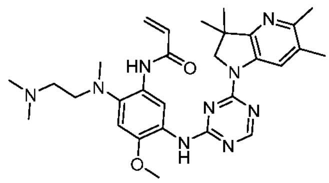 | *N*-(2-((2-(dimethylamino)ethyl)(m ethyl)amino)-4-methoxy-5-((4-(3, 3,5,6-tetramethyl-2,3-dihydro-1*H*-pyrrolo[3,2-b]pyridin-1-yl)-1,3,5-t riazin-2-yl)amino) phenyl)acrylam ide | 546.3 |
| **Example 21** | 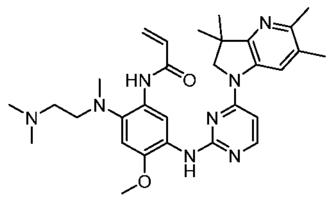 | *N*-(2-((2-(dimethylamino)ethyl)(m ethyl)amino)-4-methoxy-5-((4-(3, 3,5,6-tetramethyl-2,3-dihydro-1*H*-pyrrolo[3,2-*b*]pyridin-1-yl)pyrimi din-2-yl)amino) phenyl)acrylamid e | 545.4 |
| **Example 22** | 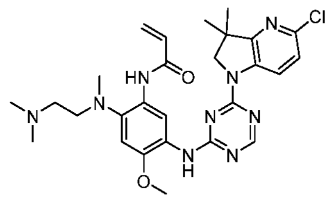 | N (5-((4-(5-chloro-3,3-dimethyl-2,3-dihydro-1*H*-pyrrolo[3,2-*b*]pyr idin-1-yl)-1,3,5-triazin-2-yl)amin o)-2-((2-(dimethylamino)ethyl)(m ethyl)amino)-4-methoxyphenyl)ac rylamide | 552.2 |
| **Example 23** | 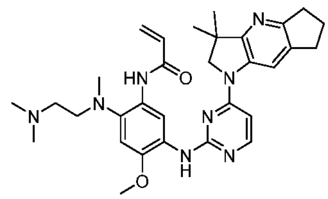 | *N*-(5-((4-(3,3-dimethyl-3,5,6,7-tet rahydrocyclopenta [*b*]pyrrolo[2,3-*e*]pyridin-1(2*H*)-yl)pyrimidin-2-y l) amino)-2-((2-(dimethylamino)et hyl)(methyl)amino)-4-methoxyph enyl)acrylamide | 557.4 |
| **Example 24** | 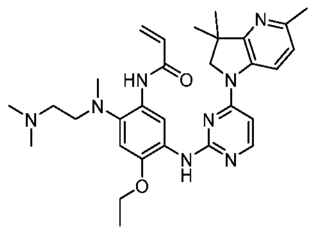 | *N*-(2-((2-(dimethylamino)ethyl)(m ethyl)amino)-4-ethoxy-5-((4-(3,3, 5-trimethyl-2,3-dihydro-1*H*-pyrro lo [3,2-*b*]pyridin-1-yl)pyrimidin-2-yl)amino)phenyl) acrylamide | 545.4 |

(continued)

| Example No. | Structural Formula | Chemical Name | ESI-MS: [M+1]$^+$ |
|---|---|---|---|
| **Example 25** | 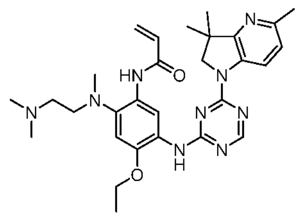 | *N*-(2-((2-(dimethylamino)ethyl)(m ethyl)amino)-4-ethoxy-5-((4-(3,3, 5-trimethyl-2,3-dihydro-1*H*-pyrro lo [3,2-*b*]pyridin-1-yl)-1,3,5-triazi n-2-yl)amino)phenyl) acrylamide | 546.3 |
| **Example 26** | 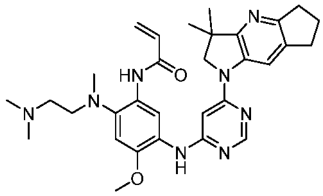 | *N*-(5-((6-(3,3-dimethyl-3,5,6,7-tet rahydrocyclopenta [*b*]pyrrolo[2,3-*e*]pyridin-1(2*H*)-yl)pyrimidin-4-y l) amino)-2-((2-(dimethylamino)et hyl)(methyl)amino)-4-methoxyph enyl)acrylamide | 557.4 |
| **Example 27** | 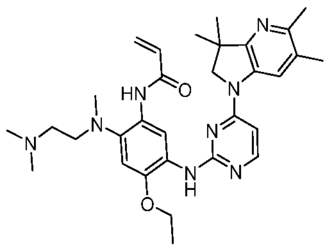 | *N*-(2-((2-(dimethylamino)ethyl)(m ethyl)amino)-4-ethoxy-5-((4-(3,3, 5,6-tetramethyl-2,3-dihydro-1*H*-p yrrolo[3,2-*b*]pyridin-1-yl)pyrimidi n-2-yl)amino) phenyl)acrylamide | 559.4 |
| **Example 28** | 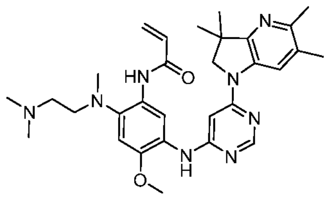 | *N*-(2-((2-(dimethylamino)ethyl)(m ethyl)amino)-4-methoxy-5-((6-(3, 3,5,6-tetramethyl-2,3-dihydro-1*H*-pyrrolo[3,2-*b*]pyridin-1-yl)pyrimi din-4-yl)amino) phenyl)acrylamid e | 545.2 |
| **Example 29** | 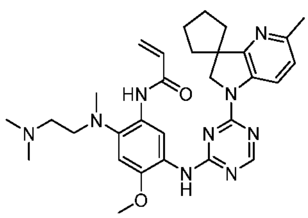 | *N*-(2-((2-(dimethylamino)ethyl)(m ethyl)amino)-4-methoxy-5-((4-(5'-methylspiro[cyclopentane-1,3'-py rrolo[3,2-*b*]pyridin]-1'(2'*H*)-yl)-1, 3,5-triazin-2-yl) amino)phenyl)acr ylamide | 558.3 |
| **Example 30** | 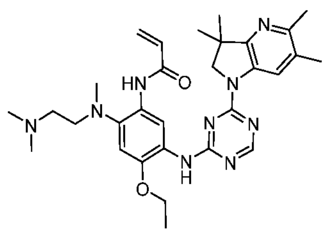 | *N*-(2-((2-(dimethylamino)ethyl)(m ethyl)amino)-4-ethoxy-5-((4-(3,3, 5,6-tetramethyl-2,3-dihydro-1*H*-p yrrolo[3,2-*b*]pyridin-1-yl)-1,3,5-tr iazin-2-yl)amino) phenyl)acrylami de | 560.4 |
| **Example 31** | 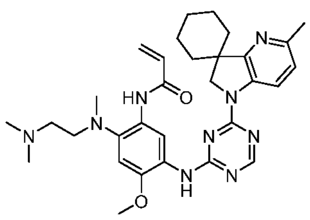 | *N*-(2-((2-(dimethylamino)ethyl)(m ethyl)amino)-4-methoxy-5-((4-(5'-methylspiro[cyclohexane-1,3'-py rrolo[3,2-*b*]pyridin]-1'(2'*H*)-yl)-1, 3,5-triazin-2-yl) amino)phenyl)acr ylamide | 572.4 |

(continued)

| Example No. | Structural Formula | Chemical Name | ESI-MS: [M+1]+ |
|---|---|---|---|
| **Example 32** | 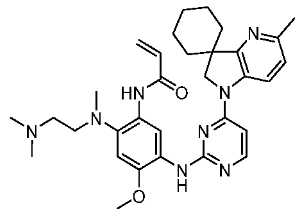 | *N*-(2-((2-(dimethylamino)ethyl)(m ethyl)amino)-4-methoxy-5-((4-(5' -methylspiro[cyclohexane-1,3'-py rrolo[3,2-*b*]pyridin]-1'(2'*H*)-yl)pyr imidin-2-yl)amino) phenyl)acryla mide | |
| **Example 33** | 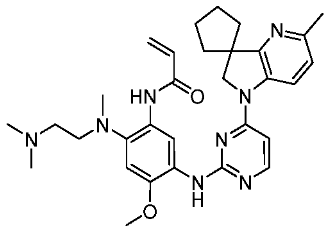 | *N*-(2-((2-(dimethylamino)ethyl)(m ethyl)amino)-4-methoxy-5-((4-(5' -methylspiro[cyclopentane-1,3'-py rrolo[3,2-*b*]pyridin]-1'(2'*H*)-yl)pyr imidin-2-yl)amino) phenyl)acryla mide | 557.4 |
| **Example 34** | 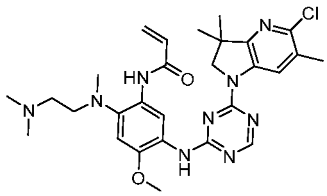 | *N*-(5-((4-(5-chloro-3,3,6-trimethyl -2,3-dihydro-1*H*-pyrrolo[3,2-*b*]py ridin-1-yl)-1,3, 5-triazin-2-yl)amin o)-2-((2-(dimethylamino)ethyl)(m ethyl)amino)-4-methoxyphenyl)ac rylamide | 566.2 |
| **Example 35** | 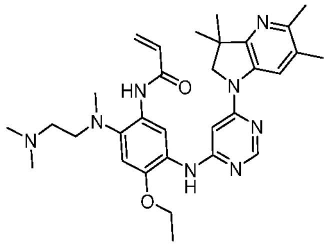 | *N*-(2-((2-(dimethylamino)ethyl)(m ethyl)amino)-4-ethoxy-5-((6-(3,3, 5,6-tetramethyl-2,3-dihydro-1*H*-p yrrolo[3,2-*b*]pyridin-1-yl)pyrimidi n-4-yl)amino) phenyl)acrylamide | 559.4 |
| **Example 36** | 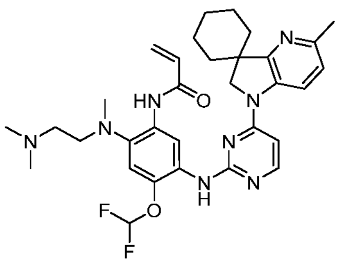 | *N*-(4-(difluoromethoxy)-2-((2-(di methylamino)ethyl)(methyl)amin o)-5-((4-(5'-methylspiro[cyclohex ane-1,3'-pyrrolo[3,2-*b*]pyridin]-1' (2'*H*)-yl)pyrimidin-2-yl) amino)ph enyl)acrylamide | 607.4 |
| **Example 37** | 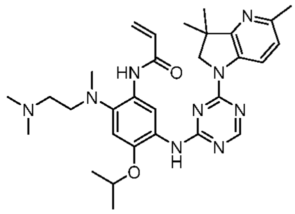 | *N*-(2-((2-(dimethylamino)ethyl)(m ethyl)amino)-4-isopropoxy-5-((4-(3,3,5-trimethyl-2,3-dihydro-1*H*-p yrrolo[3,2-*b*]pyridin-1-yl)-1,3,5-tr iazin-2-yl)amino) phenyl)acrylami de | 560.4 |

(continued)

| Example No. | Structural Formula | Chemical Name | ESI-MS: $[M+1]^+$ |
|---|---|---|---|
| **Example 38** | 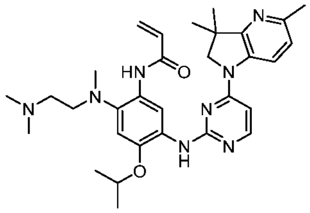 | *N*-(2-((2-(dimethylamino)ethyl)(m ethyl)amino)-4-isopropoxy-5-((4-(3,3,5-trimethyl-2,3-dihydro-1*H*-p yrrolo[3,2-*b*]pyridin-1-yl)pyrimidi n-2-yl)amino) phenyl)acrylamide | 559.4 |
| **Example 39** | 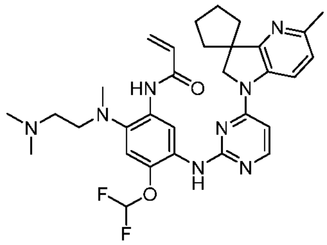 | *N*-(4-(difluoromethoxy)-2-((2-(di methylamino)ethyl)(methyl)amin o)-5-((4-(5'-methylspiro[cyclopen tane-1,3'-pyrrolo[3,2-*b*]pyridin]-1' (2'*H*)-yl)pyrimidin-2-yl) amino)ph enyl)acrylamide | 593.4 |
| **Example 40** | 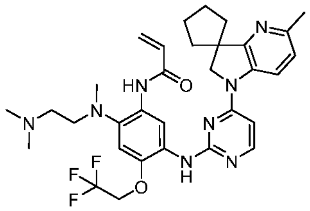 | *N*-(2-((2-(dimethylamino)ethyl)(m ethyl)amino)-5-((4-(5'-methylspir o[cyclopentane-1,3'-pyrrolo[3,2-*b*]pyridin]-1'(2'*H*)-yl)pyrimidin-2-yl)amino)-4-(2,2,2-trifluoroethox y)phenyl)acrylamide | 625.4 |
| **Example 41** | 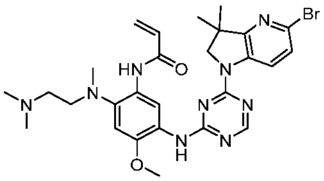 | *N*-(5-((4-(5-bromo-3,3-dimethyl-2,3-dihydro-1*H*-pyrrolo[3,2-*b*]pyr idin-1-yl)-1,3,5-triazin-2-yl)amin o)-2-((2-(dimethylamino)ethyl)(m ethyl)amino)-4-methoxyphenyl)ac rylamide | 596.2<br>598.2 |
| **Example 42** | 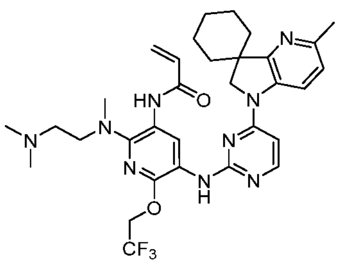 | *N*-(2-((2-(dimethylamino)ethyl)(m ethyl)amino)-5-((4-(5'-methylspir o[cyclohexane-1,3'-pyrrolo[3,2-*b*] pyridin]-1'(2'*H*)-yl)pyrimidin-2-y l)amino)-6-(2,2,2-trifluoroethoxy) pyridin-3 -yl)acrylamide | 640.4 |
| **Example 43** | 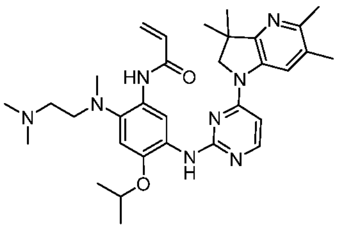 | *N*-(2-((2-(dimethylamino)ethyl)(m ethyl)amino)-4-isopropoxy-5-((4-(3,3,5,6-tetramethyl-2,3-dihydro-1 *H*-pyrrolo[3,2-*b*]pyridin-1-yl)pyri midin-2-yl)amino) phenyl)acrylam ide | 573.4 |

(continued)

| Example No. | Structural Formula | Chemical Name | ESI-MS: $[M+1]^+$ |
|---|---|---|---|
| **Example 44** | 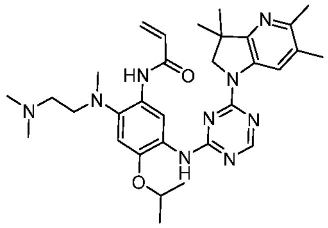 | *N*-(2-((2-(dimethylamino)ethyl)(m ethyl)amino)-4-isopropoxy-5-((4-(3,3,5,6-tetramethyl-2,3-dihydro-1 *H*-pyrrolo[3,2-*b*]pyridin-1-yl)-1,3, 5-triazin-2-yl) amino)phenyl)acryl amide | 574.4 |
| **Example 45** | 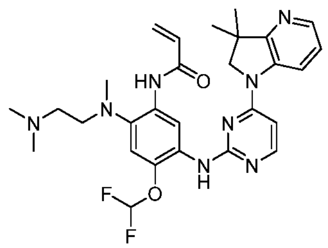 | *N*-(4-(difluoromethoxy)-5-((4-(3,3 -dimethyl-2,3-dihydro-1*H*-pyrrolo [3,2-*b*]pyridin-1-yl)pyrimidin-2-y l) amino)-2-((2-(dimethylamino)et hyl)(methyl)amino) phenyl)acryla mide | 553.2 |
| **Example 46** | 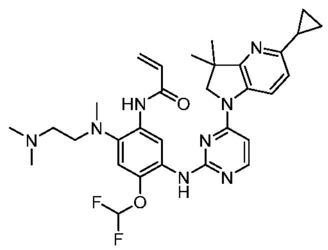 | *N*-(5-((4-(5-cyclopropyl-3,3-dimet hyl-2,3-dihydro-1*H*-pyrrolo[3,2-*b*]pyridin-1-yl)pyrimidin-2-yl)ami no)-4-(difluoromethoxy)-2-((2-(di methylamino)ethyl)(methyl)amin o)phenyl)acrylamide | 593.3 |
| **Example 47** | 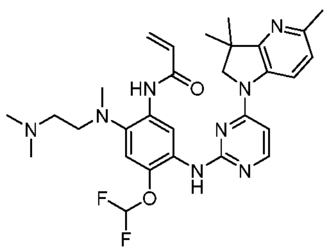 | *N*-(4-(difluoromethoxy)-2-((2-(di methylamino)ethyl)(methyl)amin o)-5-((4-(3,3,5-trimethyl-2,3-dihy dro-1*H*-pyrrolo[3,2-*b*]pyridin-1-y l)pyrimidin-2-yl)amino) phenyl)ac rylamide | 567.2 |
| **Example 48** | 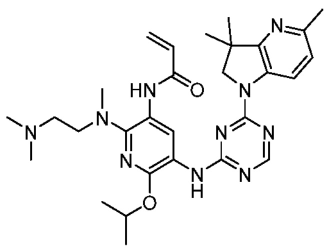 | *N*-(2-((2-(dimethylamino)ethyl)(m ethyl)amino)-6-isopropoxy-5-((4-(3,3,5-trimethyl-2,3-dihydro-1*H*-p yrrolo[3,2-*b*]pyridin-1-yl)-1,3,5-tr iazin-2-yl)amino) pyridin-3 -yl)acr ylamide | 561.3 |
| **Example 49** | 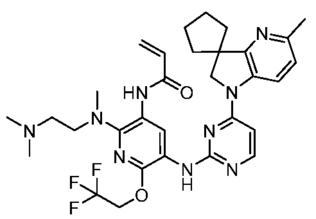 | *N*-(2-((2-(dimethylamino)ethyl)(m ethyl)amino)-5-((4-(5'-methylspir o[cyclopentane-1,3'-pyrrolo[3,2-*b*]pyridin]-1'(2'*H*)-yl)pyrimidin-2-yl)amino)-6-(2,2,2-trifluoroethox y)pyridin-3-yl)acrylamide | 626.3 |

(continued)

| Example No. | Structural Formula | Chemical Name | ESI-MS: [M+1]+ |
|---|---|---|---|
| **Example 50** | 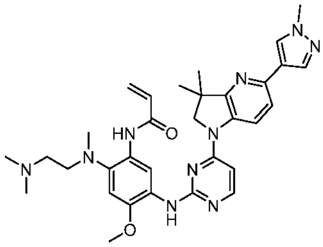 | *N*-(5-((4-(3,3-dimethyl-5-(1-meth yl-1*H*-pyrazol-4-yl)-2,3-dihydro-1 *H*-pyrrolo[3,2-*b*]pyridin-1-yl)pyri midin-2-yl)amino)-2-((2-(dimethy lamino)ethyl)(methyl) amino)-4-m ethoxyphenyl)acrylamide | 597.4 |
| **Example 51** | 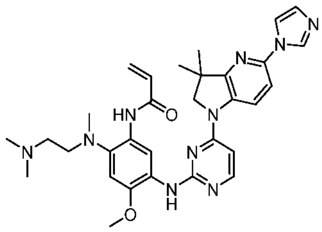 | *N*-(5-((4-(5-(1*H*-imidazole-1-yl)-3,3-dimethyl-2,3-dihydro-1*H*-pyrr olo[3,2-*b*]pyridin-1-yl)pyrimidin-2-yl) amino)-2-((2-(dimethylamin o)ethyl)(methyl)amino)-4-methox yphenyl)acrylamide | 583.2 |
| **Example 52** | 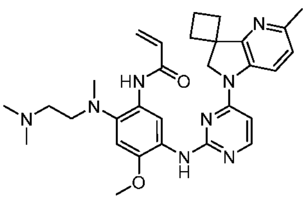 | *N*-(2-((2-(dimethylamino)ethyl)(m ethyl)amino)-4-methoxy-5-((4-(5' -methylspiro[cyclobutane-1,3'-pyr rolo[3,2-*b*]pyridin]-1'(2'*H*)-yl)pyri midin-2-yl)amino) phenyl)acrylam ide | 543.2 |
| **Example 53** | 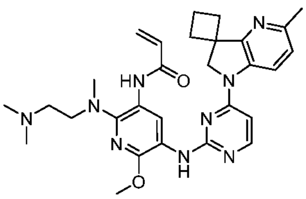 | *N*-(2-((2-(dimethylamino)ethyl)(m ethyl)amino)-6-methoxy-5-((4-(5' -methylspiro[cyclobutane-1,3'-pyr rolo[3,2-*b*]pyridin]-1'(2'*H*)-yl)pyri midin-2-yl)amino) pyridin-3-yl)acr ylamide | 544.2 |
| **Example 55** | 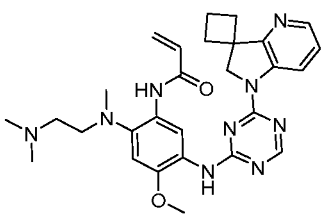 | *N*-(2-((2-(dimethylamino)ethyl)(m ethyl)amino)-4-methoxy-5-((4-(sp iro[cyclobutane-1,3'-pyrrolo[3,2-*b*] pyridin]-1'(2'*H*-yl)-1,3,5-triazin -2-yl)amino)phenyl) acrylamide | 530.2 |
| **Example 56** | 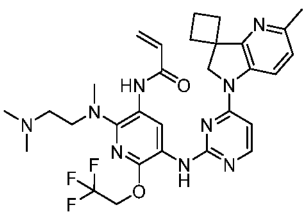 | *N*-(2-((2-(dimethylamino)ethyl)(m ethyl)amino)-5-((4-(5'-methylspir o[cyclobutane-1,3'-pyrrolo[3,2-*b*] pyridin]-1'(2'*H*)-yl)pyrimidin-2-y l)amino)-6-(2,2,2-trifluoroethoxy) pyridin-3 -yl)acrylamide | 612.3 |

(continued)

| Example No. | Structural Formula | Chemical Name | ESI-MS: [M+1]$^+$ |
|---|---|---|---|
| **Example 57** | 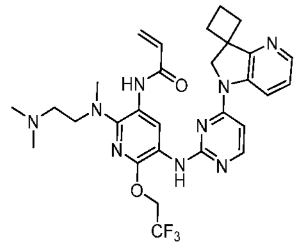 | *N*-(2-((2-(dimethylamino)ethyl)(m ethyl)amino)-5-((4-(spiro[cyclobu tane-1,3'-pyrrolo[3,2-*b*]pyridin]-1' (2'*H*)-yl)pyrimidin-2-yl)amino)-6-(2,2,2-trifluoroethoxy)pyridin-3-y l)acrylamide | 598.2 |
| **Example 61** | 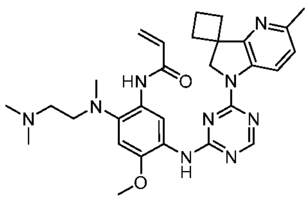 | *N*-(2-((2-(dimethylamino)ethyl)(m ethyl)amino)-4-methoxy-5-((4-(5' -methylspiro[cyclobutane-1,3'-pyr rolo[3,2-b]pyridin]-1'(2'*H*)-yl)-1, 3,5-triazin-2-yl) amino)phenyl)acr ylamide | 544.2 |
| **Example 62** | 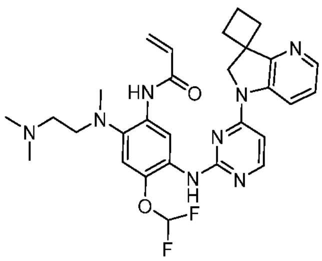 | *N*-(4-(difluoromethoxy)-2-((2-(di methylamino)ethyl) (methyl)amin o)-5-((4-(spiro[cyclobutane-1,3'-p yrrolo[3,2-*b*]pyridin]-1'(2'*H*)-yl)p yrimidin-2-yl)amino) phenyl)acryl amide | 565.2 |
| **Example 63** | 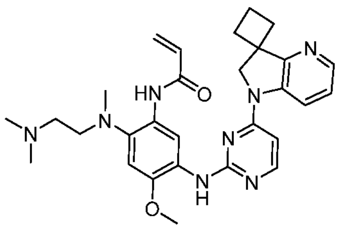 | *N*-(2-((2-(dimethylamino)ethyl)(m ethyl)amino)-4-methoxy-5-((4-(sp iro[cyclobutane-1,3'-pyrrolo[3,2-*b*] pyridin]-1'(2'*H*)-yl)pyrimidin-2-yl)amino)phenyl) acrylamide | 529.2 |
| **Example 64** | 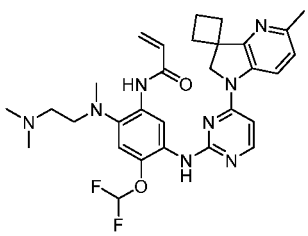 | *N*-(4-(difluoromethoxy)-2-((2-(di methylamino)ethyl) (methyl)amin o)-5-((4-(5'-methylspiro[cyclobut ane-1,3'-pyrrolo[3,2-*b*]pyridin]-1' (2'*H*)-yl)pyrimidin-2-yl) amino)ph enyl)acrylamide | 579.2 |
| **Example 65** | 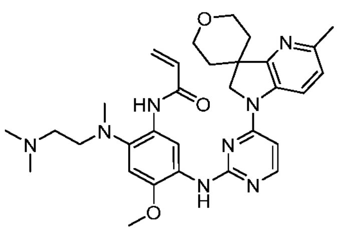 | *N*-(2-((2-(dimethylamino)ethyl)(m ethyl)amino)-4-methoxy-5-((4-(5' -methyl-2,3, 5,6-tetrahydrogenspir o[pyran-4,3'-pyrrolo[3,2-*b*]pyridi n]-1'(2'*H*)-yl) pyrimidin-2-yl)amin o)phenyl)acrylamide | 573.2 |

(continued)

| Example No. | Structural Formula | Chemical Name | ESI-MS: [M+1]+ |
|---|---|---|---|
| **Example 66** | 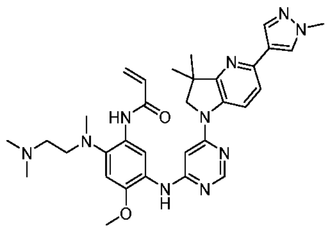 | *N*-(5-((6-(3,3-dimethyl-5-(1-meth yl-1*H*-pyrazol-4-yl)-2,3-dihydro-1 *H*-pyrrolo[3,2-*b*]pyridin-1-yl)pyri midin-4-yl)amino)-2-((2-(dimethy lamino)ethyl)(methyl) amino)-4-m ethoxyphenyl)acrylamide | 597.4 |
| **Example 67** | 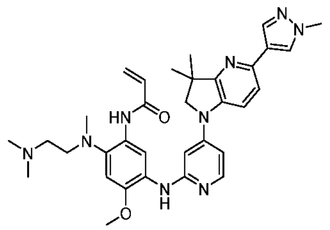 | *N*-(5-((4-(3,3-dimethyl-5-(1-meth yl-1*H*-pyrazol-4-yl)-2,3-dihydro-1 *H*-pyrrolo[3,2-*b*]pyridin-1-yl)pyri din-2-yl)amino)-2-((2-(dimethyla mino)ethyl)(methyl) amino)-4-met hoxyphenyl)acrylamide | 596.3 |
| **Example 69** | 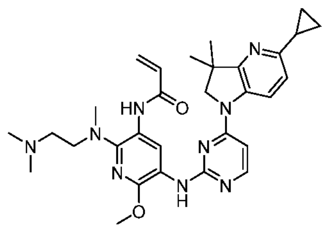 | *N*-(5-((4-(5-cyclopropyl-3,3-dimet hyl-2,3-dihydro-1H-pyrrolo[3,2-*b*]pyridin-1-yl)pyrimidin-2-yl)ami no)-2-((2-(dimethylamino)ethyl) (methyl)amino)-6-methoxypyridin -3-yl)acrylamide | 558.2 |
| **Example 70** | 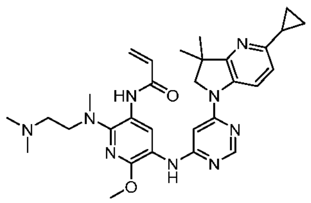 | *N*-(-((6-(5 -cyclopropyl-3,3 -dimet hyl-2,3-dihydro-1*H*-yrrolo[3,2-*b*]pyridin-1-yl)pyrimidin-4-yl)ami no)-2-((2-(dimethylamino)ethyl) (methyl)amino)-6-methoxypyridin -3-yl)acrylamide | 558.4 |
| **Example 71** | 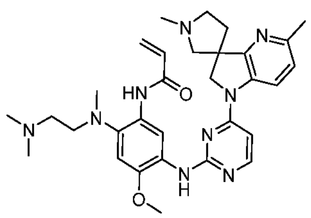 | *N*-(2-((2-(dimethylamino)ethyl)(m ethyl)amino)-5-((4-(1,5'-dimethyl spiro[pyrrolidin-3,3'-pyrrolo[3,2-*b*]pyridin]-1'(2'*H*)-yl)pyrimidin-2-yl)amino)-4-methoxyphenyl)acryl amide | 572.2 |
| **Example 72** | 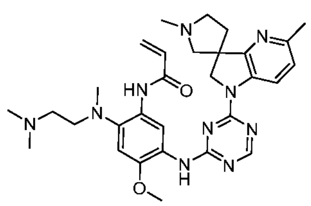 | *N*-(2-((2-(dimethylamino)ethyl)(m ethyl)amino)-5-((4-(1,5'-dimethyl spiro[pyrrolidin-3,3'-pyrrolo[3,2-*b*]pyridin]-1'(2'*H*)-yl)-1,3,5-triazin -2-yl)amino)-4-methoxyphenyl)ac rylamide | 573.2 |

(continued)

| Example No. | Structural Formula | Chemical Name | ESI-MS: [M+1]+ |
|---|---|---|---|
| Example 73 | 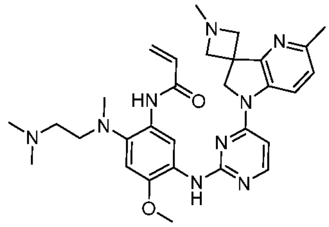 | *N*-(2-((2-(dimethylamino)ethyl)(m ethyl)amino)-5-((4-(1,5'-dimethyl spiro[azetidine-3,3'-pyrrolo[3,2-*b*] pyridin]-1'(2'*H*)-yl)pyrimidin-2-y l)amino)-4-methoxyphenyl)acryla mide | 558.3 |
| Example 74 | 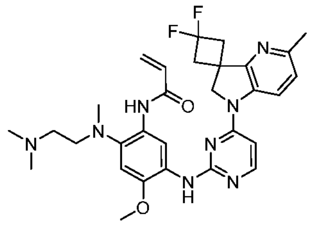 | *N*-(5-((4-(3,3-difluoro-5'-methylsp iro[cyclobutane-1,3'-pyrrolo[3,2-*b*]pyridin]-1'(2'*H*)-yl)pyrimidin-2-yl) amino)-2-((2-(dimethylamino)e thyl)(methyl)amino)-4-methoxyph enyl)acrylamide | 579.2 |

**The magnetic resonance imaging data of the compound prepared from the above example was as follows:**

| Example No. | $^1$H NMR (400 MHz) |
|---|---|
| Example 2 | (DMSO-$d_6$) δ 10.09 (s, 1H), 8.81 (s, 1H), 7.94 (d, *J* = 5.9 Hz, 1H), 7.78 (s, 1H), 7.61 (d, *J* = 8.2 Hz, 1H), 7.04 - 6.89 (m, 2H), 6.75 (d, *J* = 2.1 Hz, 1H), 6.56 (dd, *J* = 5.9, 2.1 Hz, 1H), 6.38 (dd, *J* = 16.9, 10.0 Hz, 1H), 6.22 (dd, *J* = 16.9, 2.2 Hz, 1H), 5.74 (dd, *J* = 10.0, 2.2 Hz, 1H), 3.84 (s, 3H), 3.77 (s, 2H), 2.86 (t, *J* = 5.9 Hz, 2H), 2.68 (s, 3H), 2.40 (s, 3H), 2.28 (t, *J* = 5.8 Hz, 2H), 2.20 (s, 6H), 1.30 (s, 6H) |
| Example 3 | (DMSO-$d_6$) δ 9.51 (s, 1H), 8.41 (s, 1H), 7.92 (d, *J* = 5.8 Hz, 1H), 7.75 (s, 1H), 7.59 (d, *J* = 8.2 Hz, 1H), 6.96 (d, *J* = 8.2 Hz, 1H), 6.78 (s, 1H), 6.69 (d, *J* = 2.1 Hz, 1H), 6.60 - 6.47 (m, 2H), 6.20 (dd, *J* = 16.9, 2.1 Hz, 1H), 5.71 (dd, *J* = 10.1, 2.1 Hz, 1H), 3.82 (s, 3H), 3.80 - 3.73 (m, 2H), 3.61 - 3.54 (m, 1H), 2.94-2.88(m, 1H), 2.40 (s, 3H), 2.27-2.23 (m, 1H), 2.19-2.14 (m, 2H), 2.12 (s, 6H), 2.09 - 2.04 (m, 1H), 1.91-1.84 (m, 2H), 1.69-1.61 (m, 1H), 1.30 (s, 6H) |
| Example 4 | (DMSO-$d_6$) δ 9.44 (s, 1H), 8.34 (s, 1H), 7.88 (t, *J* = 5.8 Hz, 2H), 7.71 (s, 1H), 7.59 (d, *J* = 8.1 Hz, 1H), 7.03 (dd, *J* = 8.1, 4.9 Hz, 1H), 6.77 - 6.62 (m, 2H), 6.50 (dd, *J* = 6.1, 2.2 Hz, 1H), 6.48 - 6.40 (m, 1H), 6.13 (dd, *J* = 17.0, 2.1 Hz, 1H), 5.64 (dd, *J* = 10.1, 2.1 Hz, 1H), 3.75 (s, 3H), 3.71 (t, *J* = 9.0 Hz, 2H), 3.51 (s, 1H), 2.96-2.80(m, 1H), 2.52-2.49(m, 1H), 2.19 (dd, *J* = 12.2, 5.1 Hz, 1H), 2.06 (s, 6H), 2.01 (d, *J* = 4.5 Hz, 2H), 1.85-1.76 (m, 2H), 1.62-1.54 (m, 1H), 1.25 (s, 6H) |
| Example 5 | (DMSO-$d_6$) 810.05 (s, 1H), 8.58 (s, 1H), 8.16 (d, *J* = 8.2 Hz, 1H), 8.10 (d, *J* = 5.8Hz, 1H), 8.03 (s, 1H), 7.01 (s, 1H), 6.80 (d, *J* = 8.5 Hz, 1H), 6.40 (dd, *J* = 16.9, 10.1 Hz, 1H), 6.25 (d, *J* =5.9 Hz, 1H), 6.17 (dd, *J* = 17.0, 2.1 Hz, 1H), 5.72 (dd, *J* = 10.1, 2.1 Hz, 1H), 3.82 (s, 2H), 3.78 (s, 3H),2.90 (t, *J* = 5.9 Hz, 2H), 2.73 (s, 3H), 2.36 (s, 3H), 2.32 (t, *J* = 5.9 Hz, 2H), 2.21 (s, 6H), 1.30 (s, 6H) |
| Example 6 | (DMSO-$d_6$) δ 9.47 (s, 1H), 8.26 (d, *J* = 8.0Hz, 1H), 8.17 - 8.09 (m, 2H), 8.06 (s, 1H), 8.00 (dd, *J* = 4.9, 1.4Hz, 1H), 6.99 (dd, *J* = 8.2, 4.9Hz, 1H), 6.80 (s, 1H), 6.55 (dd, *J* = 17.0, 10.2 Hz, 1H), 6.25 (d, *J* = 5.9Hz, 1H), 6.16 (dd, *J* = 17.0, 2.1Hz, 1H), 5.69 (dd, *J* = 10.1, 2.1Hz, 1H), 3.83 (d, *J* = 3.5Hz, 2H), 3.77 (s, 3H), 3.67 (s, 1H), 3.42 (q, *J* = 7.4Hz, 1H), 2.95 (q, *J* = 7.7Hz, 1H), 2.28 (dd, *J* = 12.1, 4.7Hz, 1H), 2.12 (s, 8H), 1.90 (q, *J* = 8.5, 7.6Hz, 2H), 1.75 - 1.65 (m, 1H), 1.32 (d, *J* = 2.9 Hz, 6H) |
| Example 7 | (DMSO-$d_6$) δ 9.46 (s, 1H), 8.21 - 8.11 (m, 2H), 8.09 (d, *J* = 5.8Hz, 1H), 7.98 (s, 1H), 6.85 (d, *J* = 8.2Hz, 1H), 6.79 (s, 1H), 6.55 (dd, *J* = 17.0, 10.2Hz, 1H), 6.22 (d, *J* = 5.8Hz, 1H), 6.16 (dd, *J* = 17.0, 2.1Hz, 1H), 5.69 (dd, *J* = 10.1, 2.1Hz, 1H), 3.81 (s, 2H), 3.77 (s, 3H), 3.68 (s, 1H), 3.41 (t, *J* = 7.9Hz, 1H), 3.02 - 2.89 (m, 1H), 2.38 (s, 3H), 2.28 (dd, *J* = 12.0, 4.7Hz, 1H), 2.13 (s, 8H), 1.90 (q, *J* = 7.4Hz, 2H), 1.70 (dd, *J* = 12.6, 6.5Hz, 1H), 1.30 (d, *J* = 2.9Hz, 6H) |

(continued)

| Example No. | $^1$H NMR (400 MHz) |
|---|---|
| Example 8 | (DMSO-$d_6$) δ10.06 (s, 1H), 9.06 (s, 1H), 8.34 (s, 3H), 7.03 (s, 2H), 6.42 (dd, *J* = 16.9, 10.0 Hz, 1H), 6.28-6.20 (m, 1H), 5.74 (d, *J* = 10.5 Hz, 1H), 4.18-3.66 (m, 5H), 2.89 (s, 2H), 2.73(s, 3H), 2.48-2.30 (m, 5H), 2.21 (s, 6H), 1.30 (s, 6H) |
| Example 10 | (DMSO-$d_6$) δ 10.12 (s, 1H), 9.21 (s, 1H), 8.35 (s, 2H), 7.19 (s, 1H), 6.86 (d, *J* = 159.9 Hz, 1H), 6.42 (dd, *J* = 16.9, 10.1 Hz, 1H), 6.24 (t, *J* = 24.4 Hz, 1H), 5.77 (d, *J* = 10.1 Hz, 1H), 4.73 (d, *J* = 68.1 Hz, 2H), 3.99 (d, *J* = 61.2 Hz, 2H), 2.89 (s, 2H), 2.73 (s, 3H), 2.37 (t, *J* = 18.2 Hz, 5H), 2.21 (s, 6H), 1.29 (s, 6H) |
| Example 11 | (DMSO-$d_6$) δ 10.13 (s, 1H), 8.56 (s, 1H), 8.37 (d, *J* = 8.3 Hz, 1H), 8.32 (s, 1H), 8.28 (s, 1H), 6.99 (t, *J* = 4.1 Hz, 2H), 6.39 (dd, *J* = 16.9, 10.0 Hz, 1H), 6.28 - 6.17 (m, 2H), 5.75 (dd, *J* = 9.9, 2.1 Hz, 1H), 3.82 (s, 3H), 3.76 (s, 2H), 2.87 (t, *J* = 5.8 Hz, 2H), 2.71 (s, 3H), 2.41 (s, 3H), 2.31 (t, *J* = 5.9 Hz, 2H), 2.21 (s, 6H), 1.32 (s, 6H) |
| Example 12 | (DMSO-$d_6$) δ 9.45 (s, 1H), 8.40 (d, *J* = 8.2Hz, 1H), 8.27 (s, 1H), 8.21 (s, 1H), 7.95 (d, *J* = 4.8Hz, 2H), 7.07 (dd, *J* = 8.2, 4.9Hz, 1H), 6.69 (s, 1H), 6.46 (dd, *J* = 16.9, 10.1Hz, 1H), 6.23 - 6.01 (m, 2H), 5.64 (dd, *J* = 10.0, 2.2Hz, 1H), 3.74 (s, 4H), 3.62 (dd, *J* = 18.0, 8.4Hz, 2H), 3.40 - 3.30 (m, 1H), 2.88 (td, *J* = 8.9, 8.4, 5.3Hz, 1H), 2.19 (dd, *J* = 12.0, 4.8Hz, 1H), 2.16-1.95 (m, 8H), 1.80 (tt, *J* = 12.7, 6.3Hz, 2H), 1.59 (dq, *J* = 14.0, 7.3Hz, 1H), 1.26 (s, 6H) |
| Example 13 | (DMSO-$d_6$) δ 9.53 (s, 1H), 8.37 (d, *J* = 8.2 Hz, 1H), 8.28 (d, *J* = 8.1 Hz, 2H), 8.03 (s, 1H), 6.99 (d, *J* = 8.3 Hz, 1H), 6.76 (s, 1H), 6.54 (dd, *J* = 16.9, 10.2 Hz, 1H), 6.21 (dd, *J* = 16.9, 2.2 Hz, 1H), 6.11 (s, 1H), 5.71 (dd, *J* = 10.2, 2.1 Hz, 1H), 3.81 (s, 4H), 3.69 (d, *J* = 10.3 Hz, 2H), 3.42 (dd, *J* = 9.7, 6.9 Hz, 1H), 2.96 (td, *J* = 9.1, 8.4, 5.3 Hz, 1H), 2.41 (s, 3H), 2.27 (dd, *J* = 12.0, 4.8 Hz, 1H), 2.20 (d, *J* = 14.7 Hz, 1H), 2.14 (s, 6H), 2.07 (dd, *J* = 12.0, 8.0 Hz, 1H), 1.88 (dd, *J* = 15.7, 7.9 Hz, 2H), 1.67 (dd, *J* = 12.8, 6.4 Hz, 1H), 1.32 (s, 6H) |
| Example 14 | (DMSO-$d_6$) δ 10.07 (s, 1H), 8.34 (s, 3H), 7.02 (s, 2H), 6.41 (dd, *J* = 16.9, 10.1 Hz,1H), 6.20 (t, *J* = 18.7 Hz, 1H), 5.75 (s, 1H), 4.37 (s, 1H), 4.17 (s, 1H), 3.82 (s, 1H), 3.78 (s, 2H), 2.89-2.81 (m, 2H), 2.80-2.62 (m, 3H), 2.36 (d, *J* = 11.6 Hz, 3H), 2.30 (s, 2H), 2.21 (s, 6H), 1.26-1.10 (m, 4H) |
| Example 15 | ($CDCl_3$) δ 9.94 (s, 1H), 9.06 (s, 1H), 7.97 (d,J = 6.0Hz, 1H), 7.48 (d,J = 8.3Hz, 1H), 6.81 (d,J = 8.2Hz, 1H), 6.62 (d,J = 2.2Hz, 1H), 6.51 (dd,J = 6.0, 2.1Hz, 1H), 6.41 - 6.29 (m, 2H), 5.70 - 5.60 (m, 1H), 3.88 (s, 3H), 3.77 (s, 2H), 2.98 (s, 2H), 2.67 (s, 3H), 2.42 (s, 5H), 2.34 (s, 6H), 1.34 (s, 6H) |
| Example 16 | ($CDCl_3$) δ 10.01 (s, 1H), 9.70 (s, 1H), 8.43 (d, J = 6.8 Hz, 2H), 8.01 (s, 1H), 7.74 (s, 1H), 6.79 (s, 1H), 6.45 (d, J = 16.1 Hz, 1H), 6.35 (s, 1H), 5.70 (d, J = 10.0 Hz, 1H), 4.38 (s, 2H), 3.88 (s, 3H), 2.91 (s, 2H), 2.71 (s, 3H), 2.36 (s, 4H), 2.32 - 2.25 (m, 6H), 1.47 (s, 6H) |
| Example 17 | (DMSO-$d_6$) 6 10.06 (s, 1H), 8.59 (s, 1H), 8.14 (d,J = 8.1Hz, 1H), 8.10 (d,J = 5.9 Hz, 1H), 8.02 (s, 1H), 7.02 (s, 1H), 6.77 (d,J= 8.4Hz, 1H), 6.42 (dd,J = 16.9, 10.1Hz, 1H), 6.27 - 6.11 (m, 2H), 5.74 (dd,J = 10.2, 1.8Hz, 1H), 3.81-3.79 (m, 5H), 2.90 (d,J = 6.1Hz, 2H), 2.74 (s, 3H), 2.34 (s, 2H), 2.22 (s, 6H), 1.97 (dt,J = 7.8, 3.6Hz, 1H), 1.28 (s, 6H), 0.95 - 0.75 (m, 4H) |
| Example 18 | (DMSO-$d_6$) δ 9.82 (s, 1H), 8.69 (s, 1H), 8.54 (s, 1H), 8.14 (d, J = 5.8 Hz, 1H), 8.08 (s, 1H), 8.03 (s, 1H), 7.53 (p, J = 7.4 Hz, 1H), 7.24 (t, J = 7.9 Hz, 2H), 6.89 (s, 1H), 6.46 - 6.26 (m, 2H), 6.18 (d, J = 16.8 Hz, 1H), 5.71 (d, J = 10.1 Hz, 1H), 3.98 (s, 2H), 3.77 (s, 3H), 2.77 (d, J = 5.8 Hz, 2H), 2.66 (s, 3H), 2.29 (t, J = 5.8 Hz, 2H), 2.19 (s, 6H), 1.39 (s, 6H) |
| Example 19 | ($CDCl_3$) δ 9.85 (s, 1H), 9.54 (s, 1H), 8.34 (s, 1H), 8.28 (s, 1H), 8.25 (d, J = 6.5 Hz, 1H), 7.46 (d, J = 6.5 Hz, 2H), 7.37 (q, J = 7.2, 6.7 Hz, 1H), 7.20 (dd, J = 19.7, 9.2 Hz, 2H), 6.77 (s, 1H), 6.37-6.33 (m, 3H), 5.66 (d, J = 10.0 Hz, 1H), 4.12 (s, 2H), 3.87 (s, 3H), 2.86 (d, J = 5.9 Hz, 2H), 2.69 (s, 3H), 2.29 (d, J = 16.9 Hz, 8H), 1.50 (s, 6H) |
| Example 20 | (DMSO-$d_6$) δ 10.06 (s, 1H), 9.06 (br, 1H)8.59 - 7.64 (m, 3H), 7.03 (s, 1H), 6.29 (d, J = 76.2 Hz, 2H), 5.74 (s, 1H), 4.09 (s, 1H), 3.87 (d, J = 18.7 Hz, 2H), 3.75 (s, 2H), 2.88 (s, 2H), 2.73 (s, 2H), 2.34 (d, J = 25.4 Hz, 8H), 2.21 (s, 6H), 1.27 (d, J = 8.8 Hz, 6H) |
| Example 21 | (DMSO-$d_6$) δ 10.02 (s, 1H), 8.63 (s, 1H), 8.09-8.04 (m, 3H), 7.01 (s, 1H), 6.38 (dd, J = 16.9, 10.1 Hz, 1H), 6.28 - 6.10 (m, 2H), 5.72 (dd, J = 10.0, 2.2 Hz, 1H), 3.81 (s, 2H), 3.78 (s, 3H), 2.87 (t, J = 5.8 Hz, 2H), 2.72 (s, 3H), 2.34-2.31 (m, 5H), 2.21 (s, 6H), 2.08 (s, 3H), 1.29 (s, 6H) |

(continued)

| Example No. | $^1$H NMR (400 MHz) |
|---|---|
| **Example 22** | ($CDCl_3$) δ 10.01 (s, 1H), 9.69 (s, 1H), 8.56 (d, J = 8.6 Hz, 1H), 8.41 (s, 1H), 7.76 (s, 1H), 7.13 (d, J = 8.4 Hz, 1H), 6.79 (s, 1H), 6.55 - 6.16 (m, 2H), 5.71 (d, J = 9.6 Hz, 1H), 4.40 (s, 2H), 3.88 (s, 3H), 2.91 (s, 2H), 2.72 (s, 3H), 2.31 (s, 8H), 1.45 (d, J = 22.5 Hz, 6H) |
| **Example 23** | (DMSO-$d_6$) δ 10.01 (s, 1H), 8.61 (s, 1H), 8.22 - 8.00 (m, 3H), 7.01 (s, 1H), 6.39 (dd,J = 16.9, 10.1Hz, 1H), 6.30 - 6.11 (m, 2H), 5.73 (dd,J = 10.1, 2.2Hz, 1H), 3.81 (d,J = 19.4Hz, 5H), 2.88 (t,J = 5.8Hz, 2H), 2.80 (t,J = 7.6Hz, 2H), 2.73 (d,J = 5.0Hz, 5H), 2.34 (t,J = 5.8Hz, 2H), 2.22 (s, 6H), 2.01 (t,J = 7.4Hz, 2H), 1.30 (s, 6H) |
| **Example 24** | (DMSO-$d_6$) δ 10.05 (s, 1H), 8.64 (s, 1H), 8.14 (dd,J = 13.6, 7.0Hz, 2H), 7.96 (s, 1H), 6.99 (s, 1H), 6.80 (d,J = 8.2Hz, 1H), 6.41 (dd,J = 16.9, 10.1Hz, 1H), 6.29 (d,J = 5.9Hz, 1H), 6.17 (dd,J = 17.0, 2.1Hz, 1H), 5.73 (dd,J = 10.0, 2.1Hz, 1H), 4.05 (q,J = 6.9Hz, 2H), 3.84 (s, 2H), 2.89 (t,J = 5.9Hz, 2H), 2.72 (s, 3H), 2.37 (s, 3H), 2.32 (t,J = 5.9Hz, 2H), 2.21 (s, 6H), 1.30 (d,J = 10.1 Hz, 9H) |
| **Example 25** | (DMSO-$d_6$) δ 9.98 (s, 1H), 9.04 (d,J = 95.8Hz, 1H), 8.46 - 7.66 (m, 3H), 6.94 (s, 2H), 6.35 (dd,J = 16.8, 10.0Hz, 1H), 6.27 - 6.05 (m, 1H), 5.69 (s, 1H), 4.27 - 3.70 (m, 4H), 2.92 - 2.74 (m, 2H), 2.64 (s, 3H), 2.41 - 2.23 (m, 5H), 2.13 (s, 6H), 1.24 (s, 9H) |
| **Example 26** | (DMSO-$d_6$) δ 10.05 (s, 1H), 8.49 (s, 1H), 8.37 - 8.13 (m, 3H), 6.93 (s, 1H), 6.32 (dd,J = 16.9, 10.0Hz, 1H), 6.15 (d,J = 26.9Hz, 2H), 5.68 (d,J = 10.1Hz, 1H), 3.73 (d,J = 22.2Hz, 5H), 2.85 - 2.72 (m, 6H), 2.64 (s, 3H), 2.24 (t,J = 6.1Hz, 2H), 2.14 (s, 6H), 2.03 - 1.96 (m, 2H), 1.24 (s, 6H) |
| **Example 27** | (DMSO-$d_6$) δ 10.00 (s, 1H), 8.70 (s, 1H), 8.16 - 8.01 (m, 2H), 7.95 (s, 1H), 6.99 (s, 1H), 6.38 (dd, J = 16.9, 10.0 Hz, 1H), 6.29 (d, J = 5.5 Hz, 1H), 6.22 - 6.10 (m, 1H), 5.72 (d, J = 9.9 Hz, 1H), 4.04 (q, J = 6.9 Hz, 2H), 3.83 (s, 2H), 2.86 (t, J = 5.9 Hz, 2H), 2.70 (s, 3H), 2.32 (s, 5H), 2.20 (s, 6H), 2.07 (s, 3H), 1.28 (d, J = 8.2 Hz, 9H) |
| **Example 28** | (DMSO-$d_6$) δ 10.12 (s, 1H), 8.54 (s, 1H), 8.29 (s, 2H), 8.24 (s, 1H), 6.99 (s, 1H), 6.39 (dd, \J = 16.9, 10.0 Hz, 1H), 6.23 (dd, J = 16.7, 2.0 Hz, 1H), 6.17 (s, 1H), 5.75 (dd, J = 9.9, 2.2 Hz, 1H), 3.82 (s, 3H), 3.74 (s, 2H), 2.87 (t, J = 5.8 Hz, 2H), 2.71 (s, 3H), 2.36 (s, 3H), 2.31 (t, J = 5.9 Hz, 2H), 2.22 (s, 3H), 2.21 (s, 6H), 1.30 (s, 6H) |
| **Example 29** | (DMSO-$d_6$) δ 10.07 (s, 1H), 8.69 (d, *J* = 289.2 Hz, 3H), 7.03 (s, 2H), 6.40 (dd, *J* = 17.0, 10.0 Hz, 2H), 6.20 (s, 1H), 5.74 (*d, J* = 9.9 Hz, 1H), 4.11 (s, 1H), 3.96 (s, 1H), 3.80 (d, *J* = 36.7 Hz, 3H), 2.88 (s, 2H), 2.72 (s, 3H), 2.39 (d, *J* = 26.1 Hz, 5H), 2.20 (s, 6H), 1.85 (d, *J* = 67.1 Hz, 8H) |
| **Example 30** | (DMSO-$d_6$) δ 10.04 (s, 1H), 9.11 (br, 1H), 8.35 (dd, J = 36.7, 18.7 Hz, 2H), 7.97 (br, 1H), 7.01 (s, 1H), 6.38 (d, J = 12.0 Hz, 1H), 6.24 (d, J = 34.0 Hz, 1H), 5.74 (s, 1H), 4.23 - 3.83 (m, 4H), 2.87 (t, J = 5.8 Hz,2H), 2.69 (d, J = 16.3 Hz, 3H), 2.41 - 2.24 (m, 8H), 2.21 (s, 6H), 1.42 - 1.14 (m, 9H) |
| **Example 31** | (DMSO-$d_6$) δ 10.10 (d, J = 33.9 Hz, 1H), 9.10 - 8.29 (m, 3H), 7.03 (s, 2H), 6.39 (s, 1H), 6.22 (d, J = 16.8 Hz, 1H), 5.74 (d, J = 10.1 Hz, 1H), 4.02 (d, J = 29.8 Hz, 2H), 3.80 (d, J = 38.1 Hz, 3H), 2.88 (s, 2H), 2.73 (d, J = 13.0 Hz, 3H), 2.39 (d, J = 25.0 Hz, 5H), 2.20 (s, 6H), 1.78 - 1.25 (m, 10H) |
| **Example 32** | (DMSO-$d_6$) δ 10.06 (s, 1H), 8.56 (s, 1H), 8.17 (d, J = 8.3 Hz, 1H), 8.10 (d, J = 5.8 Hz, 1H), 8.04 (s, 1H), 7.00 (s, 1H), 6.77 (d, J = 8.2 Hz, 1H), 6.45 - 6.29 (m, 2H), 6.16 (dd, J = 17.0, 2.0 Hz, 1H), 5.72 (dd, J = 10.2, 2.1 Hz, 1H), 3.86 (s, 2H), 3.78 (s, 3H), 2.89 (t, J = 5.9 Hz, 2H), 2.73 (s, 3H), 2.34 (d, J = 11.9 Hz, 5H), 2.21 (s, 6H), 1.79 - 1.62 (m, 6H), 1.50 (dd, J = 29.8, 13.2 Hz, 4H) |
| **Example 33** | (DMSO-$d_6$) δ 10.05 (s, 1H), 8.58 (s, 1H), 8.09 (dd, *J* = 27.0, 21.4 Hz, 3H), 7.01 (s, 1H), 6.78 (d, *J* = 8.5 Hz, 1H), 6.40 (dd, *J* = 17.0, 10.3 Hz, 1H), 6.32 - 6.10 (m, 2H), 5.72 (d, *J* = 10.3 Hz, 1H), 3.83 (d, *J* = 33.2 Hz, 5H), 2.90 (s, 2H), 2.73 (s, 3H), 2.34 (d, *J* = 13.4 Hz, 5H), 2.21 (s, 6H), 1.98 - 1.72 (m, 8H) |
| **Example 34** | (DMSO-$d_6$) δ 10.05 (s, 1H), 9.17 (br, 1H), 8.36-7.92 (m, 3H), 7.04 (s, 1H), 6.40 (dd, J = 16.7, 10.2 Hz, 1H), 6.21 (s, 1H), 5.74 (s, 1H), 4.05 (d, J = 70.8 Hz, 2H), 3.76 (s, 3H), 2.88 (t, J = 5.8 Hz, 2H), 2.73 (s, 3H), 2.45 - 2.24 (m, 3H), 2.21 (s, 6H), 2.03 (br, 2 H), 1.27 (s, 6H) |

(continued)

| Example No. | $^1$H NMR (400 MHz) |
|---|---|
| **Example 35** | (DMSO-$d_6$) δ 10.12 (s, 1H), 8.53 (s, 1H), 8.30 (s, 1H), 8.26 (s, 1H), 8.18 (s, 1H), 6.98 (s, 1H), 6.39 (dd, J = 16.9, 10.0 Hz, 1H), 6.23 (dd, J = 17.0, 2.2 Hz, 1H), 6.18 (s, 1H), 5.75 (dd, J = 9.9, 2.2 Hz, 1H), 4.09 (q, J = 6.9 Hz, 2H), 3.76 (s, 2H), 2.86 (t, J = 5.9 Hz, 2H), 2.70 (s, 3H), 2.36 (s, 3H), 2.30 (t, J = 5.8 Hz, 2H), 2.21 (d, J = 7.0 Hz, 9H), 1.31 (d, J = 5.8 Hz, 9H) |
| **Example 36** | (DMSO-$d_6$) δ 10.14 (s, 1H), 8.51 (d, J = 43.8 Hz, 2H), 8.13 (dd, J = 22.4, 7.1 Hz, 2H), 7.16 (s, 1H), 7.01 (t, J = 74.8 Hz, 1H), 6.76 (d, J = 8.2 Hz, 1H), 6.50 - 6.32 (m, 2H), 6.20 (dd, J = 16.9, 2.1 Hz, 1H), 5.77 (dd, J = 10.1, 2.2 Hz, 1H), 3.86 (s, 2H), 2.88 (t, J = 5.8 Hz, 2H), 2.72 (s, 3H), 2.36 (s, 5H), 2.22 (s, 6H), 1.81 - 1.42 (m, 10H) |
| **Example 37** | (DMSO-$d_6$) δ 10.06 (s, 1H), 9.30-8.80 (br, 1H), 8.40-8.05 (m3H), 7.10-6.70 (m, , 2H), 6.43 (dd,J = 16.9, 10.0Hz, 1H), 6.35 - 6.07 (m, 1H), 5.76 (s, 1H), 4.66-4.53 (m, 1H), 4.16 (s, 1H), 3.92 (s, 1H), 2.88 (s, 2H), 2.76 - 2.65 (m, 3H), 2.43 (s, 3H), 2.38 - 2.28 (m, 2H), 2.20 (s, 6H), 1.35 - 1.17 (m, 12H) |
| **Example 38** | (DMSO-$d_6$) δ 9.97 (s, 1H), 8.62 (s, 1H), 8.07 (dd,J = 10.3, 6.6Hz, 2H), 7.77 (s, 1H), 6.92 (s, 1H), 6.74 (d,J = 8.3Hz, 1H), 6.34 (dd,J = 17.0, 10.1Hz, 1H), 6.23 (d,J = 5.9Hz, 1H), 6.10 (dd,J = 17.0, 2.1Hz, 1H), 5.66 (dd,J = 10.0, 2.1Hz, 1H), 4.56 - 4.41 (m, 1H), 3.78 (s, 2H), 2.81 (t, J = 6.0Hz, 2H), 2.63 (s, 3H), 2.30 (s, 3H), 2.24 (t,J = 6.0Hz, 2H), 2.13 (s, 6H), 1.23 (s, 6H), 1.17 (d,J = 6.0Hz, 6H) |
| **Example 39** | (DMSO-$d_6$) δ 10.17 (s, 1H), 8.58 (s, 1H), 8.47 (s, 1H), 8.13 (dd, *J* = 20.8, 7.0 Hz, 2H), 7.22 - 6.82 (m, 2H), 6.77 (d, *J* = 8.2 Hz, 1H), 6.43 (dd, *J* = 16.9, 10.1 Hz, 1H), 6.30 - 6.14 (m, 2H), 5.78 (dd, *J* = 10.2, 2.0 Hz, 1H), 3.86 (s, 2H), 2.88 (t, *J* = 5.8 Hz, 2H), 2.73 (s, 3H), 2.37 (s, 5H), 2.22 (s, 6H), 1.99 - 1.72 (m, 8H) |
| **Example 40** | (DMSO-$d_6$) δ 10.11 (s, 1H), 8.57 (s, 1H), 8.10 (q, *J* = 7.6 Hz, 3H), 7.18 (s, 1H), 6.76 (d, *J* = 8.3 Hz, 1H), 6.40 (dd, *J* = 16.9, 10.1 Hz, 1H), 6.30 - 6.12 (m, 2H), 5.74 (dd, *J* = 10.0, 2.0 Hz, 1H), 4.70 (q, *J* = 8.9 Hz, 2H), 3.86 (s, 2H), 2.89 (t, *J* = 6.0 Hz, 2H), 2.72 (s, 3H), 2.34 (d, *J* = 11.1 Hz, 5H), 2.21 (s, 6H), 1.96 - 1.70 (m, 8H) |
| **Example 41** | ($CDCl_3$) δ 10.01 (s, 1H), 9.68 (s, 1H), 8.59 - 8.32 (m, 2H), 7.76 (s, 1H), 7.26 (s, 1H), 6.77 (s, 1H), 6.44 (d, J = 16.0 Hz, 2H), 5.71 (d, J = 9.3 Hz, 1H), 4.38 (s, 2H), 3.88 (s, 3H), 2.92 (s, 2H), 2.72 (s, 3H), 1.61 (s, 8H), 1.47 (s, 6H) |
| **Example 42** | (DMSO-$d_6$) δ 9.84 (s, 1H), 8.35 - 8.02 (m, 4H), 6.81 (d, J = 8.3 Hz, 1H), 6.46 (dd, J = 17.0, 10.2 Hz, 1H), 6.33 (d, J = 5.9 Hz, 1H), 6.20 (dd, J = 17.0, 2.1 Hz, 1H), 5.74 (dd, J = 10.2, 2.0 Hz, 1H), 4.92 (q, J = 9.1 Hz, 2H), 3.84 (s, 2H), 3.22 (t, J = 6.8 Hz, 2H), 2.88 (s, 3H), 2.46 (d, J = 6.7 Hz, 2H), 2.37 (s, 3H), 2.19 (s, 6H), 1.78 - 1.65 (m, 5H), 1.50 (dd, J = 29.2, 13.0 Hz, 5H) |
| **Example 43** | (DMSO-$d_6$) δ 10.01 (s, 1H), 8.78 (s, 1H), 8.17 - 8.01 (m, 2H), 7.86 (s, 1H), 6.99 (s, 1H), 6.51 - 6.26 (m, 1H), 6.17 (dd, J = 16.8, 2.1 Hz, 2H), 6.03 (s, 1H), 5.72 (dd, J = 10.0, 2.1 Hz,1H), 4.61-4.51 (m, 1H), 3.85 (s, 2H), 2.85 (t, J = 5.9 Hz, 2H), 2.69 (s, 3H), 2.31 (d, J = 6.3 Hz, 5H), 2.20 (s, 6H), 2.09 (s, 3H), 1.29 (s, 6H), 1.24 (d, J = 5.9 Hz, 6H) |
| **Example 44** | (DMSO-$d_6$) δ 10.04 (s, 1H), 9.10 (br, 1H), 8.36 (dd, *J* = 40.8, 16.9 Hz, 2H), 7.93 (br, 1H), 7.02 (s, 1H), 6.58 - 6.03 (m, 2H), 5.75 (s, 1H), 4.59 (d, *J* = 59.4 Hz, 1H), 4.14 (s, 1H), 3.90 (s, 1H), 2.87 (t, *J* = 5.9 Hz, 2H), 2.68 (s, 3H), 2.36 - 2.25 (m, 5H), 2.20 (s, 6H), 1.30-1.16 (m, 15H) |
| **Example 45** | (DMSO-$d_6$) δ 10.18 (s, 1H), 8.56 (d,J = 2.3Hz, 2H), 8.26 (d,J = 8.1Hz, 1H), 8.13 (d,J = 5.8Hz, 1H), 8.00 (dd,J = 4.9, 1.3Hz, 1H), 7.19 (d,J = 15.8Hz, 1H), 7.04 - 6.82 (m, 2H), 6.42 (dd,J = 16.9, 10.1Hz, 1H), 6.32 - 6.19 (m, 2H), 5.77 (dd,J = 10.1, 2.1Hz, 1H), 3.83 (s, 2H), 2.87 (t,J = 5.7Hz, 2H), 2.72 (s, 3H), 2.37 (d,J = 5.7Hz, 2H), 2.21 (s, 6H), 1.32 (s, 6H) |
| **Example 46** | (DMSO-$d_6$) δ 10.17 (s, 1H), 8.57 (s, 1H), 8.47 (s, 1H), 8.11 (dd,J = 13.3, 7.0Hz, 2H), 7.22 - 6.83 (m, 2H), 6.75 (d,J = 8.3Hz, 1H), 6.43 (dd,J = 16.9, 10.1Hz, 1H), 6.27 - 6.19 (m, 2H), 5.78 (dd,J = 10.1, 2. 1Hz, 1H), 3.79 (s, 2H), 2.88 (t,J = 5.8Hz, 2H), 2.72 (s, 3H), 2.38 (d,J = 6.0Hz, 2H), 2.22 (s, 6H), 1.97 (s, 1H), 1.27 (s, 6H), 0.90 - 0.79 (m, 4H) |
| **Example 47** | (DMSO-$d_6$) δ 10.16 (s, 1H), 8.57 (s, 1H), 8.48 (s, 1H), 8.16 (d,J = 8.3Hz, 1H), 8.10 (d,J = 5.9Hz, 1H), 7.25 - 6.82 (m, 2H), 6.78 (d,J = 8.3Hz, 1H), 6.42 (dd,J = 16.9, 10.2Hz, 1H), 6.30 - 6.16 (m, 2H), 5.77 (dd,J = 10.1, 2.0Hz, 1H), 3.80 (s, 2H), 2.87 (s, 2H), 2.72 (s, 3H), 2.36 (d,J = 3.6Hz, 5H), 2.21 (s, 6H), 1.30 (s, 6H) |

(continued)

| Example No. | $^1H$ NMR (400 MHz) |
|---|---|
| **Example 48** | (DMSO-$d_6$) δ 9.76 (s, 1H), 8.96 (s, 1H), 8.50-8.15 (m, 2H), 7.97-7.75 (m, 1H), 7.06-6.77 (m, 1H), 6.47 (dd, J = 16.8, 10.2 Hz, 1H), 6.22 (brs, 1H), 5.75 (s, 1H), 5.20 (s, 1H), 4.00-3.93 (m, 2H), 3.23-3.14 (m, 2H), 2.85 (d, J = 19.0 Hz, 3H), 2.42 (s, 5H), 2.19 (s, 6H), 1.33-1.23(m, 12H) |
| **Example 49** | (DMSO-$d_6$) δ 10.02 (s, 1H), 8.24 (d, *J* = 25.6 Hz, 2H), 8.08 (d, *J* = 5.8 Hz, 2H), 6.87 (d, *J* = 8.3 Hz, 1H), 6.66 (s, 1H), 6.30 - 6.17 (m, 2H), 5.83 - 5.68 (m, 1H), 4.94 (q, *J* = 9.1 Hz, 2H), 3.84 (s, 2H), 3.65 - 3.51 (m, 2H), 2.83 (s, 3H), 2.77-2.68 (m, 2H),2.50 (s, 6H), 2.38 (s, 3H), 1.98 - 1.74 (m, 8H) |
| **Example 50** | (DMSO-$d_6$) δ 10.04 (s, 1H), 8.59 (s, 1H), 8.27 (d,J = 8.4Hz, 1H), 8.15 - 8.02 (m, 3H), 7.83 (s, 1H), 7.18 (d,J = 8.4Hz, 1H), 7.02 (s, 1H), 6.44 (dd,J = 17.0, 10.2Hz, 1H), 6.27 (d,J = 5.9 Hz, 1H), 6.17 (dd,J = 16.9, 2.1Hz, 1H), 5.70 (dd,J = 10.1, 2. 1Hz, 1H), 3.88 (s, 3H), 3.85 (s, 2H), 3.80 (s, 3H), 2.91 (d,J = 6.0Hz, 2H), 2.75 (s, 3H), 2.34 (t,J = 6.1Hz, 2H), 2.19 (s, 6H), 1.34 (s, 6H) |
| **Example 51** | (DMSO-$d_6$) δ 9.99 (s, 1H), 8.55 (s, 1H), 8.50 (d,J = 8.8Hz, 1H), 8.38 (d,J = 1.1 Hz, 1H), 8.21 - 8.12 (m, 2H), 7.82 (t,J = 1.4Hz, 1H), 7.30 (d,J = 8.7Hz, 1H), 7.10(t,J = 1.2Hz, 1H), 7.02 (s, 1H), 6.43 (dd,J= 17.0, 10.2Hz, 1H), 6.29 (d,J = 5.8Hz, 1H), 6.15 (dd,J = 16.9, 2.0Hz, 1H), 5.68 (dd,J = 10.1, 2.1Hz, 1H), 3.91 (s, 2H), 3.80 (s, 3H), 2.91 (t,J = 6.1Hz, 2H), 2.75 (s, 3H), 2.33 (t,J = 6.0Hz, 2H), 2.17 (s, 6H), 1.37 (s, 6H) |
| **Example 52** | (DMSO-$d_6$) δ 10.05 (s, 1H), 8.60 (s, 1H), 8.11 (d, *J* = 5.7 Hz, 2H), 8.01 (s, 1H), 7.00 (s, 1H), 6.81 (d, *J* = 8.3 Hz, 1H), 6.40 (dd, *J* = 16.9, 10.2 Hz, 1H), 6.28 (d, *J* = 5.8 Hz, 1H), 6.16 (dd, *J* = 16.8, 2.1 Hz, 1H), 5.72 (dd, *J* = 10.1, 2.1 Hz, 1H), 4.17 (s, 2H), 3.78 (s, 3H), 2.90 (t, *J* = 6.0 Hz, 2H), 2.73 (s, 3H), 2.46-2.43 (m, 2H), 2.41 (s, 3H), 2.32 (t, *J* = 5.9 Hz, 2H), 2.24-2.17 (m, 8H), 2.09 - 1.98 (m, 2H) |
| **Example 53** | (DMSO-$d_6$) δ 9.76 (s, 1H), 8.28 (s, 1H), 8.17 - 8.06 (m, 3H), 6.87 (d, *J* = 8.3 Hz, 1H), 6.46 (dd, *J* = 17.0, 10.2 Hz, 1H), 6.29 - 6.14 (m, 2H), 5.72 (dd, *J* = 10.2, 2.1 Hz, 1H), 4.14 (s, 2H), 3.84 (s, 3H), 3.18 (t, *J* = 6.7 Hz, 2H), 2.86 (s, 3H), 2.45-2.43 (m, 5H), 2.24-2.197(m, 9H), 2.11 - 1.98 (m, 3H) |
| **Example 55** | (DMSO-$d_6$) δ 10.10 (s, 1H), 9.20 (d, J = 65.4 Hz, 1H), 8.40 (s, 3H), 8.16 (d, J = 14.6 Hz, 1H), 7.23 (s, 1H), 7.04 (s, 1H), 6.41 (d, J = 11.2 Hz, 1H), 6.23 (s, 1H), 5.76 (s, 1H), 4.37 (d, J = 76.4 Hz, 2H), 3.81 (d, J = 34.9 Hz, 4H), 2.90 (s, 2H), 2.74 (s, 3H), 2.55 - 2.38 (m, 3H), 2.33 (s, 2H), 2.21 (s, 6H), 2.14 - 1.80 (m, 2H) |
| **Example 56** | (DMSO-$d_6$) δ 9.84 (s, 1H), 8.23 (d, *J* = 13.7 Hz, 2H), 8.11 - 8.00 (m, 2H), 6.84 (d, *J* = 8.3 Hz, 1H), 6.46 (dd, *J* = 17.0, 10.2 Hz, 1H), 6.27 - 6.15 (m, 2H), 5.74 (dd, *J* = 10.1, 2.1 Hz, 1H), 4.93 (q, *J* = 9.1 Hz, 2H), 4.13 (s, 2H), 3.21 (t, *J* = 6.8 Hz, 2H), 2.88 (s, 3H), 2.46-2.42 (m, 5H), 2.23- 2.17 (m, 9H), 2.11 - 1.97 (m, 3H) |
| **Example 57** | (DMSO-$d_6$) δ 9.85 (s, 1H), 8.32 (s, 1H), 8.20 (s, 1H), 8.11 (d, *J* = 5.8 Hz, 2H), 8.07 (d, J = 4.9 Hz, 1H), 6.98 (dd, J = 8.1, 4.9 Hz, 1H), 6.46 (dd, J = 17.0, 10.2 Hz, 1H), 6.28 (d, J = 5.9 Hz, 1H), 6.20 (dd, J = 17.1, 2.0 Hz, 1H), 5.86 - 5.62 (m, 1H), 4.92 (q, J = 9.1 Hz, 2H), 4.15 (s, 2H), 3.22 (t, J = 6.7 Hz, 2H), 2.88 (s, 3H), 2.45 (d, J = 13.2 Hz, 2H), 2.19 (s, 6H), 2.07 (s, 2H), 2.00 (q, J = 7.8 Hz, 2H), 1.24 (s, 2H) |
| **Example 61** | (DMSO-$d_6$) δ 10.09 (d, *J* = 23.0 Hz, 1H), 9.28-9.05 (m, 1H), 8.37 - 7.77( m, 3H), 7.03-6.73 (m, 2H), 6.43 (t, *J* = 13.4 Hz, 1H), 6.34 - 6.11 (m, 1H), 5.75 (s, 1H), 4.35 (d, *J* = 81.6 Hz, 2H), 3.81 (d, *J* = 44.9 Hz, 3H), 2.89 (s, 2H), 2.73 (s, 3H), 2.47- 2.33 (m, 6H), 2.25-2.21 (m, 8H), 2.13 - 1.92 (m, 3H) |
| **Example 62** | (DMSO-$d_6$) δ 10.18 (s, 1H), 8.57 (s, 1H), 8.53 (s, 1H), 8.21 (d, J = 8.2 Hz, 1H), 8.14 (d, J = 5.8 Hz, 1H), 8.07 (dd, J = 4.9, 1.4 Hz, 1H), 7.16 (s, 1H), 7.02 (t, J = 76.0 Hz, 1H), 6.93 (dd, J = 8.2, 4.9 Hz, 1H), 6.42 (dd, J = 17.0, 10.1 Hz, 1H), 6.31 (d, J = 5.9 Hz, 1H), 6.21 (dd, J = 17.0, 2.0 Hz, 1H), 5.77 (dd, J = 10.1, 2.0 Hz, 1H), 4.17 (s, 2H), 2.87 (t, J = 5.8 Hz, 2H), 2.72 (s, 3H), 2.44 (ddd, J = 11.7, 5.7, 2.2 Hz,2H), 2.36 (t, J = 5.8 Hz, 2H), 2.30 - 2.23 (m, 2H), 2.21 (s, 6H), 2.15 - 1.95 (m, 2H) |
| **Example 63** | (DMSO-$d_6$) δ 10.06 (s, 1H), 8.59 (s, 1H), 8.21 (d, J = 8.2 Hz, 1H), 8.14 (d, J = 5.8 Hz, 1H), 8.10 (s, 1H), 8.06 (dd, J = 4.8, 1.4 Hz, 1H), 7.01 (s, 1H), 6.95 (dd, J = 8.1, 4.9 Hz, 1H), 6.40 (dd, J = 16.9, 10.2 Hz, 1H), 6.31 (d, J = 5.9 Hz, 1H), 6.17 (dd, J = 17.0, 2.0 Hz, 1H), 5.72 (dd, J = 10.1, 2.1 Hz, 1H), 4.18 (s, 2H), 3.78 (s, 3H), 2.89 (t, J = 5.9 Hz, 2H), 2.73 (s, 3H), 2.47 - 2.38 (m, 2H), 2.32 |
| **Example 64** | (DMSO-$d_6$) δ 10.16 (s, 1H), 8.58 (s, 1H), 8.46 (s, 1H), 8.13-8.10 (m, 2H), 7.27 - 6.81 (m, 2H), 6.79 (d, *J* = 8.3 Hz, 1H), 6.42 (dd, *J* = 16.9, 10.2 Hz, 1H), 6.31 - 6.15 (m, 2H), 5.77 (dd, *J* = 10.1, 2.0 Hz, 1H), 4.15 (s, 2H), 2.87 (t, *J* = 5.8 Hz, 2H), 2.72 (s, 3H), 2.41-2.35(m, 6H), 2.21 -2.17(m, 8H), 2.12 - 1.93 (m, 3H) |

(continued)

| Example No. | $^1$H NMR (400 MHz) |
|---|---|
| **Example 65** | (DMSO-$d_6$) δ 10.05 (s, 1H), 8.57 (s, 1H), 8.19 (d, J = 8.3 Hz, 1H), 8.12 (d, J = 5.8 Hz, 1H), 8.04 (s, 1H), 7.01 (s, 1H), 6.81 (d, J = 8.3 Hz, 1H), 6.46 - 6.32 (m, 2H), 6.16 (dd, J = 17.0, 2.1 Hz, 1H), 5.72 (dd, J = 10.1, 2.1 Hz, 1H), 4.01 (s, 2H), 3.90 (dt, J = 11.3, 3.7 Hz, 2H), 3.78 (s, 3H), 3.57 (td, J = 11.8, 2.0 Hz, 2H), 2.90 (t, J = 5.9 Hz, 2H), 2.73 (s, 3H), 2.37 (s, 3H), 2.32 (t, J = 5.9 Hz, 2H), 2.21 (s, 6H), 1.97 (td, J = 12.7, 4.5 Hz, 2H), 1.50 (d, J = 13.1 Hz, 2H) |
| **Example 66** | (DMSO-*d*$_6$) δ 0.12 (s, 1H), 8.57 (s, 1H), 8.45 (d, J = 8.5 Hz, 1H), 8.32 (d, J = 12.2 Hz, 2H), 8.13 (s, 1H), 7.88 (s, 1H), 7.39 (d, J = 8.4 Hz, 1H), 7.00 (s, 1H), 6.40 (dd, J = 16.9, 10.1 Hz, 1H), 6.29 - 6.18 (m, 2H), 5.75 (dd, J = 10.0, 2.2 Hz, 1H), 3.92 - 3.78 (m, 9H), 2.87 (t, J = 5.9 Hz, 2H), 2.71 (s, 3H), 2.31 (t, J = 5.8 Hz, 2H), 2.21 (s, 6H), 1.36 (s, 6H) |
| **Example 67** | (DMSO-*d*$_6$) δ 10.11 (s, 1H), 8.83 (s, 1H), 8.12 (s, 1H), 7.96 (d,J = 5.9Hz, 1H), 7.87 (s, 1H), 7.80 (s, 1H), 7.68 (d,J = 8.5Hz, 1H), 7.33 (d,J = 8.4Hz, 1H), 6.97 (s, 1H), 6.79 (d,J = 2.1Hz, 1H), 6.59 (dd,J = 6.0, 2.1Hz, 1H), 6.39 (dd,J = 16.9, 10.1Hz, 1H), 6.23 (dd,J = 16.9, 2.2Hz, 1H), 5.75 (dd,J = 9.9, 2.2Hz, 1H), 3.88 (s, 3H), 3.84 (s, 3H), 3.81 (s, 2H), 2.85 (t,J = 5.8Hz, 2H), 2.69 (s, 3H), 2.27 (t,J = 5.9Hz, 2H), 2.20 (s, 6H), 1.35 (s, 6H) |
| **Example 69** | (DMSO-*d*$_6$) δ 9.78 (s, 1H), 8.27 (s, 1H), 8.17 - 8.03 (m, 3H), 6.82 (d, J = 8.4 Hz, 1H), 6.47 (dd, J = 17.0, 10.3 Hz, 1H), 6.28 - 6.13 (m, 2H), 5.73 (dd, J = 10.1, 2.2 Hz, 1H), 3.85 (s, 3H), 3.78 (s, 2H), 3.19 (t, J = 6.8 Hz, 2H), 2.87 (s, 3H), 2.46 (d, J = 6.8 Hz, 2H), 2.20 (s, 6H), 1.98 (td, J = 8.1, 4.2 Hz, 1H), 1.27 (s, 6H), 0.91 - 0.79 (m, 4H) |
| **Example 70** | (DMSO-*d*$_6$) δ .81 (s, 1H), 8.42 (s, 1H), 8.38 - 8.22 (m, 3H), 6.99 (d, J = 8.4 Hz, 1H), 6.44 (dd, J = 17.0, 10.2 Hz, 1H), 6.23 (dd, J = 17.0, 2.1 Hz, 1H), 6.11 (s, 1H), 5.74 (dd, J = 10.1, 2.1 Hz, 1H), 3.89 (s, 3H), 3.71 (s, 2H), 3.17 (t, J = 6.7 Hz, 2H), 2.84 (s, 3H), 2.43 (d, J = 6.7 Hz, 2H), 2.19 (s, 6H), 2.02 (ddt, J = 10.7, 7.9, 4.0 Hz, 1H), 1.29 (s, 6H), 0.90 - 0.82 (m, 4H) |
| **Example 71** | (DMSO-*d*$_6$) δ 10.04 (s, 1H), 8.54 (s, 1H), 8.09 (d, J = 5.8 Hz, 1H), 8.04 (s, 1H), 7.00 (s, 1H), 6.80 (d, J = 8.3 Hz, 1H), 6.40 (dd, J = 16.9, 10.1 Hz, 1H), 6.25 (d, J = 5.9 Hz, 1H), 6.16 (dd, J = 16.9, 2.1 Hz, 1H), 5.72 (d, J = 10.2 Hz, 1H), 5.33 (t, J = 4.8 Hz, 1H), 4.04 (d, J = 10.8 Hz, 1H), 3.91 (d, J = 10.7 Hz, 1H), 3.78 (s, 3H), 2.95 (d, J = 4.5 Hz, 3H), 2.90 (t, J = 5.9 Hz, 3H), 2.79 (d, J = 8.9 Hz, 2H), 2.73 (s, 3H), 2.69 - 2.65 (m, 3H), 2.37 (s, 3H), 2.33 (s, 3H), 2.29 (s, 3H), 2.21 (s, 3H), 2.06 - 1.93 (m, 2H) |
| **Example 72** | (DMSO-*d*$_6$) δ 10.06 (s, 1H), 9.05 (s, 1H), 8.35 (s, 2H), 7.02 (s, 2H), 6.41 (dd, J = 17.0, 9.9 Hz, 1H), 6.20 (s, 1H), 5.74 (d, J = 10.1 Hz, 1H), 5.32 (t, J = 4.8 Hz, 1H), 4.21 (d, J = 11.3 Hz, 2H), 3.76 (s, 3H), 2.90 (s, 8H), 2.74 (s, 4H), 2.33 (p, J = 1.9 Hz, 2H), 2.27 (s, 3H), 2.21 (s, 6H), 1.99 (dt, J = 13.4, 7.0 Hz, 2H) |
| **Example 73** | (DMSO-*d*$_6$) δ 10.03 (s, 1H), 8.55 (s, 1H), 8.17 (d, J = 8.3 Hz, 1H), 8.11 (d, J = 5.8 Hz, 1H), 8.05 (s, 1H), 7.00 (s, 1H), 6.82 (d, J = 8.3 Hz, 1H), 6.40 (dd, J = 16.9, 10.1 Hz, 1H), 6.28 (d, J = 5.9 Hz, 1H), 6.15 (dd, J = 16.9, 2.1 Hz, 1H), 5.71 (dd, J = 10.1, 2.1 Hz, 1H), 4.31 (s, 2H), 3.77 (s, 3H), 3.46 (d, J = 7.4 Hz, 2H), 3.39 (s, 2H), 2.90 (t, J = 5.9 Hz, 2H), 2.73 (s, 3H), 2.41 (s, 3H), 2.33 (t, J = 5.9 Hz, 2H), 2.29 (s, 3H), 2.21 (s, 6H) |
| **Example 74** | (DMSO-*d*$_6$) δ 10.04 (s, 1H), 8.58 (s, 1H), 8.17 (d, *J* = 8.3 Hz, 1H), 8.12 (d, *J* = 5.8 Hz, 1H), 8.07 (s, 1H), 7.01 (s, 1H), 6.87 (d, *J* = 8.3 Hz, 1H), 6.39 (dd, *J* = 16.9, 10.1 Hz, 1H), 6.28 (d, *J* = 5.9 Hz, 1H), 6.16 (dd, *J*= 16.9, 2.1 Hz, 1H), 5.72 (dd, *J* = 10.1, 2.1 Hz, 1H), 4.29 (s, 2H), 3.78 (s, 3H), 3.10-2.99 (m, 2H), 2.96 - 2.87 (m, 4H), 2.73 (s, 3H), 2.41 (s, 3H), 2.32 (t, *J*= 5.8 Hz, 2H), 2.21 (s, 6H) |

**Example 54: Preparation of *N*-(2-((2-(dimethylamino)ethyl)(methyl)amino)-5-((4-(5-ethynyl-3,3-dimethyl-2,3-dihydro-1*H*-pyrrolo[3,2-*b*]pyridin-1-yl)pyrimidin-2-yl)amino)-4-methoxyphenyl)acrylamide**

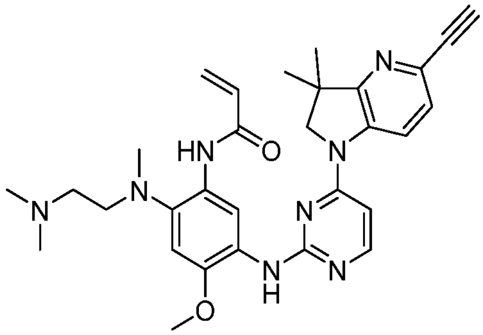

*N*-(5-((4-(3,3-dimethyl-5-((trimethylsilyl)ethynyl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide (100 mg, 0.07 mmol) was dissolved in methanol (5mL), and potassium carbonate (19.3mg, 0.14mmol) was added. The mixture was stirred for 1 h at room temperature, and filtered. The solvent was removed from the filtrate. The residue was separated by reversed column chromatography [40-50% acetonitrile/water] to obtain *N*-(2-((2-(dimethylamino)ethyl)(methyl)amino)-5-((4-(5-ethynyl-3,3-dimethyl-2,3-dihydro-1*H*-pyrrolo[3,2-*b*]pyridin-1-yl)pyrimidin-2-yl)amino)-4-methoxyphenyl)acrylamide (3.3 mg, yield: 7.83%). ESI-MS: 541.2 $[M+1]^+$.

$^1H$ NMR (400MHz, DMSO-$d_6$) δ 10.05 (s, 1H), 8.51 (s, 1H), 8.26 - 8.08 (m, 3H), 7.10 (d,J = 8.4Hz, 1H), 7.02 (s, 1H), 6.41 (dd,J = 16.9, 10.1Hz, 1H), 6.29 (d,J = 5.8Hz, 1H), 6.17 (dd,J = 16.9, 2.1Hz, 1H), 5.72 (dd,J = 10.1, 2.0Hz, 1H), 4.13 (s, 1H), 3.86 (s, 2H), 3.77 (s, 3H), 2.90 (t,J = 5.9Hz, 2H), 2.73 (s, 3H), 2.33 (t,J = 5.9Hz, 2H), 2.21 (s, 6H), 1.31 (s, 6H).

**Example 68 was prepared according to the synthesis method for Example 54:**

| Example No. | Structural Formula | Chemical Name | ESI-MS: $[M+1]^+$ |
|---|---|---|---|
| **Example 68** | 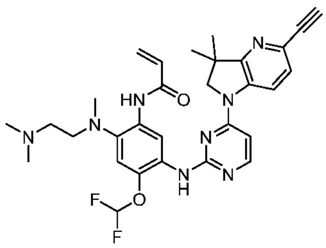 | *N*-(4-(difluoromethoxy)-2-((2-(d imethylamino)ethyl)(methyl)ami no)-5-((4-(5-ethynyl-3,3-dimeth yl-2,3-dihydro-1*H*-pyrrolo[3,2-*b*]pyridin-1-yl)pyrimidin-2-yl)a mino)phenyl)acrylamide | 577.2 |

**The magnetic resonance imaging data of the compound prepared from the above example was as follows:**

| Example No. | $^1H$ NMR (400 MHz) |
|---|---|
| Example 68 | (DMSO-$d_6$) δ 10.13 (s, 1H), 8.63 (s, 1H), 8.52 (s, 1H), 8.24 (d,J = 8.4Hz, 1H), 8.16 (d,J = 5.8Hz, 1H), 7.25 - 6.82 (m, 3H), 6.44 (dd,J = 16.9, 10.1Hz, 1H), 6.31 (d,J = 5.8Hz, 1H), 6.21 (dd,J = 17.0, 2.0Hz, 1H), 5.77 (dd,J = 10.1, 2.0Hz, 1H), 4.13 (s, 1H), 3.85 (s, 2H), 2.89 (s, 2H), 2.72 (s, 3H), 2.39 (s, 2H), 2.23 (s, 6H), 1.31 (s, 6H) |

**Example 58: Preparation of *N*-(5-((4-(3,3-dimethyl-5-(1*H*-pyrazol-4-yl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide**

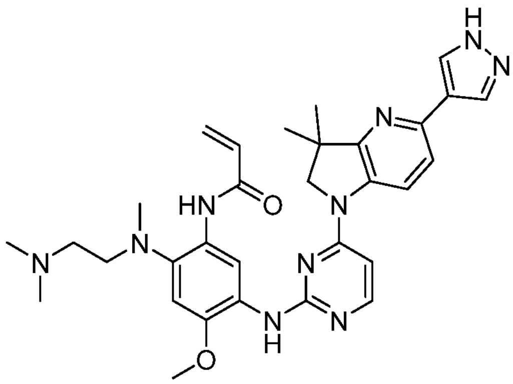

**Step 1: Synthesis of tert-butyl 4-(1-(2-((4-((2-(dimethylamino)ethyl)(methyl)amino)-2-methoxy-5-nitrophenyl)amino)pyrimidin-4-yl)-3,3-dimethyl-2,3-dihydro-1*H*-pyrrolo[3,2-*b*]pyridin-5-yl)-1*H*-pyrazol-1-carboxylate**

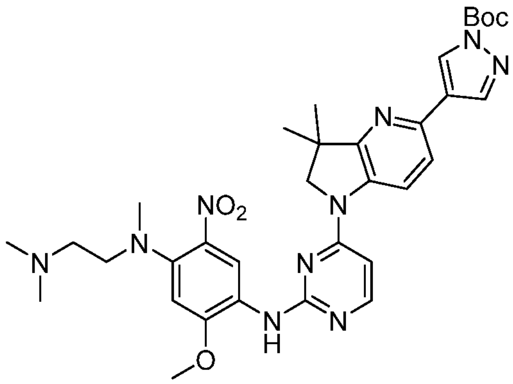

$N^1$-(4-(5-bromo-3,3-dimethyl-2,3-dihydro-1*H*-pyrrolo[3,2-*b*]pyridin-1-yl)pyrimidin-2-yl)-$N^4$-(2-(dimethylamino)ethyl)-2-methoxy-$N^4$-methyl-5-nitrobenzene-1,4-diamine (40 mg, 0.67 mmol), tert-butyl 4-(4,4,5,5-tetramethyl-1,3,2-dioxaborolane-2-yl)-1*H*-pyrazol-1-carboxylate (19.6 mg, 0.67 mmol), potassium phosphate (42 mg, 2.0 mmol), tricyclohexylphosphine (7.5 mg, 0.03 mmol), palladium acetate (3.0 mg, 0.013 mmol), and toluene (3 mL) were added to a reaction flask. The mixture was subjected to nitrogen displacement three times, and under the protection of nitrogen, was heated to 110 °C and stirred for 16 h. The reaction mixture was filtered. The filtrate was washed with water and extracted with ethyl acetate. The organic layer was dried over anhydrous sodium sulfate, and distilled under reduced pressure to obtain a crude product, which was separated by flash silicagel columns [eluent: ethyl acetate/petroleum ether: 0-30%] to obtain tert-butyl 4-(1-(2-((4-((2-(dimethylamino)ethyl)(methyl)amino)-2-methoxy-5-nitrophenyl)amino)pyrimidin-4-yl)-3,3-dimethyl-2,3-dihydro-1*H*-pyrrolo[3,2-*b*]pyridin-5-yl)-1*H*-pyrazol-1-carboxylate(36 mg, yield: 78.1%). ESI-MS: 659.3 $[M+1]^+$.

**Step 2: Synthesis of tert-butyl 4-(1-(2-((5-amino-4-((2-(dimethylamino)ethyl)(methyl)amino)-2-methoxyphenyl)amino)pyrimidin-4-yl)-3,3-dimethyl-2,3-dihydro-1*H*-pyrrolo[3,2-*b*]pyridin-5-yl)-1*H*-pyrazol-1-carboxylate**

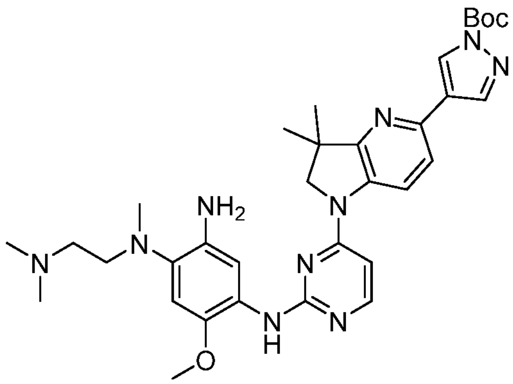

Tert-butyl 4-(1-(2-((4-((2-(dimethylamino)ethyl)(methyl)amino)-2-methoxy-5-nitrophenyl)amino)pyrimidin-4-yl)-3,3-dimethyl-2,3-dihydro-1*H*-pyrrolo[3,2-b]pyridin-5-yl)-1*H*-pyrazol-1-carboxylate (36 mg, 0.055 mmol) was dissolved in 5 mL of methanol. 10% palladium on carbon (10 mg) was added to the mixture. The reaction mixture was subjected to hydrogen displacement three times, and stirred for 1 hr at room temperature in the atmosphere of hydrogen. After the reaction was completed, the resultant was filtered with diatomite. The solvent was removed to subsequently obtain tert-butyl 4-(1-(2-((5-amino-4-((2-(dimethylamino)ethyl)(methyl)amino)-2-methoxyphenyl)amino)pyrimidin-4-yl)-3,3-dimethyl-2,3-dihydro-1H-pyrrolo[3,2-*b*]pyridin-5-yl)-1H-pyrazol-1-carboxylate (30 mg, yield: 82.1%). ESI-MS: 629.4 $[M+1]^+$.

**Step 3: Synthesis of tert-butyl 4-(1-(2-((5-acrylamido-4-((2-(dimethylamino)ethyl)(methyl)amino)-2-methoxyphenyl)amino)pyrimidin-4-yl)-3,3-dimethyl-2,3-dihydro-1*H*-pyrrolo[3,2-*b*]pyridin-5-yl)-1*H*-pyrazol-1-carboxylate**

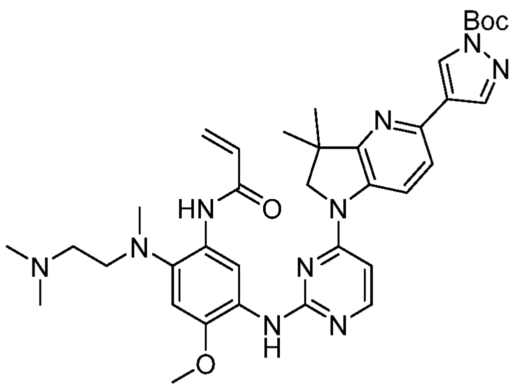

Tert-butyl 4-(1-(2-((5-amino-4-((2-(dimethylamino)ethyl)(methyl)amino)-2-methoxyphenyl)amino)pyrimidin-4-yl)-3,3-dimethyl-2,3-dihydro-1*H*-pyrrolo[3,2-*b*]pyridin-5-yl)-1*H*-pyrazol-1-carboxylate (30 mg, 0.048 mmol) was dissolved in anhydrous acetonitrile/water (1mL/0.3mL). *N*,*N*-diisopropylethylamine (18 mg, 0.143 mmol) was added to the mixture. Acryloyl chloride (13.0 mg, 0.143 mmol) was added to the reaction mixture at 0 °C. After the reaction was completed, stratification was conducted with dichloromethane and water. The organic phase was washed with water and saturated sodium chloride in sequence, then dried over anhydrous sodium sulfate, filtered, concentrated, and then separated by reversed column chromatography [40-50% acetonitrile/water] to obtain tert-butyl 4-(1-(2-((5-acryloylamino-4-((2-(dimethylamino)ethyl)(methyl)amino)-2-methoxyphenyl)amino)pyrimidin-4-yl)-3,3-dimethyl-2,3-dihydro-1*H*-pyrrolo[3,2-*b*]pyridin-5-yl)-1*H*-pyrazol-1-carboxylate (300 mg, yield: 64.5%). ESI-MS: 683.4 $[M+1]^+$.

**Step 4: Synthesis of *N*-(5-((4-(3.3-dimethyl-5-(1*H*-pyrazol-4-yl)-2.3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide**

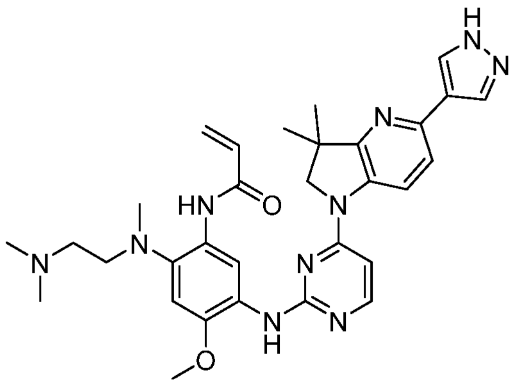

Tert-butyl 4-(1-(2-((5-acryloylamino-4-((2-(dimethylamino)ethyl)(methyl)amino)-2-methoxyphenyl)amino)pyrimidin-4-yl)-3,3-dimethyl-2,3-dihydro-1H-pyrrolo[3,2-*b*]pyridin-5-yl)-1*H*-pyrazol-1-carboxylate (30 mg, 0.044 mmol) was dissolved in dichloromethane (3mL), and trifluoroacetic acid (1 mL) was added. The reaction mixture was stirred for 2 hrs at room temperature, the solvent was removed, and the residue was treated by reversed column chromatography to obtain N-(5-((4-(3,3-dimethyl-5-(1H-pyrazol-4-yl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide (5 mg, yield: 18.5%). ESI-MS: 583.3 $[M+1]^+$.

$^1$HNMR(400MHz, DMSO-$d_6$) $\delta$ 12.92 (s, 1H), 10.03 (s, 1H), 8.59 (s, 1H), 8.27 (d,J = 8.4Hz, 1H), 8.12 (d,J = 5.8Hz, 2H), 8.05 (s, 1H), 7.91 (s, 1H), 7.22 (d,J = 8.4Hz, 1H), 7.02 (s, 1H), 6.44 (dd,J = 16.9, 10.1Hz, 1H), 6.28 (d,J = 5.8Hz, 1H), 6.17 (dd,J = 16.8, 2.1Hz, 1H), 5.70 (dd,J = 10.1, 2.1Hz, 1H), 3.86 (s, 2H), 3.80 (s, 3H), 2.92 (t,J = 6.0Hz, 2H), 2.75 (s, 3H), 2.39 - 2.31 (m, 2H), 2.20 (s, 6H), 1.35 (s, 6H).

**Example 59: Preparation of *N*-(5-((4-(3,3-dimethyl-5-(5-methyl-1*H*-imidazole-1-yl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide**

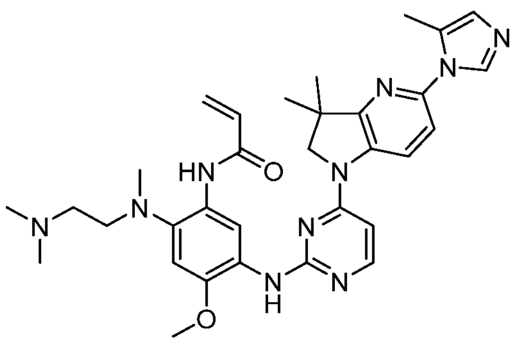

**Step 1: Synthesis of *N*$^1$-(4-(3,3-dimethyl-5-(5-methyl-1H-imidazole-1-yl)-2,3-dihydro-1*H*-pyrrolo[3,2-*b*]pyridin-1-yl)pyrimidin-2-yl)-*N*$^4$-(2-(dimethylamino)ethyl)-2-methoxy-*N*$^4$-methyl-5-nitrobenzene-1,4-diamine** and ***N*$^1$-(4-(3,3-dimethyl-5-(4-methyl-1*H*-imidazole-1-yl)-2,3-dihydro-1*H*-pyrrolo[3,2-*b*]pyridin-1-yl)pyrimidin-2-yl)-*N*$^4$-(2-(dimethylamino)ethyl)-2-methoxy-*N*$^4$-methyl-5-nitrobenzene-1,4-diamine**

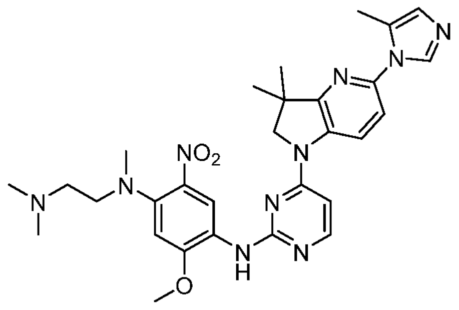

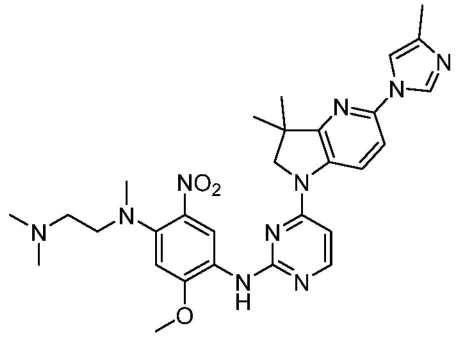

$N^1$-(4-(5-bromo-3,3-dimethyl-2,3-dihydro-1*H*-pyrrolo[3,2-*b*]pyridin-1-yl)pyrimidin-2-yl)-$N^4$-(2-(dimethylamino)ethyl)-2-methoxy-$N^4$-methyl-5-nitrobenzene-1,4-diamine (100 mg, 0.166 mmol), 4-methyl-1*H*-imidazole (13.7 mg, 0.166 mmol), potassium carbonate (68.9 mg, 0.5 mmol), cuprous iodide (6.3 mg, 0.033 mmol), (1S,2*S*)-$N^1$,$N^2$-dimethyl cyclohexane-1,2-diamine (9.5 mg, 0.066 mmol), and dimethylsulfoxide (2 mL) were added to a reaction flask. The mixture was subjected to nitrogen displacement three times, and under the protection of nitrogen, was heated to 110 °C and stirred for 16 h. The reaction mixture was filtered. The filtrate was washed with water and extracted with ethyl acetate. The organic layer was dried over anhydrous sodium sulfate, and distilled under reduced pressure to obtain a crude product, which was separated by flash silicagel columns [eluent: methanol/dichloromethane: 0-10%] to obtain N$^1$-(4-(3,3-dimethyl-5-(5-methyl-1H-imidazole-1-yl)-2,3-dihydro-1H-pyrrolo[3,2-b]pyridin-1-yl)pyrimidin-2-yl)-$N^4$-(2-(dimethylamino)ethyl)-2-methoxy-$N^4$-methyl-5-nitrobenzene-1,4-diamine (19 mg, yield: 18.1%). ESI-MS: 287.0 [M+1]$^+$.

$N^1$-(4-(3,3-dimethyl-5-(4-methyl-1*H*-imidazole-1-yl)-2,3-dihydro-1*H*-pyrrolo[3,2-*b*]pyiidin-1-yl)pyrimidin-2-yl)-$N^4$-(2-(dimethylamino)ethyl)-2-methoxy-$N^4$-methyl-5 - nitrobenzene-1,4-diamine (53 mg, yield: 50.4%). ESI-MS: 287.1 [M+1]$^+$.

**Step 2: Synthesis of *N*$^4$-(4-(3,3-dimethyl-5-(5-methyl-1*H*-imidazole-1-yl)-2,3-dihydro-1*H*-pyrrolo[3,2-*b*]pyridin-1-yl)pyrimidin-2-yl)-*N*$^1$-(2-(dimethylamino)ethyl)-5-methoxy-*N*$^1$-methylbenzene-1,2,4-triamine**

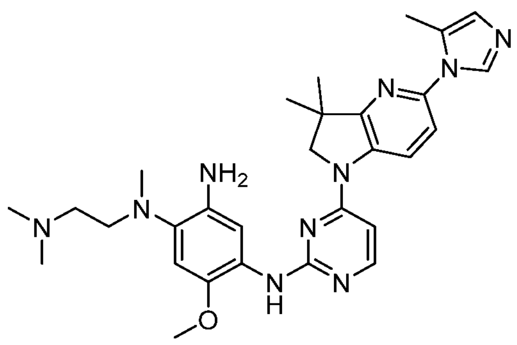

$N^1$-(4-(3,3-dimethyl-5-(5-methyl-1*H*-imidazole-1-yl)-2,3-dihydro-1*H*-pyrrolo[3,2-*b*]pyridin-1-yl)pyrimidin-2-yl)-$N^4$-(2-(dimethylamino)ethyl)-2-methoxy-$N^4$-methyl-5-nitrobenzene-1,4-diamine (17 mg, 0.055 mmol) was dissolved in 5 mL of methanol. 10% palladium on carbon (10 mg) was added to the mixture. The reaction mixture was subjected to hydrogen displacement three times, and stirred for 1 hr at room temperature in the atmosphere of hydrogen. After the reaction was completed, the resultant was filtered with diatomite. The solvent was removed to subsequently obtain $N^4$-(4-(3,3-dimethyl-5-(5-methyl-1*H*-imidazole-1-yl)-2,3-dihydro-1H-pyrrolo[3,2-b]pyridin-1-yl)pyrimidin-2-yl)-$N^1$-(2-(dimethylamino)ethyl)-5-methoxy-$N^1$-methylbenzene-1,2,4-triamine (10 mg, yield: 56.5%). ESI-MS: 543.2[M+1]$^+$.

**Step 3: Synthesis of *N*-(5-((4-(3,3-dimethyl-5-(5-methyl-1*H*-imidazole-1-yl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide**

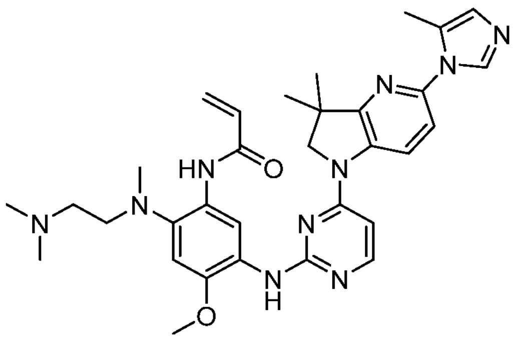

$N^4$-(4-(3,3-dimethyl-5-(5-methyl-1*H*-imidazole-1-yl)-2,3-dihydro-1*H*-pyrrolo[3,2-*b*]pyridin-1-yl)pyrimidin-2-yl)-$N^1$-(2-(dimethylamino)ethyl)-5-methoxy-$N^1$-methylbenzene-1,2,4-triamine (10 mg, 0.018 mmol) was dissolved in anhydrous acetonitrile/water (1 mL/0.3 mL). N,N-diisopropylethylamine (7 mg, 0.055 mmol) was added to the mixture. Acryloyl chloride (5 mg, 0.055 mmol) was added to the reaction mixture at 0 °C After the reaction was completed, stratification was conducted with dichloromethane and water. The organic phase was washed with water and saturated sodium chloride in sequence, then dried over anhydrous sodium sulfate, filtered, concentrated, and then separated by reversed column chromatography [40-50% acetonitrile/water] to obtain *N*-(5-((4-(3,3-dimethyl-5-(5-methyl-1*H*-imidazole-1-yl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide (1.7 mg, yield: 14.7%). ESI-MS: 597.4 $[M+1]^+$.

$^1$HNMR (DMSO-$d_6$) δ 9.61 (s, 1H), 9.29 (s, 1H), 8.53 (d,J = 8.6Hz, 1H), 8.28 (s, 1H), 8.24 - 8.15 (m, 2H), 7.89 (s, 1H), 7.17 (d,J = 8.5Hz, 1H), 6.82 (s, 1H), 6.60 (dd,J = 16.6, 10.4Hz, 1H), 6.33 (d,J = 5.9Hz, 1H), 6.23 (dd,J = 16.8, 2.0Hz, 1H), 5.73 (dd,J = 10.1, 2.0Hz, 1H), 3.93 (s, 2H), 3.85 (s, 3H), 3.25 (s, 2H), 2.76 (s, 6H), 2.64 (s, 3H), 2.52 (s, 2H), 2.29 (s, 3H), 1.36 (s, 6H).

**Example 60: Preparation of *N*-(5-((4-(3,3-dimethyl-5-(4-methyl-1*H*-imidazole-1-yl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide**

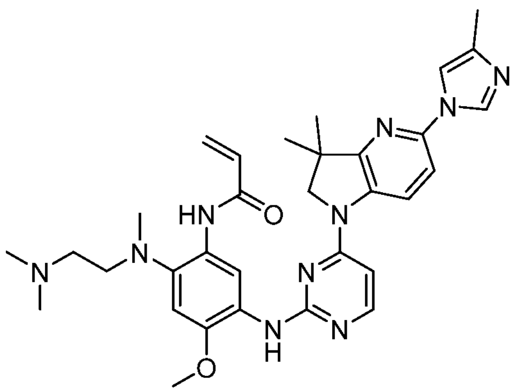

**Step 1: Synthesis of $N^4$-(4-(3,3-dimethyl-5-(4-methyl-1*H*-imidazole-1-yl)-2,3-dihydro-1*H*-pyrrolo[3,2-*b*]pyridin-1-yl)pyrimidin-2-yl)-1$N^1$-(2-(dimethylamino)ethyl)-5-methoxy-$N^1$-methylbenzene-1,2,4-triamine**

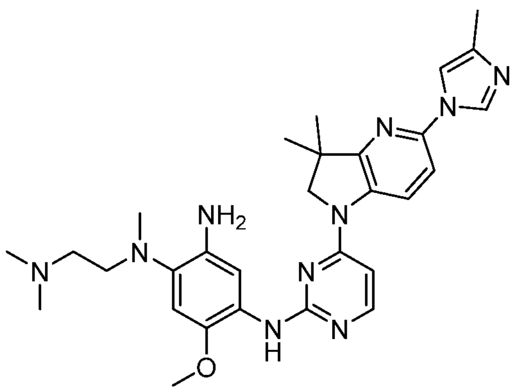

$N^1$-(4-(3,3-dimethyl-5-(4-methyl-1*H*-imidazole-1-yl)-2,3-dihydro-1*H*-pyrrolo[3,2-b]pyridin-1-yl)pyrimidin-2-yl)-$N^4$-(2-(dimethylamino)ethyl)-2-methoxy-$N^4$-methyl-5-nitrobenzene-1,4-diamine (53 mg, 0.093 mmol) was dissolved in 5 mL of methanol. 10% palladium on carbon (10 mg) was added to the mixture. The reaction mixture was subjected to hydrogen displacement three times, and stirred for 1 hr at room temperature in the atmosphere of hydrogen. After the reaction was completed, the resultant was filtered with diatomite. The solvent was removed to subsequently obtain $N^4$-(4-(3,3-dimethyl-5-(4-methyl-1*H*-imidazole-1-yl)-2,3-dihydro-1*H*-pyrrolo[3,2-*b*]pyridin-1-yl)pyrimidin-2-yl)-$N^1$-(2-(dimethylamino)ethyl)-5-methoxy-$N^1$-methylbenzene-1,2,4-triamine (32 mg, yield: 61.8%). ESI-MS: 543.2[M+1]$^+$.

**Step 2: Synthesis of *N*-(5-((4-(3,3-dimethyl-5-(4-methyl-1*H*-imidazole-1-yl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide**

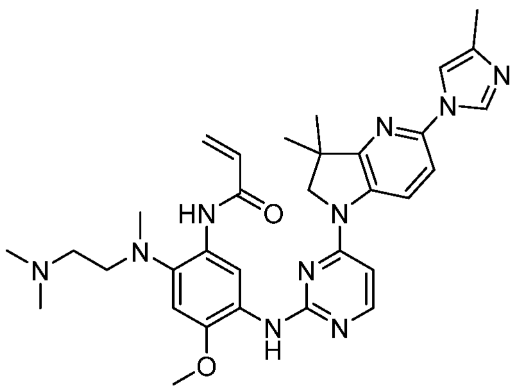

$N^4$-(4-(3,3-dimethyl-5-(4-methyl-1*H*-imidazole-1-yl)-2,3-dihydro-1*H*-pyrrolo[3,2-*b*]pyiidin-1-yl)pyrimidin-2-yl)-$N^1$-(2-(dimethylamino)ethyl)-5-methoxy-$N^1$-methylbenzene-1,2,4-triamine (32 mg, 0.059 mmol) was dissolved in anhydrous acetonitrile/water (1 mL/0.3 mL). *N*,*N*-diisopropylethylamine (22.8 mg, 0.177 mmol) was added to the mixture. Acryloyl chloride (16 mg, 0.177 mmol) was added to the reaction mixture at 0 °C. After the reaction was completed, stratification was conducted with dichloromethane and water. The organic phase was washed with water and saturated sodium chloride in sequence, then dried over anhydrous sodium sulfate, filtered, concentrated, and then separated by reversed column chromatography [40-50% acetonitrile/water] to obtain *N*-(5-((4-(3,3-dimethyl-5-(4-methyl-1H-imidazole-1-yl)-2,3-dihydro-1*H*-pyrrolo[3,2-*b*]pyridin-1-yl)pyrimidin-2-yl)amino)-2-((2-(dimethylamino)ethyl)(methyl)amino)-4-methoxyphenyl)acrylamide (13 mg, yield: 35.1%). ESI-MS: 597.3 [M+1]$^+$.

$^1$HNMR(400MHz, DMSO-$d_6$) δ 9.98 (s, 1H), 8.55 (s, 1H), 8.48 (d,J = 8.7Hz, 1H), 8.26 (d,J = 1.4 Hz, 1H), 8.20 - 8.11 (m, 2H), 7.52 (t,J = 1.3Hz, 1H), 7.23 (d,J = 8.7Hz, 1H), 7.01 (s, 1H), 6.43 (dd,J = 16.9, 10.1Hz, 1H), 6.28 (d,J = 5.8Hz, 1H), 6.16 (dd,J = 16.9, 2.1Hz, 1H), 5.68 (dd,J = 10.1, 2.1Hz, 1H), 3.90 (s, 2H), 3.80 (s, 3H), 2.91 (s, 2H), 2.75 (s, 3H), 2.33 (d,J = 6.1Hz, 2H), 2.25 - 2.15 (m, 9H), 1.36 (s, 6H).

## Biological Test Evaluation

### I. Cell proliferation assay

#### (i) Reagents and consumables

Fetal bovine serum (FBS) (GBICO, Cat#10099-141);
CellTiter-Glo® Luminescent Cell Viability Assay Kit (Promega, Cat#G7572);
Black transparent flat-bottomed 96-well plate (Corning®, Cat# 3603).

#### (ii) Instruments

SpectraMax Multi-Label Microplate Detector MD, 2104-0010A;
$CO_2$ incubator, Thermo Scientific 3100 Series;
Biosafety cabinet, Thermo Scientific, 1300 Series type A2;
Inverted microscope, Olympus, CKX41SF;
Siemens refrigerator KK25E76TI.

#### (iii) Cell lines and culture conditions

| No. | Cell lines | Cell culture medium | Cell density |
|---|---|---|---|
| 1 | A431 | DMEM+15%FBS | 5000 |
| 2 | Ba/F3 EGFR-D770-N771ins_SVD | RPMI 1640+10%FBS | 3000 |
| 3 | Ba/F3 EGFR-V769_D770insASV | RPMI 1640+10%FBS | 3000 |

#### (iv) Experimental procedures

**1. Cell culture and seeding:**

(1) Cells in the logarithmic growth phase were harvested, and counted using a platelet counter. Cell viability was detected by the trypan blue exclusion assay to ensure that the cell viability was above 90%.
(2) The cell concentration was adjusted to achieve the final density; and 90 μL of the cell suspension was added to the 96-well plate.
(3) The cells were incubated in the 96-well plate overnight at 37°C in the presence of 5% $CO_2$ with 95% humidity.

**2. T0 benchmark data:**

(1) 10 μL of PBS was added to each well of the T0 flat plate containing the cells.
(2) The CTG reagent was thawed, and the cell plate was equilibrated to room temperature for 30 min.
(3) An equal volume of CTG solution was added to each well.
(4) The cells were shaken for 5 min on an orbital shaker for lysis.
(5) The cell plate was left at room temperature for 20 min to stabilize luminescent signals.
(6) The values of T0 luminescent signals were read.

### 3. Compound dilution and addition

(1) According to the compound information table, a corresponding volume of DMSO was added to the corresponding compound powder to prepare a 10 mM stock solution.
(2) Compound solutions diluted at 1000 fold and 3.16 fold were prepared.
(3) The compound solution diluted at 1000 fold was diluted at 100 fold with PBS, to prepare the compound solutions at 10 fold in 9 concentrations, with the maximum concentration of 10 μM; and the compound solution was diluted at 3.16 fold. 10 μL of medicament solution was added to each well of the seeded 96-well plate. Three replicate wells were provided for each concentration of the compound, and the final concentration of DMSO was 0.1%.
(4) Cells were placed in the 96-well plate containing the medicament and subsequently cultured for 72 hrs at 37°C in the presence of 5% $CO_2$ with 95% humidity, and then, CTG assay was conducted.

### 4. Reading of luminescent signals

(1) The CTG reagent was thawed, and the cell plate was equilibrated to room temperature for 30 min.
(2) An equal volume of CTG solution was added to each well.
(3) The cells were shaken for 5 min on an orbital shaker for lysis.
(4) The cell plate was left at room temperature for 20 min to stabilize the luminescent signals.
(5) Luminescent values were read.

### 5. Data processing

The data were analyzed using software GraphPad Prism 7.0, and fitted by the nonlinear S-curve regression to obtain a dose-effect curve, based on which the $IC_{50}$ value (unit: nM) was calculated, with the specific test results shown in Table 1.

Cell viability (%) = (Lum test medicament - Lum culture medium control)/(Lum cell control - Lum culture medium control) × 100%.

**Table 1: Biological test results**

| Example No. | A431 (EGFR-WT) (nM) | Ba/F3 EGFR-D770-N771ins_ SVD(nM) | Ba/F3 EGFR-V769_D770insASV(nM) |
|---|---|---|---|
| Example 1 | 897.60 | 60.20 | 69.80 |
| Example 2 | 1069.00 | 60.40 | 97.90 |
| Example 3 | 841.40 | 66.30 | 111.44 |
| Example 4 | 964.50 | 47.25 | 83.05 |
| Example 5 | 410.63 | 26.60 | 31.40 |
| Example 6 | NT | NT | NT |
| Example 7 | NT | NT | NT |
| Example 8 | 71.80 | 12.50 | 17.90 |
| Example 9 | NT | NT | NT |
| Example 10 | NT | NT | NT |
| Example 11 | 293.10 | 40.40 | 73.90 |
| Example 12 | 89.91 | 35.25 | 70.79 |
| Example 13 | 101.20 | 42.06 | 87.39 |

(continued)

| Example No. | A431 (EGFR-WT) (nM) | Ba/F3 EGFR-D770-N771ins_SVD(nM) | Ba/F3 EGFR-V769_D770insASV(nM) |
|---|---|---|---|
| **Example 14** | NT | NT | NT |
| **Example 15** | 815.3 | 37.0 | 74.8 |
| **Example 16** | 47.7 | 9.5 | 27.7 |
| **Example 17** | 125.3 | 33.9 | 54.6 |
| **Example 18** | NT | 60.0 | 142.3 |
| **Example 19** | NT | 41.6 | 141.6 |
| **Example 20** | 15.2 | 2.9 | 14.0* |
| **Example 21** | 80.4* | 3.8 | 19.0* |
| **Example 22** | 121.2 | 15.6 | 58.2* |
| **Example 23** | NT | 13.8 | 26.6 |
| **Example 24** | 849.8 | 25.4 | 44.3 |
| **Example 25** | NT | 56.5 | 105.6 |
| **Example 26** | NT | 22.9 | 76.1 |
| **Example 27** | NT | 31.5 | 68.8 |
| **Example 28** | 26.3 | 28.9 | 37.1 |
| **Example 29** | 22.1 | 20.0 | 45.5 |
| **Example 30** | 42.2 | 38.1 | 65.3 |
| **Example 31** | 11.1 | 13.0* | 33.0* |
| **Example 32** | 53.7 | 35.7 | 95.8 |
| **Example 33** | 264.1 | 35.5 | 84.3 |
| **Example 34** | 47.7 | 22.5 | 68.4 |
| **Example 35** | NT | 40.1 | 94.8 |
| **Example 36** | NT | 33.2 | 51.5 |
| **Example 37** | NT | 92.2 | 158.7 |
| **Example 38** | NT | 123.6 | 266.2 |
| **Example 39** | 396.3* | 24.9 | 35.8 |
| **Example 40** | NT | 37.0 | NT |
| **Example 41** | NT | 35.5 | NT |
| **Example 42** | 488.1 | 11.6 | 23.5 |
| **Example 43** | NT | 38.5 | NT |
| **Example 44** | 108.5 | 20.1 | 35.0 |
| **Example 45** | 1066.0 | 35.8 | 11.7 |
| **Example 46** | 401.5 | 22.6 | 21.6 |
| **Example 47** | 377.8 | 12.9 | 5.6 |
| **Example 48** | 66.0 | 23.7 | 9.1 |
| **Example 49** | 1017.0 | 25.2 | NT |
| **Example 50** | 82.4* | 14.6* | 32.1 |

(continued)

| Example No. | A431 (EGFR-WT) (nM) | Ba/F3 EGFR-D770-N771ins_ SVD(nM) | Ba/F3 EGFR-V769_D770insASV(nM) |
|---|---|---|---|
| **Example 51** | 620.4 | 34.8 | 122.7 |
| **Example 52** | 188.6* | 32.9 | 54.2 |
| **Example 53** | 96.9 | 14.8 | 26.3 |
| **Example 54** | 72.9* | 17.8 | 30.6* |
| **Example 55** | 311.6 | 23.8 | 47.0 |
| **Example 56** | 347.6 | 12.0 | 35.4 |
| **Example 57** | 672.8 | 32.7 | 79.7 |
| **Example 58** | NT | 68.2 | NT |
| **Example 59** | NT | 43.8 | 139.3 |
| **Example 60** | 747.0 | 34.5 | 110.5 |
| **Example 61** | 18.7 | 14.1 | 46.4 |
| **Example 62** | 581.9 | 21.7 | 36.3 |
| **Example 63** | 531.6 | 39.6 | 116.5 |
| **Example 64** | 493.6 | 17.1 | 31.9 |
| **Example 65** | NT | 108.4 | NT |
| **Example 66** | NT | 89.1 | NT |
| **Example 67** | NT | 108.5 | NT |
| **Example 68** | 673.2 | 6.5 | 17.1 |
| **Example 69** | 316.8* | 24.8 | 53.7 |
| **Example 70** | NT | 121.5 | NT |
| **Example 71** | NT | 372.1 | NT |
| **Example 72** | NT | 298.0 | NT |
| **Example 73** | NT | 810.7 | NT |
| **Example 74** | NT | 83.6 | NT |
| **Positive compound** | 280.1 * | 66.8* | 58.6* |
| Note | "NT", i.e., "Not Tested", means that the compound was not tested yet. " *" means the average of multiple measurements.<br>The positive compound is a compound in Example 4 of WO2018210246A1, with a structure as follows:<br>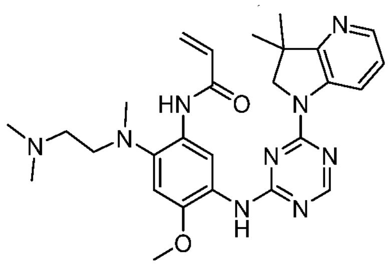 | | |

It can be seen from the biological activity data of the compounds of the specific examples that the compounds of the present invention have a strong inhibitory effect on EGFR exon 20 insertion mutations at a cellular level, and show high selectivity for EGFR WT. Under the same test conditions, the cell inhibition activity of the compounds in some

examples of the present invention is improved several times compared with the positive compound.

All documents mentioned in the present invention are hereby incorporated by reference in their entirety, just as each document is cited separately as a reference. In addition, it should be understood that various modifications and changes may be made by those skilled in the art after reading the above disclosure of the present invention and these equivalent forms also fall within the scope defined by the claims appended hereto.

## Claims

1. A compound of formula (I), or a stereoisomer or pharmaceutically acceptable salt thereof:

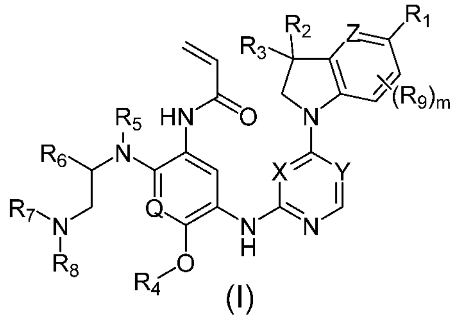

(I)

,

wherein, X and Y are each independently $CR_{10}$ or N; Z is $CR_{11}$ or N; Q is CH or N; $R_1$ is selected from the group consisting of hydrogen, deuterium, halogen, cyano, nitro, azido, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -SFs, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$, the above groups are optionally further substituted by one or more substituents selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, -SFs, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$,

or, when $m \geq 1$, $R_1$ and adjacent $R_9$, together with the moiety to which they are directly attached, form a $C_{5-6}$ cycloalkyl or 5-6 membered heterocyclyl;

$R_2$ and $R_3$ are each independently selected from the group consisting of hydrogen, deuterium, halogen, cyano, nitro, azido, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl and 5-10 membered heteroaryl,

or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, nitro, azido, $C_{1-10}$ alkyl, $C_{2-10}$ alkenyl, $C_{2-10}$ alkynyl, $C_{1-10}$ haloalkyl, $C_{1-10}$ deuterioalkyl, $C_{3-12}$ cycloalkyl, 3-12 membered heterocyclyl, $C_{6-10}$ aryl, 5-10 membered heteroaryl, =O, -SFs, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

$R_4$ is selected from the group consisting of hydrogen, deuterium, $C_{1-10}$ alkyl, $C_{2-10}$ alkenyl, $C_{3-12}$ cycloalkyl, 3-12 membered heterocyclyl, $C_{6-10}$ aryl and 5-10 membered heteroaryl, the above groups are optionally further substituted by one or more substituents selected from the group consisting of deuterium, halogen, hydroxy, =O, cyano, $C_{1-10}$ alkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

$R_5$ is selected from the group consisting of hydrogen, deuterium, hydroxy, $C_{1-10}$ alkyl, $C_{1-10}$ haloalkyl, $C_{1-10}$ deuterioalkyl, $C_{2-4}$ alkenyl, $C_{3-6}$ cycloalkyl and 3-6 membered heterocyclyl;

$R_6$ is selected from the group consisting of hydrogen, deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -SFs, $-S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

or, Rs and $R_6$, together with the moiety to which they are directly attached, form a 4-6 membered heterocyclyl, the above 4-6 membered heterocyclyl is optionally further substituted by one or more substituents selected from the group consisting of deuterium, halogen, cyano, nitro, azido, $C_{1-10}$ alkyl, $C_{1-10}$ haloalkyl, $C_{1-10}$ deute-

rioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ and $-N(R_{15})-C(O)R_{14}$;

$R_7$ and Rs are each independently selected from the group consisting of hydrogen, deuterium, hydroxy, $C_{1-10}$ alkyl, $C_{2-4}$ alkenyl, $C_{3-6}$ cycloalkyl and 3-6 membered heterocyclyl, or, $R_7$ and $R_8$, together with the nitrogen atom to which they are directly attached, form a 3-12 membered heterocyclyl, the above groups are optionally further substituted by one or more substituents selected from the group consisting of deuterium, halogen, hydroxy, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

each $R_9$ is independently selected from the group consisting of hydrogen, deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ and $-N(R_{15})-C(O)R_{14}$, or, when m=2, two $R_9$, together with the moiety to which they are directly attached, form a $C_{3-12}$ cycloalkyl or 3-12 membered heterocyclyl, the above groups are optionally further substituted by one or more substituents selected from the group consisting of deuterium, halogen, hydroxy, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

each $R_{10}$ is independently selected from the group consisting of hydrogen, deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ and $-N(R_{15})-C(O)R_{14}$;

$R_{11}$ is selected from the group consisting of hydrogen, deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -SFs, $-S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

each $R_{12}$ is independently selected from the group consisting of hydrogen, deuterium, hydroxy, $C_{1-10}$ alkyl, $C_{2-10}$ alkenyl, $C_{3-12}$ cycloalkyl, 3-12 membered heterocyclyl, $C_{6-10}$ aryl, 5-10 membered heteroaryl and $-NR_{15}R_{16}$, the above groups are optionally further substituted by one or more substituents selected from the group consisting of deuterium, halogen, hydroxy, oxo, $C_{1-10}$ alkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

each $R_{13}$ is independently selected from the group consisting of hydrogen, deuterium, $C_{1-10}$ alkyl, $C_{2-10}$ alkenyl, $C_{3-12}$ cycloalkyl, 3-12 membered heterocyclyl, $C_{6-10}$ aryl and 5-10 membered heteroaryl, the above groups are optionally further substituted by one or more substituents selected from the group consisting of deuterium, halogen, hydroxy, oxo, cyano, $C_{1-10}$ alkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

each $R_{14}$ is independently selected from the group consisting of hydrogen, deuterium, hydroxy, $C_{1-10}$ alkyl, $C_{1-10}$ alkoxy, $C_{2-10}$ alkenyl, $C_{2-10}$ alkynyl, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$, the above groups are optionally further substituted by one or more substituents selected from the group consisting of deuterium, halogen, hydroxy, cyano, $C_{1-10}$ alkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{15}R_{16}$;

$R_{15}$ and $R_{16}$ are each independently selected from the group consisting of hydrogen, deuterium, hydroxy, $C_{1-10}$ alkoxy, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl, amino, mono$C_{1-10}$ alkylamino, di$C_{1-10}$ alkylamino and $C_{1-10}$ alkanoyl, the above groups are optionally further substituted by one or more substituents selected from the group consisting of deuterium, halogen, hydroxy, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy, amino, mono$C_{1-10}$ alkylamino, di$C_{1-10}$ alkylamino and $C_{1-10}$ alkanoyl,

or, $R_{15}$ and $R_{16}$, together with the nitrogen atom to which they are directly attached, form a 4-10 membered heterocyclyl or 5-10 membered heteroaryl, the above groups are optionally further substituted by one or more

substituents selected from the group consisting of deuterium, halogen, hydroxy, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkyloxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy, amino, mono$C_{1-10}$ alkylamino, di$C_{1-10}$ alkylamino and $C_{1-10}$ alkanoyl;

m is 0, 1 or 2;

each r is independently 0, 1 or 2.

**2.** The compound of formula (I), the stereoisomer or pharmaceutically acceptable salt thereof of claim 1, wherein, Ri is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -SFs, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$,

or, when m≥1, $R_1$ and adjacent $R_9$, together with the moiety to which they are directly attached, form a $C_{5-6}$ cycloalkyl or 5-6 membered heterocyclyl;

$R_2$ and $R_3$ are each independently selected from the group consisting of hydrogen, deuterium, halogen, cyano, nitro, azido, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl and 5-8 membered heteroaryl, or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

$R_4$ is selected from the group consisting of hydrogen, deuterium, $C_{1-4}$ alkyl, $C_{2-4}$ alkenyl, $C_{3-6}$ cycloalkyl, 3-6 membered heterocyclyl, $C_{6-8}$ aryl and 5-8 membered heteroaryl, the above groups are optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_5$ is selected from the group consisting of hydrogen, deuterium, hydroxy, $C_{1-4}$ alkyl, $C_{1-4}$ haloalkyl, $C_{1-4}$ deuterioalkyl, $C_{2-4}$ alkenyl, $C_{3-6}$ cycloalkyl and 3-6 membered heterocyclyl;

$R_6$ is selected from the group consisting of hydrogen, deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -SFs, $-S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

or, $R_5$ and $R_6$, together with the moiety to which they are directly attached, form a 4-6 membered heterocyclyl, the above 4-6 membered heterocyclyl is optionally further substituted by one or more substituents selected from the group consisting of deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

$R_7$ and $R_8$ are each independently selected from the group consisting of hydrogen, deuterium, hydroxy, $C_{1-4}$ alkyl, $C_{2-4}$ alkenyl, $C_{3-6}$ cycloalkyl and 3-6 membered heterocyclyl, or, $R_7$ and $R_8$, together with the nitrogen atom to which they are directly attached, form a 3-6 membered heterocyclyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

each $R_9$ is independently selected from the group consisting of hydrogen, deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$, or, when m=2, two $R_9$, together with the moiety to which they are directly attached, form a $C_{3-6}$ cycloalkyl or 3-6 membered heterocyclyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$

alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;
each $R_{10}$ is independently selected from the group consisting of hydrogen, deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ and $-N(R_{15})-C(O)R_{14}$;
$R_{11}$ is selected from the group consisting of hydrogen, deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -SFs, - $S(O)_rR_{12}$, $-OR_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;
wherein, $R_{12}$, $R_{13}$, $R_{14}$, $R_{15}$, $R_{16}$ and r are defined as in claim 1.

3. The compound of formula (I), the stereoisomer or pharmaceutically acceptable salt thereof of claim 1, wherein, the compound of formula (I) is a compound of formula (IIa) as below:

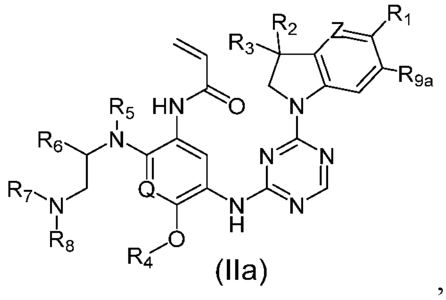

(IIa)

,

wherein, Z is $CR_{11}$ or N; Q is CH or N;
$R_1$ is selected from hydrogen, chlorine, bromine and $C_{1-4}$ alkyl, the $C_{1-4}$ alkyl is optionally further substituted by one or more substituents selected from the group consisting of deuterium, halogen, cyano, hydroxy, amino, dimethylamino, $C_{3-6}$ cycloalkyl and 3-6 membered heterocyclyl;
$R_2$ and $R_3$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, -SFs, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;
$R_4$ is selected from the group consisting of hydrogen, deuterium, $C_{1-4}$ alkyl, $C_{2-4}$ alkenyl, $C_{3-6}$ cycloalkyl and 3-6 membered heterocyclyl, the above groups are optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;
$R_5$ is selected from the group consisting of hydrogen, deuterium, $C_{1-4}$ alkyl, $C_{1-4}$ haloalkyl, $C_{1-4}$ deuterioalkyl and $C_{2-4}$ alkenyl;
$R_6$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl and 5-8 membered heteroaryl;
$R_7$ and $R_8$ are each independently selected from the group consisting of hydrogen, deuterium, hydroxy, $C_{1-4}$ alkyl and $C_{2-4}$ alkenyl, or, $R_7$ and $R_8$, together with the nitrogen atom to which they are directly attached, form a 3-6 membered heterocyclyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;
$R_{9a}$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl and $C_{6-8}$ aryl, the above groups are optionally further substituted by one or more substituents selected from the group consisting of deuterium, halogen, hydroxy,

$C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;
$R_{11}$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl and 3-6 membered heterocyclyl;
wherein, $R_{12}$, $R_{13}$, $R_{14}$, $R_{15}$, $R_{16}$ and r are defined as in claim 1.

**4.** The compound of formula (I), the stereoisomer or pharmaceutically acceptable salt thereof of claim 3, wherein, the compound of formula (I) is a compound of formula ($IIIa_1$) as below:

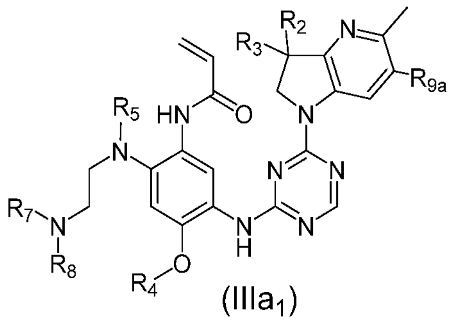

($IIIa_1$) ,

wherein, $R_2$ and $R_3$ are each independently selected from the group consisting of hydrogen, deuterium, halogen, $C_{1-4}$ alkyl, $C_{1-4}$ haloalkyl, $C_{1-4}$ deuterioalkyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl;
$R_4$ is selected from hydrogen, deuterium, $C_{1-4}$ alkyl and $C_{3-6}$ cycloalkyl, the above groups are optionally further substituted by one or more substituents selected from the group consisting of deuterium, halogen, hydroxy, cyano, $C_{1-4}$ alkyl, $C_{1-4}$ alkoxy, $C_{3-6}$ cycloalkyl, $C_{3-6}$ cycloalkyloxy, 3-6 membered heterocyclyl and 3-6 membered heterocyclyloxy;
$R_5$, $R_7$ and $R_8$ are each independently hydrogen or methyl;
$R_{9a}$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl and $C_{6-8}$ aryl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$.

**5.** The compound of formula (I), the stereoisomer or pharmaceutically acceptable salt thereof of claim 4, wherein, $R_2$ and $R_3$ are each independently selected from the group consisting of hydrogen, deuterium, fluorine, chlorine, bromine, methyl, ethyl, propyl, isopropyl, trifluoromethyl, difluoromethyl, trideuteriomethyl, dideuteriomethyl, cyclopropyl, cyclobutyl, oxacyclobutyl and azacyclobutyl, or, $R_2$ and $R_3$, together with the carbon atom to which they are directly attached, form a cyclopropyl, cyclobutyl, cyclopentyl, cyclohexyl, oxacyclobutyl, azacyclobutyl, oxacyclopentyl or azacyclopentyl, the above cyclopropyl, cyclobutyl, cyclopentyl, cyclohexyl, oxacyclobutyl, azacyclobutyl, oxacyclopentyl or azacyclopentyl is optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl and cyclobutyl;

$R_4$ is selected from the group consisting of hydrogen, deuterium, methyl, ethyl, propyl, isopropyl, cyclopropyl and cyclobutyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl;
$R_5$, $R_7$ and $R_8$ are each independently hydrogen or methyl;
$R_{9a}$ is selected from the group consisting of hydrogen, deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl, cyclobutyl and phenyl, the phenyl is optionally further substituted by one or more substituents selected from the group consisting of deuterium, fluorine, chlorine, bromine, hydroxy, methyl, ethyl, propyl, isopropyl, vinyl, ethynyl, trifluoromethyl, difluoromethyl, trideuteriomethyl, dideuteriomethyl, methoxy, ethoxy, cyclopropyl,

cyclobutyl, oxacyclobutyl and azacyclobutyl.

6. The compound of formula (I), the stereoisomer or pharmaceutically acceptable salt thereof of claim 3, wherein, the compound of formula (I) is a compound of formula ($IIIa_2$) as below:

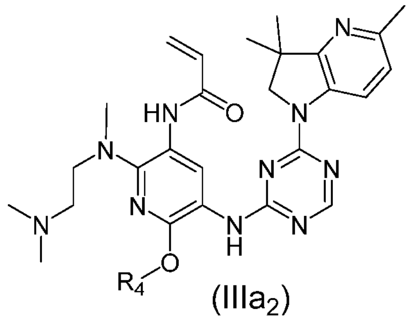

($IIIa_2$) ,

wherein, $R_4$ is isopropyl and cyclopropyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl.

7. The compound of formula (I), the stereoisomer or pharmaceutically acceptable salt thereof of claim 3, wherein, the compound of formula (I) is a compound of formula ($IIIa_3$) as below:

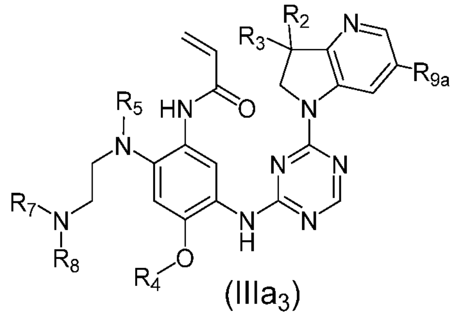

($IIIa_3$) ,

wherein, $R_2$ and $R_3$ are each independently selected from the group consisting of hydrogen, deuterium, fluorine, chlorine, bromine, methyl, ethyl, propyl, isopropyl, trifluoromethyl, difluoromethyl, trideuteriomethyl, dideuteriomethyl, cyclopropyl, cyclobutyl, oxacyclobutyl and azacyclobutyl, or, $R_2$ and $R_3$, together with the carbon atom to which they are directly attached, form a cyclobutyl, cyclopentyl, cyclohexyl, oxacyclobutyl, azacyclobutyl, oxacyclopentyl or azacyclopentyl, the above cyclopropyl, cyclobutyl, cyclopentyl, cyclohexyl, oxacyclobutyl, azacyclobutyl, oxacyclopentyl or azacyclopentyl is optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl and cyclobutyl;
$R_4$ is selected from the group consisting of hydrogen, deuterium, methyl, ethyl, propyl, isopropyl, cyclopropyl and cyclobutyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl;
$R_5$, $R_7$ and $R_8$ are each independently hydrogen or methyl;
$R_{9a}$ is selected from the group consisting of hydrogen, deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl and cyclobutyl,
provided that, when $R_{9a}$ is hydrogen, $R_2$ and $R_3$, together with the carbon atom to which they are directly attached, form a cyclobutyl, cyclopentyl, cyclohexyl, oxacyclobutyl, azacyclobutyl, oxacyclopentyl or azacyclopentyl.

8. The compound of formula (I), the stereoisomer or pharmaceutically acceptable salt thereof of claim 3, wherein, the

compound of formula (I) is a compound of formula ($IIIa_4$) as below:

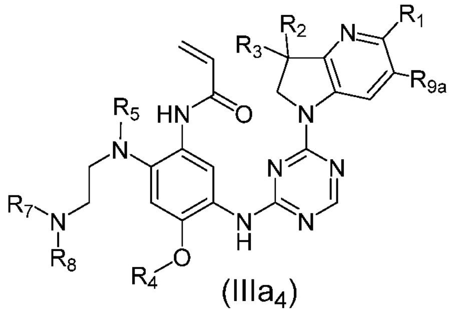

($IIIa_4$)

,

wherein, $R_1$ is chlorine or bromine;

$R_2$ and $R_3$ are each independently selected from the group consisting of hydrogen, deuterium, fluorine, chlorine, bromine, methyl, ethyl, propyl, isopropyl, trifluoromethyl, difluoromethyl, trideuteriomethyl, dideuteriomethyl, cyclopropyl, cyclobutyl, oxacyclobutyl and azacyclobutyl, or, $R_2$ and $R_3$, together with the carbon atom to which they are directly attached, form a cyclopropyl, cyclobutyl, cyclopentyl, cyclohexyl, oxacyclobutyl, azacyclobutyl, oxacyclopentyl or azacyclopentyl, the above cyclopropyl, cyclobutyl, cyclopentyl, cyclohexyl, oxacyclobutyl, azacyclobutyl, oxacyclopentyl or azacyclopentyl is optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl and cyclobutyl;

$R_4$ is selected from the group consisting of hydrogen, deuterium, methyl, ethyl, propyl, isopropyl, cyclopropyl and cyclobutyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl;

$R_5$, $R_7$ and $R_8$ are each independently hydrogen or methyl;

$R_{9a}$ is selected from the group consisting of hydrogen, deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl, cyclobutyl and phenyl, the phenyl is optionally further substituted by one or more substituents selected from the group consisting of deuterium, fluorine, chlorine, bromine, hydroxy, methyl, ethyl, propyl, isopropyl, vinyl, ethynyl, trifluoromethyl, difluoromethyl, trideuteriomethyl, dideuteriomethyl, methoxy, ethoxy, cyclopropyl, cyclobutyl, oxacyclobutyl and azacyclobutyl.

**9.** The compound of formula (I), the stereoisomer or pharmaceutically acceptable salt thereof of claim 1, wherein, the compound of formula (I) is a compound of formula (IIb) as below:

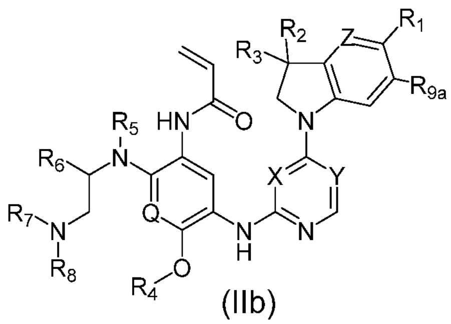

(IIb)

,

wherein, one of X and Y is CH, the other is N; Z is $CR_{11}$ or N; Q is CH or N;

$R_1$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl and $-SF_5$, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$,$-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and

$-N(R_{15})-C(O)R_{14}$,

or, $R_1$ and $R_{9a}$, together with the moiety to which they are directly attached, form a $C_{5-6}$ cycloalkyl or 5-6 membered heterocyclyl;

$R_2$ and $R_3$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;

$R_4$ is selected from the group consisting of hydrogen, deuterium, $C_{1-4}$ alkyl, $C_{2-4}$ alkenyl, $C_{3-6}$ cycloalkyl and 3-6 membered heterocyclyl, the above groups are optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_5$ is selected from the group consisting of hydrogen, deuterium, $C_{1-4}$ alkyl, $C_{1-4}$ haloalkyl, $C_{1-4}$ deuterioalkyl and $C_{2-4}$ alkenyl;

$R_6$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl and 5-8 membered heteroaryl;

or, $R_5$ and $R_6$, together with the moiety to which they are directly attached, form a 4-6 membered heterocyclyl, the above 4-6 membered heterocyclyl is optionally further substituted by one or more substituents selected from the group consisting of deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ and $-N(R_{15})-C(O)R_{14}$;

$R_7$ and $R_8$ are each independently selected from the group consisting of hydrogen, deuterium, hydroxy, $C_{1-4}$ alkyl and $C_{2-4}$ alkenyl, or, $R_7$ and $R_8$, together with the nitrogen atom to which they are directly attached, form a 3-6 membered heterocyclyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_{9a}$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl and 5-8 membered heteroaryl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_{11}$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl and 3-6 membered heterocyclyl;

wherein, $R_{12}$, $R_{13}$, $R_{14}$, $R_{15}$, $R_{16}$ and r are defined as in claim 1.

**10.** The compound of formula (I), the stereoisomer or pharmaceutically acceptable salt thereof of claim 9, wherein, the compound of formula (I) is a compound of formula ($IIIb_1$) or ($IIIb_2$) as below:

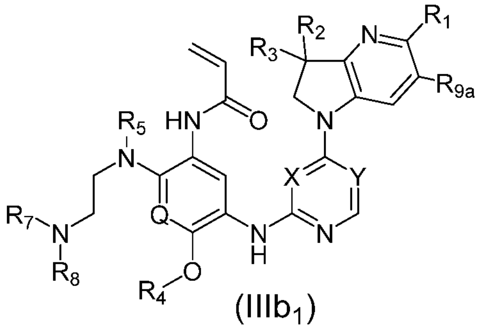

(IIIb1) or

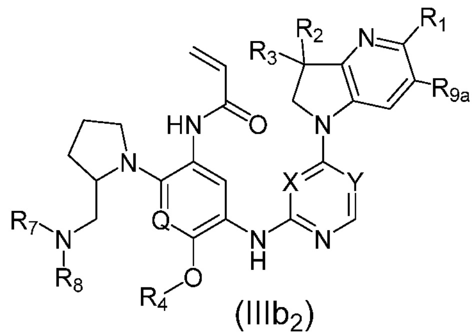

(IIIb2) ,

wherein, one of X and Y is CH, the other is N; each Q is CH or N;

each $R_1$ is independently selected from the group consisting of hydrogen, deuterium, halogen, cyano, $C_{1-4}$ alkyl, $C_{2-4}$ alkynyl, $C_{3-6}$ cycloalkyl and 5-8 membered heteroaryl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl and 3-6 membered heterocyclyl, or, $R_1$ and $R_{9a}$, together with the moiety to which they are directly attached, form a $C_{5-6}$ cycloalkyl or 5-6 membered heterocyclyl;

$R_2$ and $R_3$ are each independently selected from the group consisting of hydrogen, deuterium, halogen, $C_{1-4}$ alkyl, $C_{1-4}$ haloalkyl, $C_{1-4}$ deuterioalkyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, $C_{1-4}$ alkyl, $C_{2-4}$ alkenyl, $C_{2-4}$ alkynyl, $C_{1-4}$ haloalkyl, $C_{1-4}$ deuterioalkyl and $C_{3-6}$ cycloalkyl;

each $R_4$ is independently selected from hydrogen, deuterium, $C_{1-4}$ alkyl and $C_{3-6}$ cycloalkyl, the above groups are optionally further substituted by one or more substituents selected from the group consisting of deuterium, halogen, hydroxy, cyano, $C_{1-4}$ alkyl, $C_{1-4}$ alkoxy, $C_{3-6}$ cycloalkyl, $C_{3-6}$ cycloalkyloxy, 3-6 membered heterocyclyl and 3-6 membered heterocyclyloxy;

in the compound of formula (IIIbi), $R_5$ is selected from hydrogen, deuterium, $C_{1-4}$ alkyl, $C_{1-4}$ haloalkyl and $C_{1-4}$ deuterioalkyl;

$R_7$ and $R_8$ are each independently selected from hydrogen, deuterium and $C_{1-4}$ alkyl, or, $R_7$ and $R_8$, together with the nitrogen atom to which they are directly attached, form a 3-6 membered heterocyclyl;

each $R_{9a}$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl and $C_{6-8}$ aryl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl and 3-6 membered heterocyclyloxy.

**11.** The compound of formula (I), the stereoisomer or pharmaceutically acceptable salt thereof of claim 10, wherein, each $R_1$ is independently selected from the group consisting of hydrogen, deuterium, methyl, ethyl, propyl, isopropyl, ethynyl, cyclopropyl, cyclobutyl, cyclopentyl,

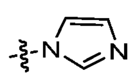 and 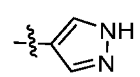 ,

the above groups are optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl, cyclobutyl, oxacyclobutyl and azacyclobutyl;

or, $R_1$ and $R_{9a}$, together with the moiety to which they are directly attached, form a cyclopentyl;

$R_2$ and $R_3$ are each independently selected from the group consisting of hydrogen, deuterium, fluorine, chlorine, bromine, methyl, ethyl, propyl, isopropyl, trifluoromethyl, difluoromethyl, trideuteriomethyl, dideuteriomethyl, cyclopropyl, cyclobutyl, oxacyclobutyl and azacyclobutyl, or, $R_2$ and $R_3$, together with the carbon atom to which they are directly attached, form a $C_{3-6}$ cycloalkyl or 3-6 membered heterocyclyl, the above $C_{3-6}$ cycloalkyl or 3-6 membered heterocyclyl is optionally further substituted by one or more substituents selected from the group

consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl and cyclobutyl;
each $R_4$ is independently selected from the group consisting of hydrogen, deuterium, methyl, ethyl, propyl, isopropyl, cyclopropyl and cyclobutyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl;
in the compound of formula (IIIbi), $R_5$ is selected from hydrogen, deuterium and methyl;
$R_7$ and $R_8$ are each independently selected from hydrogen, deuterium and methyl;
each $R_{9a}$ is independently selected from the group consisting of hydrogen, deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl, cyclobutyl and phenyl, the phenyl is optionally further substituted by one or more substituents selected from the group consisting of deuterium, fluorine, chlorine, bromine, hydroxy, methyl, ethyl, propyl, isopropyl, vinyl, ethynyl, trifluoromethyl, difluoromethyl, trideuteriomethyl, dideuteriomethyl, methoxy, ethoxy, cyclopropyl, cyclobutyl, oxacyclobutyl and azacyclobutyl.

**12.** The compound of formula (I), the stereoisomer or pharmaceutically acceptable salt thereof of claim 1, wherein, the compound of formula (I) is a compound of formula (IIc) as below:

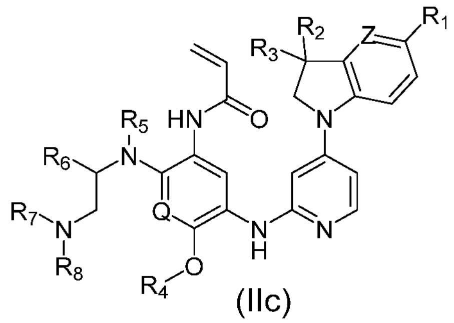

(IIc)

,

wherein, Z is $CR_{11}$ or N; Q is CH or N;
$R_1$ is selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl and $-SF_5$, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-O-R_{13}$, $-C(O)OR_{13}$, $-C(O)R_{14}$, $-O-C(O)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;
$R_2$ and $R_3$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-O-R_{13}$, $-C(O)OR_{13}$, $-C(O)R_{14}$, $-O-C(0)R_{14}$, $-NR_{15}R_{16}$, $-C(=NR_{15})R_{14}$, $-N(R_{15})-C(=NR_{16})R_{14}$, $-C(O)NR_{15}R_{16}$ and $-N(R_{15})-C(O)R_{14}$;
$R_4$ is selected from the group consisting of hydrogen, deuterium, $C_{1-4}$ alkyl, $C_{2-4}$ alkenyl, $C_{3-6}$ cycloalkyl and 3-6 membered heterocyclyl, the above groups are optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;
$R_5$ is selected from the group consisting of hydrogen, deuterium, $C_{1-4}$ alkyl, $C_{1-4}$ haloalkyl, $C_{1-4}$ deuterioalkyl and $C_{2-4}$ alkenyl;
$R_6$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl and 5-8 membered heteroaryl;
or, $R_5$ and $R_6$, together with the moiety to which they are directly attached, form a 4-6 membered heterocyclyl, the above 4-6 membered heterocyclyl is optionally further substituted by one or more substituents selected

from the group consisting of deuterium, halogen, cyano, nitro, azido, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, $-SF_5$, $-S(O)_rR_{12}$, $-OR_{13}$, $-C(O)OR_{13}$, $-C(O)R_{14}$, $-O-C(O)R_{14}$, $-NR_{15}R_{16}$, - $C(=NR_{15})R_{14}$, $-N(R_{15})-C(=NR_{16})R_{14}$, $-C(O)NR_{15}R_{16}$ and $-N(R_{15})-C(O)R_{14}$;

$R_7$ and $R_8$ are each independently selected from the group consisting of hydrogen, deuterium, hydroxy, $C_{1-4}$ alkyl and $C_{2-4}$ alkenyl, or, $R_7$ and $R_8$, together with the nitrogen atom to which they are directly attached, form a 3-6 membered heterocyclyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

$R_{11}$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl and 3-6 membered heterocyclyl;

wherein, $R_{12}$, $R_{13}$, $R_{14}$, $R_{15}$, $R_{16}$ and r are defined as in claim 1.

**13.** The compound of formula (I), the stereoisomer or pharmaceutically acceptable salt thereof of claim 12, wherein, the compound of formula (I) is a compound of formula (IIIci) or ($IIIc_2$) as below:

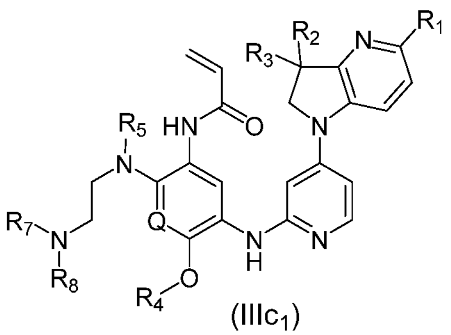
($IIIc_1$)

or

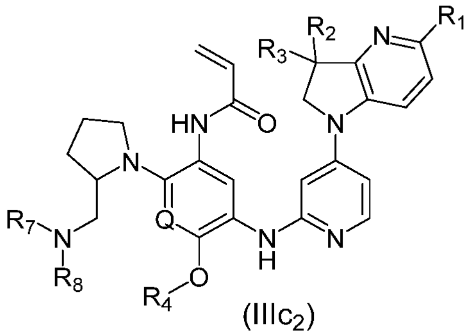
($IIIc_2$)

,

wherein, each Q is CH or N;

each $R_1$ is independently selected from the group consisting of hydrogen, deuterium, halogen, cyano, $C_{1-4}$ alkyl and 5-8 membered heteroaryl, the above groups are optionally further substituted by one or more substituents selected from the group consisting of deuterium, halogen, cyano, nitro, azido, $C_{1-4}$ alkyl, $C_{2-4}$ alkenyl, $C_{2-4}$ alkynyl, $C_{1-4}$ haloalkyl, $C_{1-4}$ deuterioalkyl, $C_{3-6}$ cycloalkyl and 3-6 membered heterocyclyl;

$R_2$ and $R_3$ are each independently selected from the group consisting of hydrogen, deuterium, halogen, $C_{1-4}$ alkyl, $C_{1-4}$ haloalkyl, $C_{1-4}$ deuterioalkyl, $C_{3-6}$ cycloalkyl and 3-6 membered heterocyclyl, or, $R_2$ and $R_3$, together with the carbon atom to which they are directly attached, form a $C_{3-6}$ cycloalkyl or 3-6 membered heterocyclyl;

each $R_4$ is independently selected from hydrogen, deuterium, $C_{1-4}$ alkyl and $C_{3-6}$ cycloalkyl, the above groups are optionally further substituted by one or more substituents selected from the group consisting of deuterium, halogen, hydroxy, cyano, $C_{1-4}$ alkyl, $C_{1-4}$ alkoxy, $C_{3-6}$ cycloalkyl, $C_{3-6}$ cycloalkyloxy, 3-6 membered heterocyclyl and 3-6 membered heterocyclyloxy;

in the compound of formula ($IIIc_1$), $R_5$ is selected from hydrogen, deuterium, $C_{1-4}$ alkyl, $C_{1-4}$ haloalkyl and $C_{1-4}$ deuterioalkyl;

$R_7$ and $R_8$ are each independently selected from hydrogen, deuterium and $C_{1-4}$ alkyl, or, $R_7$ and $R_8$, together with the nitrogen atom to which they are directly attached, form a 3-6 membered heterocyclyl.

**14.** The compound of formula (I), the stereoisomer or pharmaceutically acceptable salt thereof of claim 13, wherein, each $R_1$ is independently selected from the group consisting of hydrogen, deuterium, fluorine, chlorine, bromine, cyano, methyl, ethyl, propyl, isopropyl,

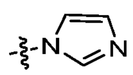
and
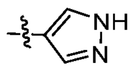
,

the above groups are optionally further substituted by one or more substituents selected from the group consisting of deuterium, fluorine, chlorine, bromine, cyano, methyl, ethyl, propyl, isopropyl, vinyl, ethynyl, trifluoromethyl, difluoromethyl, trideuteriomethyl, dideuteriomethyl, cyclopropyl, cyclobutyl, oxacyclobutyl and azacyclobutyl;

$R_2$ and $R_3$ are each independently selected from the group consisting of hydrogen, deuterium, fluorine, chlorine, bromine, methyl, ethyl, propyl, isopropyl, trifluoromethyl, difluoromethyl, trideuteriomethyl, dideuteriomethyl, cyclopropyl, cyclobutyl, oxacyclobutyl and azacyclobutyl;
each $R_4$ is independently selected from the group consisting of hydrogen, deuterium, methyl, ethyl, propyl, isopropyl, cyclopropyl and cyclobutyl, the above groups are optionally further substituted by one or more substituents selected from the group consisting of deuterium, fluorine, chlorine, bromine, hydroxy, cyano, methyl, ethyl, propyl, isopropyl, methoxy, ethoxy, cyclopropyl, cyclobutyl, oxacyclobutyl and azacyclobutyl;
in the compound of formula (IIIci), $R_5$ is selected from hydrogen, deuterium and methyl;
$R_7$ and $R_8$ are each independently selected from hydrogen, deuterium and methyl.

**15.** The compound of formula (I), the stereoisomer or pharmaceutically acceptable salt thereof of claim 1, wherein, each $R_{12}$ is independently selected from the group consisting of hydrogen, deuterium, hydroxy, $C_{1-4}$ alkyl, $C_{2-4}$ alkenyl, $C_{3-6}$ cycloalkyl, 3-6 membered heterocyclyl, $C_{6-8}$ aryl, 5-8 membered heteroaryl and $-NR_{15}R_{16}$, the above groups are optionally further substituted by one or more substituents selected from the group consisting of deuterium, halogen, hydroxy, oxo, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;

each $R_{13}$ is independently selected from the group consisting of hydrogen, deuterium, $C_{1-4}$ alkyl, $C_{2-4}$ alkenyl, $C_{3-6}$ cycloalkyl, 3-6 membered heterocyclyl, $C_{6-8}$ aryl and 5-8 membered heteroaryl, the above groups are optionally further substituted by one or more substituents selected from the group consisting of deuterium, halogen, hydroxy, oxo, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;
each $R_{14}$ is independently selected from the group consisting of hydrogen, deuterium, hydroxy, $C_{1-4}$ alkyl, $C_{1-4}$ alkoxy, $C_{2-4}$ alkenyl, $C_{2-4}$ alkynyl, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$, the above groups are optionally further substituted by one or more substituents selected from the group consisting of deuterium, halogen, hydroxy, cyano, $C_{1-4}$ alkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and $-NR_{15}R_{16}$;
$R_{15}$ and $R_{16}$ are each independently selected from the group consisting of hydrogen, deuterium, hydroxy, $C_{1-4}$ alkoxy, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl, amino, mono$C_{1-4}$ alkylamino, di$C_{1-4}$ alkylamino and $C_{1-4}$ alkanoyl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy, amino, mono$C_{1-4}$ alkylamino, di$C_{1-4}$ alkylamino and $C_{1-4}$ alkanoyl,
or, $R_{15}$ and $R_{16}$, together with the nitrogen atom to which they are directly attached, form a 5-8 membered heterocyclyl or 5-8 membered heteroaryl, the above groups are optionally further substituted by one or more substituents selected from the group consisting of deuterium, halogen, hydroxy, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkyloxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy, amino, mono$C_{1-4}$ alkylamino, di$C_{1-4}$ alkylamino and $C_{1-4}$ alkanoyl.

**16.** The compound of formula (I), the stereoisomer or pharmaceutically acceptable salt thereof of any one of claims 1-15, wherein, the compound is selected from the group consisting of the following compounds:

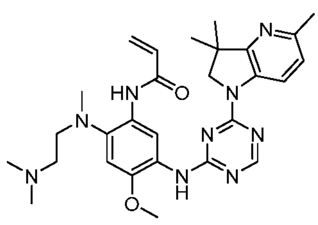
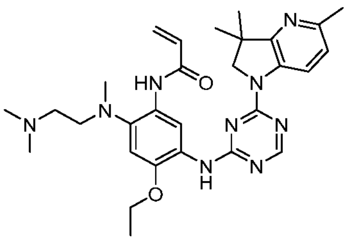
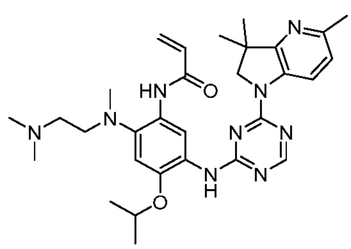
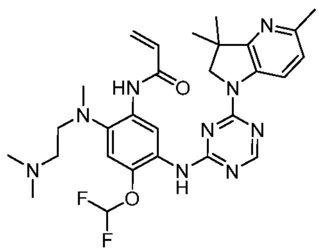
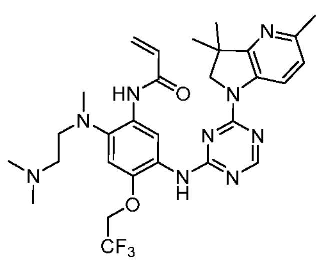
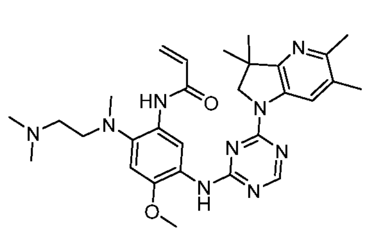
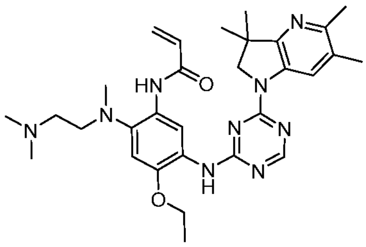
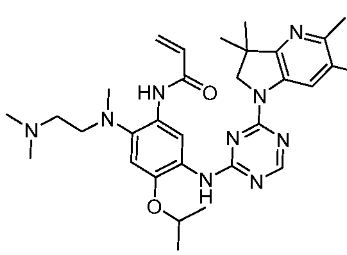
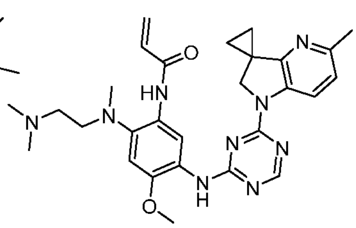
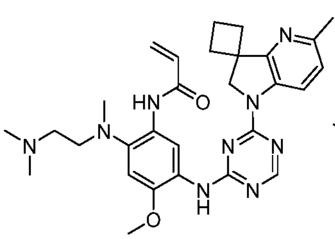
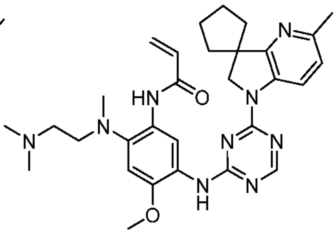
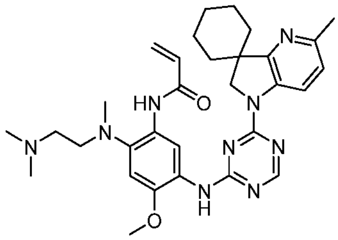
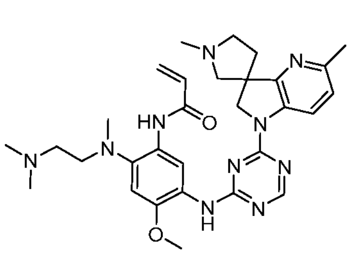
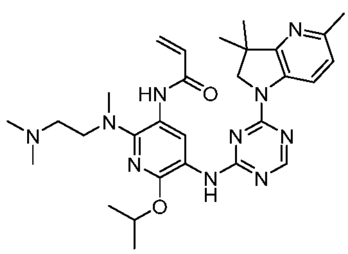
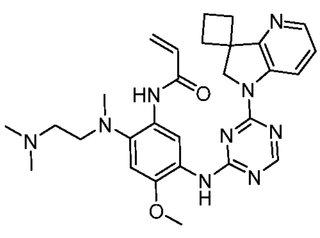
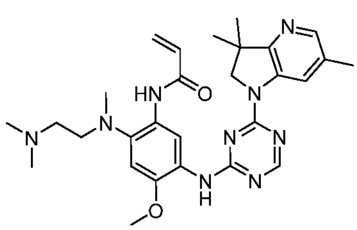

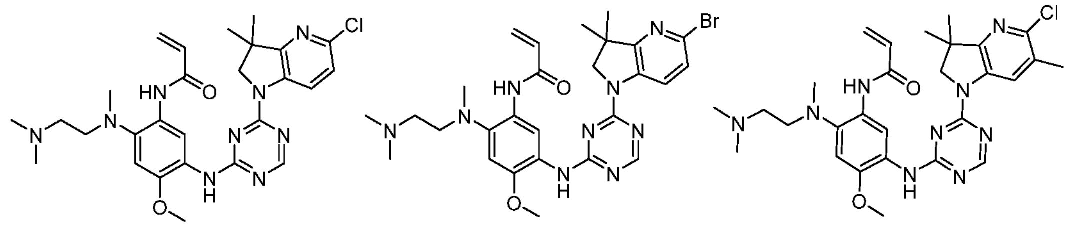

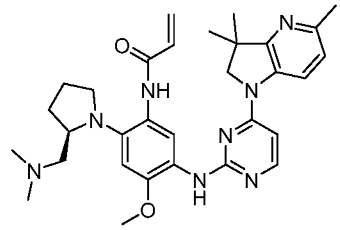

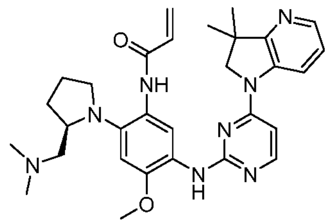

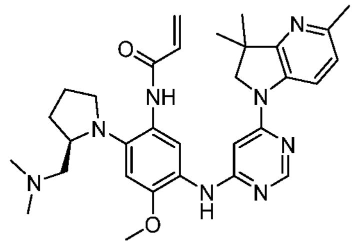

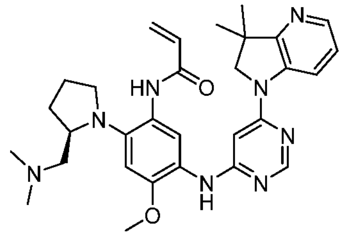

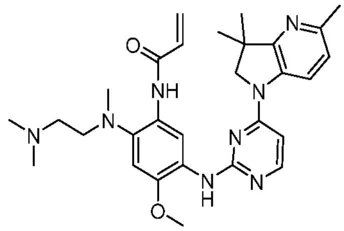

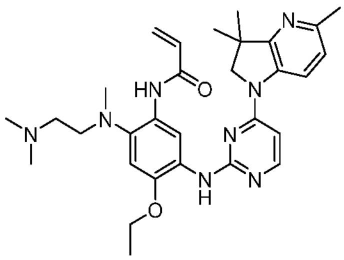

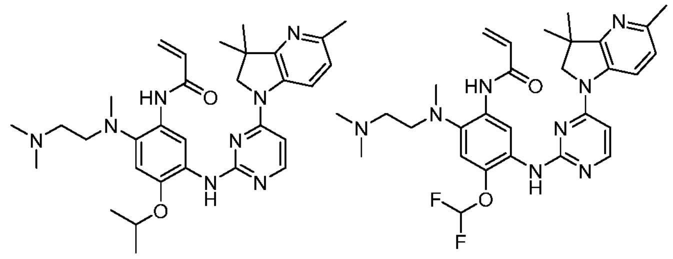

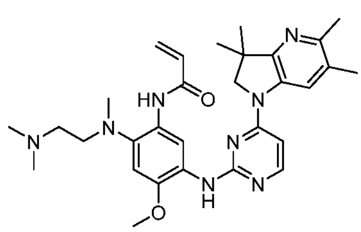

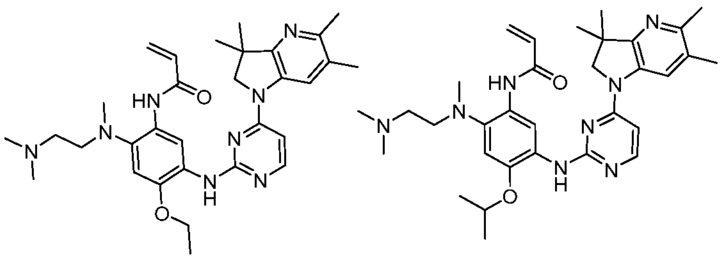

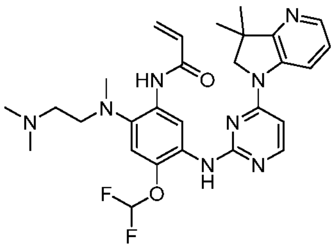

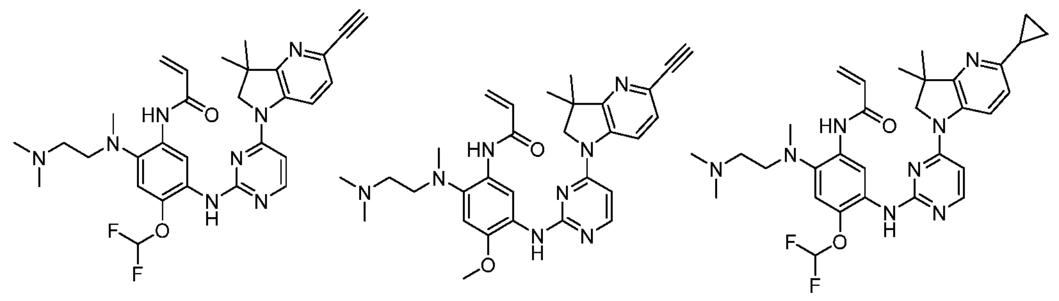

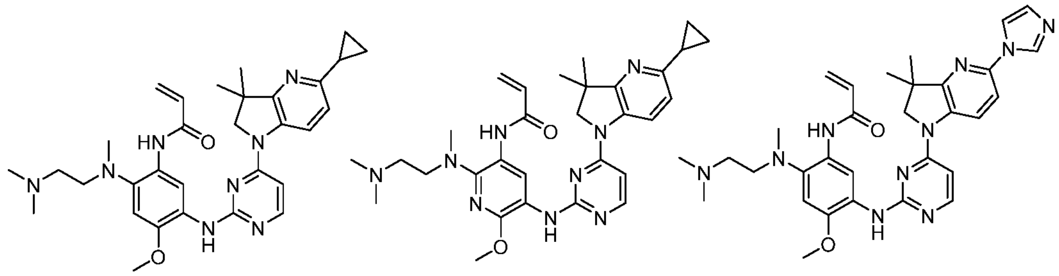

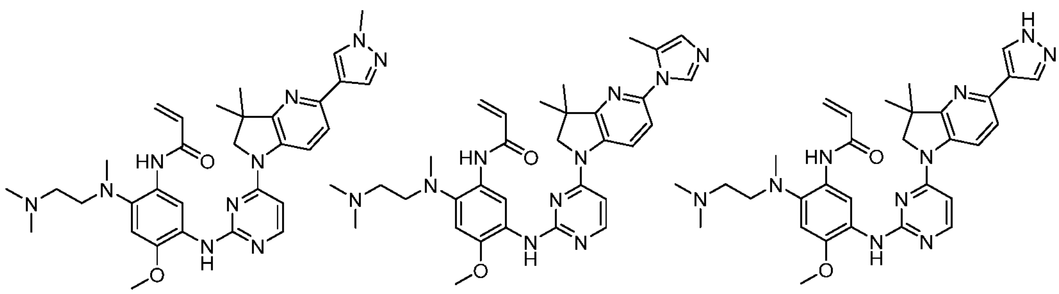

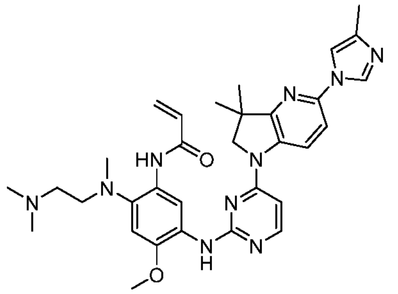

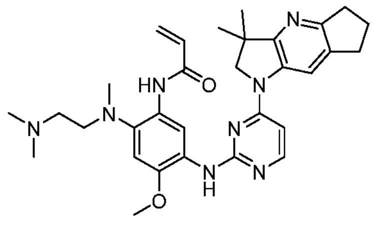

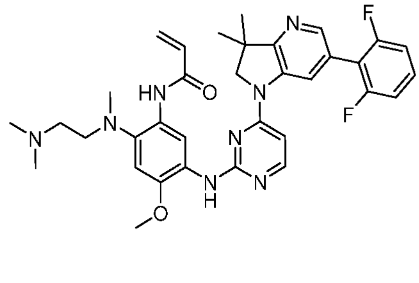

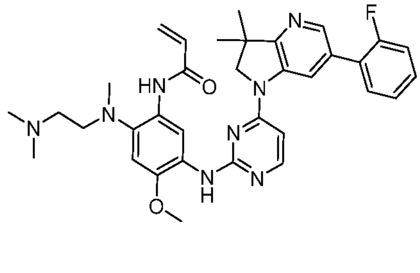

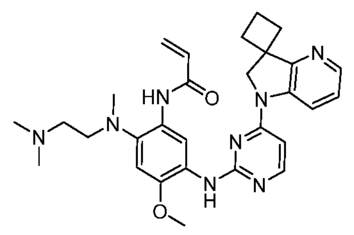

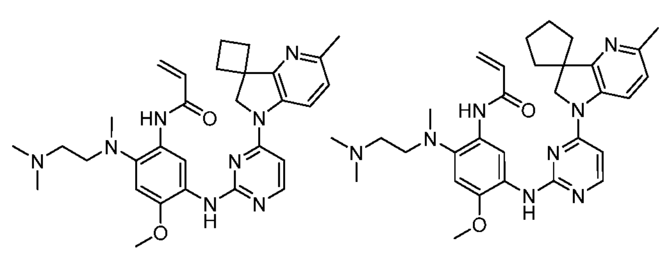

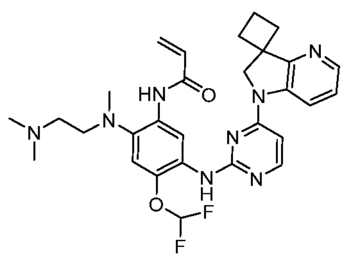

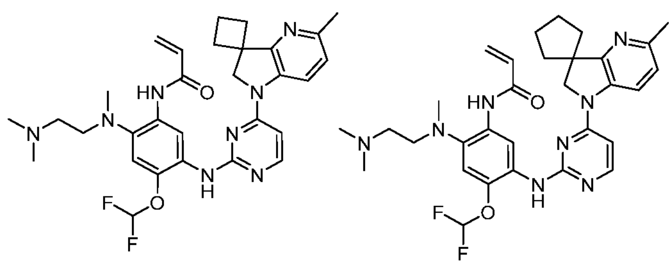

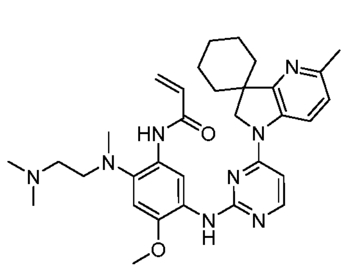

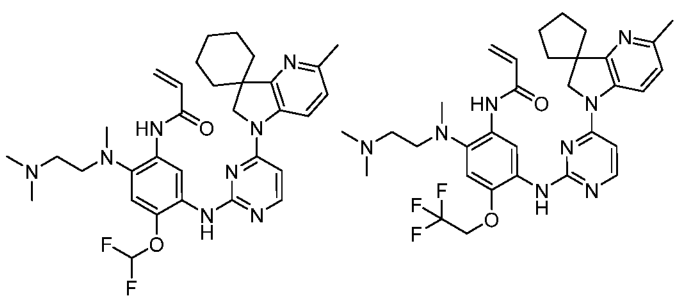

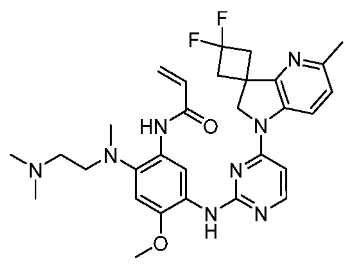

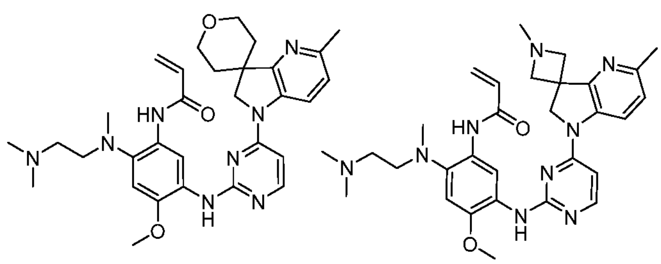

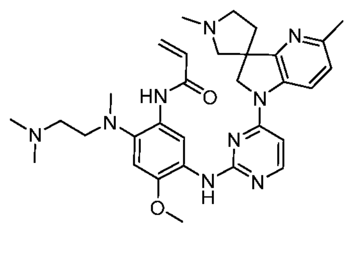

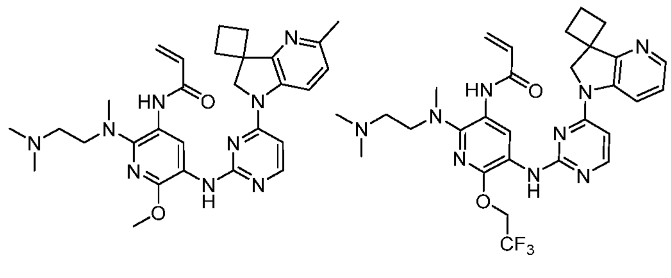

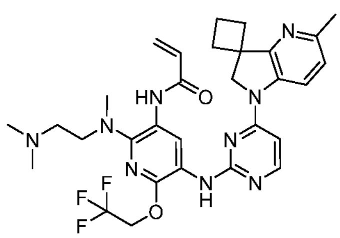
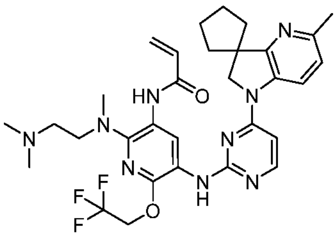
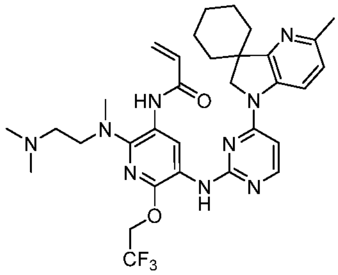

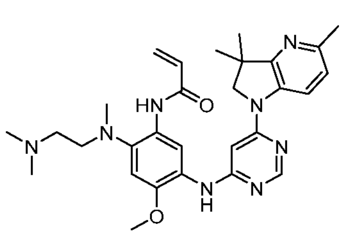
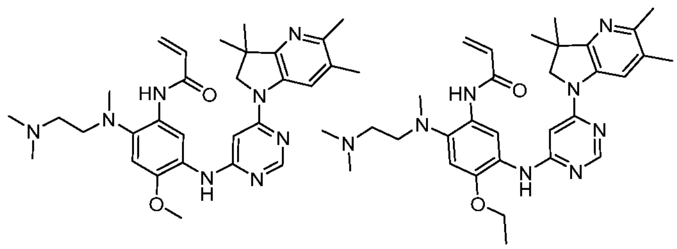

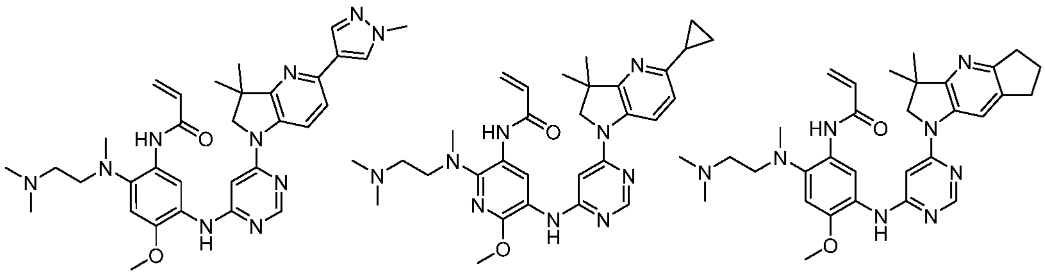

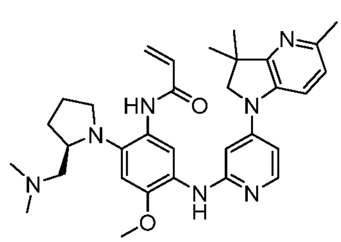
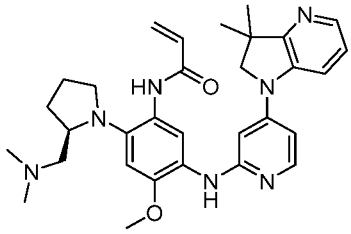
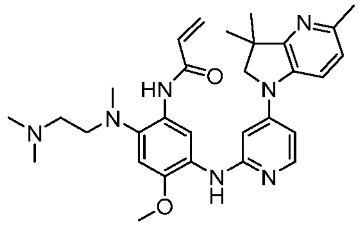

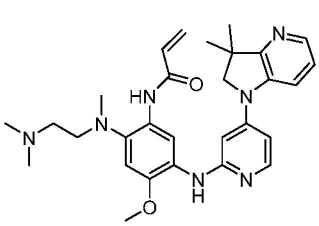
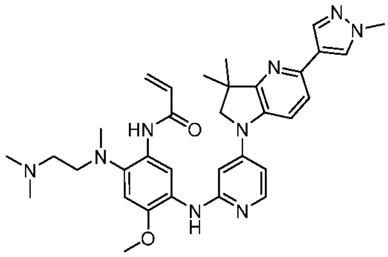
and
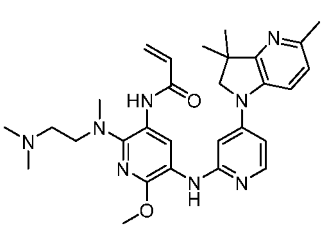
.

**17.** A preparation method for the compound of formula (I), the stereoisomer or pharmaceutically acceptable salt thereof of claim 1, comprising the following step:

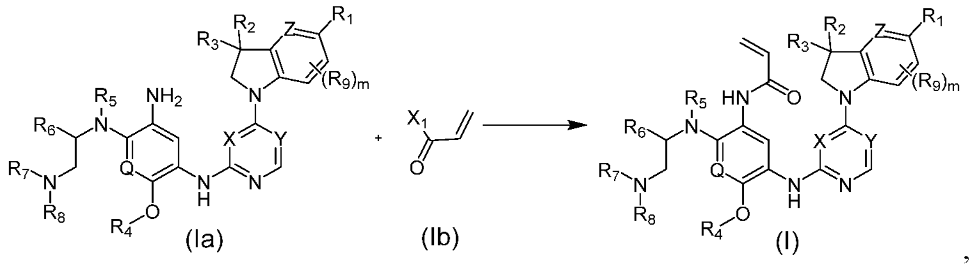

wherein, Xi is halogen, preferably selected from fluorine, chlorine and bromine; $R_1$, $R_2$, $R_3$, $R_4$, $R_5$, $R_6$, $R_7$, $R_8$, $R_9$, X, Y, Z, Q and m are defined as in claim 1.

**18.** A pharmaceutical composition, comprising the compound of formula (I), the stereoisomer or pharmaceutically acceptable salt thereof of any one of claims 1-16, and a pharmaceutically acceptable carrier.

**19.** Use of the compound of formula (I), the stereoisomer or pharmaceutically acceptable salt thereof of any one of claims 1-16 in preparation of a medicament for treatment and/or prevention of cancers, tumors or metastatic diseases at least partially associated with the insertion, deletion or other mutations of EGFR exon 20.

**20.** Use of the compound of formula (I), the stereoisomer or pharmaceutically acceptable salt thereof of any one of claims 1-16 in preparation of a medicament for treatment and/or prevention of tumors, cancers or metastatic diseases caused by hyperproliferation and dysfunction in cell death induction.

**21.** Use of the compound of formula (I), the stereoisomer or pharmaceutically acceptable salt thereof of any one of claims 1-16 in the preparation of a medicament for treatment and/or prevention of lung cancer, colon cancer, pancreatic cancer, head and neck cancer, breast cancer, ovarian cancer, uterine cancer, gastric cancer, non-small cell lung cancer, leukemia, myelodysplastic syndrome, malignant lymphoma, head and neck tumor, thoracic tumor, gastrointestinal tumor, endocrine tumor, breast and other gynecological tumor, urological tumor, skin tumor, sarcoma, sinonasal inverted papilloma, or sinonasal squamous cell carcinoma associated with sinonasal inverted papilloma, which is at least partially associated with the insertion, deletion or other mutations of EGFR exon 20.

**22.** The compound of formula (I), the stereoisomer or pharmaceutically acceptable salt thereof of any one of claims 1-16, for use in the treatment and/or prevention of lung cancer, colon cancer, pancreatic cancer, head and neck cancer, breast cancer, ovarian cancer, uterine cancer, gastric cancer, non-small cell lung cancer, leukemia, myelodysplastic syndrome, malignant lymphoma, head and neck tumor, thoracic tumor, gastrointestinal tumor, endocrine tumor, breast and other gynecological tumor, urological tumor, skin tumor, sarcoma, sinonasal inverted papilloma, or sinonasal squamous cell carcinoma associated with sinonasal inverted papilloma, which is at least partially associated with the insertion, deletion or other mutations of EGFR exon 20.

**INTERNATIONAL SEARCH REPORT**

International application No.
**PCT/CN2021/134790**

**A. CLASSIFICATION OF SUBJECT MATTER**

C07D 401/04(2006.01)i； C07D 471/04(2006.01)i； C07D 487/04(2006.01)i； C07D 403/04(2006.01)i； A61K 31/506(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNPAT WPI EPODOC CNKI STN(REGISTRY) STN(CAPLUS): EGFR, 抑制剂, 癌症, 肿瘤, inhibitor?, cancer, tumor, STN structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 105085489 A (SHANGHAI YEYAN TECHNOLOGY CO., LTD.) 25 November 2015 (2015-11-25)<br>claims 1, 10, 12, description paragraphs [0029]-[0113], embodiments 5, 37, 38, 57, 62 | 1-22 |
| X | WO 2016029839 A1 (SICHUAN HAISCO PHARMACEUTICAL CO., LTD.) 03 March 2016 (2016-03-03)<br>description, pages 4-22, embodiment 67 | 1-22 |
| X | CN 106928150 A (ANCUREALL BIOMEDICAL TECHNOLOGY (SHANGHAI) CO., LTD.) 07 July 2017 (2017-07-07)<br>description paragraphs [0010]-[0085], [0200], embodiment 1 | 1-22 |
| X | CN 109328059 A (CS PHARMASCIENCES INC.) 12 February 2019 (2019-02-12)<br>claims 1-153, embodiment 13 | 1-22 |
| X | CN 108864079 A (SHENZHEN FORWARD PHARMACEUTICAL CO., LTD.) 23 November 2018 (2018-11-23)<br>claims 1-10, description paragraphs [0046]-[0047], [0066] -[0075] | 1-22 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 February 2022** | **01 March 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2021/134790**

| Patent document cited in search report | Publication date (day/month/year) | Patent family member(s) | Publication date (day/month/year) |
|---|---|---|---|
| CN 105085489 A | 25 November 2015 | EP 3216786 A1 | 13 September 2017 |
| | | EP 3216786 A4 | 04 July 2018 |
| | | EP 3216786 B1 | 12 May 2021 |
| | | JP 2017533266 A | 09 November 2017 |
| | | JP 6637058 B2 | 29 January 2020 |
| | | ES 2881622 T3 | 30 November 2021 |
| | | EP 3885344 A2 | 29 September 2021 |
| | | EP 3885344 A3 | 08 December 2021 |
| | | CA 2966376 A1 | 12 May 2016 |
| | | IL 286165 D0 | 31 October 2021 |
| | | US 2017355696 A1 | 14 December 2017 |
| | | US 10179784 B2 | 15 January 2019 |
| | | CN 107548391 A8 | 05 January 2018 |
| | | KR 20170101908 A | 06 September 2017 |
| | | HK 1243702 A1 | 20 July 2018 |
| | | CN 111170998 A | 19 May 2020 |
| | | IL 252036 D0 | 29 June 2017 |
| | | IL 252036 A | 30 September 2021 |
| | | US 2019152969 A1 | 23 May 2019 |
| | | US 11203589 B2 | 21 December 2021 |
| | | US 2021261543 A1 | 26 August 2021 |
| | | WO 2016070816 A1 | 12 May 2016 |
| | | CN 110054618 A | 26 July 2019 |
| WO 2016029839 A1 | 03 March 2016 | CN 113121575 A | 16 July 2021 |
| | | CN 106458969 A | 22 February 2017 |
| CN 106928150 A | 07 July 2017 | EP 3398939 A1 | 07 November 2018 |
| | | EP 3398939 A4 | 13 February 2019 |
| | | WO 2017114500 A1 | 06 July 2017 |
| | | RU 2018122662 A | 31 January 2020 |
| | | RU 2018122662 A3 | 17 February 2020 |
| | | RU 2742372 C2 | 05 February 2021 |
| | | BR 112018013218 A2 | 11 December 2018 |
| | | JP 2021098715 A | 01 July 2021 |
| | | US 2019015413 A1 | 17 January 2019 |
| | | US 10342796 B2 | 09 July 2019 |
| | | KR 20180098361 A | 03 September 2018 |
| | | AU 2016382515 A1 | 16 August 2018 |
| | | AU 2016382515 B2 | 17 December 2020 |
| | | JP 2019506449 A | 07 March 2019 |
| CN 109328059 A | 12 February 2019 | EP 3399968 A1 | 14 November 2018 |
| | | EP 3399968 A4 | 24 July 2019 |
| | | EP 3399968 B1 | 20 October 2021 |
| | | EP 3399968 B8 | 01 December 2021 |
| | | JP 2019501222 A | 17 January 2019 |
| | | JP 2021091703 A | 17 June 2021 |
| | | KR 20180105161 A | 27 September 2018 |
| | | US 2019023689 A1 | 24 January 2019 |
| | | US 10435388 B2 | 08 October 2019 |
| | | WO 2017120429 A1 | 13 July 2017 |
| | | TW 201734013 A | 01 October 2017 |

Form PCT/ISA/210 (patent family annex) (January 2015)

**INTERNATIONAL SEARCH REPORT**
**Information on patent family members**

International application No.
**PCT/CN2021/134790**

| Patent document cited in search report | | | Publication date (day/month/year) | Patent family member(s) | | | Publication date (day/month/year) |
|---|---|---|---|---|---|---|---|
| | | | | TW | I726968 | B | 11 May 2021 |
| CN | 108864079 | A | 23 November 2018 | WO | 2018210246 | A1 | 22 November 2018 |
| | | | | MA | 50506 | A | 09 September 2020 |
| | | | | CN | 110891950 | A | 17 March 2020 |
| | | | | EP | 3705478 | A1 | 09 September 2020 |
| | | | | EP | 3705478 | A4 | 28 October 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018210246 A1